# EUROPÄISCHE PATENTSCHRIFT

(11) **EP 4 482 913 B1**
(45) Veröffentlichungstag und Bekanntmachung des Hinweises auf die Patenterteilung: **24.12.2025**
(21) Anmeldenummer: 23705036.4
(22) Anmeldetag: 20.02.2023
(51) Int. Cl.: C09K 11/06, C07B 59/00, C07D 471/16, C07D 471/22, C07D 487/22, C07D 491/22, C07F 7/08, H10K 50/12, H10K 71/16, H10K 85/60, H10K 50/11, H10K 50/15

(54) **STICKSTOFFHALTIGE HETEROCYCLEN FÜR ORGANISCHE ELEKTROLUMINESZENZVORRICHTUNGEN**
NITROGENOUS HETEROCYCLES FOR ORGANIC ELECTROLUMINESCENT DEVICES
HÉTÉROCYCLES AZOTÉS POUR DISPOSITIFS ÉLECTROLUMINESCENTS ORGANIQUES

(30) Priorität: 23.02.2022 EP 22158139
(43) Veröffentlichungstag der Anmeldung: 01.01.2025
(73) Patentinhaber: Merck Patent GmbH, 64293 Darmstadt (DE)
(72) Erfinder: STOESSEL, Philipp, 64293 Darmstadt (DE)
(74) Vertreter: Merck Patent Association
(86) Internationale Anmeldenummer: PCT/EP2023/054130
(87) Internationale Veröffentlichungsnummer: WO 2023/161167

(56) Entgegenhaltungen:
- CN-A- 112 778 309
- KR-A- 20180 028 010

## Beschreibung

Die vorliegende Erfindung betrifft stickstoffhaltige Heterocyclen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese heterocyclischen Verbindungen.

In organischen Elektrolumineszenzvorrichtungen werden als emittierende Materialien häufig phosphoreszierende metallorganische Komplexe oder fluoreszierende Verbindungen eingesetzt. Generell gibt es bei Elektrolumineszenzvorrichtungen immer noch Verbesserungsbedarf.

Aus US 2010/0051928, WO 2010/104047 A1, WO 2017/175690, WO 2019/132506 A1, WO 2019/111971 A1, CN112778309 A und WO 2020/064666 A1 sind polycyclische Verbindungen bekannt, die in organischen Elektrolumineszenzvorrichtungen eingesetzt werden können. Verbindungen gemäß der vorliegenden Erfindung sind nicht offenbart.

In WO 2019/111971 A1 werden insbesondere Verbindungen als bevorzugt dargelegt, die an Position R⁵ und R¹⁴ in Formeln (3-11) durch eine Diarylaminogruppe substituiert sind und an der jeweiligen aromatischen Gruppe, an der die Diarylaminogruppe bindet, keine weiteren Substituenten aufweisen (vgl. WO 2019/111971 A1, Formel (3-13).

Ferner sind aus der Druckschrift CN 109761981 Verbindungen mit Anthracen-Gruppen bekannt, die als Matrixmaterial eingesetzt werden können. Eine Verwendung dieser Verbindung als Emitter wird nicht beschrieben und ist nicht zweckmäßig. Ähnliche Verbindungen sind des Weiteren in John B. Henrv et al., J. Phys. Chem. A 2011. 115. 5435-5442 Verbindungen beschrieben.

Generell besteht bei diesen heterocyclischen Verbindungen, beispielsweise für die Verwendung als Emitter, insbesondere als fluoreszierender Emitter, noch Verbesserungsbedarf, insbesondere in Bezug auf die Lebensdauer, die Farbreinheit, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen.

Weiterhin sollten die Verbindungen eine ausgezeichnete Verarbeitbarkeit aufweisen, wobei die Verbindungen insbesondere eine gute Löslichkeit zeigen sollten.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Emitter. Insbesondere ist es eine Aufgabe der vorliegenden Erfindung, Emitter bereitzustellen, welche sich für rote, grüne oder blaue Elektrolumineszenzvorrichtungen, vorzugweise für blaue Elektrolumineszenzvorrichtungen eignen.

Weiterhin sollten die Verbindungen, insbesondere bei ihrem Einsatz als Emitter in organischen Elektrolumineszenzvorrichtungen, zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich sehr gut für die Verwendung in Elektrolumineszenzvorrichtungen eignen und zu organischen Elektrolumineszenzvorrichtungen führen, die insbesondere in Bezug auf die Lebensdauer, der Farbreinheit, der Effizienz und der Betriebsspannung sehr gute Eigenschaften vorweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur der Formel (I), vorzugsweise eine Verbindung gemäß der Formel (I), 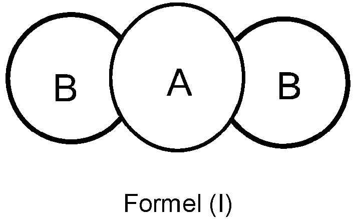 wobei A bei jedem Auftreten gleich oder verschieden für eine Teilstruktur der Formel (A1) oder (A2), vorzugsweise der Formel (A1), 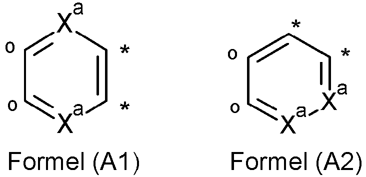 an die zwei Teilstrukturen B kondensiert sind, steht und die Symbole o und * die zwei Kondensationsstellen der jeweiligen Teilstruktur B darstellen, wobei eine Teilstruktur B über die mit o gekennzeichneten Positionen an A kondensiert ist und eine Teilstruktur B über die mit * gekennzeichneten Positionen an A kondensiert ist und B bei jedem Auftreten gleich oder verschieden für eine Teilstruktur der Formel (B) steht 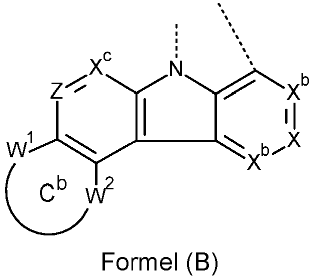 wobei die gestrichelten Bindungen die Kondensationsstellen der Teilstruktur B an A darstellen,
der Ring C^{b} bei jedem Auftreten gleich oder verschieden für einen kondensierten aliphatischen oder heteroaliphatischen Ring mit 5 bis 60 Ringatomen, der mit einem oder mehreren Resten R substituiert sein kann, vorzugsweise für einen aliphatischen oder heteroaliphatischen Ring mit 5 bis 20, besonders bevorzugt 5 bis 18, ganz besonders bevorzugt 5 bis 12 Ringatomen, der mit einem oder mehreren Resten R substituiert sein kann,
und für die weiteren Symbole gilt:
   - Z: steht bei jedem Auftreten gleich oder verschieden für N, C-CN oder CR^{c}, vorzugsweise für N oder C-CN und besonders bevorzugt für C-CN;
   - W¹, W²: steht bei jedem Auftreten gleich oder verschieden für C(R)₂, O, S, Si(R)₂, vorzugsweise für C(R)₂;
   - X: steht bei jedem Auftreten gleich oder verschieden für N oder CR, vorzugsweise für CR mit der Maßgabe, dass nicht mehr als zwei der Gruppen X, X^{b} in einem Cyclus für N stehen;
   - X^{a}: steht bei jedem Auftreten gleich oder verschieden für N oder CR^{a} vorzugsweise für CR^{a};
   - X^{b}: steht bei jedem Auftreten gleich oder verschieden für N oder CR^{b}, vorzugsweise für CR^{b} mit der Maßgabe, dass nicht mehr als zwei der Gruppen X, X^{b} in einem Cyclus für N stehen;
   - X^{c}: steht bei jedem Auftreten gleich oder verschieden für N oder CR^{c}, vorzugsweise für CR°;
   - R: ist bei jedem Auftreten gleich oder verschieden H, D, OH, F, Cl, Br, I, CN, NO₂, N(Ar)₂, N(R^{d})₂, C(=O)N(Ar)₂, C(=O)N(R^{d})₂, C(Ar)₃, C(R^{d})₃, Si(Ar)₃, Si(R^{d})₃, B(Ar)₂, B(R^{d})₂, C(=O)Ar, C(=O)R^{d}, P(=O)(Ar)₂, P(=O)( R^{d})₂, P(Ar)₂, P(R^{d})₂, S(=O)Ar, S(=O)R^{d}, S(=O)₂Ar, S(=O)₂R^{d}, OSO₂Ar, OSO₂R^{d}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR^{d}, -C(=O)O-, -C(=O)NR^{d}-, NR^{d}, P(=O)( R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Arylthio- oder Heteroarylthiogruppe mit 5 bis 60 aromatischen Ringatomen, die durch einen oder mehrere Reste R^{d} substituiert sein kann, oder eine Diarylamino-, Arylheteroarylamino-, Diheteroarylaminogruppe mit 5 bis 60 aromatischen Ringatomen, die durch einen oder mehrere Reste R^{d} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{d} substituiert sein kann; dabei kann ein Rest R mit einer weiteren Gruppe, vorzugsweise R oder R^{b} ein Ringsystem bilden;
   - Ar: ist bei jedem Auftreten gleich oder verschieden ein aromatisches oder heteroaromatisches Ringsystem mit 5 bis 60 aromatischen Ringatomen, das mit einem oder mehreren Resten R^{d} substituiert sein kann, dabei können zwei Reste Ar, welche an dasselbe C-Atom, Si-Atom, N-Atom, P-Atom oder B-Atom binden, auch durch eine Einfachbindung oder eine Brücke, ausgewählt aus B(R^{d}), C(R^{d})₂, Si(R^{d})₂, C=O, C=NR^{d}, C=C(R^{d})₂, O, S, S=O, SO₂, N(R^{d}), P(R^{d}) und P(=O)R^{d}, miteinander verbrückt sein;
   - R^{a}, R^{b}, R^{c}, R^{d}: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d} auch miteinander oder einer weiteren Gruppe, vorzugsweise R ein Ringsystem bilden;
   - Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C- Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R', miteinander verbrückt sein;
   - R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
   - Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
   - R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden;

   mit der Maßgabe, dass die Struktur/Verbindung der Formel (I) mindestens eine Teilstruktur B umfasst, in der die Gruppe Z für N oder C-CN steht, vorzugsweise die Gruppe Z in beiden Teilstrukturen B für N oder C-CN steht.

In einer bevorzugten Ausgestaltung können die erfindungsgemäßen Verbindungen eine Struktur der Formeln (I-1) und/oder (I-2) umfassen, besonders bevorzugt können die erfindungsgemäßen Verbindungen ausgewählt sein aus den Verbindungen der Formeln (I-1) und/oder (I-2), 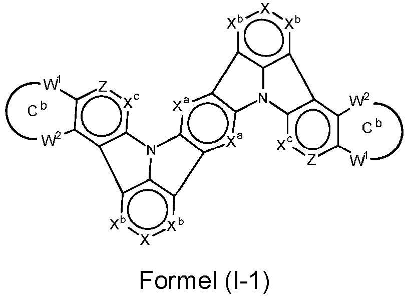 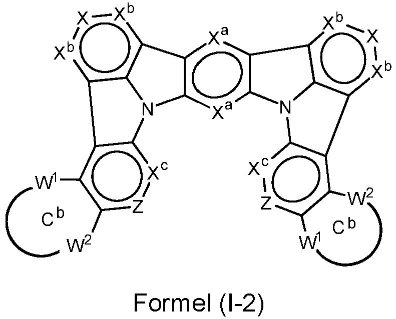 wobei die Symbole C^{b}, W¹, W², Z, X, X^{a}, X^{b} und X^{c} die zuvor, insbesondere für Formel (I) genannten Bedeutungen aufweisen, wobei Strukturen der Formel (I-1) bevorzugt sind.

Vorzugsweise kann vorgesehen sein, dass mindestens ein vorzugsweise mindestens zwei der Reste R, R^{a}, R^{b}, R^{c}, R^{d} ungleich H sind, vorzugsweise ungleich H, D, OH, NO₂, F, Cl, Br, I. Demgemäß ist der Rest R, welcher vorzugsweise benachbart zu einer Gruppe X^{b} beziehungsweise R^{b} ist, vorzugsweise ausgewählt aus CN, N(Ar)₂, N(R^{d})₂, C(=O)N(Ar)₂, C(=O)N(R^{d})₂, C(Ar)₃, C(R^{d})₃, Si(Ar)₃, Si(R^{d})₃, B(Ar)₂, B(R^{d})₂, C(=O)Ar, C(=O)R^{d}, P(=O)(Ar)₂, P(=O)(R^{d})₂, P(Ar)₂, P(R^{d})₂, S(=O)Ar, S(=O)R^{d}, S(=O)₂Ar, S(=O)₂R^{d}, OSO₂Ar, OSO₂R^{d}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR^{d}, -C(=O)O-, -C(=O)NR^{d}-, NR^{d}, P(=O)( R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Arylthio- oder Heteroarylthiogruppe mit 5 bis 60 aromatischen Ringatomen, die durch einen oder mehrere Reste R^{d} substituiert sein kann, oder eine Diarylamino-, Arylheteroarylamino-, Diheteroarylaminogruppe mit 5 bis 60 aromatischen Ringatomen, die durch einen oder mehrere Reste R^{d} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{d} substituiert sein kann; dabei kann ein Rest R mit einer weiteren Gruppe, vorzugsweise R oder R^{b} ein Ringsystem bilden; und/oder mindestens einer der Reste R^{a}, R^{b}, R^{c}, R^{d} ist vorzugsweise bei jedem Auftreten gleich oder verschieden ausgewählt aus CN, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R', S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR'-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d} auch miteinander oder einer weiteren Gruppe ein Ringsystem bilden. Hierbei sind Reste N(Ar)₂, N(R^{d})₂, gegenüber den weiteren genannten Gruppen weniger bevorzugt.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem, vorzugsweise 6 bis 40 C-Atome im Ringsystem. Ein heteroaromatisches Ringsystem im Sinne dieser Erfindung enthält 2 bis 60 C-Atome, vorzugsweise 3 bis 40 C-Atome, und mindestens ein Heteroatom im Ringsystem, mit der Maßgabe, dass die Summe aus C-Atomen und Heteroatomen mindestens 5 ergibt. Die Heteroatome sind bevorzugt ausgewählt aus **N,** O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

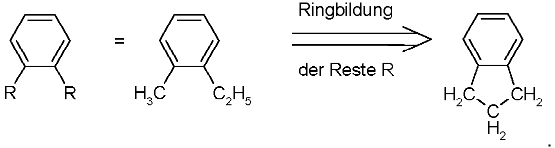

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

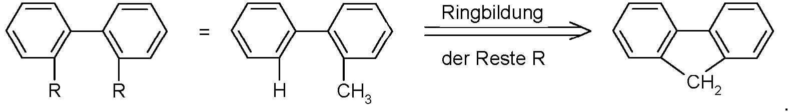

Vorzugsweise kann vorgesehen sein, dass mindestens einer der Reste R, R^{d} ungleich H ist/sind, bevorzugt mindestens einer der Reste R, R^{d} ungleich H, D, F, Cl, Br, I ist/sind.

Bevorzugt kann weiterhin vorgesehen sein, dass mindestens einer der Reste R^{c}, bevorzugt beide Reste R^{c} gleich H oder D ist/sind.

In einer bevorzugten Ausgestaltung kann vorgesehen sein, dass ein Rest R, vorzugsweise der Rest R, der zu einer Gruppe X^{b} oder einem Rest R^{b} benachbart ist, ein aromatisches oder heteroaromatisches Ringsystem mit 5 bis 13 aromatischen Ringatomen darstellt, das mit einem oder mehreren Resten R^{d} substituiert sein kann.

In einer weiterhin bevorzugten Ausgestaltung kann vorgesehen sein, dass eine erfindungsgemäß Verbindung mindestens eine Teilstruktur der Formeln (B1-1) bis (B1-30) umfasst, 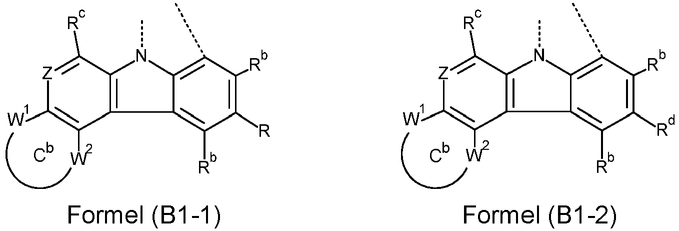 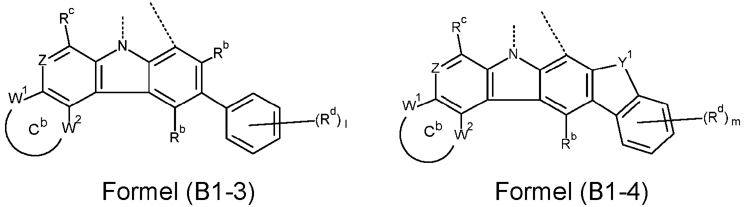 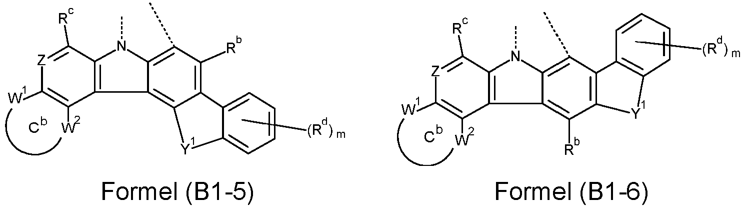 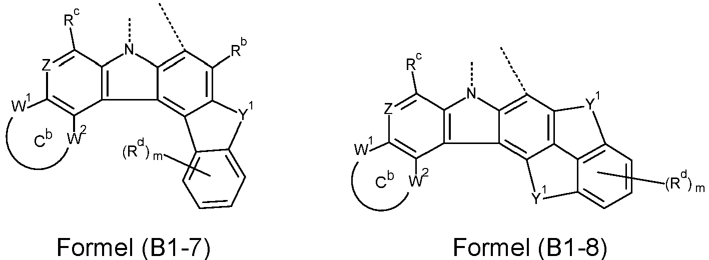 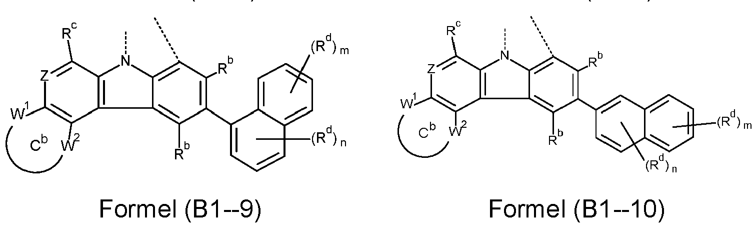 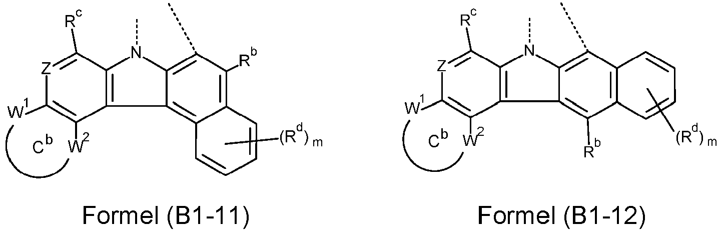 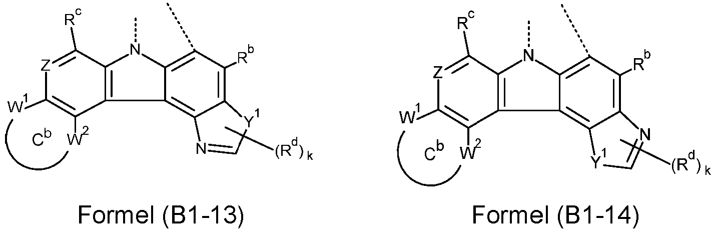 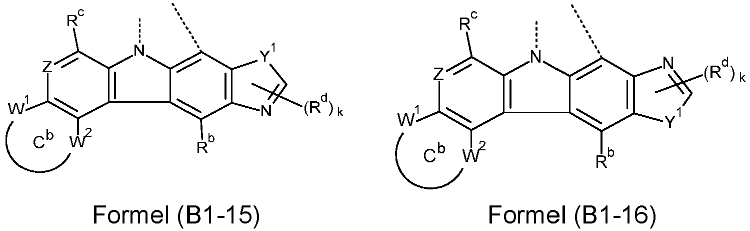 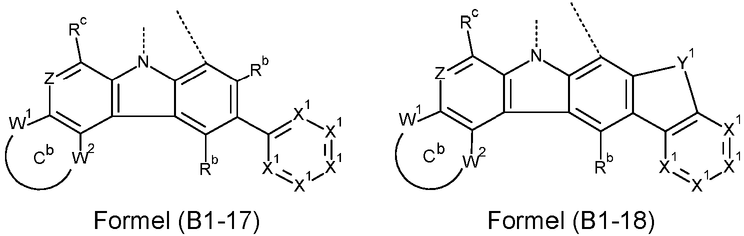 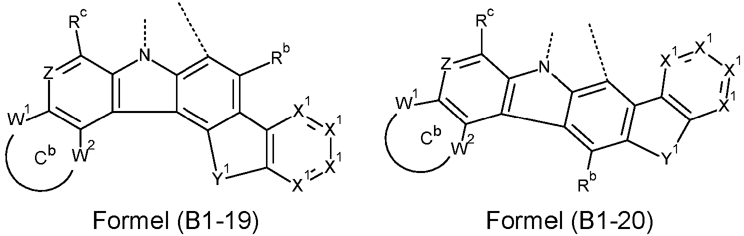 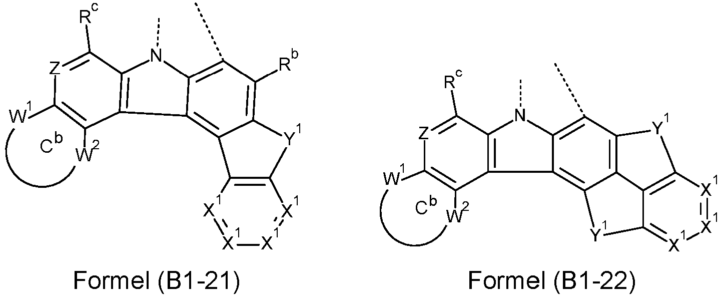 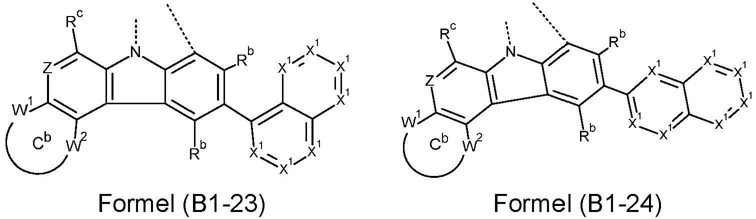 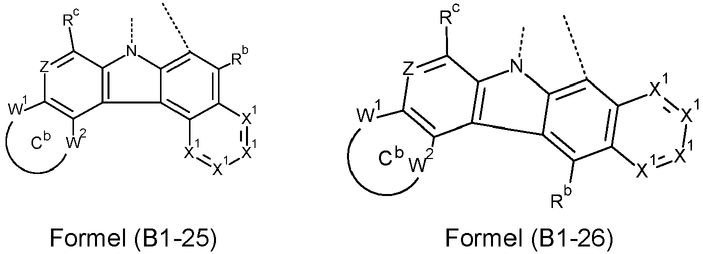 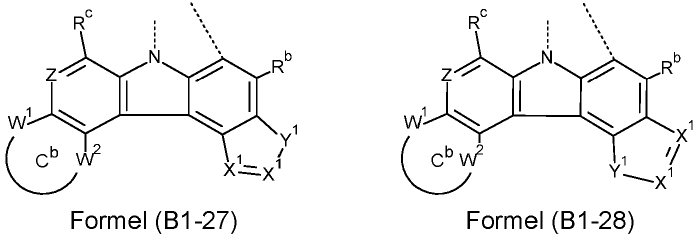 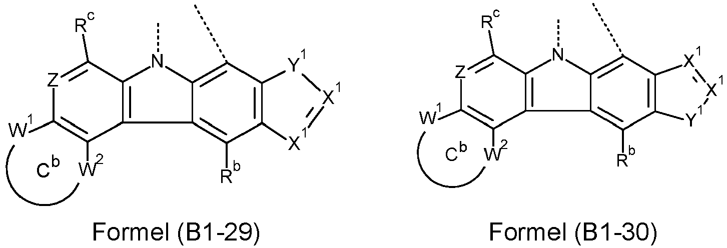 wobei die Symbole C^{b}, W¹, W², Z, R, R^{b}, R^{c} und R^{d} die zuvor, insbesondere für Formel (I) genannten Bedeutungen aufweisen, die gestrichelten Bindungen die Kondensationsstellen der Teilstruktur an A darstellen und für die weiteren verwendeten Symbole und Indices gilt:
- X¹: steht bei jedem Auftreten gleich oder verschieden für N oder CR^{d}, vorzugsweise für CR^{d} mit der Maßgabe, dass nicht mehr als zwei der Gruppen X¹ in einem Cyclus für N stehen;
- Y¹: ist bei jedem Auftreten gleich oder verschieden C(R^{d})₂, (R^{d})₂C-C(R^{d})₂, (R^{d})C=C(R^{d}), NR^{d}, NAr', O, S, SO, SO₂, Se, P(O)R^{d}, BR^{d} oder Si(R^{d})₂, vorzugsweise C(R^{d})₂, (R^{d})₂C-C(R^{d})₂, (R^{d})C=C(R^{d}), O oder S, besonders bevorzugt C(R^{d})₂;
- k: ist 0 oder 1;
- n: ist 0, 1, 2 oder 3, vorzugsweise 0, 1 oder 2;
- m: ist 0, 1, 2, 3 oder 4, vorzugsweise 0, 1 oder 2;
- l: ist 0, 1, 2, 3, 4 oder 5, vorzugsweise 0, 1 oder 2.

Hierbei sind Strukturen der Formeln (B1-1) bis (B1-18) bevorzugt, Strukturen der Formeln (B1-1) bis (B1-3) besonders bevorzugt und Strukturen der Formeln (B1-2) und (B1-3) besonders speziell bevorzugt.

In einer bevorzugten Ausgestaltung können die erfindungsgemäßen Verbindungen eine Struktur der Formeln (II-1) bis (II-15) umfassen, besonders bevorzugt können die erfindungsgemäßen Verbindungen ausgewählt sein aus den Verbindungen der Formeln (II-1) bis (II-15), 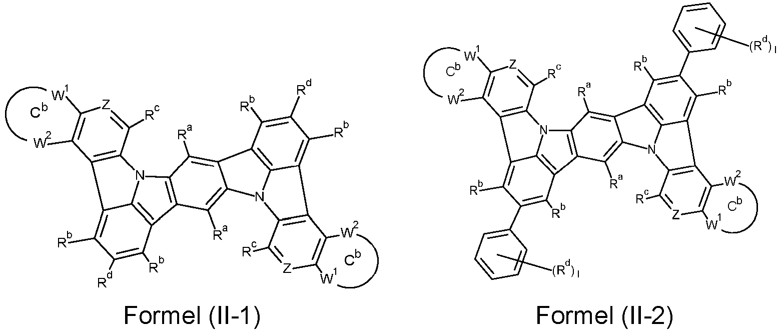 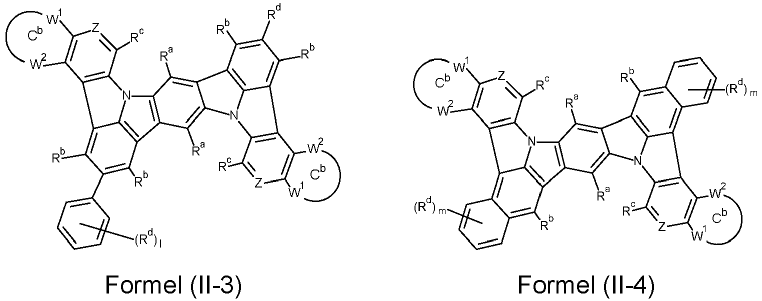 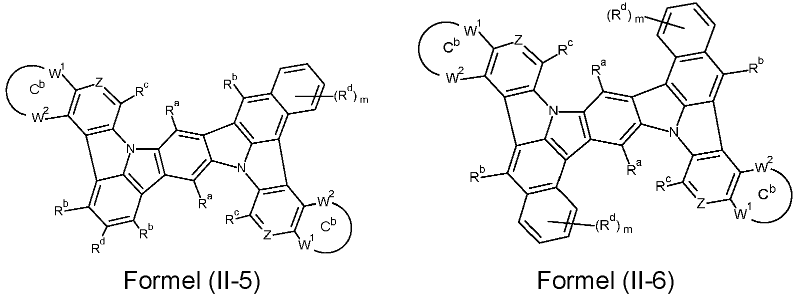 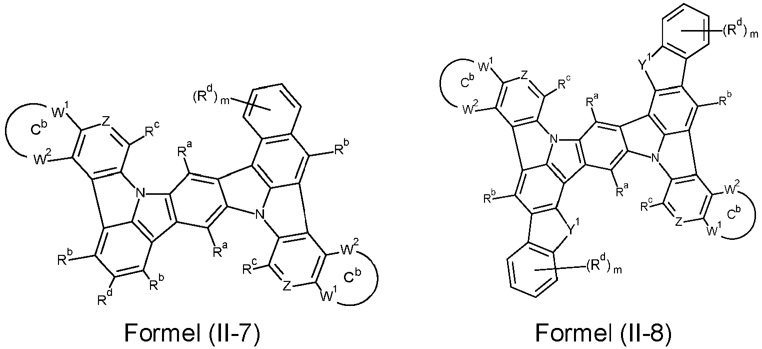 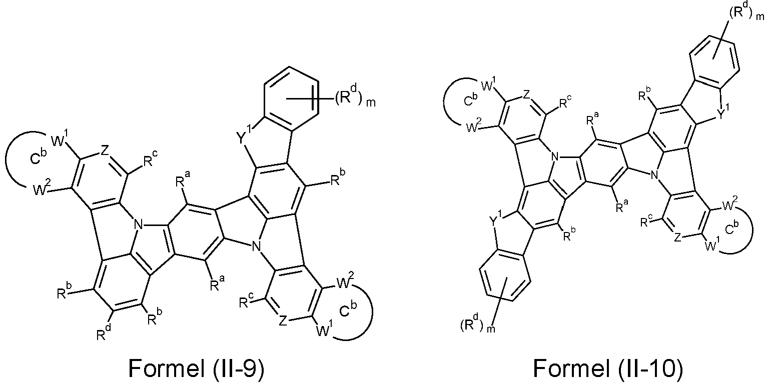 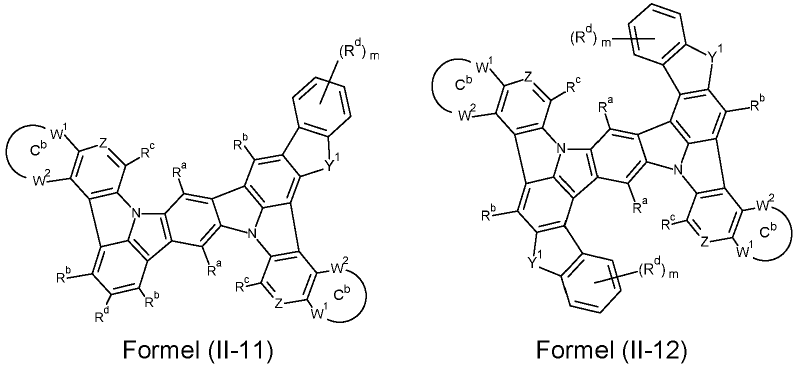 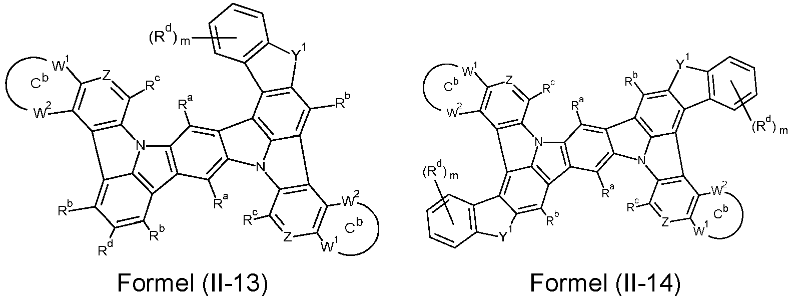 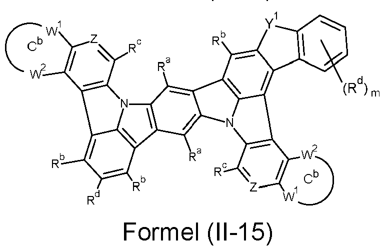 wobei die Symbole C^{b}, W¹, W², Z, R, R^{a}, R^{b}, R^{c} und R^{d} die zuvor, insbesondere für Formel (I) genannten Bedeutungen aufweisen, das Symbol Y¹ die zuvor, insbesondere für Formeln (B1-1) bis (B1-30) genannte Bedeutung aufweist und für die weiteren verwendeten Indices gilt:
- m: ist 0, 1, 2, 3 oder 4, vorzugsweise 0, 1 oder 2;
- l: ist 0, 1, 2, 3, 4 oder 5, vorzugsweise 0, 1 oder 2.

Hierbei sind Strukturen/Verbindungen der Formeln (II-1) und (II-2) bevorzugt und Strukturen/Verbindungen der Formel (II-1) besonders bevorzugt.

Ferner kann vorgesehen sein, dass der kondensierte Ring C^{b} ausgewählt ist aus einer Struktur der Formeln (BCY-1) bis (BCY-10), 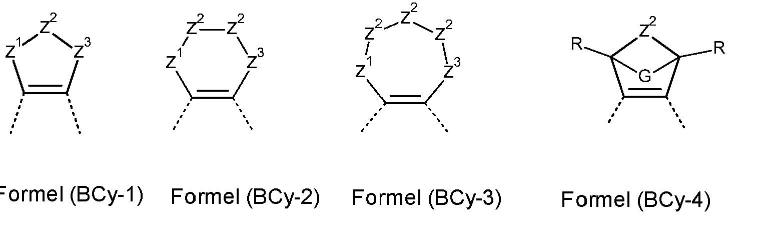 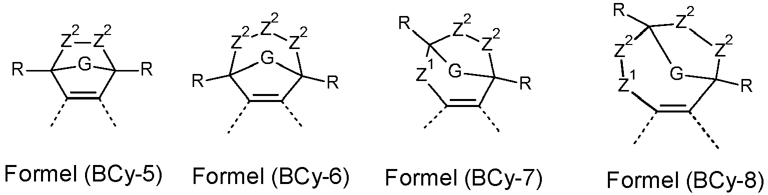 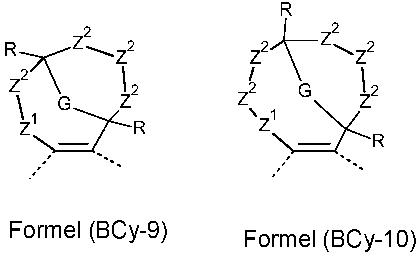 wobei R die zuvor, insbesondere für Formel (I) genannte Bedeutung aufweist, die gestrichelten Bindungen die Bindungsstellen des kondensierten Rings an die weiteren Gruppen darstellen und weiterhin gilt:
- Z¹, Z³: ist gleich oder verschieden bei jedem Auftreten C(R³)₂, O, S oder Si(R³)₂, vorzugsweise C(R³)₂,
- Z²: ist C(R)₂, O, S, NR oder C(=O), wobei zwei benachbarte Gruppen Z² für -CR=CR- oder eine ortho-verknüpfte Arylen- oder Heteroarylengruppe mit 5 bis 14 aromatischen Ringatomen, welche durch einen oder mehrere Reste R substituiert sein kann, stehen können;
- G: ist eine Alkylengruppe mit 1, 2 oder 3 C-Atomen, welche mit einem oder mehreren Resten R substituiert sein kann, -CR=CR- oder eine ortho-verknüpfte Arylen- oder Heteroarylengruppe mit 5 bis 14 aromatischen Ringatomen, welche durch einen oder mehrere Reste R substituiert sein kann;
- R³: ist bei jedem Auftreten gleich oder verschieden H, D, F, Cl, Br, I, CN, NO₂, N(Ar')₂, N(R^{d})₂, C(=O)Ar', C(=O)R^{d}, P(=O)(Ar')₂, P(Ar')₂, B(Ar')₂, B(R^{d})₂, C(Ar')₃, C(R^{d})₃, Si(Ar')₃, Si(R^{d})₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R^{d} substituiert sein kann, wobei eine oder mehrere nicht benachbarte CH₂-Gruppen durch -R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Aralkyl- oder Heteroaralkylgruppe mit 5 bis 60 aromatischen Ringatomen, die mit einem oder mehreren Resten R^{d} substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem, vorzugsweise benachbarten Rest R, R^{a}, R^{c} oder R³ ein aliphatisches Ringsystem bilden, wobei Ar' und R^{d} die zuvor, insbesondere für Formel (I) genannten Bedeutungen aufweisen;
mit der Maßgabe, dass in diesen Gruppen nicht zwei Heteroatome direkt aneinander gebunden sind und nicht zwei Gruppen C=O direkt aneinander gebunden sind.

**In** einer bevorzugten Ausführungsform der Erfindung ist R³ ungleich H und/oder D.

Wenn benachbarte Reste in den erfindungsgemäßen Strukturen ein aliphatisches Ringsystem bilden, dann ist es bevorzugt, wenn dieses keine aziden benzylischen Protonen aufweist. Unter benzylischen Protonen werden Protonen verstanden, die an ein Alkyl-Kohlenstoffatom binden, welches direkt an eine Aryl- oder Heteroarylgruppe gebunden sind. Dies kann dadurch erreicht werden, dass die Kohlenstoffatome des aliphatischen Ringsystems, die direkt an eine Aryl- oder Heteroarylgruppe binden, vollständig substituiert sind und keine Wasserstoffatome gebunden enthalten. So wird die Abwesenheit von aziden benzylischen Protonen in den Formeln (BCy-1) bis (BCy-3) dadurch erreicht, dass Z¹ und Z³, wenn diese für C(R³)₂ stehen, so definiert sind, dass R³ ungleich Wasserstoff ist. Dies kann weiterhin auch dadurch erreicht werden, dass die Kohlenstoffatome des aliphatischen Ringsystems, die direkt an eine Aryl- oder Heteroarylgruppe binden, die Brückenköpfe einer bi- oder polycyclischen Struktur sind. Die an Brückenkopfkohlenstoffatome gebundenen Protonen sind aufgrund der räumlichen Struktur des Bi- oder Polycyclus wesentlich weniger azide als benzylische Protonen an Kohlenstoffatomen, die nicht in einer bi- oder polycyclischen Struktur gebunden sind, und werden im Sinne der vorliegenden Erfindung als nichtazide Protonen angesehen. So wird die Abwesenheit von aziden benzylischen Protonen ist in Formeln (BCy-4) bis (BCy-10) dadurch erreicht, dass es sich dabei um eine bicyclische Struktur handelt, wodurch R¹, wenn es für H steht, deutlich weniger azide als benzylische Protonen, da das korrespondierende Anion der bicyclischen Struktur nicht mesomeriestabilisiert ist. Auch wenn R¹ in Formeln (BCy-4) bis (BCy-10) für H steht, handelt es sich dabei daher um ein nicht-azides Proton im Sinne der vorliegenden Anmeldung.

Vorzugsweise kann vorgesehen sein, dass insbesondere in Formeln (BCy-1) bis (BCy-3) gilt:
- R³: ist bei jedem Auftreten gleich oder verschieden F, Cl, Br, I, CN, NO₂, N(Ar')₂, N(R^{d})₂, C(=O)Ar', C(=O)R^{d}, P(=O)(Ar')₂, P(Ar')₂, B(Ar')₂, B(R^{d})₂, C(Ar')₃, C(R^{d})₃, Si(Ar')₃, Si(R^{d})₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R^{d} substituiert sein kann, wobei eine oder mehrere nicht benachbarte CH₂-Gruppen durch - R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Aralkyl- oder Heteroaralkylgruppe mit 5 bis 60 aromatischen Ringatomen, die mit einem oder mehreren Resten R^{d} substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem, vorzugsweise benachbarten Rest R, R^{a}, R^{c} oder R³ ein aliphatisches Ringsystem bilden, wobei Ar' und R^{d} die zuvor, insbesondere für Formel (I) genannten Bedeutungen aufweisen.

Vorzugsweise kann vorgesehen sein, dass insbesondere in Formeln (BCy-1) bis (BCy-3) gilt:
- R³: ist bei jedem Auftreten gleich oder verschieden F, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R^{d} substituiert sein kann, wobei eine oder mehrere nicht benachbarte CH₂-Gruppen durch -R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann; dabei können zwei Reste R³ auch miteinander oder ein Rest R³ mit einem Rest R, R^{a}, R^{c} oder mit einer weiteren Gruppe ein Ringsystem, vorzugsweise ein aliphatisches Ringsystem bilden.

In einer bevorzugten Ausführungsform der Struktur gemäß Formel (BCy-1) bis (BCy-10) steht maximal eine der Gruppen Z¹, Z² und Z³ für ein Heteroatom, insbesondere für O oder NR, und die anderen Gruppen stehen für C(R³)₂ bzw. C(R)₂ oder Z¹ und Z³ stehen gleich oder verschieden bei jedem Auftreten für O und Z² steht für C(R)₂. In einer besonders bevorzugten Ausführungsform der Erfindung stehen Z¹ und Z³ gleich oder verschieden bei jedem Auftreten für C(R³)₂ und Z² steht für C(R)₂ und besonders bevorzugt für C(R³)₂ oder CH₂.

In einer bevorzugten Ausführungsform der Erfindung ist der Rest R, der an das Brückenkopfatom, vorzugsweise an das Brückenkopfatom gemäß Formeln (BCy-4) bis (BCy-10) gebunden ist, gleich oder verschieden bei jedem Auftreten gewählt aus der Gruppe bestehend aus H, D, F, einer geradkettigen Alkylgruppe mit 1 bis 10 C-Atomen, die mit einem oder mehreren Resten R¹ substituiert sein kann, bevorzugt aber unsubstituiert ist, einer verzweigten oder cyclischen Alkylgruppe mit 3 bis 10 C-Atomen, die mit einem oder mehreren Resten R¹ substituiert sein kann, bevorzugt aber unsubstituiert ist, oder einem aromatischen oder heteroaromatischen Ringsystem mit 5 bis 12 aromatischen Ringatomen, das jeweils durch einen oder mehrere Reste R¹ substituiert sein kann. Besonders bevorzugt ist der Rest R, der an das Brückenkopfatom gemäß Formel (BCy-4) gebunden ist, gleich oder verschieden bei jedem Auftreten gewählt aus der Gruppe bestehend aus **H, F,** einer geradkettigen Alkylgruppe mit 1 bis 4 C-Atomen, einer verzweigten Alkylgruppe mit 3 oder 4 C-Atomen oder einer Phenylgruppe, die durch eine Alkylgruppe mit 1 bis 4 C-Atomen substituiert sein kann, bevorzugt aber unsubstituiert ist. Ganz besonders bevorzugt ist der Rest R bei jedem Auftreten gleich oder verschieden gewählt aus der Gruppe bestehend aus **H,** Methyl oder tert-Butyl.

**In** einer bevorzugten Ausgestaltung der vorliegenden Erfindung kann vorgesehen sein, dass der kondensierte Ring C^{b} ausgewählt ist aus einer Struktur der Formeln (BRA-1) bis (BRA-12) 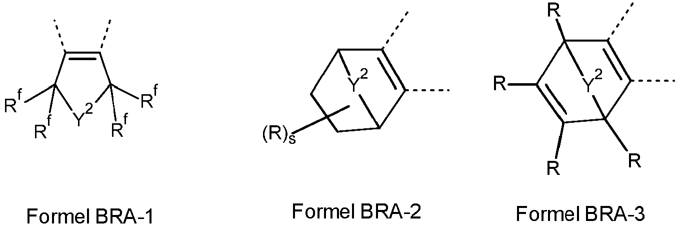 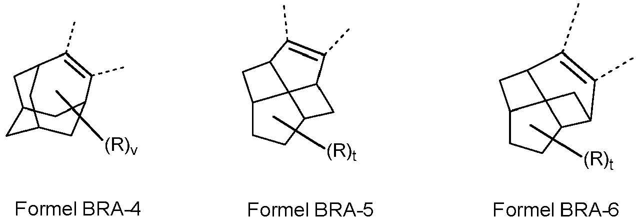 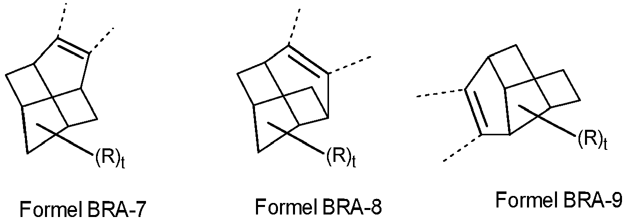 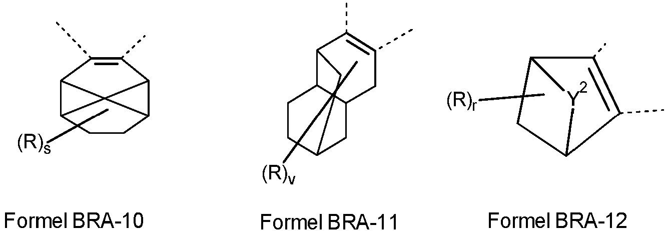 wobei R die zuvor, insbesondere für Formel (I) genannte Bedeutung aufweist, die gestrichelten Bindungen die Anbindungsstellen des kondensierten Rings an die weiteren Gruppen darstellen und die weiteren Symbole und Indices die folgende Bedeutung aufweisen:
- Y²: ist bei jedem Auftreten gleich oder verschieden C(R)₂, (R)₂C-C(R)₂, (R)C=C(R), NR, NAr', O oder S, vorzugsweise C(R)₂, (R)₂C-C(R)₂, (R)C=C(R), O oder S;
- R^{f}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{d} substituiert sein kann, wobei eine oder mehrere nicht benachbarte CH₂-Gruppen durch R^{d}C=CR^{d}, C≡C, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann; dabei können zwei Reste R^{f} auch miteinander oder ein Rest R^{f} mit einem Rest R oder mit einer weiteren Gruppe ein Ringsystem bilden, wobei R^{d} die zuvor, insbesondere für Formel (I) genannte Bedeutung aufweist;
- r: ist 0, 1, 2, 3 oder 4, vorzugsweise 0, 1, oder 2, besonders bevorzugt 0 oder 1;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2,

Hierbei sind Strukturen der Formeln BRA-1 bis RBA-4 bevorzugt und Strukturen der Formeln BRA-3 und BRA-4 besonders bevorzugt.

Besonders bevorzugt kann vorgesehen sein, dass der kondensierte Ring C^{b} ausgewählt ist aus einer Struktur der Formeln (BRA-1a) bis (BRA-3f) 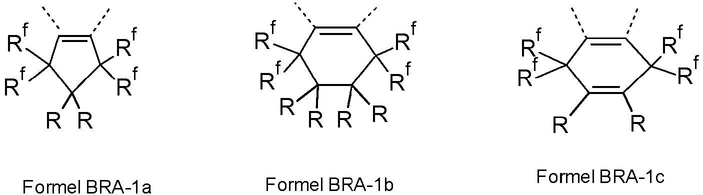 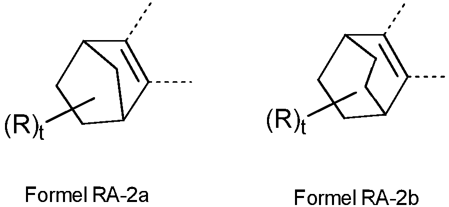 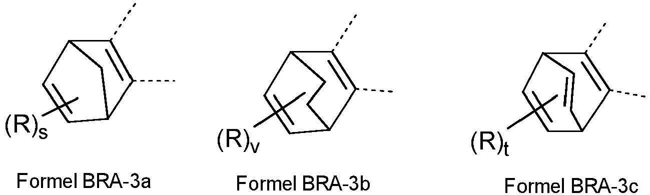 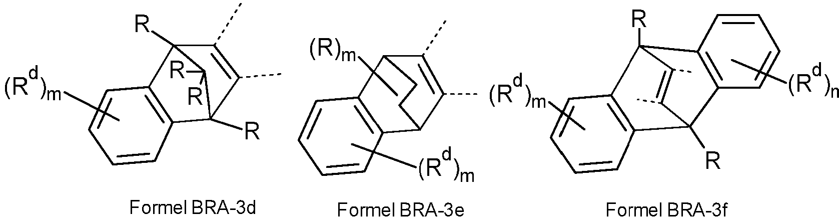 wobei die gestrichelten Bindungen die Anbindungsstellen des kondensierten Rings an die weiteren Gruppen darstellen, der Index m 0, 1, 2, 3 oder 4, vorzugsweise 0, 1 oder 2 ist und die Symbole R, R^{d}, R^{f} und die Indices s, t und v die zuvor, insbesondere für Formel (I) und/oder Formeln (BRA-1) bis (BRA-12) dargelegten Bedeutungen haben.

Hierbei sind Strukturen der Formeln BRA-3f bevorzugt.

In einer bevorzugten Ausgestaltung kann vorgesehen sein, dass der zuvor und nachfolgend dargelegte Ring C^{b} durch Substituten R^{d} anstatt R substituiert ist. In dieser bevorzugten Ausgestaltung sind beispielsweise die Substituenten R und R^{d} der zuvor und nachfolgend dargelegten Gruppen W¹, W², Z¹ bis Z³, G, Y², R³ und R^{f} durch R^{d} beziehungsweise R¹ zu ersetzen. Dies gilt insbesondere für die Formeln (BCy-1) bis (BCy-10), (BRA-1 bis BRA-12) und (BRA-1a) bis (BRA-3f) in denen beispielsweise die Substituenten R und R^{d} durch R^{d} beziehungsweise R¹ zu ersetzen sind.

In einer weiterhin bevorzugten Ausführungsform kann vorgesehen sein, dass der zuvor und nachfolgend dargelegte Ring C^{b} durch Substituten R¹ anstatt R substituiert ist. In dieser bevorzugten Ausgestaltung sind beispielsweise die Substituenten R und R^{d} der zuvor und nachfolgend dargelegten Gruppen W¹, W², Z¹ bis Z³, G, Y², R³ und R^{f} durch R¹ beziehungsweise R² zu ersetzen, wobei diese Definitionen für die nachfolgend dargelegten Gruppen Z⁵ bis Z⁷, G¹, Y⁴ und R^{g} beispielhaft dargelegt sind und entsprechend gelten. Dies gilt insbesondere für die Formeln (BCy-1) bis (BCy-10), (BRA-1 bis BRA-12) und (BRA-1a) bis (BRA-3f) in denen beispielsweise die Substituenten R und R^{d} durch R¹ beziehungsweise R² zu ersetzen sind.

Der Ring C^{b} umfasst Gruppen W¹, W², wobei diese Gruppen bewirken, dass aromatische oder heteraromatische Substituenten R, die von diesen Gruppen stammen können, keine durchgängige Konjugation mit dem Grundgerüst der Teilstruktur B, insbesondere mit dem Ring ausbilden können, der die Gruppe Z beziehungsweise X^{c} aufweist.

In einer bevorzugten Ausgestaltung der vorliegenden Erfindung kann vorgesehen sein, dass mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} mindestens eine Struktur der folgenden Formeln (Cy-1) bis (Cy-10) formen, 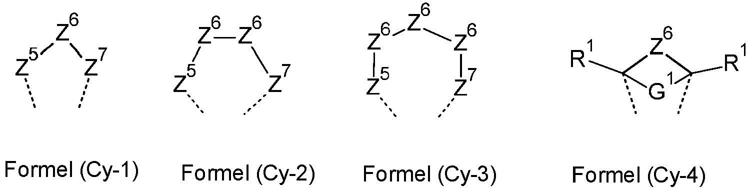 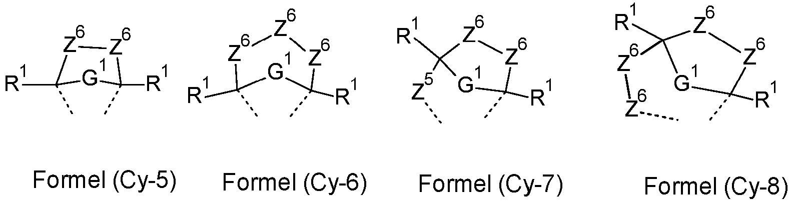 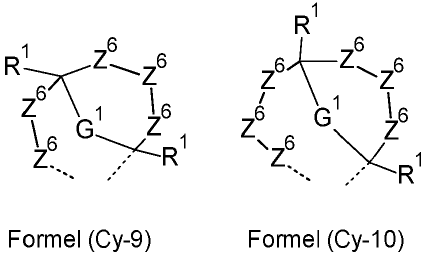 wobei R¹ die zuvor, insbesondere für Formel (I) dargelegte Bedeutung aufweist, die gestrichelten Bindungen die Anbindungsstellen an die Atome der Gruppen, an die die zwei Reste R, R^{a}, R^{b}, R^{c}, R^{d} binden, darstellen und weiterhin gilt:
- Z⁵, Z⁷: ist gleich oder verschieden bei jedem Auftreten C(R⁴)₂, O, S, NR⁴ oder C(=O);
- Z⁶: ist C(R¹)₂, O, S, NR¹ oder C(=O), wobei zwei benachbarte Gruppen Z² für -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann, stehen können;
- G¹: ist eine Alkylengruppe mit 1, 2 oder 3 C-Atomen, welche mit einem oder mehreren Resten R¹ substituiert sein kann, -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann;
- R⁴: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R⁴, welche an dasselbe Kohlenstoffatom gebunden sind, miteinander ein aliphatisches oder aromatisches Ringsystem bilden und so ein Spirosystem aufspannen; weiterhin kann R⁴ mit einem, vorzugsweise benachbarten Rest R, R^{a}, R^{b}, R^{c}, R^{d} oder R¹ ein aliphatisches Ringsystem bilden, wobei die Symbole R¹, R² und Ar" die zuvor, insbesondere für Formel (I) genannten Bedeutungen aufweisen;
mit der Maßgabe, dass in diesen Gruppen nicht zwei Heteroatome direkt aneinander gebunden sind und nicht zwei Gruppen C=O direkt aneinander gebunden sind.

In einer bevorzugten Ausführungsform der Erfindung ist R⁴ ungleich H und/oder D.

Die Abwesenheit von aziden benzylischen Protonen wird in den Formeln (Cy-1) bis (Cy-3) vorzugsweise dadurch erreicht, dass Z⁵ und Z⁷, wenn diese für C(R⁴)₂ stehen, so definiert sind, dass R⁴ ungleich Wasserstoff ist. Dies kann weiterhin auch dadurch erreicht werden, dass die Kohlenstoffatome des aliphatischen Ringsystems, die direkt an eine Aryl- oder Heteroarylgruppe binden, die Brückenköpfe einer bi- oder polycyclischen Struktur sind. Die an Brückenkopfkohlenstoffatome gebundenen Protonen sind aufgrund der räumlichen Struktur des Bi- oder Polycyclus wesentlich weniger azide als benzylische Protonen an Kohlenstoffatomen, die nicht in einer bi- oder polycyclischen Struktur gebunden sind, und werden im Sinne der vorliegenden Erfindung als nichtazide Protonen angesehen. So wird die Abwesenheit von aziden benzylischen Protonen ist in Formeln (Cy-4) bis (Cy-10) vorzugsweise dadurch erreicht, dass es sich dabei um eine bicyclische Struktur handelt, wodurch R¹, wenn es für H steht, deutlich weniger azide als benzylische Protonen, da das korrespondierende Anion der bicyclischen Struktur nicht mesomeriestabilisiert ist. Auch wenn R¹ in Formeln (Cy-4) bis (Cy-10) für H steht, handelt es sich dabei daher um ein nicht-azides Proton im Sinne der vorliegenden Anmeldung.

Vorzugsweise kann vorgesehen sein, dass insbesondere in Formeln (Cy-1) bis (Cy-3) gilt:
- R⁴: ist bei jedem Auftreten gleich oder verschieden F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch - R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R⁴, welche an dasselbe Kohlenstoffatom gebunden sind, miteinander ein aliphatisches oder aromatisches Ringsystem bilden und so ein Spirosystem aufspannen; weiterhin kann R⁴ mit einem vorzugsweise benachbarten Rest R, R^{a}, R^{c}, R^{d}, R¹ oder mit einer weiteren Gruppe ein Ringsystem, vorzugsweise ein aliphatisches Ringsystem bilden.

Vorzugsweise kann vorgesehen sein, dass insbesondere in Formeln (Cy-1) bis (Cy-3) gilt:
- R⁴: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R⁴ auch miteinander oder ein Rest R⁴ mit einem Rest R, R^{a}, R^{c}, R^{d}, R¹ oder mit einer weiteren Gruppe ein Ringsystem, vorzugsweise ein aliphatisches Ringsystem bilden.

In einer bevorzugten Ausführungsform der Struktur gemäß Formel (Cy-1) bis (Cy-10) steht maximal eine der Gruppen Z⁵, Z⁶ und Z⁷ für ein Heteroatom, insbesondere für O oder NR⁴, beziehungsweise für O oder NR¹, und die anderen Gruppen stehen für C(R⁴)₂ bzw. C(R¹)₂ oder Z⁵ und Z⁷ stehen gleich oder verschieden bei jedem Auftreten für O oder NR⁴ und Z⁶ steht für C(R¹)₂. In einer besonders bevorzugten Ausführungsform der Erfindung stehen Z⁵ und Z⁷ gleich oder verschieden bei jedem Auftreten für C(R⁴)₂ und Z⁶ steht für C(R¹)₂ und besonders bevorzugt für C(R⁴)₂ oder CH₂.

In einer bevorzugten Ausführungsform der Erfindung ist der Rest R¹, der an das Brückenkopfatom, vorzugsweise an das Brückenkopfatom gemäß Formeln (Cy-4) bis (Cy-10) gebunden ist, gleich oder verschieden bei jedem Auftreten gewählt aus der Gruppe bestehend aus H, D, F, einer geradkettigen Alkylgruppe mit 1 bis 10 C-Atomen, die mit einem oder mehreren Resten R² substituiert sein kann, bevorzugt aber unsubstituiert ist, einer verzweigten oder cyclischen Alkylgruppe mit 3 bis 10 C-Atomen, die mit einem oder mehreren Resten R² substituiert sein kann, bevorzugt aber unsubstituiert ist, oder einem aromatischen oder heteroaromatischen Ringsystem mit 5 bis 12 aromatischen Ringatomen, das jeweils durch einen oder mehrere Reste R² substituiert sein kann. Besonders bevorzugt ist der Rest R¹, der an das Brückenkopfatom gemäß Formel (CY-4) gebunden ist, gleich oder verschieden bei jedem Auftreten gewählt aus der Gruppe bestehend aus H, F, einer geradkettigen Alkylgruppe mit 1 bis 4 C-Atomen, einer verzweigten Alkylgruppe mit 3 oder 4 C-Atomen oder einer Phenylgruppe, die durch eine Alkylgruppe mit 1 bis 4 C-Atomen substituiert sein kann, bevorzugt aber unsubstituiert ist. Ganz besonders bevorzugt ist der Rest R¹ bei jedem Auftreten gleich oder verschieden gewählt aus der Gruppe bestehend aus H, Methyl oder tert-Butyl.

In einer bevorzugten Weiterbildung der vorliegenden Erfindung kann vorgesehen sein, dass mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} mindestens eine Struktur der Formeln (RA-1) bis (RA-13) formen 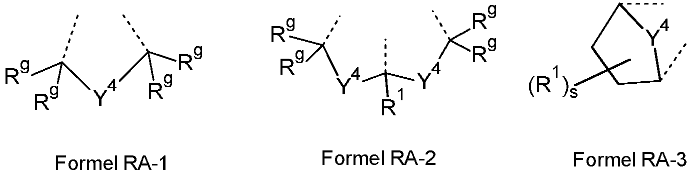 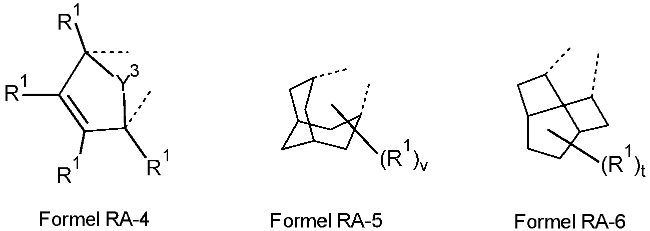 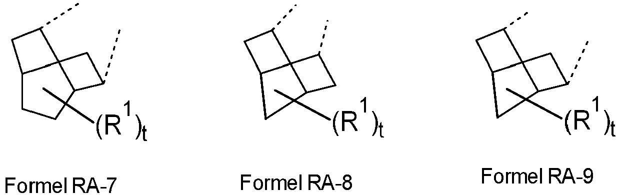 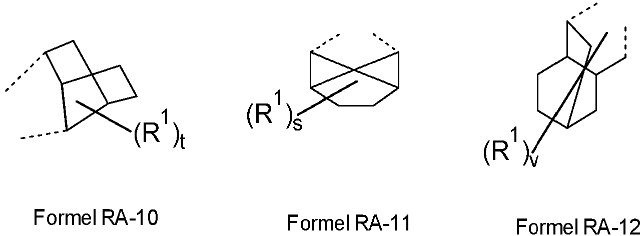 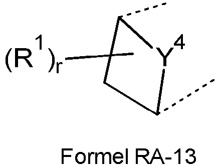 wobei R¹ die zuvor dargelegte Bedeutung hat, die gestrichelten Bindungen die Anbindungsstellen an die Atome der Gruppen, an die die zwei Reste R, R^{a}, R^{b}, R^{c}, R^{d} binden, darstellen, und die weiteren Symbole die folgende Bedeutung aufweisen:
- Y⁴: ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
- R^{g}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R²), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{g} auch miteinander oder ein Rest R^{g} mit einem Rest R¹ oder mit einer weiteren Gruppe ein Ringsystem bilden, wobei R² die in Anspruch 1 genannte Bedeutung aufweist;
- r: ist 0, 1, 2, 3 oder 4, vorzugsweise 0, 1, oder 2, besonders bevorzugt 0 oder 1;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

Hierbei sind Strukturen der Formeln RA-1, RA-3, RA-4 und RA-5 bevorzugt und Strukturen der Formeln RA-4 und RA-5 besonders bevorzugt.

In einer bevorzugten Ausführungsform der Erfindung bilden mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} Strukturen der Formeln (RA-1a) bis (RA-4f) formen 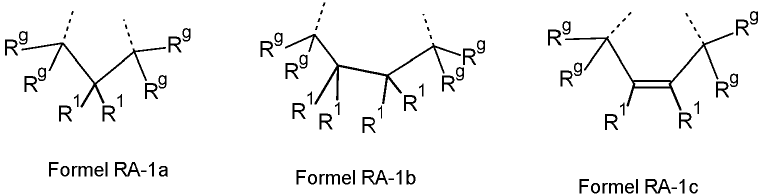 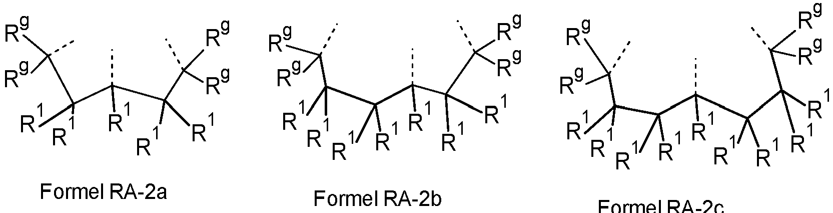 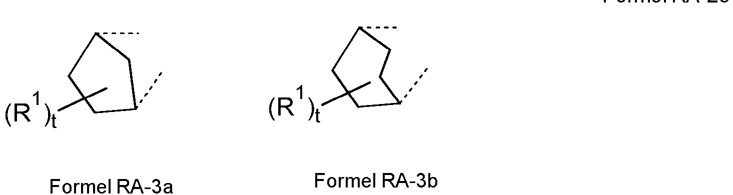 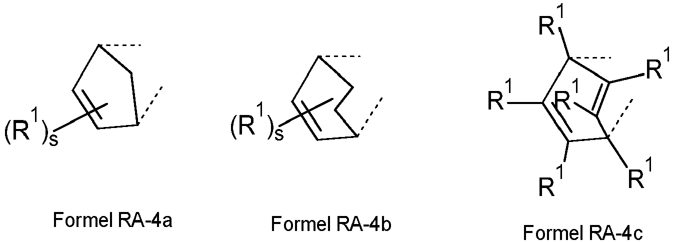 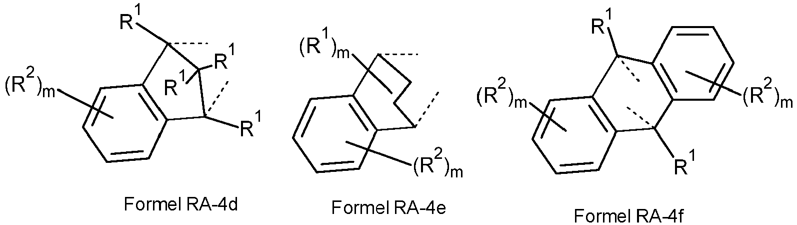 wobei die gestrichelten Bindungen die Anbindungsstellen darstellen, über die die zwei Reste R, R^{a}, R^{b}, R^{c}, R^{d} binden, der Index m 0, 1, 2, 3 oder 4, vorzugsweise 0, 1 oder 2 ist und die Symbole R¹, R², R^{g} und die Indices s, und t die zuvor, insbesondere für Formel (I) und/oder Formeln (RA-1) bis (RA-13) dargelegte Bedeutung haben.

Hierbei sind Strukturen der Formeln RA-4f bevorzugt.

Ferner kann vorgesehen sein, dass ein Rest R^{b} und ein Rest R^{d} die Strukturen der Formeln (Cy-1) bis (Cy-10), (RA-1) bis (RA-13) und/oder (RA-1a) bis (RA-4f) formen und einen kondensierten Ring bilden, wobei der Rest R^{b} und der Rest R^{d} vorzugsweise benachbart sind.

Weiterhin kann vorgesehen sein, dass zwei Reste R^{d} die Strukturen der Formeln (Cy-1) bis (Cy-10), (RA-1) bis (RA-13) und/oder (RA-1a) bis (RA-4f) formen und einen kondensierten Ring bilden, wobei die Reste R^{d} vorzugsweise benachbart sind. Des weiteren können die zwei Reste R^{d} auch von unterschiedlichen Ringen stammen.

In einer weiteren Ausgestaltung kann vorgesehen sein, dass ein Rest R^{b} mit einem Rest R oder R^{d} die Strukturen der Formeln (Cy-1) bis (Cy-10), (RA-1) bis (RA-13) und/oder (RA-1a) bis (RA-4f) formen und einen kondensierten Ring bilden.

In einer weiterhin bevorzugten Ausgestaltung bilden mindestens zwei Reste R, R^{a}, R^{b}, R^{c}, R^{d} , vorzugsweise mindestens zwei Reste R, R^{b}, R^{d} mit den weiteren Gruppen, an die die zwei Reste R, R^{a}, R^{b}, R^{c}, R^{d} beziehungsweise die zwei Reste R, R^{b}, R^{d} binden, einen kondensierten Ring bilden, wobei die zwei Reste R, R^{a}, R^{b}, R^{c}, R^{d}, vorzugsweise die zwei Reste R, R^{b}, R^{d} Strukturen der Formel (RB) formen 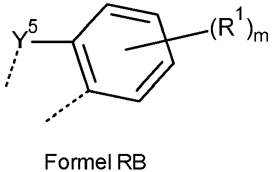 wobei R¹ die zuvor, insbesondere für Formel (I) genannte Bedeutung aufweist, die gestrichelten Bindungen die Anbindungsstellen darstellen, über die die zwei Reste R, R^{a}, R^{b}, R^{c}, R^{d} beziehungsweise die zwei Reste R, R^{b}, R^{d} binden, der Index m 0, 1, 2, 3 oder 4, vorzugsweise 0, 1 oder 2 ist, und Y⁵ C(R¹)₂, NR¹, NAr', BR¹, BAr', O oder S ist, vorzugsweise C(R¹)₂, NAr' oder O, besonders bevorzugt C(R¹)₂ oder O, wobei Ar' die zuvor, insbesondere für Formel (I) genannte Bedeutung aufweist.

Hierbei kann vorgesehen sein, dass ein Rest R^{b} mit einem Rest R oder R^{d} die Strukturen der Formel (RB) formen und einen kondensierten Ring bilden. Ferner kann vorgesehen sein, dass zwei Reste R^{d} die Strukturen der Formel (RB) formen und einen kondensierten Ring bilden, wobei die Reste R^{d} vorzugsweise benachbart sind. Ferner kann vorgesehen sein, dass ein Rest R^{b} und ein Rest R^{d} die Strukturen der Formel (RB) formen und einen kondensierten Ring bilden, wobei die Reste R^{b} und Rest R^{d} vorzugsweise benachbart sind.

Insbesondere kann vorgesehen sein, dass in bevorzugten Strukturen/Verbindungen die Summe der Indices r, s, t, v, m und n vorzugsweise 0, 1, 2 oder 3, besonders bevorzugt 1 oder 2 ist.

Besonders bevorzugt umfassen die Verbindungen mindestens eine Struktur der Struktur der Formeln (III-1) bis (III-8), besonders bevorzugt sind die Verbindungen ausgewählt aus Verbindungen der Formeln (III-1) bis (III-8), wobei die Verbindungen mindestens einen kondensierten Ring aufweisen, 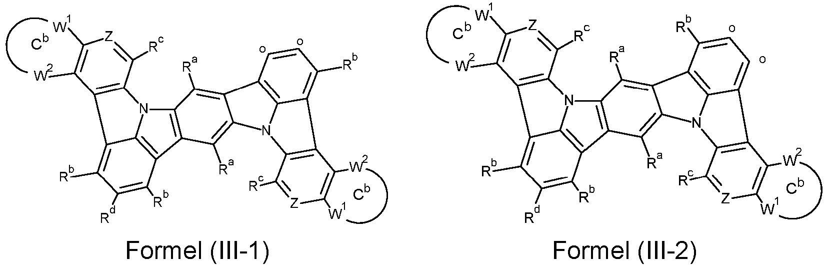 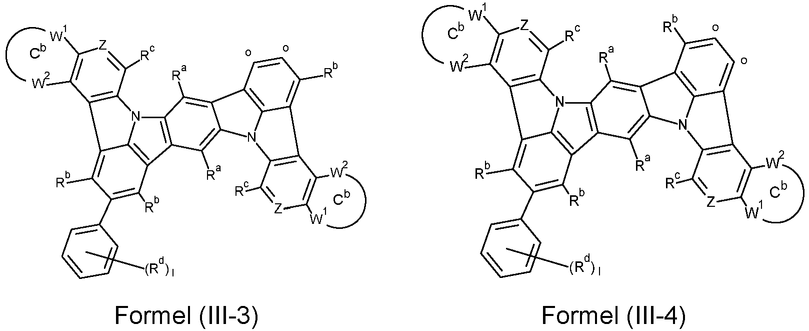 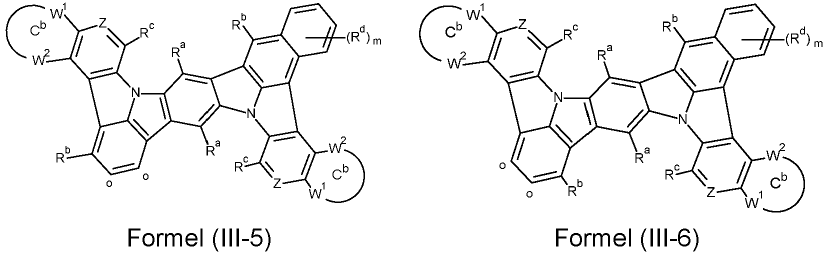 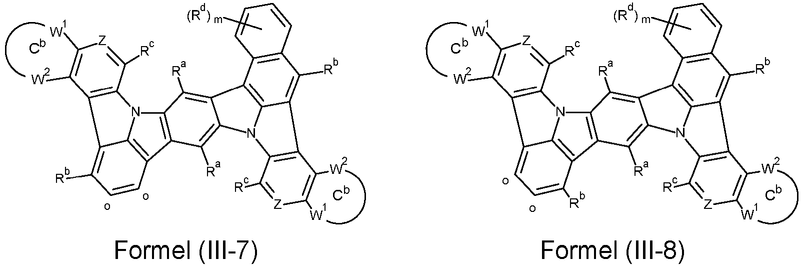 wobei die Symbole C^{b}, W¹, W², Z, R^{a}, R^{b}, R^{c} und R^{d} die zuvor, insbesondere für Formel (I) genannten Bedeutungen aufweisen, das Symbol o für die Kondensationsstellen des mindestens einen kondensierten Rings steht und die weiteren Indices die folgende Bedeutung haben:
- m: ist 0, 1, 2, 3 oder 4, vorzugsweise 0, 1 oder 2;
- l: ist 0, 1, 2, 3, 4 oder 5, vorzugsweise 0, 1 oder 2.

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-13) und/oder (RA-1a) bis (RA-4f) gebildet ist und ein weiterer Ring durch Strukturen der Formeln (RA-1) bis (RA-13), (RA-1a) bis (RA-4f) oder (RB) gebildet ist.

Besonders bevorzugt umfassen die Verbindungen mindestens eine Struktur der Formeln (IV-1) bis (IV-3), besonders bevorzugt sind die Verbindungen ausgewählt aus Verbindungen der Formeln (IV-1) bis (IV-3), wobei die Verbindungen mindestens zwei kondensierte Ringe aufweisen 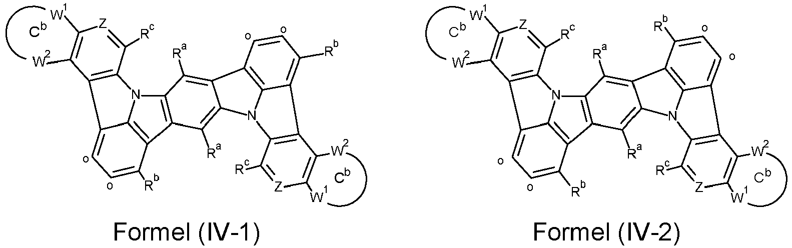 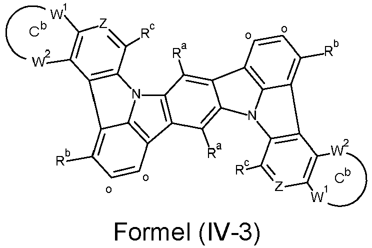 wobei die Symbole C^{b}, W¹, W², Z, R^{a}, R^{b} und R^{c} die zuvor, insbesondere für Formel (I) genannten Bedeutungen aufweisen, das Symbol o für die Kondensationsstellen der mindestens zwei kondensierten Ringe steht.

Bevorzugt wird mindestens einer der kondensierte Ring, besonders bevorzugt werden beide der kondensierten Ringe, insbesondere in Formeln (IV-1) bis (IV-3), durch mindestens zwei Reste R, R^{a}, R^{b}, R^{c}, R^{d} und den weiteren Gruppen, an die die zwei Reste R, R^{a}, R^{b}, R^{c}, R^{d} binden, gebildet, wobei die mindestens zwei Reste R, R^{a}, R^{b}, R^{c}, R^{d} Strukturen der Formeln (RA-1) bis (RA-12) und/oder der Formel (RB) formen, vorzugsweise Strukturen der Formeln (RA-1) bis (RA-12).

Weiterhin kann vorgesehen sein, dass die Substituenten R, R^{a}, R^{b}, R^{c}, R^{d}, R^{f}, R^{g}, R¹, R², R³ und R⁴ gemäß obigen Formeln mit den Ringatomen des Ringsystems, an das die Substituenten R, R^{a}, R^{b}, R^{c}, R^{d}, R^{f}, R^{g}, R¹, R², R³ und R⁴ binden, kein kondensiertes aromatisches oder heteroaromatisches Ringsystem bilden. Dies schließt die Bildung eines kondensierten aromatischen oder heteroaromatischen Ringsystems mit möglichen Substituenten R^{d}, R¹ und R² ein, die an die Substituenten R, R^{a}, R^{b}, R^{c}, R^{d}, R^{f}, R^{g}, R¹, R³ und R⁴ gebunden sein können.

Wenn die erfindungsgemäße Verbindung mit aromatischen oder heteroaromatischen Gruppen R, R^{a}, R^{b}, R^{c}, R^{d}, R^{f}, R^{g}, R¹, R², R³ beziehungsweise R⁴ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Vorzugsweise kann daher vorgesehen sein, dass der Rest R keine durchgängig konjungierte Anthracen-Gruppe umfasst, bevorzugt keiner der Reste R, R^{a}, R^{b}, R^{c}, R^{d}, R^{f}, R^{g}, R¹, R², R³ und R⁴ eine durchgängig konjungierte Anthracen-Gruppe umfasst.

Eine durchgängige Konjugation der Anthracen-Gruppe wird ausgebildet, sobald direkte Bindungen zwischen der Anthracen-Gruppe, dem erfindungsgemäßen Grundgerüst, welches in Formel (I) dargestellt ist, und einer optionalen aromatischen oder heteroaromatischen Verbindungsgruppe gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das sp³ hybridisierte Kohlenstoffatom in Position 9 zwar eine Kondensation dieser Ringe unterbindet, jedoch eine Konjugation erfolgen kann, da dieses sp³ hybridisierte Kohlenstoffatom in Position 9 nicht zwingend zwischen den Gruppen, die über eine Verbindungsgruppe verbunden sind, liegt. Im Gegensatz hierzu kann bei einer Spirobifluorenstruktur eine durchgängige Konjugation ausgebildet werden, falls die Verbindung zwischen den Gruppen, die über die Spirobifluorengruppe verbunden sind, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen den Gruppen, die über eine Spirobifluorengruppe verbunden sind, über verschiedene Phenylgruppen der zweiten Spirobifluorenstruktur erfolgt, die über das sp³ hybridisierte Kohlenstoffatom in Position 9 verbunden sind, ist die Konjugation unterbrochen.

Besonders bevorzugt kann weiterhin vorgesehen sein, dass der Rest R keine Anthracen-Gruppe umfasst, bevorzugt keiner der Reste R, R^{a}, R^{b}, R^{c} und R^{d}, besonders bevorzugt keiner der Reste R, R^{a}, R^{b}, R^{c}, R^{d}, R^{f}, R^{g}, R¹, R², R³ und R⁴ eine Anthracen-Gruppe umfasst.

Ganz speziell bevorzugt kann ferner vorgesehen sein, dass der Reste R kein aromatisches oder heteroaromatisches Ringsystem umfasst, welches drei linear kondensierte aromatische 6 Ringe aufweist, wobei vorzugsweise keiner der Reste R, R^{a}, R^{b}, R^{c} und R^{d}, besonders bevorzugt keiner der Reste R, R^{a}, R^{b}, R^{c}, R^{d}, R^{f}, R^{g}, R¹, R², R³ und R⁴ ein aromatisches oder heteroaromatisches Ringsystem umfasst, welches drei linear kondensierte aromatische 6 Ringe aufweist.

Weiterhin kann vorgesehen sein, dass keiner der Reste R, R^{a}, R^{b}, R^{c} und R^{d}, besonders bevorzugt keiner der Reste R, R^{a}, R^{b}, R^{c}, R^{d}, R^{f}, R^{g}, R¹, R², R³ und R⁴ eine Fluorenon-Gruppe umfasst oder bildet. Dies schließt Substituenten ein, die an die Reste R, R^{a}, R^{b}, R^{c}, R^{d}, etc. binden. Eine Fluorenon umfasst einen 5-Ring mit einer CO-Gruppe an den zwei aromatische 6-Ringe kondensiert sind.

Wenn zwei Reste, die insbesondere ausgewählt sein können aus R, R^{a}, R^{b}, R^{c}, R^{d}, R^{f}, R^{g}, R¹, R², R³ und R⁴, miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R^{a}, R^{b}, R^{c}, R^{d}, R^{f}, R^{g}, R¹, R², R³ und/oder R⁴ versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R^{a}, R^{b}, R^{c}, R^{d}, R^{f}, R^{g}, R¹, R², R³ und/oder R⁴ versehen.

Vorzugsweise kann vorgesehen sein, dass die Struktur/Verbindung in Bezug auf die Teilstrukturen B symmetrisch ist.

Symmetrisch in Bezug auf die Teilstrukturen B bedeutet insbesondere, dass die entsprechenden Reste R, R^{a}, R^{b}, R^{c}, R^{d}, R^{f}, R^{g}, R¹, R², R³ und R⁴ gleich sind und sich nicht unterscheiden.

Strukturen/Verbindungen, bei denen die Teilstrukturen B symmetrisch sind, zeichnen sich durch eine überraschend hohe Farbreinheit aus, die sich insbesondere in einem engen Emissionsspektrum widerspiegelt.

In einer weiteren Ausgestaltung kann die Struktur/Verbindung in Bezug auf die Verbindung in Bezug auf die Teilstrukturen B unsymmetrisch sein.

Ferner kann vorgesehen sein, dass ein Rest R, vorzugsweise der Rest R, der zu einer Gruppe X^{b} oder einem Rest R^{b} benachbart ist, mindestens eine Gruppe ausgewählt aus C(Ar)₃, C(R^{d})₃, Si(Ar)₃, Si(R^{d})₃, B(R^{d})₂, vorzugsweise ausgewählt aus C(Ar)₃, C(R^{d})₃, Si(Ar)₃, Si(R^{d})₃, darstellt, besonders bevorzugt eine Fluorengruppe, die mit einem oder mehreren Resten R^{d} substituiert sein kann, umfasst, darstellt oder mit einem Rest R^{b} bildet.

Weiterhin kann vorgesehen sein, dass der Rest R^{b} und/oder R^{d} mindestens eine Gruppe ausgewählt aus C(Ar')₃, C(R¹)₃, Si(Ar')₃, Si(R¹)₃, B(R¹)₂, vorzugsweise ausgewählt aus C(Ar')₃, C(R¹)₃, Si(Ar')₃, Si(R¹)₃, darstellt, vorzugsweise eine Fluorengruppe, die mit einem oder mehreren Resten R¹ substituiert sein kann, umfasst, darstellt oder mit einem Rest R^{b} beziehungsweise R^{d} bildet.

Strukturen/Verbindungen mit einer der zuvor genannten Gruppen ausgewählt aus C(Ar)₃, C(R^{d})₃, Si(Ar)₃, Si(R^{d})₃, B(R^{d})₂ beziehungsweise C(Ar')₃, C(R¹)₃, Si(Ar')₃, Si(R¹)₃, B(R¹)₂, besonders bevorzugt einer Fluorengruppe zeichnen sich durch eine überraschend hohe Effizienz aus.

Gemäß einer bevorzugten Ausgestaltung ist eine erfindungsgemäße Verbindung durch mindestens eine der Strukturen gemäß Formeln (I), (I-1) und/oder (I-2) darstellbar. Vorzugsweise weisen erfindungsgemäße Verbindungen, bevorzugt umfassend Strukturen gemäß Formeln (I), (I-1) und/oder (I-2) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Bevorzugte aromatische bzw. heteroaromatische Ringsysteme Ar, R, R^{a}, R^{b}, R^{c}, R^{d}, R^{f}, R^{g}, R³, R⁴ und/oder Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{d}, R¹ oder R² substituiert sein können.

Vorzugsweise kann vorgesehen sein, dass mindestens ein Substituent R, R^{a}, R^{b}, R^{c}, R^{d} gleich oder verschieden bei jedem Auftreten ausgewählt ist aus der Gruppe bestehend aus H, D, einer verzweigte oder cyclische Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-75, vorzugsweise die Substituenten R, R^{a}, R^{b}, R^{c}, R^{d} entweder einen kondensierten Ring, vorzugsweise gemäß den Strukturen der Formeln (RA-1) bis (RA-13) oder (RB) bilden oder der Substituent R, R^{a}, R^{b}, R^{c}, R^{d} gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-75, und/oder die Gruppe Ar' gleich oder verschieden bei jedem Auftreten ausgewählt ist aus den Gruppen der folgenden Formeln Ar-1 bis Ar-75, 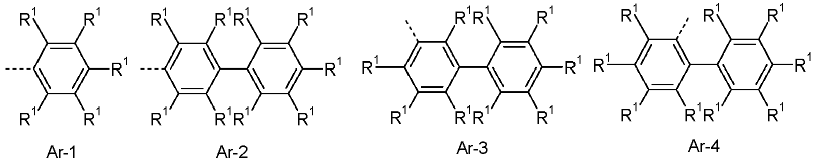 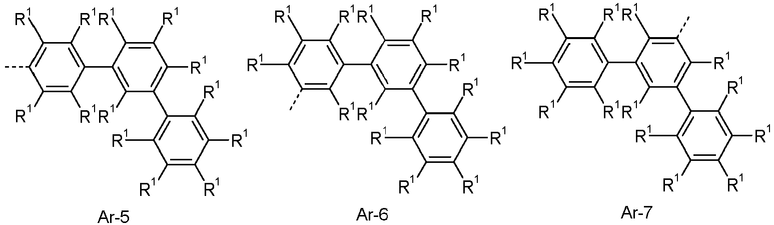 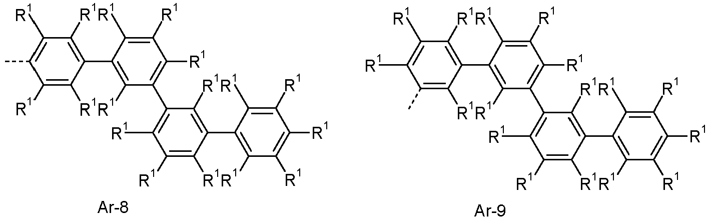 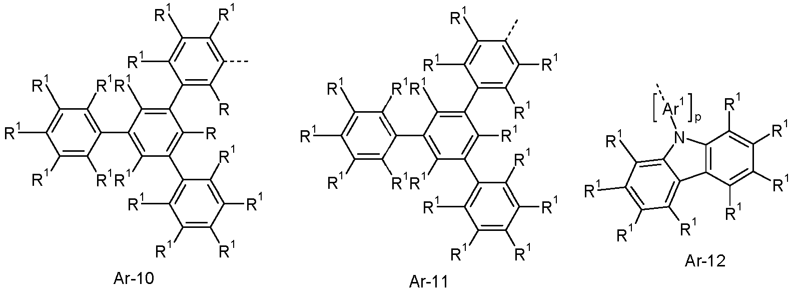 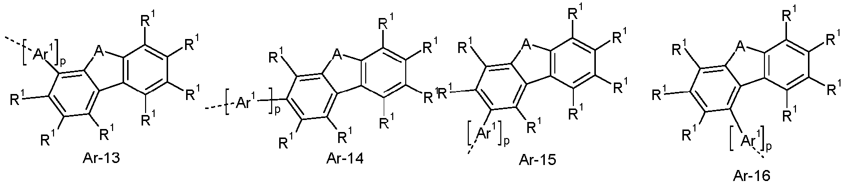 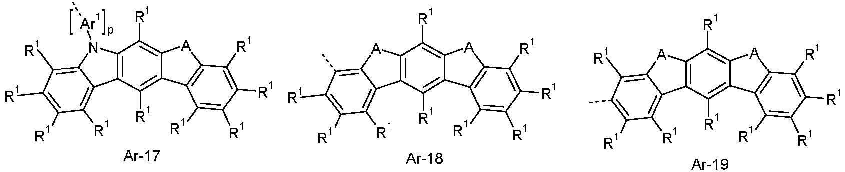 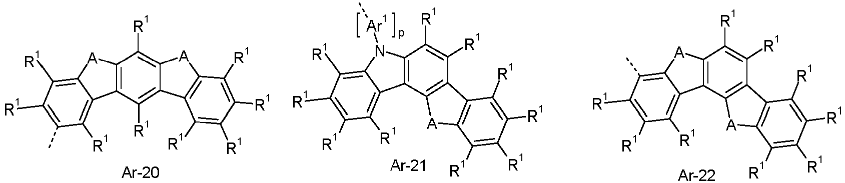 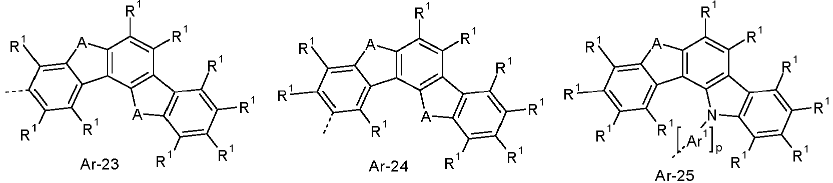 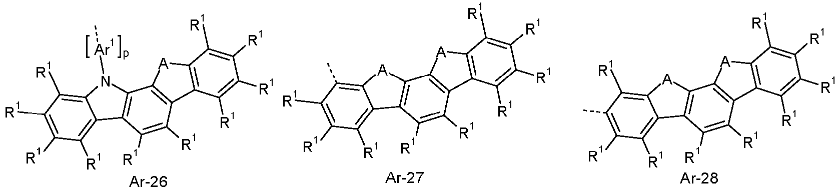 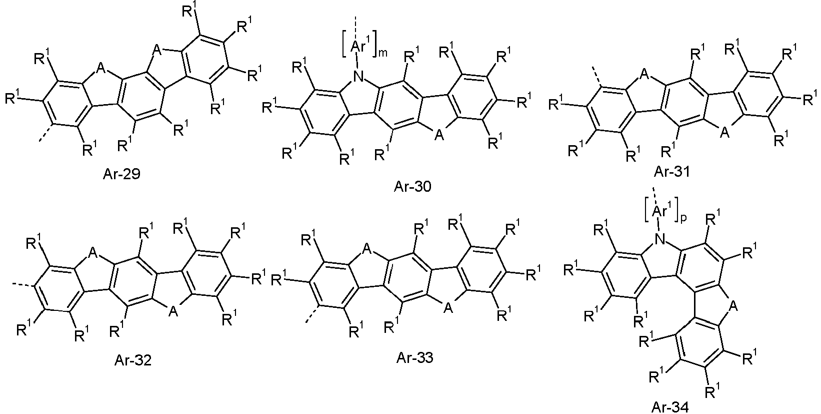 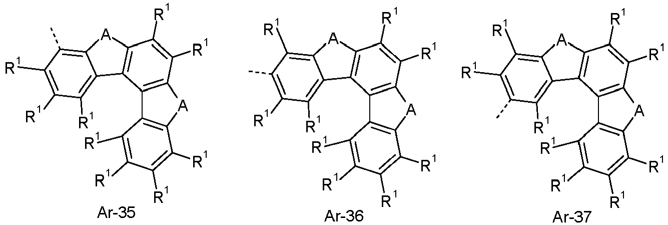 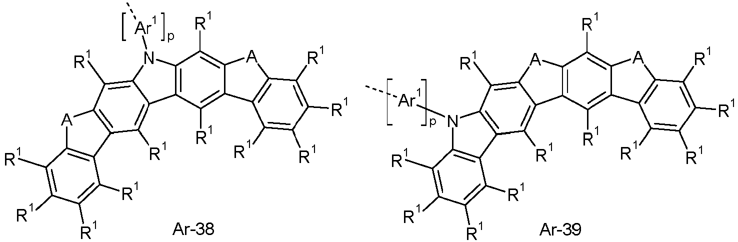 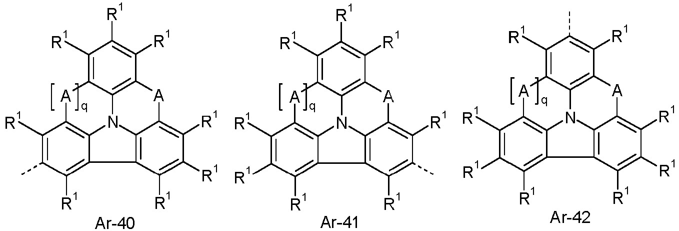 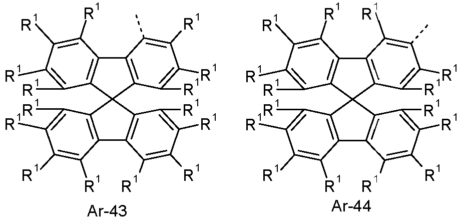 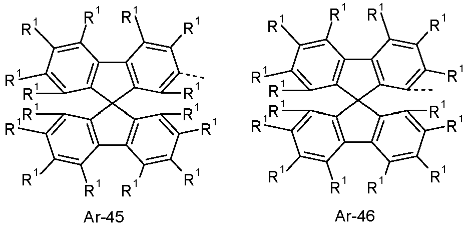 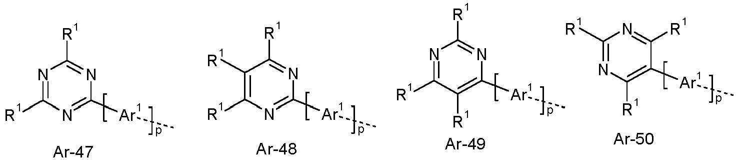 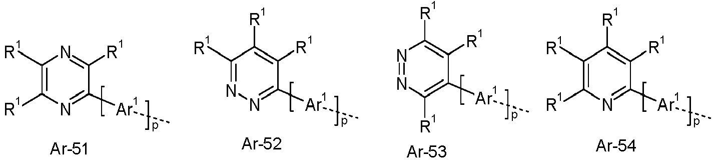 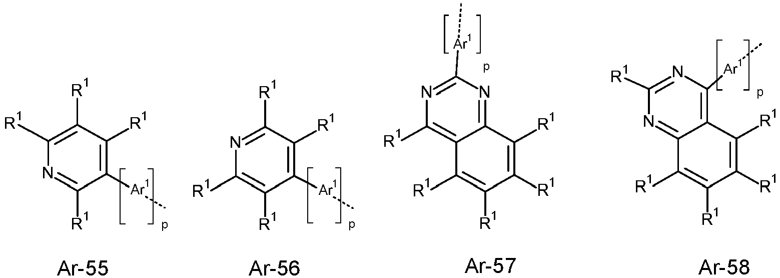 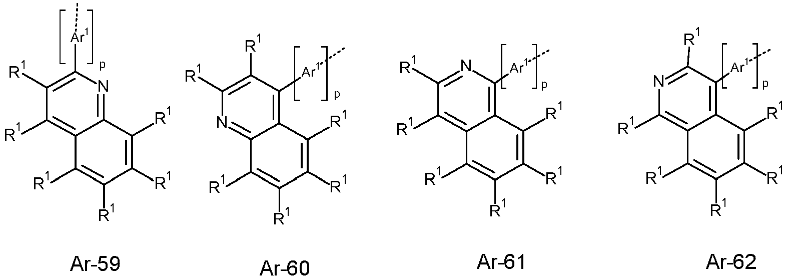 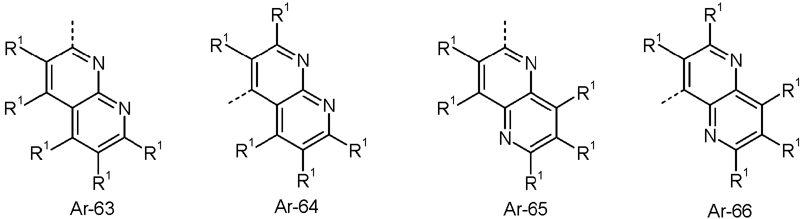 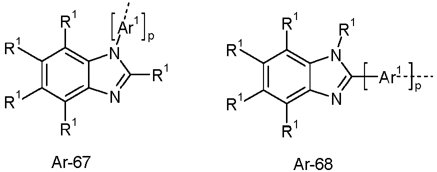 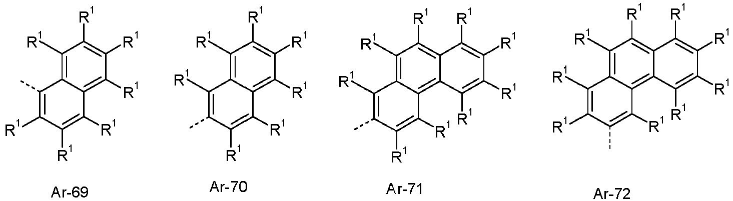 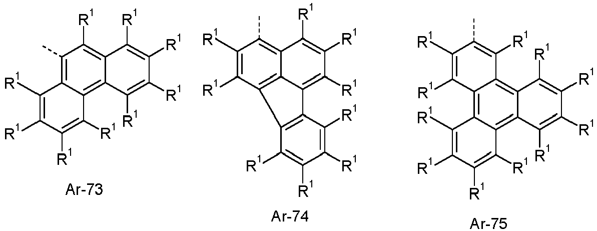 wobei R¹ die oben genannten Bedeutungen aufweist, die gestrichelte Bindung die Anbindungstelle an die entsprechende Gruppe darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Hierbei sind die Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Im Folgenden werden bevorzugte Substituenten R, R^{a}, R^{b}, R^{c}, R^{d}, R^{f} und R^{g} beschrieben.

In einer bevorzugten Ausführungsform der Erfindung ist R, R^{a}, R^{b}, R^{c}, R^{d} gleich oder verschieden bei jedem Auftreten ausgewählt aus der Gruppe bestehend aus H, D, F, CN, NO₂, Si(R¹)₃, B(OR¹)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist Substituent R, R^{a}, R^{b}, R^{c}, R^{d} gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

Ferner kann vorgesehen sein, dass mindestens ein Substituent R, R^{a}, R^{b}, R^{c}, R^{d} gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann. In einer weiterhin bevorzugten Ausführungsform der Erfindung bilden die Substituenten R, R^{a}, R^{b}, R^{c}, R^{d} entweder einen Ring gemäß den Strukturen der Formeln (RA-1) bis (RA-13), (RA-1 a) bis (RA-4f) oder (RB) oder R, R^{a}, R^{b}, R^{c}, R^{d} ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann. Besonders bevorzugt ist Substituent R, R^{a}, R^{b}, R^{c}, R^{d} gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

In einer bevorzugten Ausführungsform der Erfindung ist R^{f} beziehungsweise R^{g} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R^{d} beziehungsweise R² substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R^{d} beziehungsweise R² substituiert sein kann.

In einer weiterhin bevorzugten Ausführungsform der Erfindung ist R^{f} beziehungsweise R^{g} gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R^{d} beziehungsweise R² substituiert sein kann, einem aromatischen oder heteroaromatischen Ringsystem mit 6 bis 30 aromatischen Ringatomen, welches mit einem oder mehreren Resten R^{d} beziehungsweise R² substituiert sein kann.

Besonders bevorzugt ist der Rest R, welcher vorzugsweise einer Gruppe X^{b} oder R^{b} benachbart ist, beziehungsweise R^{d} gleich oder verschieden bei jedem Auftreten ausgewählt aus der Gruppe bestehend aus einer geradkettigen Alkylgruppe mit 1 bis 5 C-Atomen oder einer verzweigten oder cyclischen Alkylgruppe mit 3 bis 5 C-Atomen, wobei die Alkylgruppe jeweils mit einem oder mehreren Resten R^{d} beziehungsweise R¹ substituiert sein kann oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R^{d} beziehungsweise R¹ substituiert sein kann.

In einer bevorzugten Ausführungsform der Erfindung ist R^{f} beziehungsweise R^{g} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R^{d} beziehungsweise R² substituiert sein kann, oder einem aromatischen oder heteroaromatischen Ringsystem mit 6 bis 24 aromatischen Ringatomen, das jeweils durch einen oder mehrere Reste R^{d} beziehungsweise R² substituiert sein kann; dabei können zwei Reste R^{f} beziehungsweise R^{g} auch miteinander ein Ringsystem bilden. Besonders bevorzugt ist R^{f} beziehungsweise R^{g} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R^{d} beziehungsweise R² substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R^{d} beziehungsweise R² substituiert sein kann, bevorzugt aber unsubstituiert ist; dabei können zwei Reste R^{f} beziehungsweise R^{g} miteinander ein Ringsystem bilden. Ganz besonders bevorzugt ist R^{f} beziehungsweise R^{g} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{f} beziehungsweise R^{g} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme für die die Substituenten R, R³, R^{a}, R^{b}, R^{c}, R^{d}, R^{f}, R^{g} beziehungsweise Ar, Ar' oder Ar" stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{d}, R¹ beziehungsweise R² substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-75 ist festzuhalten, dass diese mit einem Substituenten R¹ dargestellt sind. Im Falle des Ringsystems R, R³, R^{f} oder Ar sind diese Substituenten R¹ durch R^{d} und im Falle Ar", R^{g} sind diese Substituenten R¹ durch R² zu ersetzen.

Weitere geeignete Gruppen R, R^{a}, R^{b}, R^{c}, R^{d} sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40. Diese Gruppen der Formel -Ar⁴-N(Ar²)(Ar³) sind jedoch nicht bevorzugt.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formel (I), (I-1), (I-2) und/oder (II-1) bis (II-15) umfasst, wobei vorzugsweise eines der aromatischen oder heteroaromatischen Ringsysteme, die durch die mindestens einer der Gruppen R, R^{b}, R^{d} darstellbar ist oder an das die Gruppen R, R^{b}, R^{d} binden, von beiden Strukturen geteilt wird.

In einer bevorzugten Ausgestaltung sind die Verbindungen ausgewählt aus Verbindungen gemäß der Formel (D-1), (D2) oder (D-3), 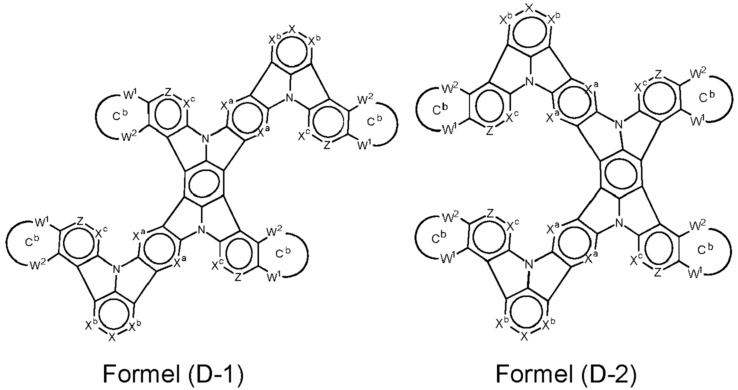 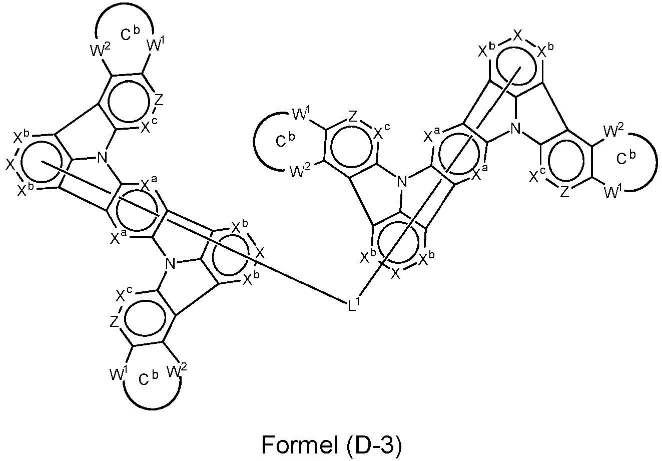 wobei die Gruppe L¹ eine Verbindungsgruppe, vorzugsweise eine Bindung oder ein aromatisches oder heteroaromatisches Ringsystem mit 5 bis 40, bevorzugt 5 bis 30 aromatischen Ringatomen darstellt, welches durch einen oder mehrere Reste R substituiert sein kann, und die weiteren verwendeten Symbole die zuvor, insbesondere für Formel (I) genannten Bedeutungen aufweisen.

In einer weiteren bevorzugten Ausführungsform der Erfindung steht L¹ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste R substituiert sein kann, bevorzugt aber unsubstituiert ist, wobei R die zuvor, insbesondere für Formel (I) genannte Bedeutung aufweisen kann. Besonders bevorzugt steht L¹ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R¹ substituiert sein kann, bevorzugt aber unsubstituiert ist, wobei R¹ die zuvor, insbesondere für Formel (I) genannte Bedeutung aufweisen kann.

Weiterhin bevorzugt steht das unter anderem in Formel (D3) dargelegte Symbol L¹ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (D3) dargelegte Gruppe L¹ ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L¹ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R¹ substituiert sein können, bevorzugt aber unsubstituiert sind.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

In einer weiteren Ausgestaltung der vorliegenden Erfindung sind Verbindungen umfassend eine Struktur gemäß Formel (I), vorzugsweise Verbindungen gemäß Formel (I) bevorzugt, bei denen mindestens ein Ring C^{b} die folgenden Eigenschaften aufweist:

| Formel des Ringes C^{b} | Z¹ | Z² | Z³ |
|---|---|---|---|
| BCy-1 | C(R³)₂ | C(R)₂ | C(R³)₂ |
| BCy-2 | C(R³)₂ | C(R)₂ | C(R³)₂ |
| BCy-3 | C(R³)₂ | C(R)₂ | C(R³)₂ |
| BCy-1 | Si(R³)₂ | C(R)₂ | Si(R³)₂ |
| BCy-2 | Si(R³)₂ | C(R)₂ | Si(R³)₂ |
| BCy-3 | Si(R³)₂ | C(R)₂ | Si(R³)₂ |

In einer weiteren Ausgestaltung der vorliegenden Erfindung sind Verbindungen umfassend eine Struktur gemäß Formel (I), vorzugsweise Verbindungen gemäß Formel (I) bevorzugt, bei denen mindestens ein Ring C^{b} die folgenden Eigenschaften aufweist:

| Formel des Ringes C^{b} | G | R | Z² |
|---|---|---|---|
| BCy-4 | Alkylengruppe mit 1, 2 oder 3 C-Atomen | H, Methyl oder Ar-1 bis Ar-75, vorzugsweise H | C(R)₂ |
| BCy-5 | Alkylengruppe mit 1, 2 oder 3 C-Atomen | H, Methyl oder Ar-1 bis Ar-75, vorzugsweise H | C(R)₂ |
| BCy-6 | Alkylengruppe mit 1, 2 oder 3 C-Atomen | H, Methyl oder Ar-1 bis Ar-75, vorzugsweise H | C(R)₂ |
| BCy-7 | Alkylengruppe mit 1, 2 oder 3 C-Atomen | H, Methyl oder Ar-1 bis Ar-75, vorzugsweise H | C(R)₂ |
| BCy-8 | Alkylengruppe mit 1, 2 oder 3 C-Atomen | H oder Ar-1 bis H, Methyl oder Ar-1 bis Ar-75, vorzugsweise H | C(R)₂ |
| BCy-9 | Alkylengruppe mit 1, 2 oder 3 C-Atomen | H, Methyl oder Ar-1 bis Ar-75, vorzugsweise H | C(R)₂ |
| BCy-10 | Alkylengruppe mit 1, 2 oder 3 C-Atomen | H, Methyl oder Ar-1 bis Ar-75, vorzugsweise H | C(R)₂ |
| BCy-4 | -CR=CR- | H, Methyl oder Ar-1 bis Ar-75, vorzugsweise H | C(R)₂ |
| BCy-5 | -CR=CR- | H, Methyl oder Ar-1 bis Ar-75, vorzugsweise H | C(R)₂ |
| BCy-6 | -CR=CR- | H, Methyl oder Ar-1 bis Ar-75, vorzugsweise H | C(R)₂ |
| BCy-7 | -CR=CR- | H, Methyl oder Ar-1 bis Ar-75, vorzugsweise H | C(R)₂ |
| BCy-8 | -CR=CR- | H oder Ar-1 bis H, Methyl oder Ar-1 bis Ar-75, vorzugsweise H | C(R)₂ |
| BCy-9 | -CR=CR- | H, Methyl oder Ar-1 bis Ar-75, vorzugsweise H | C(R)₂ |
| BCy-10 | -CR=CR- | H, Methyl oder Ar-1 bis Ar-75, vorzugsweise H | C(R)₂ |
| BCy-4 | Arylen- oder Heteroarylengruppe mit 5 bis 14 aromatischen Ringatomen | H, Methyl oder Ar-1 bis Ar-75, vorzugsweise H | C(R)₂ |
| BCy-5 | Arylen- oder Heteroarylengruppe mit 5 bis 14 aromatischen Ringatomen | H, Methyl oder Ar-1 bis Ar-75, vorzugsweise H | C(R)₂ |
| BCy-6 | Arylen- oder Heteroarylengruppe mit 5 bis 14 aromatischen Ringatomen | H, Methyl oder Ar-1 bis Ar-75, vorzugsweise H | C(R)₂ |
| BCy-7 | Arylen- oder Heteroarylengruppe mit 5 bis 14 aromatischen Ringatomen | H, Methyl oder Ar-1 bis Ar-75, vorzugsweise H | C(R)₂ |
| BCy-8 | Arylen- oder Heteroarylengruppe mit 5 bis 14 aromatischen Ringatomen | H oder Ar-1 bis H, Methyl oder Ar-1 bis Ar-75, vorzugsweise H | C(R)₂ |
| BCy-9 | Arylen- oder Heteroarylengruppe mit 5 bis 14 aromatischen Ringatomen | H, Methyl oder Ar-1 bis Ar-75, vorzugsweise H | C(R)₂ |
| BCy-10 | Arylen- oder Heteroarylengruppe mit 5 bis 14 aromatischen Ringatomen | H, Methyl oder Ar-1 bis Ar-75, vorzugsweise H | C(R)₂ |

In einer weiteren Ausgestaltung der vorliegenden Erfindung sind Verbindungen umfassend eine Struktur gemäß Formel (II-1), vorzugsweise Verbindungen gemäß Formel (II-1) bevorzugt, bei denen der Ring C^{b} und die Reste R^{a}, R^{b}, R^{c} und R^{d} bei jedem Auftreten gleich oder verschieden folgenden Bedeutungen aufweisen:

| C^{b} | R^{a} | R^{c} | R^{b} | R^{d} |
|---|---|---|---|---|
| BRA-5 | H, D, Alkyl | H, D, Alkyl | H, D, Alkyl | H, D, Alkyl |
| BRA-4 | H, D, Alkyl | H, D, Alkyl | H, D, Alkyl | H, D, Alkyl |
| BRA-3f | H, D, Alkyl | H, D, Alkyl | H, D, Alkyl | H, D, Alkyl |
| BRA-3 | H, D, Alkyl | H, D, Alkyl | H, D, Alkyl | H, D, Alkyl |
| BRA-2 | H, D, Alkyl | H, D, Alkyl | H, D, Alkyl | H, D, Alkyl |
| BRA-1 | H, D, Alkyl | H, D, Alkyl | H, D, Alkyl | H, D, Alkyl |
| BRA-5 | H, D, Alkyl | H, D, Alkyl | H, D, Alkyl und Phenyl-Ringbildung mit R^{d} | Phenyl-Ringbildung mit R^{b} |
| BRA-4 | H, D, Alkyl | H, D, Alkyl | H, D, Alkyl und Phenyl-Ringbildung mit R^{d} | Phenyl-Ringbildung mit R^{b} |
| BRA-3f | H, D, Alkyl | H, D, Alkyl | Aryl-, Heteroaryl und Phenyl-Ringbildung mit R^{d} | Phenyl-Ringbildung mit R^{b} |
| BRA-3 | H, D, Alkyl | H, D, Alkyl | H, D, Alkyl und Phenyl-Ringbildung mit R^{d} | Phenyl-Ringbildung mit R^{b} |
| BRA-2 | H, D, Alkyl | H, D, Alkyl | Aryl-, Heteroaryl und Phenyl-Ringbildung mit R^{d} | Phenyl-Ringbildung mit R^{b} |
| BRA-1 | H, D, Alkyl | H, D, Alkyl | H, D, Alkyl und Phenyl-Ringbildung mit R^{d} | Phenyl-Ringbildung mit R^{b} |
| BRA-5 | H, D, Alkyl | H, D, Alkyl | H, D, Alkyl und Heteroaryl-Ringbildung mit R^{d} | Heteroaryl-Ringbildung mit R^{b} |
| BRA-4 | H, D, Alkyl | H, D, Alkyl | Aryl-, Heteroaryl und Heteroaryl-Ringbildung mit R^{d} | Heteroaryl-Ringbildung mit R^{b} |
| BRA-3f | H, D, Alkyl | H, D, Alkyl | H, D, Alkyl und Heteroaryl-Ringbildung mit R^{d} | Heteroaryl-Ringbildung mit R^{b} |
| BRA-3 | H, D, Alkyl | H, D, Alkyl | H, D, Alkyl und Heteroaryl-Ringbildung mit R^{d} | Heteroaryl-Ringbildung mit R^{b} |
| BRA-2 | H, D, Alkyl | H, D, Alkyl | H, D, Alkyl und Heteroaryl-Ringbildung mit R^{d} | Heteroaryl-Ringbildung mit R^{b} |
| BRA-1 | H, D, Alkyl | H, D, Alkyl | H, D, Alkyl und Heteroaryl-Ringbildung mit R^{d} | Heteroaryl-Ringbildung mit R^{b} |
| BRA-5 | H, D, Alkyl | H, D, Alkyl | H, D, Alkyl | C(Ar')₃, Si(Ar')₃ |
| BRA-4 | H, D, Alkyl | H, D, Alkyl | H, D, Alkyl | C(Ar')₃, Si(Ar')₃ |
| BRA-3f | H, D, Alkyl | H, D, Alkyl | H, D, Alkyl | C(Ar')₃, Si(Ar')₃ |
| BRA-3 | H, D, Alkyl | H, D, Alkyl | H, D, Alkyl | C(Ar')₃, Si(Ar')₃ |
| BRA-2 | H, D, Alkyl | H, D, Alkyl | H, D, Alkyl | C(Ar')₃, Si(Ar')₃ |
| BRA-1 | H, D, Alkyl | H, D, Alkyl | H, D, Alkyl | C(Ar')₃, Si(Ar')₃ |
| BRA-5 | H, D, Alkyl | H, D, Alkyl | H, D, Alkyl und Ringbildung mit R^{d} | C(Ar')₃, Si(Ar')₃ |
| BRA-4 | H, D, Alkyl | H, D, Alkyl | H, D, Alkyl und Ringbildung mit R^{d} | C(Ar')₃, Si(Ar')₃ |
| BRA-3f | H, D, Alkyl | H, D, Alkyl | H, D, Alkyl und Ringbildung mit R^{d} | C(Ar')₃, Si(Ar')₃ |
| BRA-3 | H, D, Alkyl | H, D, Alkyl | H, D, Alkyl und Ringbildung mit R^{d} | C(Ar')₃, Si(Ar')₃ |
| BRA-2 | H, D, Alkyl | H, D, Alkyl | H, D, Alkyl und Ringbildung mit R^{d} | C(Ar')₃, Si(Ar')₃ |
| BRA-1 | H, D, Alkyl | H, D, Alkyl | H, D, Alkyl und Ringbildung mit R^{d} | C(Ar')₃, Si(Ar')₃ |
| BRA-5 | H, D, Alkyl | H, D, Alkyl | H, D, Alkyl und RA-5-Ringbildung mit R^{d} | RA-5-Ringbildung mit R^{b} |
| BRA-4 | H, D, Alkyl | H, D, Alkyl | H, D, Alkyl und RA-4-Ringbildung mit R^{d} | RA-4-Ringbildung mit R^{b} |
| BRA-3f | H, D, Alkyl | H, D, Alkyl | H, D, Alkyl und RA-4f-Ringbildung mit R^{d} | RA-4f-Ringbildung mit R^{b} |
| BRA-3 | H, D, Alkyl | H, D, Alkyl | H, D, Alkyl und RA-3-Ringbildung mit R^{d} | RA-3-Ringbildung mit R^{b} |
| BRA-2 | H, D, Alkyl | H, D, Alkyl | H, D, Alkyl und RA-4f-Ringbildung mit R^{d} | RA-4f-Ringbildung mit R^{b} |
| BRA-1 | H, D, Alkyl | H, D, Alkyl | H, D, Alkyl und RA-3-Ringbildung mit R^{d} | RA-3-Ringbildung mit R^{b} |
| BRA-5 | H, D, Alkyl | H, D, Alkyl | H, D, Alkyl | Ar-1 bis Ar-75 |
| BRA-4 | H, D, Alkyl | H, D, Alkyl | H, D, Alkyl | Ar-1 bis Ar-75 |
| BRA-3f | H, D, Alkyl | H, D, Alkyl | H, D, Alkyl | Ar-1 bis Ar-75 |
| BRA-3 | H, D, Alkyl | H, D, Alkyl | H, D, Alkyl | Ar-1 bis Ar-75 |
| BRA-2 | H, D, Alkyl | H, D, Alkyl | H, D, Alkyl und RA-4f-Ringbildung mit R^{d} | RA-4f-Ringbildung mit R^{b} |
| BRA-1 | H, D, Alkyl | H, D, Alkyl | H, D, Alkyl und RA-3-Ringbildung mit R^{d} | RA-3-Ringbildung mit R^{b} |
| BRA-5 | Ar-1 bis Ar-75 | H, D, Alkyl | H, D, Alkyl | H, D, Alkyl |
| BRA-4 | Ar-1 bis Ar-75 | H, D, Alkyl | H, D, Alkyl | H, D, Alkyl |
| BRA-3f | Ar-1 bis Ar-75 | H, D, Alkyl | H, D, Alkyl | H, D, Alkyl |
| BRA-3 | Ar-1 bis Ar-75 | H, D, Alkyl | H, D, Alkyl | H, D, Alkyl |
| BRA-2 | Ar-1 bis Ar-75 | H, D, Alkyl | H, D, Alkyl | H, D, Alkyl |
| BRA-1 | Ar-1 bis Ar-75 | H, D, Alkyl | H, D, Alkyl | H, D, Alkyl |
| BRA-5 | Ar-1 bis Ar-75 | H, D, Alkyl | H, D, Alkyl und Phenyl-Ringbildung mit R^{d} | Phenyl-Ringbildung mit R^{b} |
| BRA-4 | Ar-1 bis Ar-75 | H, D, Alkyl | Aryl-, Heteroaryl und Phenyl-Ringbildung mit R^{d} | Phenyl-Ringbildung mit R^{b} |
| BRA-3f | Ar-1 bis Ar-75 | H, D, Alkyl | H, D, Alkyl und Phenyl-Ringbildung mit R^{d} | Phenyl-Ringbildung mit R^{b} |
| BRA-3 | Ar-1 bis Ar-75 | H, D, Alkyl | Aryl-, Heteroaryl und Phenyl-Ringbildung mit R^{d} | Phenyl-Ringbildung mit R^{b} |
| BRA-2 | Ar-1 bis Ar-75 | H, D, Alkyl | H, D, Alkyl und Phenyl-Ringbildung mit R^{d} | Phenyl-Ringbildung mit R^{b} |
| BRA-1 | Ar-1 bis Ar-75 | H, D, Alkyl | Aryl-, Heteroaryl und Phenyl-Ringbildung mit R^{d} | Phenyl-Ringbildung mit R^{b} |
| BRA-5 | Ar-1 bis Ar-75 | H, D, Alkyl | H, D, Alkyl und Heteroaryl-Ringbildung mit R^{d} | Heteroaryl-Ringbildung mit R^{b} |
| BRA-4 | Ar-1 bis Ar-75 | H, D, Alkyl | Aryl-, Heteroaryl und Heteroaryl-Ringbildung mit R^{d} | Heteroaryl-Ringbildung mit R^{b} |
| BRA-3f | Ar-1 bis Ar-75 | H, D, Alkyl | H, D, Alkyl und Heteroaryl-Ringbildung mit R^{d} | Heteroaryl-Ringbildung mit R^{b} |
| BRA-3 | Ar-1 bis Ar-75 | H, D, Alkyl | Aryl-, Heteroaryl und Heteroaryl-Ringbildung mit R^{d} | Heteroaryl-Ringbildung mit R^{b} |
| BRA-2 | Ar-1 bis Ar-75 | H, D, Alkyl | H, D, Alkyl und Heteroaryl-Ringbildung mit R^{d} | Heteroaryl-Ringbildung mit R^{b} |
| BRA-1 | Ar-1 bis Ar-75 | H, D, Alkyl | Aryl-, Heteroaryl und Heteroaryl-Ringbildung mit R^{d} | Heteroaryl-Ringbildung mit R^{b} |
| BRA-5 | Ar-1 bis Ar-75 | H, D, Alkyl | H, D, Alkyl | C(Ar')₃, Si(Ar')₃ |
| BRA-4 | Ar-1 bis Ar-75 | H, D, Alkyl | H, D, Alkyl | C(Ar')₃, Si(Ar')₃ |
| BRA-3f | Ar-1 bis Ar-75 | H, D, Alkyl | H, D, Alkyl | C(Ar')₃, Si(Ar')₃ |
| BRA-3 | Ar-1 bis Ar-75 | H, D, Alkyl | H, D, Alkyl | C(Ar')₃, Si(Ar')₃ |
| BRA-2 | Ar-1 bis Ar-75 | H, D, Alkyl | H, D, Alkyl | C(Ar')₃, Si(Ar')₃ |
| BRA-1 | Ar-1 bis Ar-75 | H, D, Alkyl | H, D, Alkyl | C(Ar')₃, Si(Ar')₃ |
| BRA-5 | Ar-1 bis Ar-75 | H, D, Alkyl | H, D, Alkyl und Ringbildung mit R^{d} | C(Ar')₃, Si(Ar')₃ |
| BRA-4 | Ar-1 bis Ar-75 | H, D, Alkyl | H, D, Alkyl und Ringbildung mit R^{d} | C(Ar')₃, Si(Ar')₃ |
| BRA-3f | Ar-1 bis Ar-75 | H, D, Alkyl | H, D, Alkyl und Ringbildung mit R^{d} | C(Ar')₃, Si(Ar')₃ |
| BRA-3 | Ar-1 bis Ar-75 | H, D, Alkyl | H, D, Alkyl und Ringbildung mit R^{d} | C(Ar')₃, Si(Ar')₃ |
| BRA-2 | Ar-1 bis Ar-75 | H, D, Alkyl | H, D, Alkyl und Ringbildung mit R^{d} | C(Ar')₃, Si(Ar')₃ |
| BRA-1 | Ar-1 bis Ar-75 | H, D, Alkyl | H, D, Alkyl und Ringbildung mit R^{d} | C(Ar')₃, Si(Ar')₃ |
| BRA-5 | Ar-1 bis Ar-75 | H, D, Alkyl | H, D, Alkyl und RA-5-Ringbildung mit R^{d} | RA-5-Ringbildung mit R^{b} |
| BRA-4 | Ar-1 bis Ar-75 | H, D, Alkyl | H, D, Alkyl und RA-4-Ringbildung mit R^{d} | RA-4-Ringbildung mit R^{b} |
| BRA-3f | Ar-1 bis Ar-75 | H, D, Alkyl | H, D, Alkyl und RA-4f-Ringbildung mit R^{d} | RA-4f-Ringbildung mit R^{b} |
| BRA-3 | Ar-1 bis Ar-75 | H, D, Alkyl | H, D, Alkyl und RA-3-Ringbildung mit R^{d} | RA-3-Ringbildung mit R^{b} |
| BRA-2 | Ar-1 bis Ar-75 | H, D, Alkyl | H, D, Alkyl und RA-4f-Ringbildung mit R^{d} | RA-4f-Ringbildung mit R^{b} |
| BRA-1 | Ar-1 bis Ar-75 | H, D, Alkyl | H, D, Alkyl und RA-3-Ringbildung mit R^{d} | RA-3-Ringbildung mit R^{b} |
| BRA-5 | Ar-1 bis Ar-75 | H, D, Alkyl | H, D, Alkyl | Ar-1 bis Ar-75 |
| BRA-4 | Ar-1 bis Ar-75 | H, D, Alkyl | H, D, Alkyl | Ar-1 bis Ar-75 |
| BRA-3f | Ar-1 bis Ar-75 | H, D, Alkyl | H, D, Alkyl | Ar-1 bis Ar-75 |
| BRA-3 | Ar-1 bis Ar-75 | H, D, Alkyl | H, D, Alkyl | Ar-1 bis Ar-75 |
| BRA-2 | Ar-1 bis Ar-75 | H, D, Alkyl | H, D, Alkyl | Ar-1 bis Ar-75 |
| BRA-1 | Ar-1 bis Ar-75 | H, D, Alkyl | H, D, Alkyl | Ar-1 bis Ar-75 |

In einer weiteren Ausgestaltung der vorliegenden Erfindung sind Verbindungen umfassend eine Struktur gemäß Formel (II-2), vorzugsweise Verbindungen gemäß Formel (II-2) bevorzugt, wobei der Index I vorzugsweise jeweils kleiner oder gleich 3, besonders bevorzugt jeweils 0, 1 oder 2 und speziell bevorzugt jeweils 0 oder 1 ist, und bei denen der Ring C^{b} und die Reste R^{a}, R^{b}, R^{c} und R^{d} bei jedem Auftreten gleich oder verschieden folgenden Bedeutungen aufweisen:

| C^{b} | R^{a} | R^{c} | R^{b} | R^{d} (nur falls I ungleich 0 ist mindestens ein Rest R^{d}, andernfalls sind alle R^{e} H) |
|---|---|---|---|---|
| BRA-5 | H, D, Alkyl | H, D, Alkyl | H, D, Alkyl | D, Alkyl |
| BRA-4 | H, D, Alkyl | H, D, Alkyl | H, D, Alkyl | D, Alkyl |
| BRA-3f | H, D, Alkyl | H, D, Alkyl | H, D, Alkyl | D, Alkyl |
| BRA-3 | H, D, Alkyl | H, D, Alkyl | H, D, Alkyl | D, Alkyl |
| BRA-5 | H, D, Alkyl | H, D, Alkyl | H, D, Alkyl | Ar-1 bis Ar-75 |
| BRA-4 | H, D, Alkyl | H, D, Alkyl | H, D, Alkyl | Ar-1 bis Ar-75 |
| BRA-3f | H, D, Alkyl | H, D, Alkyl | H, D, Alkyl | Ar-1 bis Ar-75 |
| BRA-3 | H, D, Alkyl | H, D, Alkyl | H, D, Alkyl | Ar-1 bis Ar-75 |
| BRA-5 | H, D, Alkyl | H, D, Alkyl | Ar-1 bis Ar-75 | D, Alkyl |
| BRA-4 | H, D, Alkyl | H, D, Alkyl | Ar-1 bis Ar-75 | D, Alkyl |
| BRA-3f | H, D, Alkyl | H, D, Alkyl | Ar-1 bis Ar-75 | D, Alkyl |
| BRA-3 | H, D, Alkyl | H, D, Alkyl | Ar-1 bis Ar-75 | D, Alkyl |
| BRA-5 | H, D, Alkyl | H, D, Alkyl | Ar-1 bis Ar-75 | Ar-1 bis Ar-75 |
| BRA-4 | H, D, Alkyl | H, D, Alkyl | Ar-1 bis Ar-75 | Ar-1 bis Ar-75 |
| BRA-3f | H, D, Alkyl | H, D, Alkyl | Ar-1 bis Ar-75 | Ar-1 bis Ar-75 |
| BRA-3 | H, D, Alkyl | H, D, Alkyl | Ar-1 bis Ar-75 | Ar-1 bis Ar-75 |
| BRA-5 | H, D, Alkyl | H, D, Alkyl | H, D, Alkyl und Ar-1 bis Ar-75 | D, Alkyl |
| BRA-4 | H, D, Alkyl | H, D, Alkyl | H, D, Alkyl und Ar-1 bis Ar-75 | D, Alkyl |
| BRA-3f | H, D, Alkyl | H, D, Alkyl | H, D, Alkyl und Ar-1 bis Ar-75 | D, Alkyl |
| BRA-3 | H, D, Alkyl | H, D, Alkyl | H, D, Alkyl und Ar-1 bis Ar-75 | D, Alkyl |
| BRA-5 | H, D, Alkyl | H, D, Alkyl | H, D, Alkyl und Ar-1 bis Ar-75 | Ar-1 bis Ar-75 |
| BRA-4 | H, D, Alkyl | H, D, Alkyl | H, D, Alkyl und Ar-1 bis Ar-75 | Ar-1 bis Ar-75 |
| BRA-3f | H, D, Alkyl | H, D, Alkyl | H, D, Alkyl und Ar-1 bis Ar-75 | Ar-1 bis Ar-75 |
| BRA-3 | H, D, Alkyl | H, D, Alkyl | H, D, Alkyl und Ar-1 bis Ar-75 | Ar-1 bis Ar-75 |
| BRA-5 | H, D, Alkyl | H, D, Alkyl | H, D, Alkyl | C(Ar')₃, Si(Ar')₃ |
| BRA-4 | H, D, Alkyl | H, D, Alkyl | H, D, Alkyl | C(Ar')₃, Si(Ar')₃ |
| BRA-3f | H, D, Alkyl | H, D, Alkyl | H, D, Alkyl | C(Ar')₃, Si(Ar')₃ |
| BRA-3 | H, D, Alkyl | H, D, Alkyl | H, D, Alkyl | C(Ar')₃, Si(Ar')₃ |
| BRA-5 | H, D, Alkyl | H, D, Alkyl | Ar-1 bis Ar-75 | C(Ar')₃, Si(Ar')₃ |
| BRA-4 | H, D, Alkyl | H, D, Alkyl | Ar-1 bis Ar-75 | C(Ar')₃, Si(Ar')₃ |
| BRA-3f | H, D, Alkyl | H, D, Alkyl | Ar-1 bis Ar-75 | C(Ar')₃, Si(Ar')₃ |
| BRA-3 | H, D, Alkyl | H, D, Alkyl | Ar-1 bis Ar-75 | C(Ar')₃, Si(Ar')₃ |
| BRA-5 | H, D, Alkyl | H, D, Alkyl | H, D, Alkyl und Ar-1 bis Ar-75 | C(Ar')₃, Si(Ar')₃ |
| BRA-4 | H, D, Alkyl | H, D, Alkyl | H, D, Alkyl und Ar-1 bis Ar-75 | C(Ar')₃, Si(Ar')₃ |
| BRA-3f | H, D, Alkyl | H, D, Alkyl | H, D, Alkyl und Ar-1 bis Ar-75 | C(Ar')₃, Si(Ar')₃ |
| BRA-3 | H, D, Alkyl | H, D, Alkyl | H, D, Alkyl und Ar-1 bis Ar-75 | C(Ar')₃, Si(Ar')₃ |

**In** obigen Tabellen stehen die in der Spalte unter der Gruppe R^{d} genannten Reste für die Substituenten am Phenylring des Grundgerüsts, der ebenfalls durch die genannten Rest R^{b} substituiert ist (siehe beispielsweise Formel (II-1)), oder für die Substituenten am Phenylring, der an den Phenylring des Grundgerüsts, der ebenfalls durch die genannten Rest R^{b} substituiert ist, bindet (siehe beispielsweise Formel (II-2). Ganz besonders bevorzugt steht R^{d} für eine Methylgruppe oder für eine Phenylgruppe. Dabei können die Reste R^{d} auch miteinander ein Ringsystem bilden, was zu einem Spirosystem führt.

Der Begriff "Alkyl" in obigen Tabellen umfasst insbesondere geradkettige Alkylgruppen oder verzweigte oder cyclische Alkylgruppen gemäß der zuvor dargelegten Definition für die jeweilige Gruppe.

Der Begriff "Aryl-, Heteroaryl" in obigen Tabellen umfasst insbesondere Aryl- oder Heteroarylgruppen mit 5 bis 40 aromatischen Ringatomen gemäß der zuvor dargelegten Definition für die jeweilige Gruppe, wobei die Arylgruppen vorzugsweise 6 bis 12, besonders bevorzugt 6 Ringatome und die Heteroarylgruppen vorzugsweise 5 bis 13, besonders bevorzugt 5 Ringatome aufweisen. Besonders bevorzugt umfassen Heteroarylgruppen ein oder zwei Heteroatome, vorzugsweise N, O oder S.

Die Bezeichnungen "BRA-1", "BRA-2", "BRA-3", "BRA-3f", "BRA-4", "BRA-5", "Ar-1", "Ar-75" beziehen sich auf die zuvor und nachfolgend dargelegten Strukturformeln.

Phenyl-Ringbildung mit einer Gruppe bedeutet, dass die beiden Gruppen zusammen eine Phenylgruppe bilden, die jeweils gemäß der zuvor dargelegten Definition für die jeweilige Gruppe mit Resten R¹ substituiert sein kann. Üblich bilden sich hierdurch mit der an das Stickstoffatom gebundenen Phenylgruppe, die durch die Reste R^{b} und R beziehungsweise R^{d} substituiert ist, eine Naphthylgruppe. Für die weiteren Ringbildungsdefinitionen gilt entsprechendes.

Der Begriff "und" insbesondere bei der Beschreibung bevorzugter Gruppen R^{b} bedeutet, dass die beiden Reste unterschiedlich sind, wobei einer der Reste R^{b} einer ersten und der zweite Rest R^{b} einer zweiten Definition entspricht. Der Ausdruck "Aryl-, Heteroaryl und Phenyl-Ringbildung mit R^{d}" bedeutet, dass einer der Reste R^{b} für eine Aryl-, Heteroarylgruppe steht und der zweite Rest R^{b} mit R^{d}" einen Phenylring bildet. Falls ein Feld keinen Ausdruck "und" umfasst, so stellen alle Reste eine entsprechende Gruppe dar. Der Ausdruck "Ar-1 bis Ar-75" für die Gruppe R^{d} bedeutet, dass beide Reste R^{b} einen Aryl- oder Heteroarylrest gemäß obigen oder nachfolgenden Formeln Ar-1 bis Ar-75 darstellen.

Für die weitere Verwendung des Begriffs "und" in obigen Tabellen gilt entsprechendes.

Die für die Formeln (II-1) und (II-2) dargelegten Bevorzugungen hinsichtlich des Rings C^{b} und der verschiedenen Substituenten R^{a}, R^{b}, R^{c} und R^{d} gelten selbstverständlich auch für die weiteren der zuvor dargelegten Formeln (II-3) bis (II-15) entsprechend.

Beispiele für bevorzugte Verbindungen gemäß den oben aufgeführten Ausführungsformen sind die in der folgenden Tabelle aufgeführten Verbindungen:

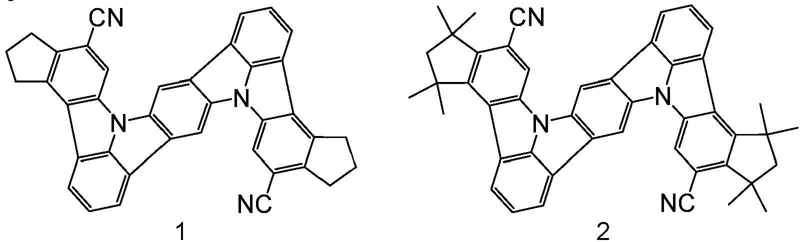

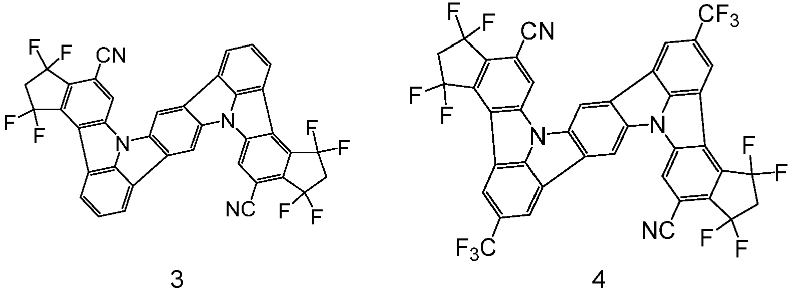

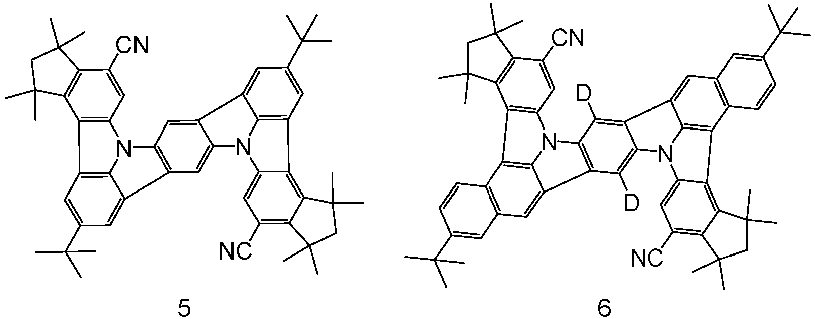

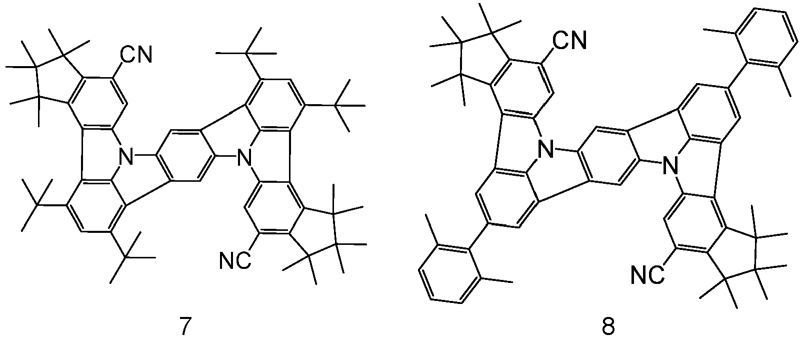

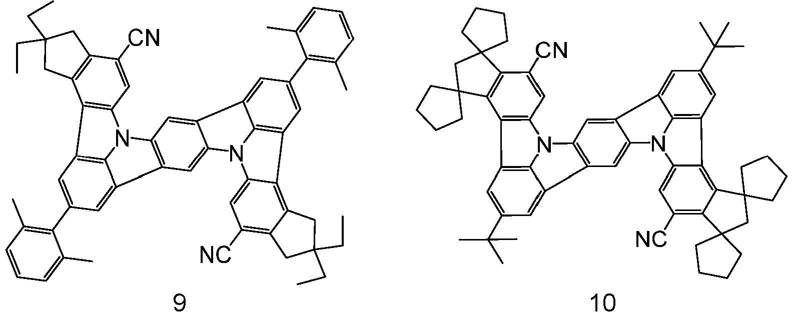

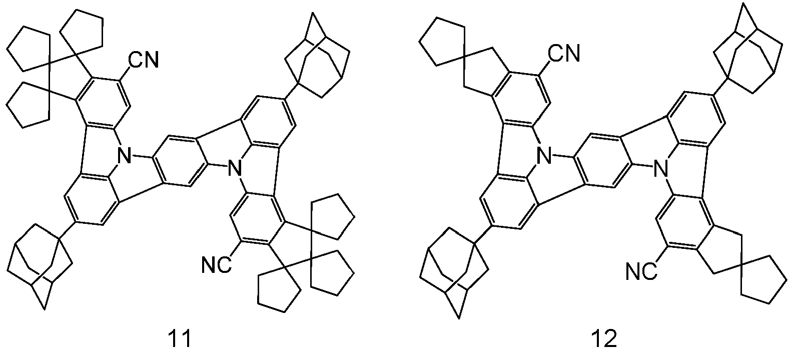

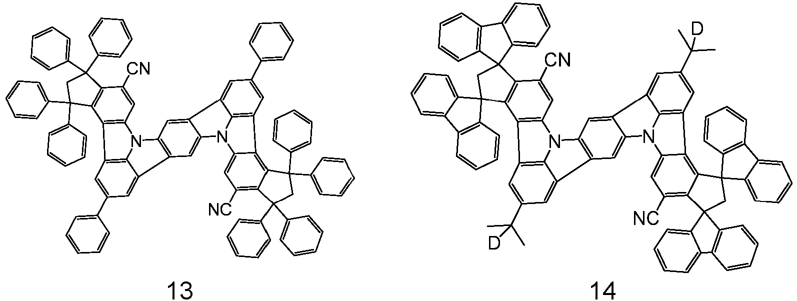

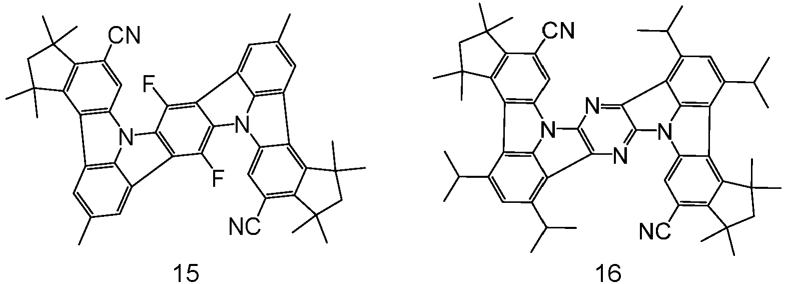

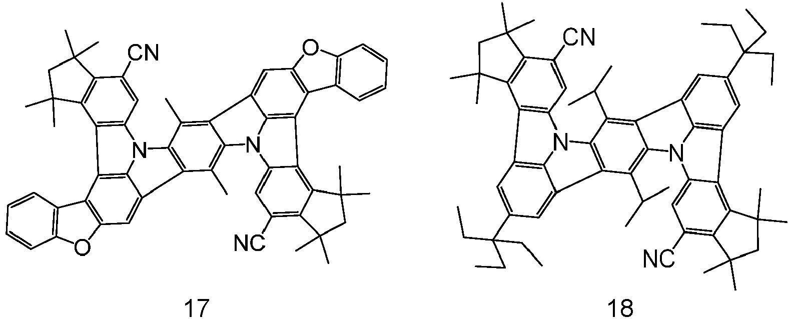

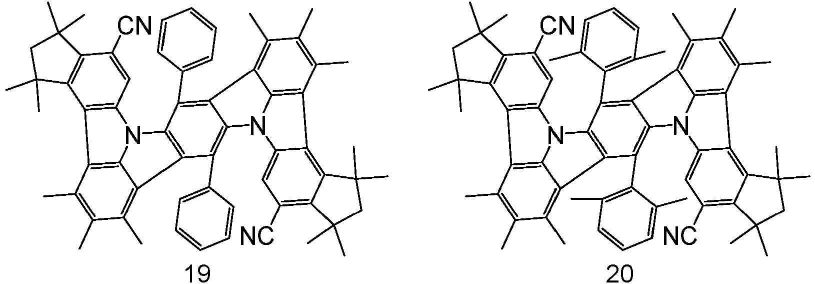

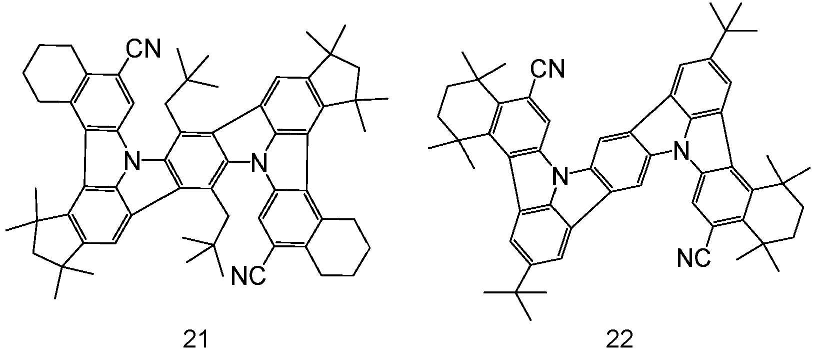

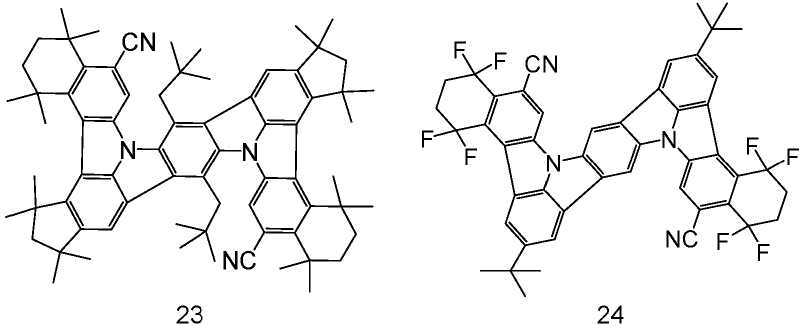

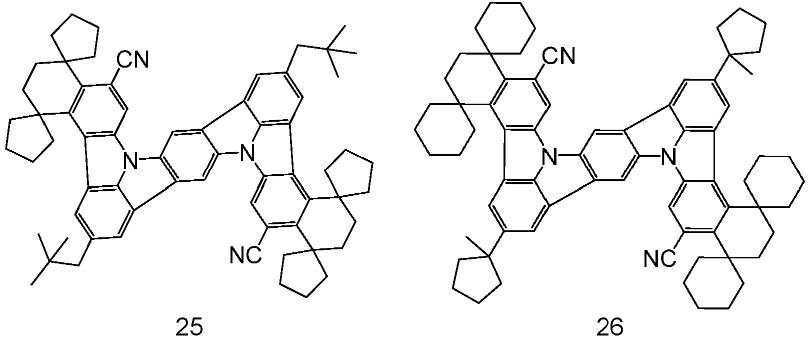

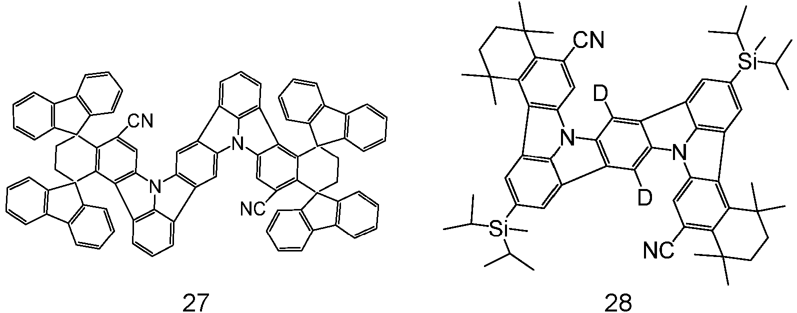

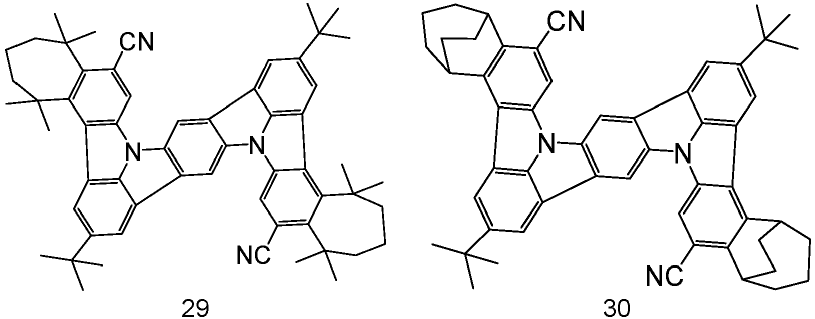

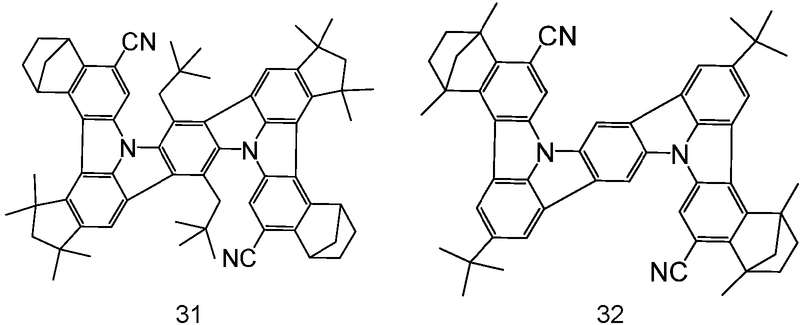

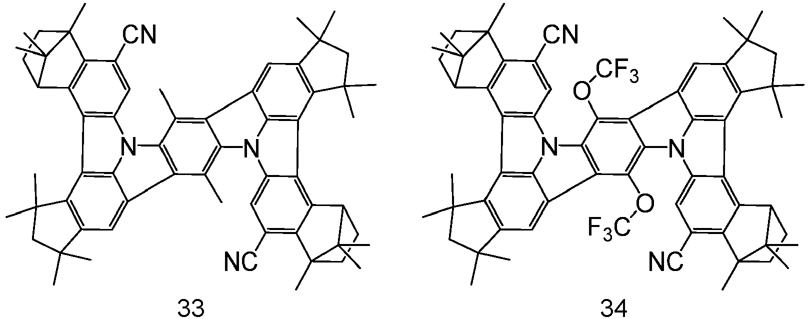

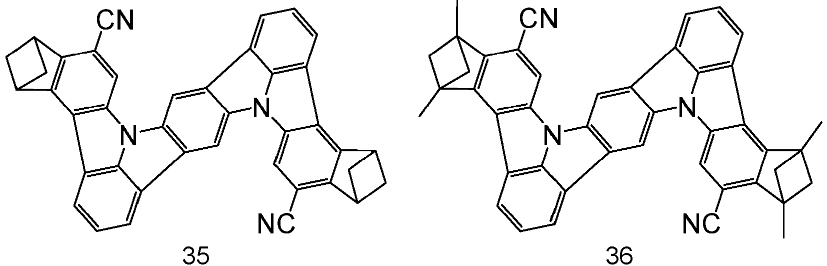

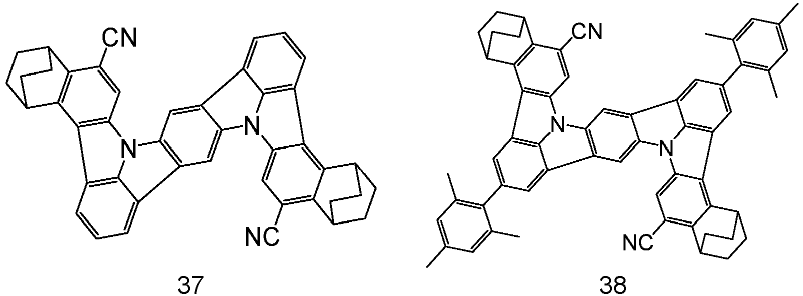

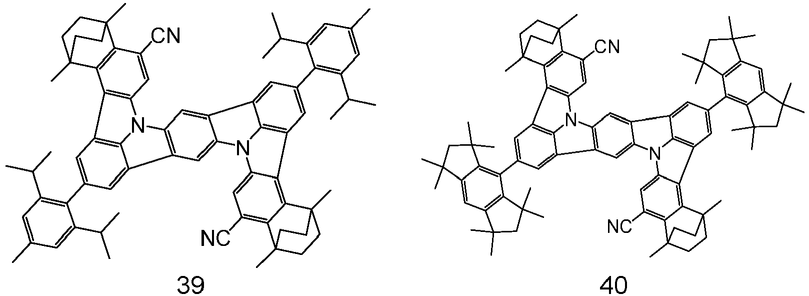

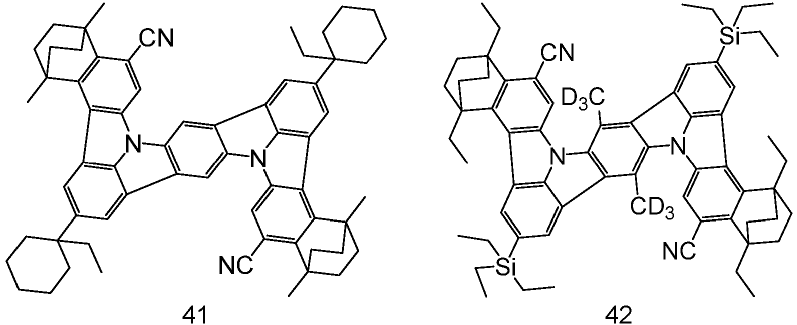

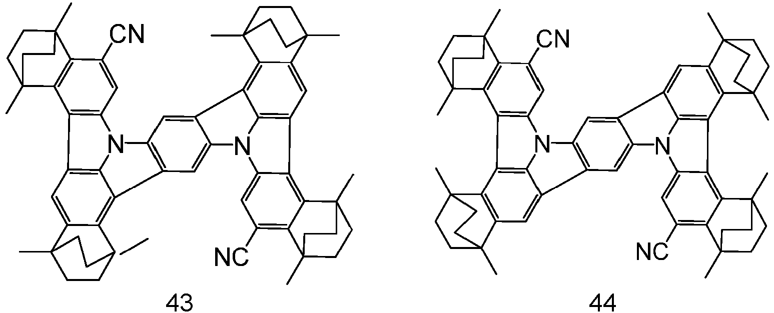

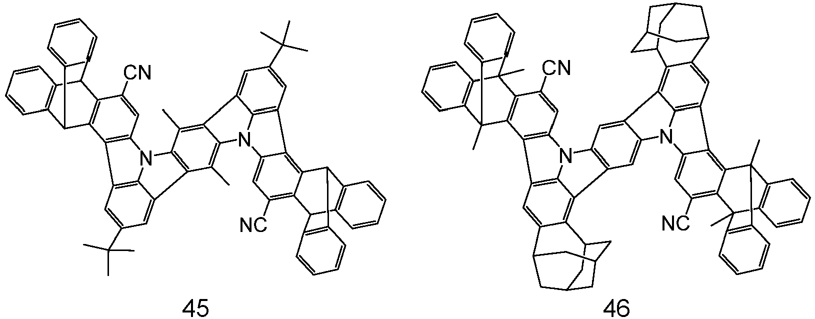

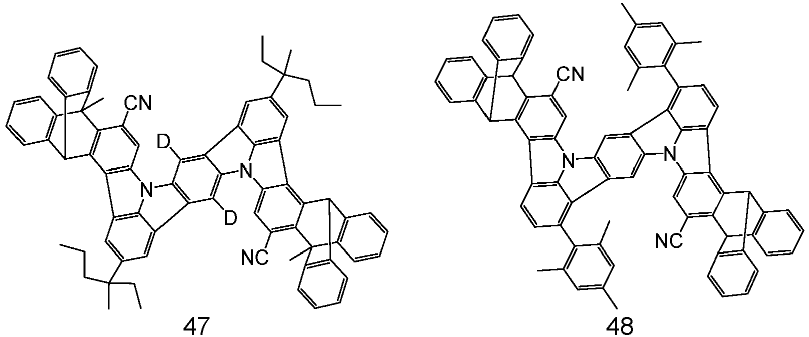

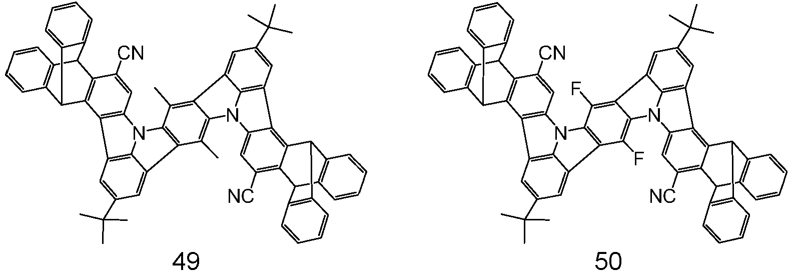

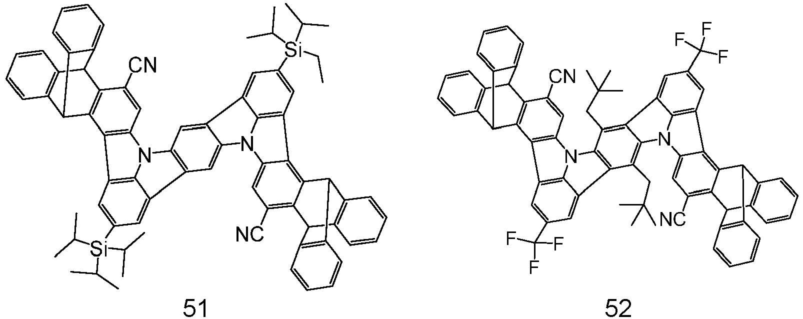

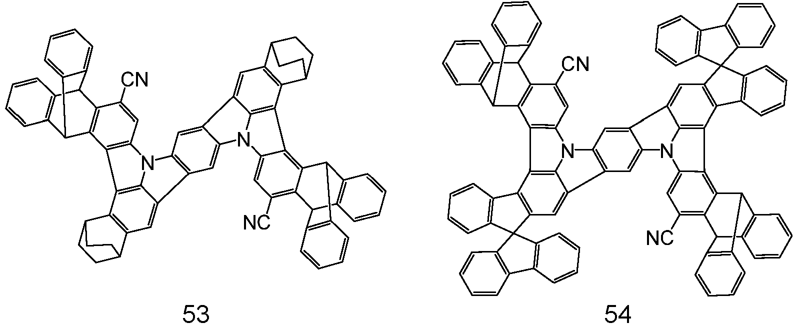

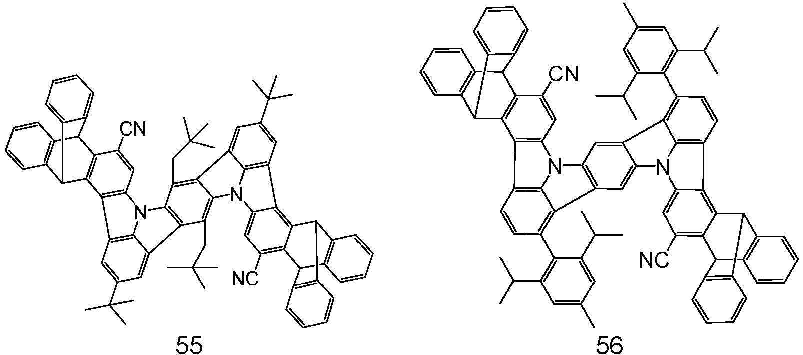

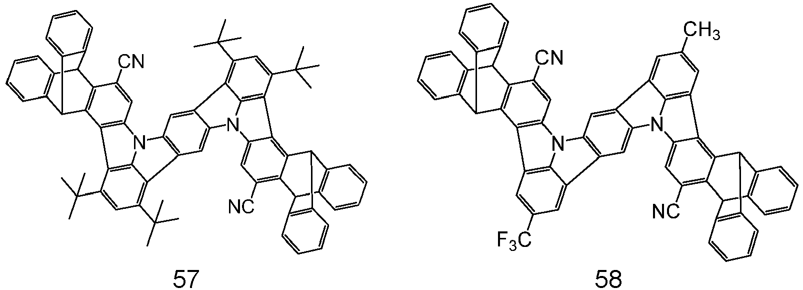

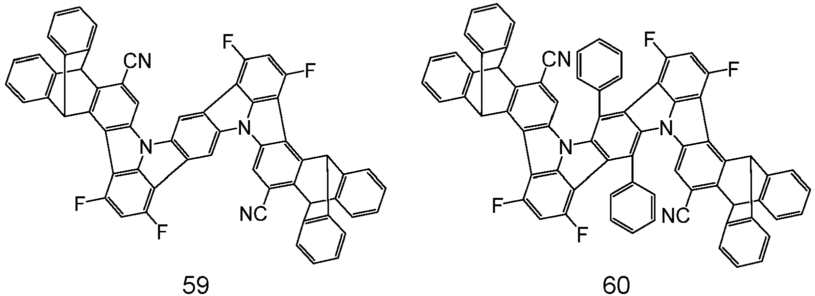

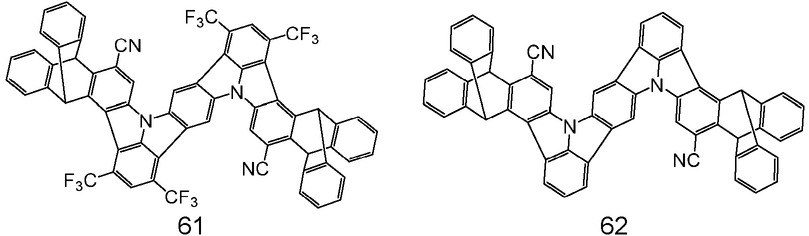

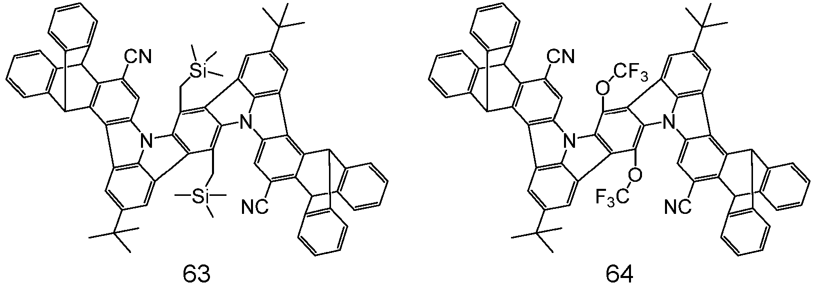

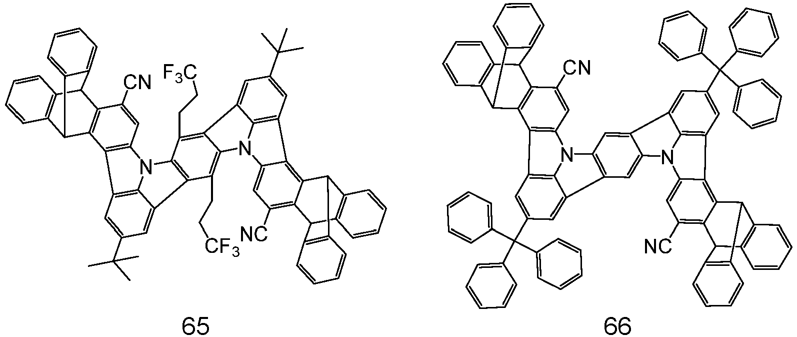

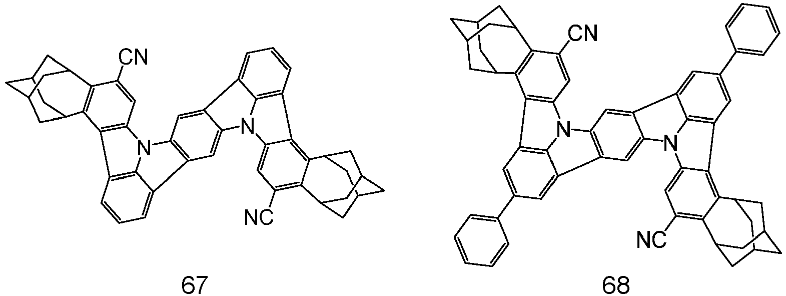

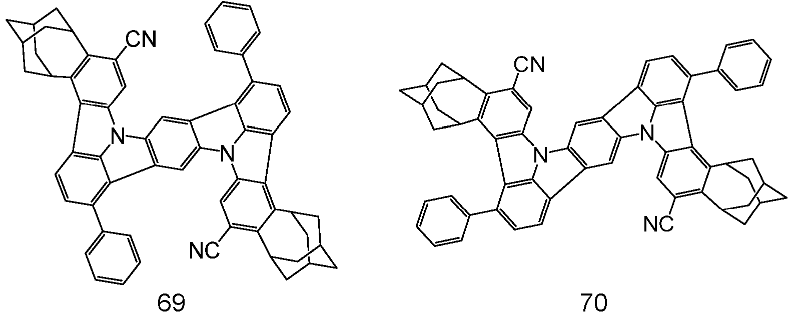

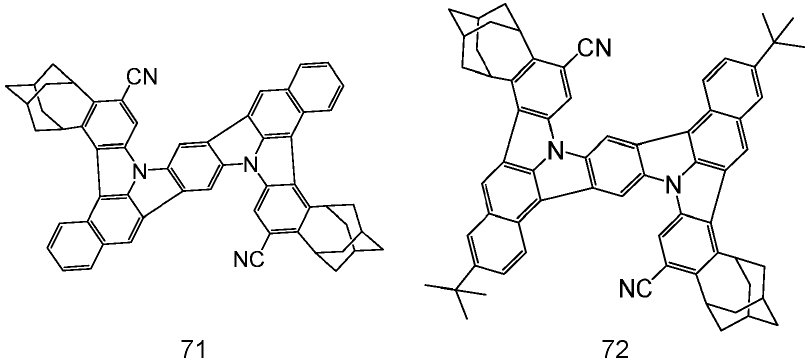

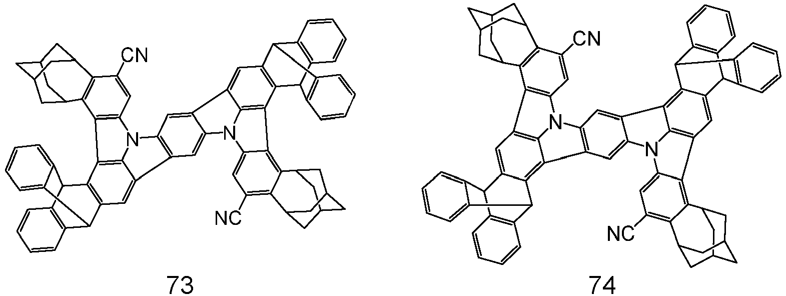

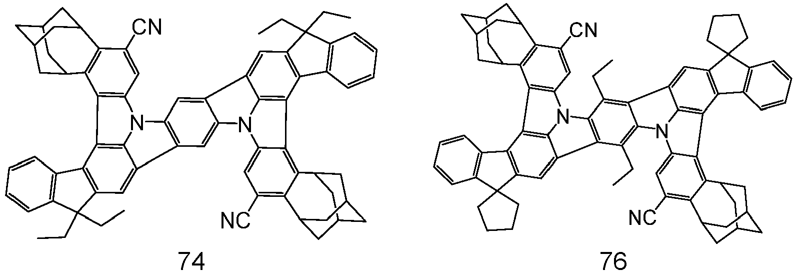

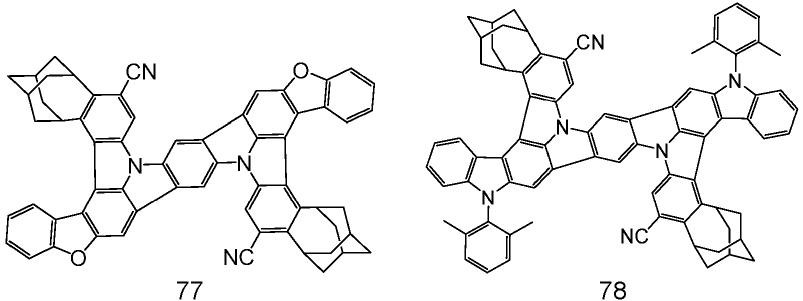

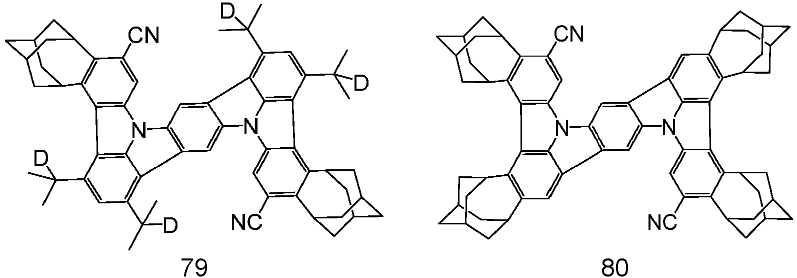

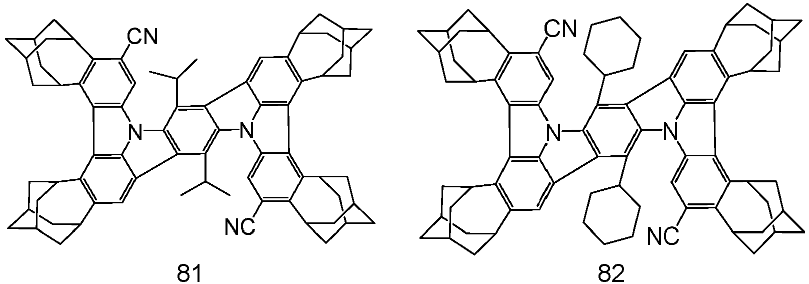

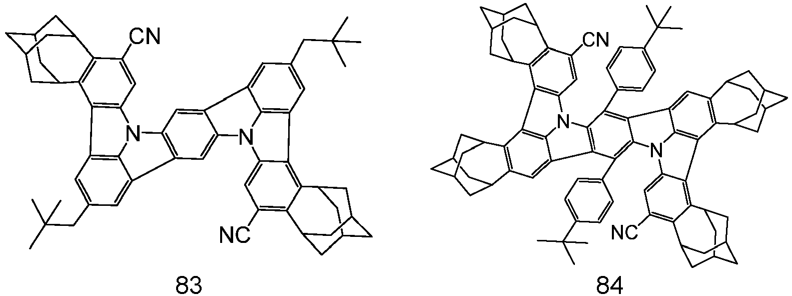

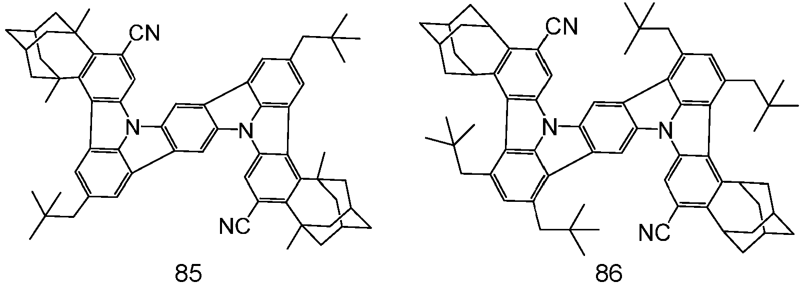

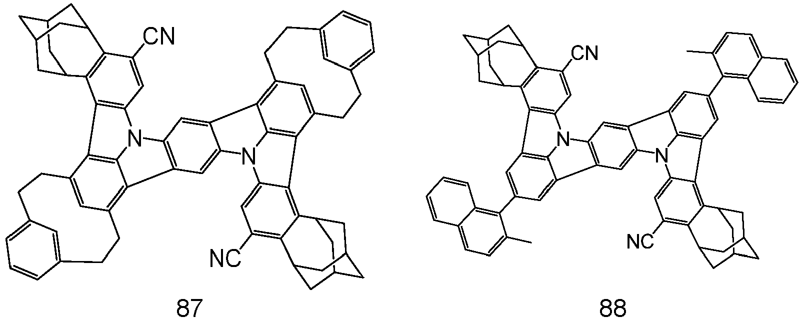

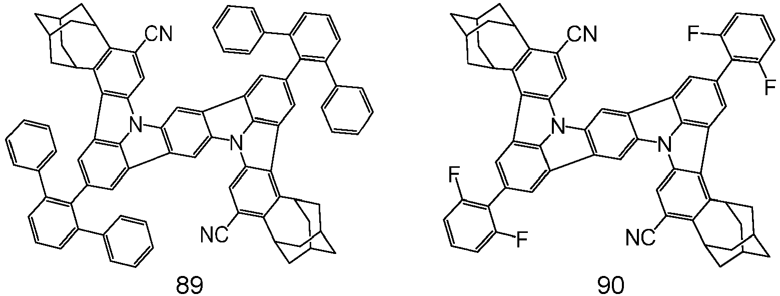

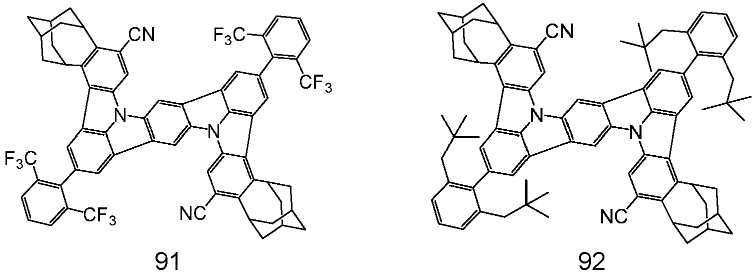

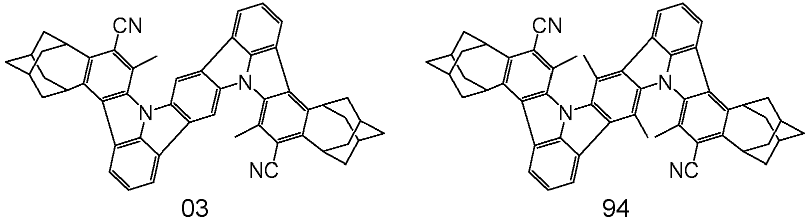

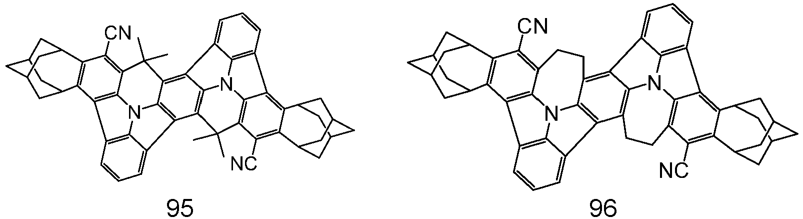

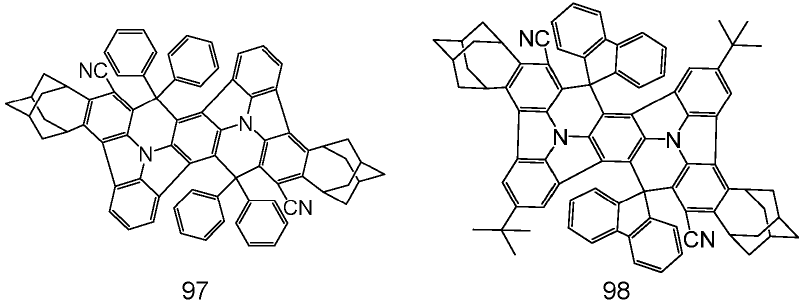

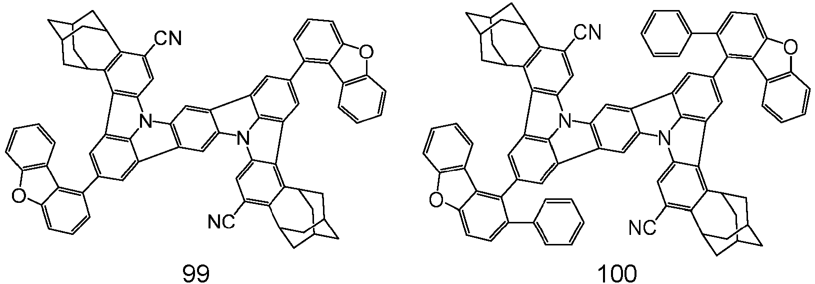

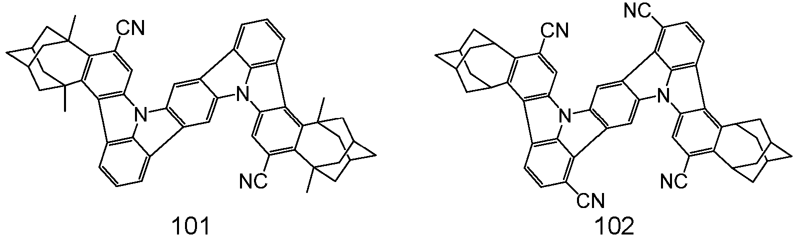

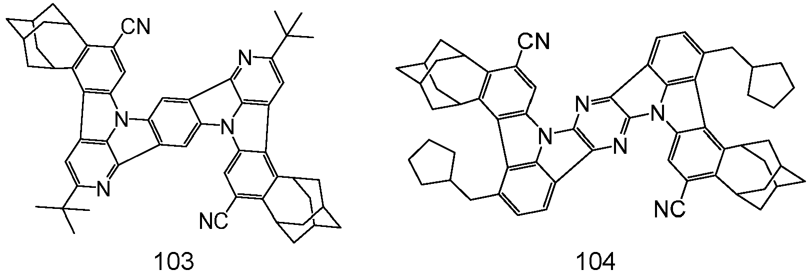

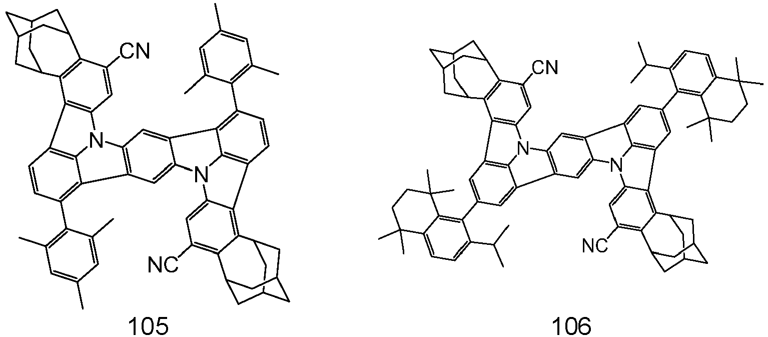

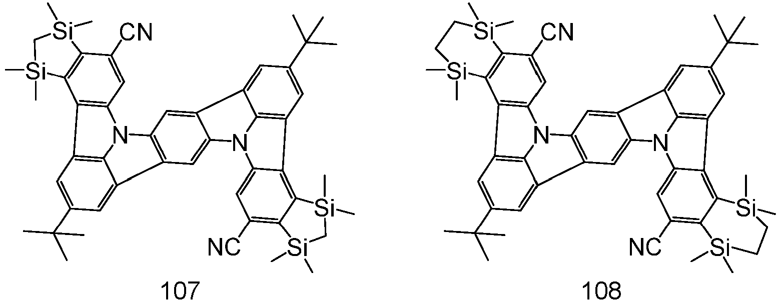

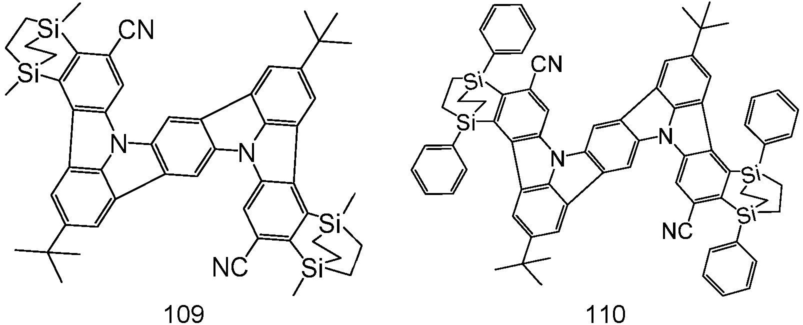

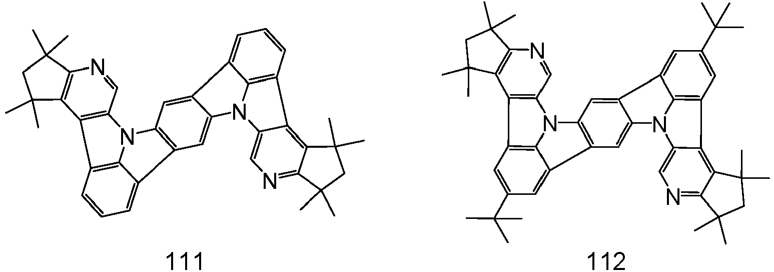

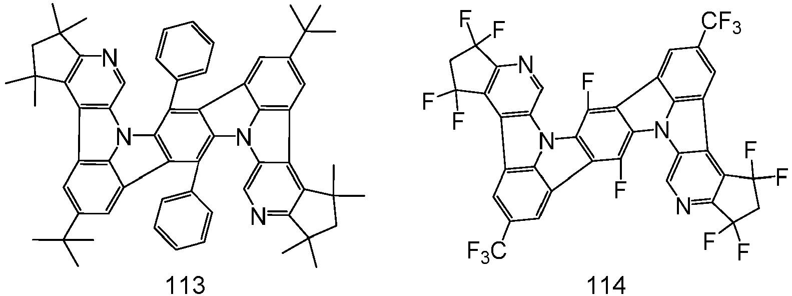

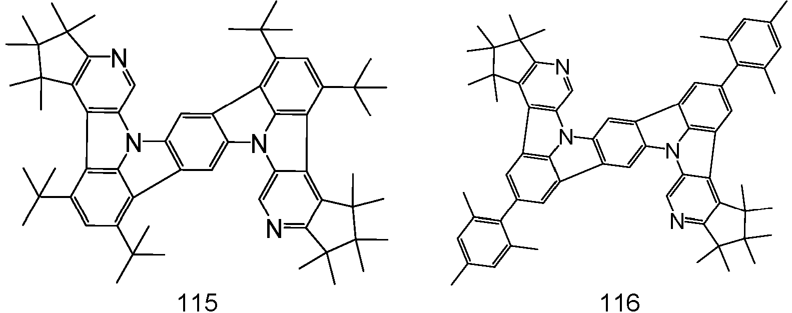

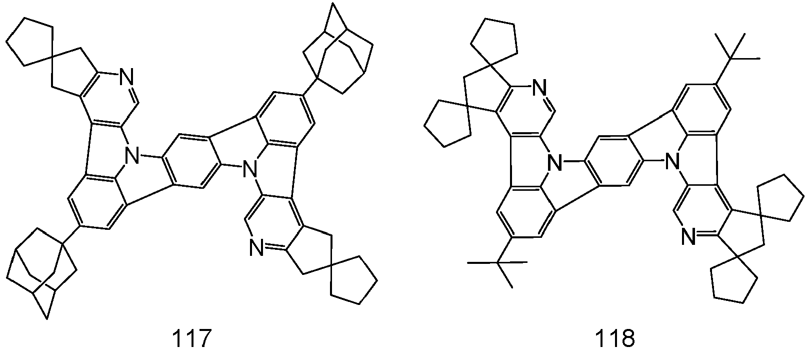

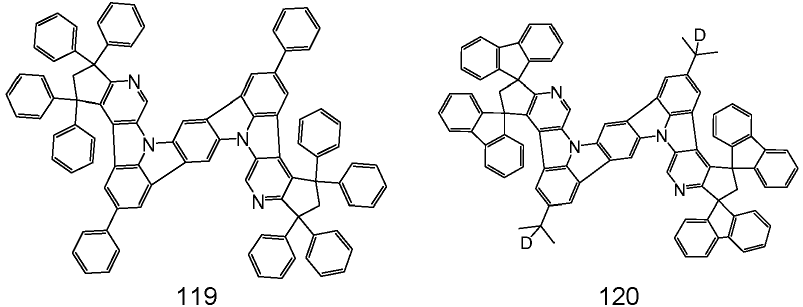

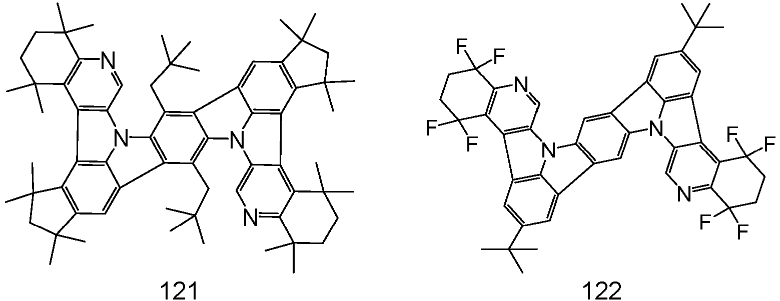

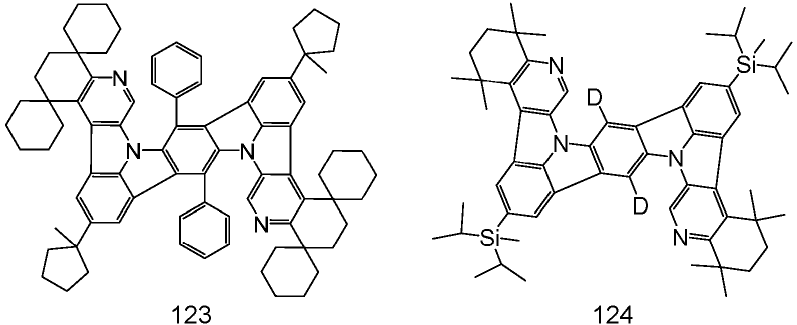

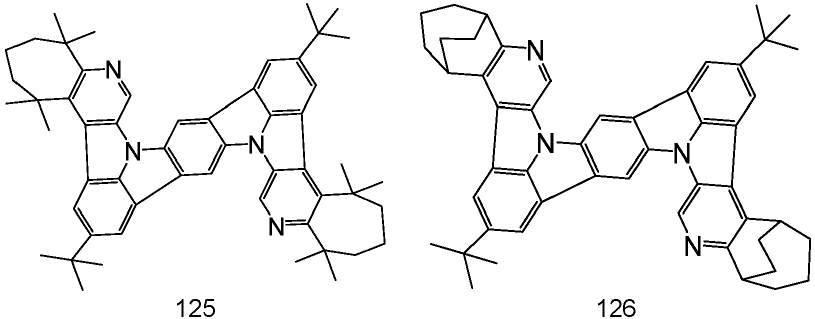

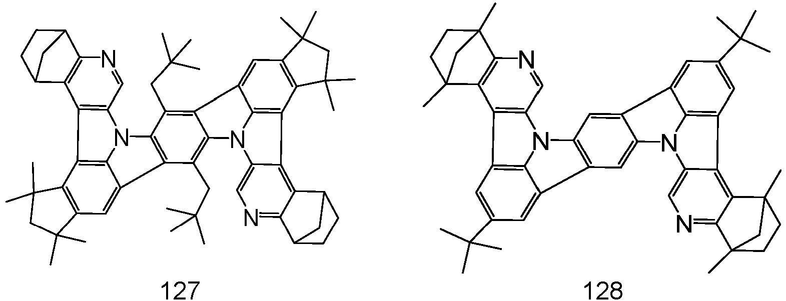

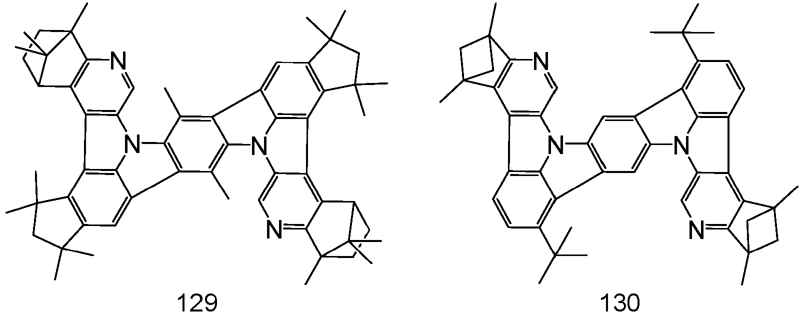

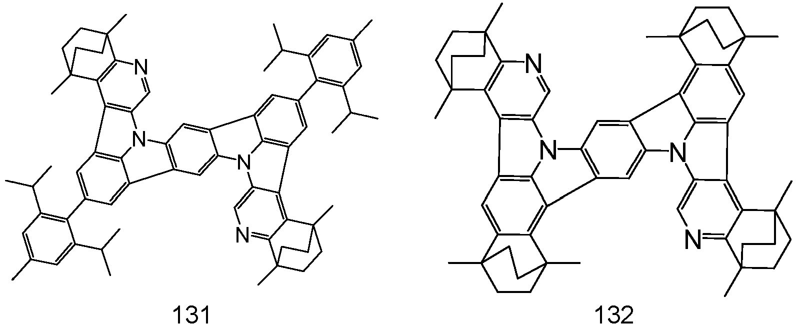

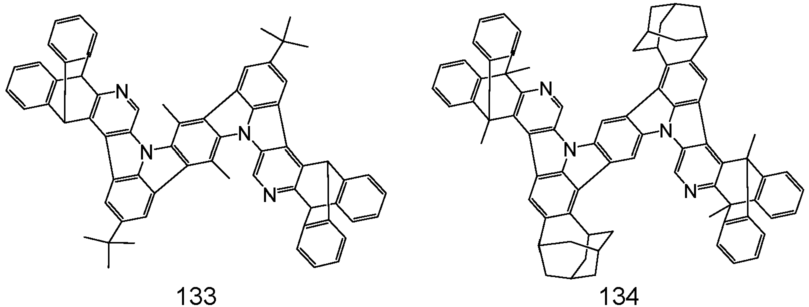

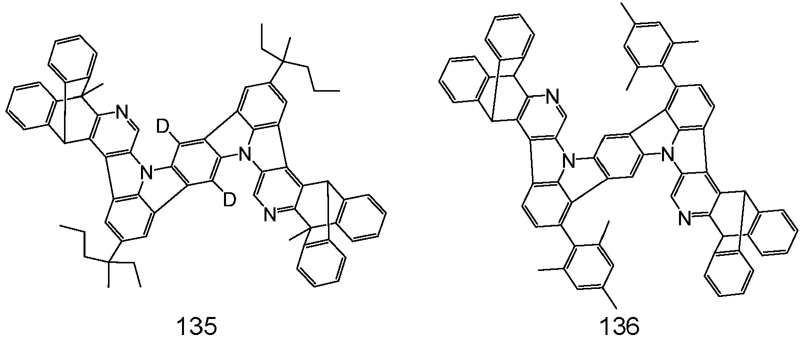

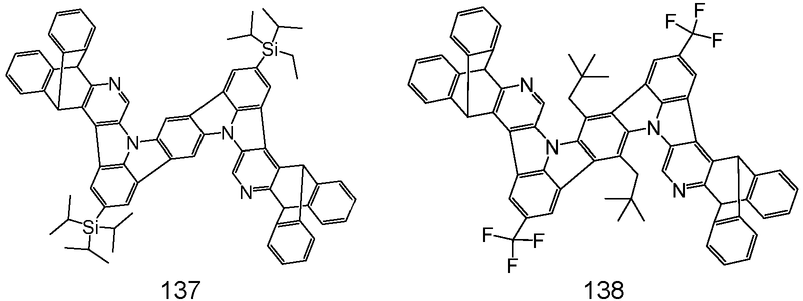

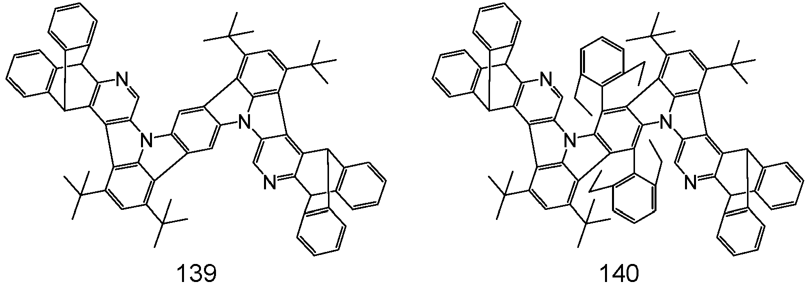

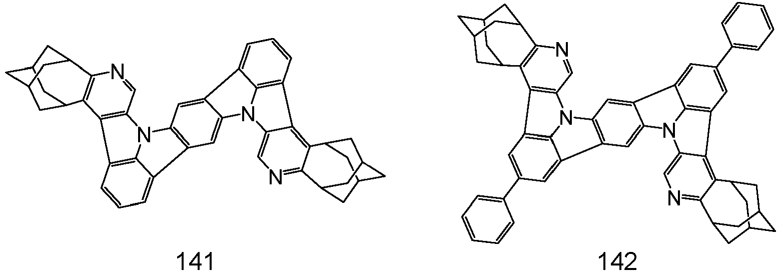

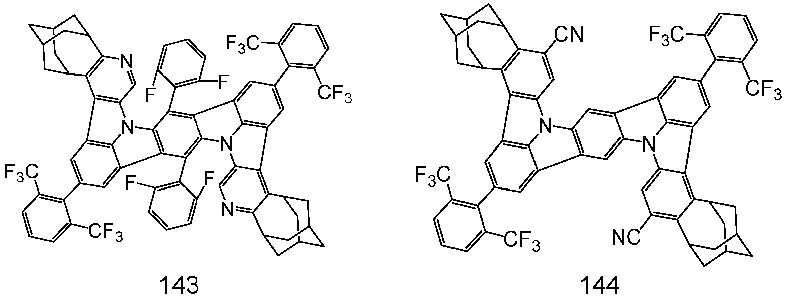

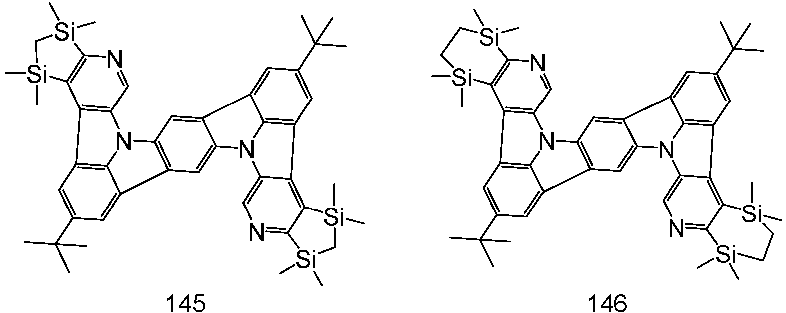

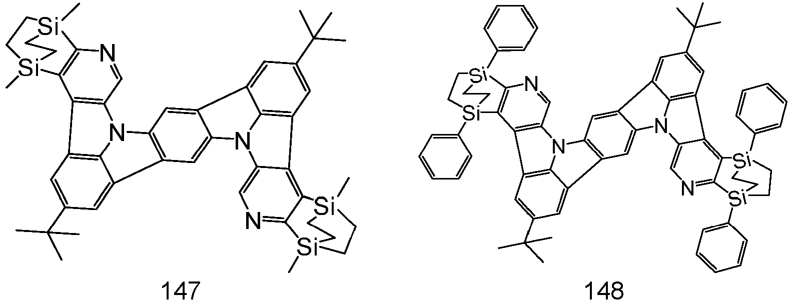

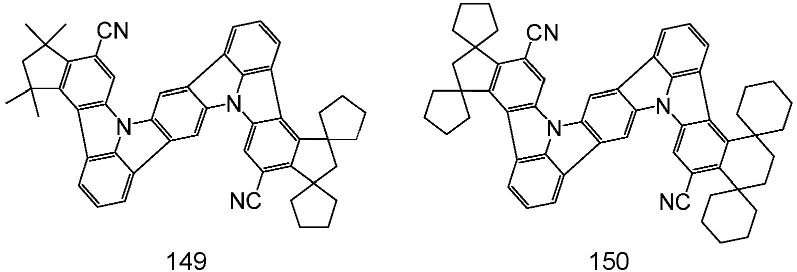

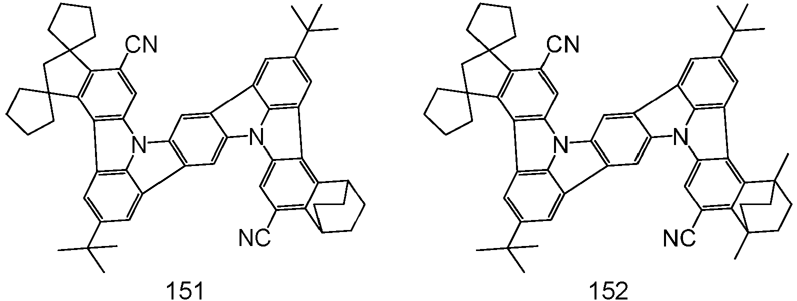

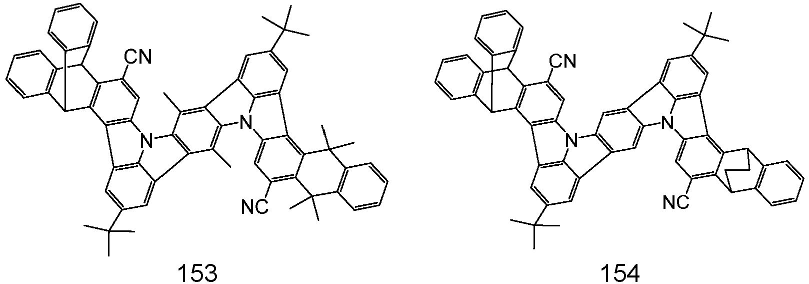

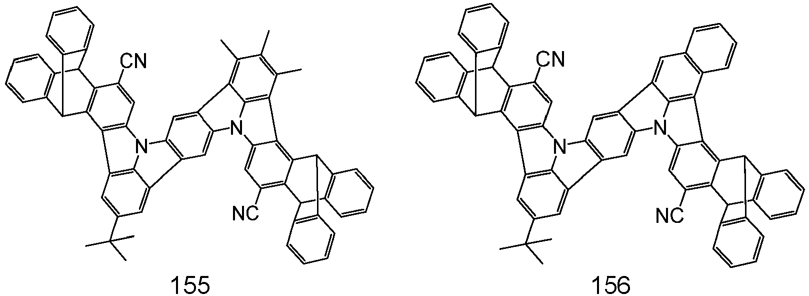

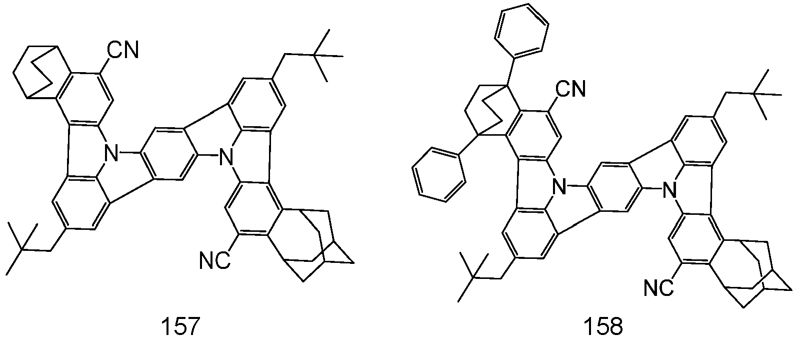

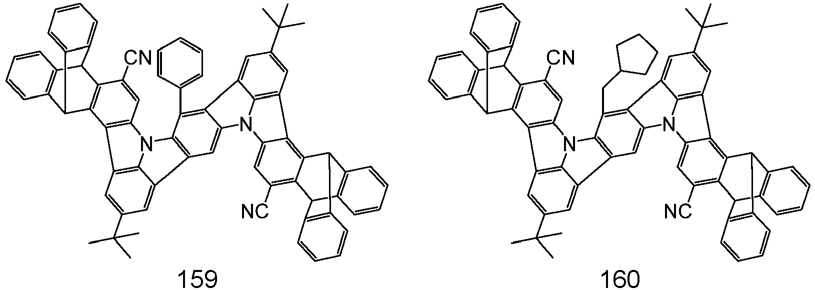

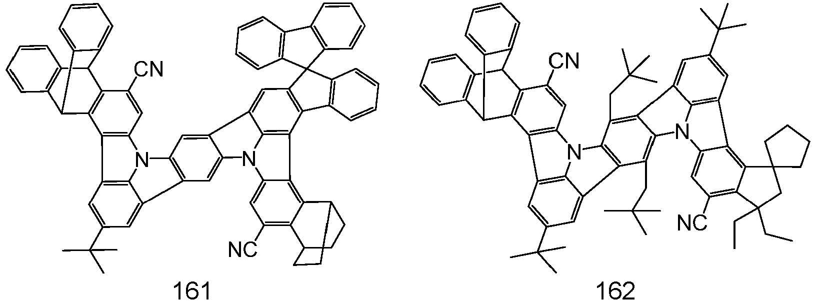

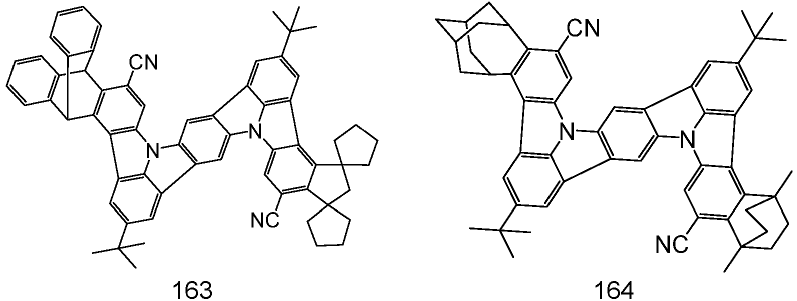

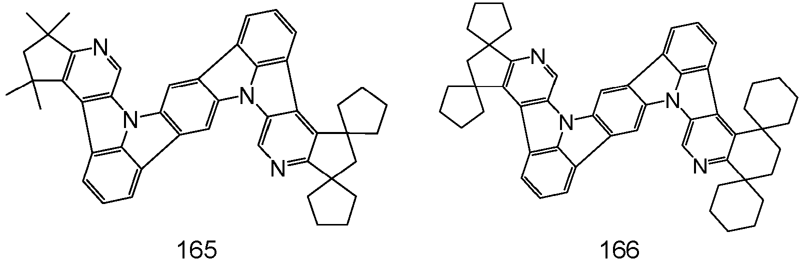

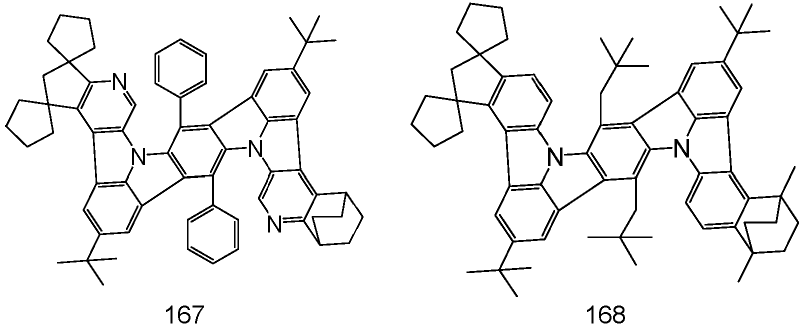

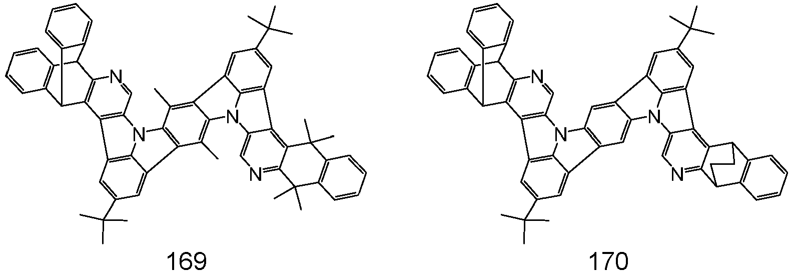

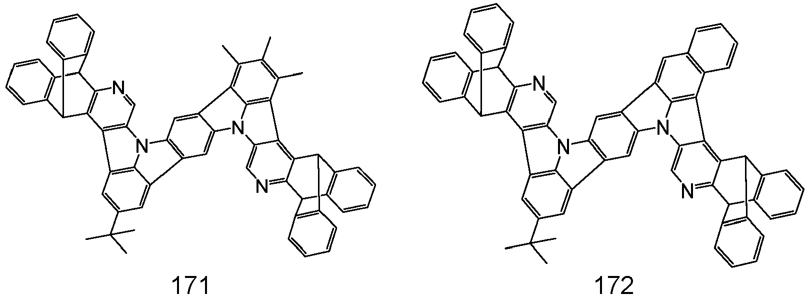

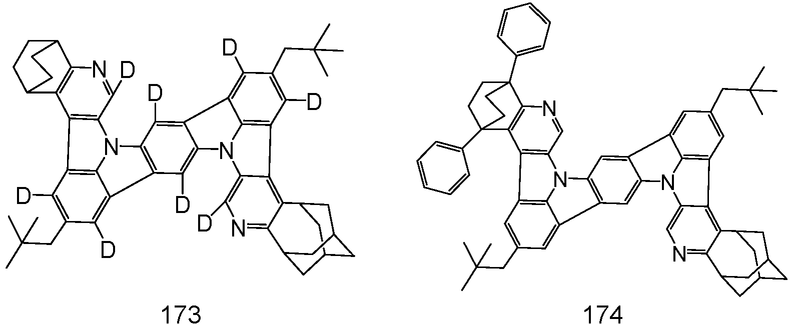

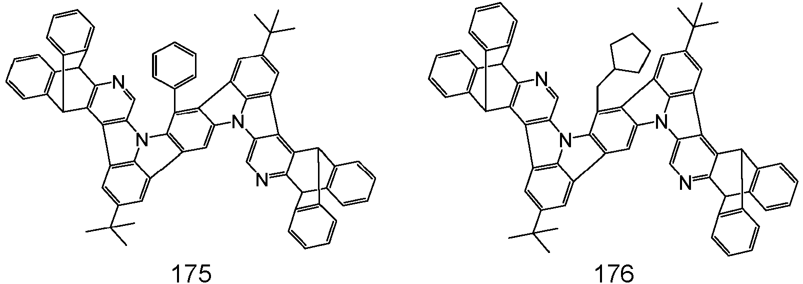

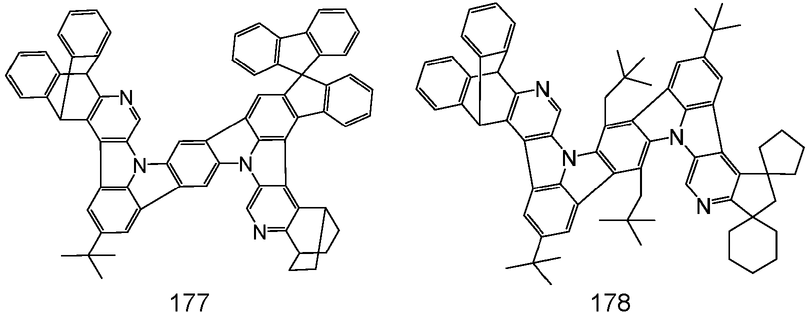

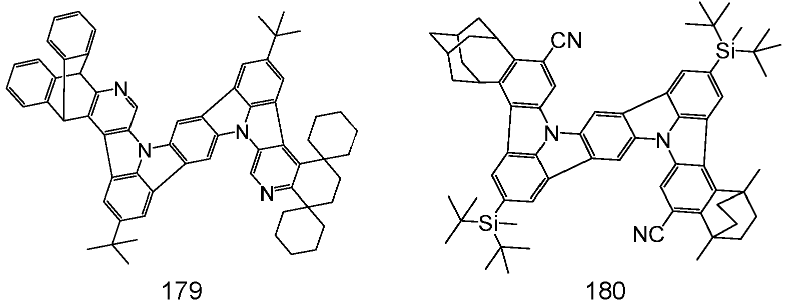

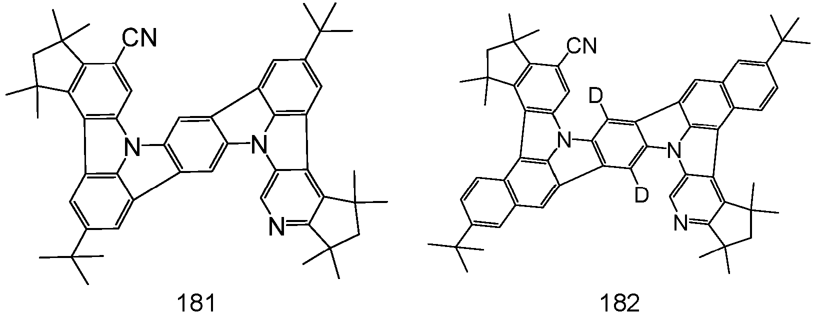

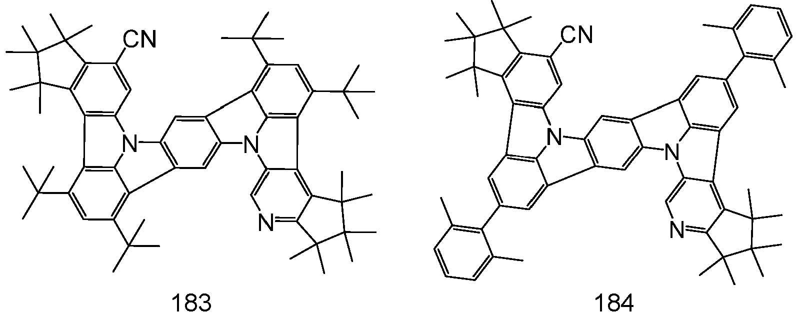

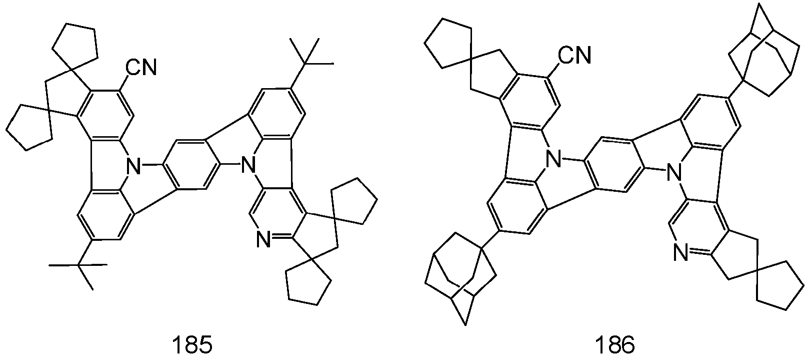

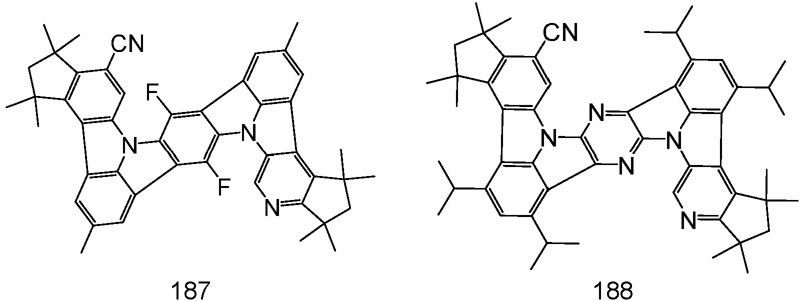

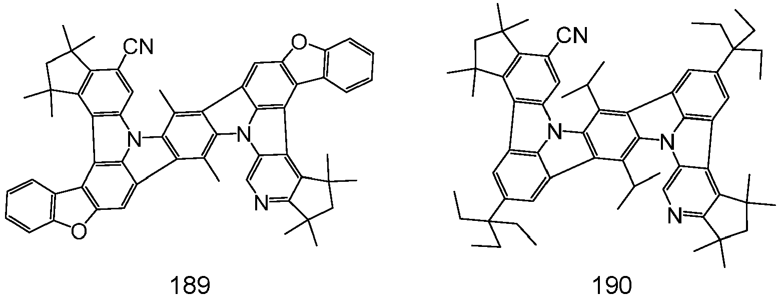

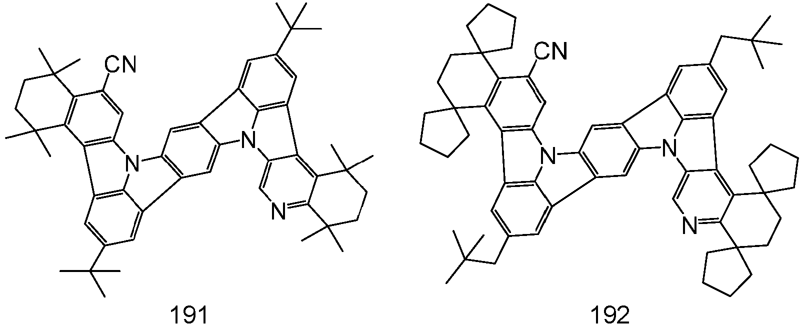

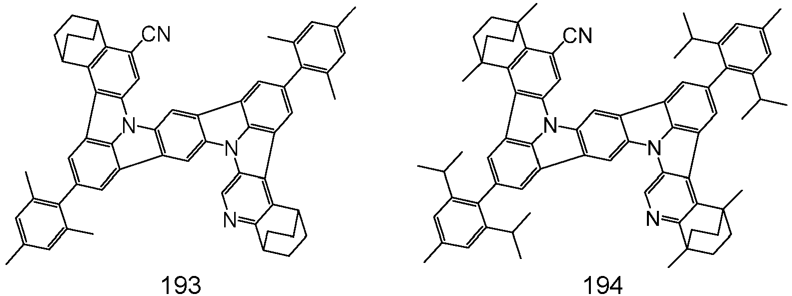

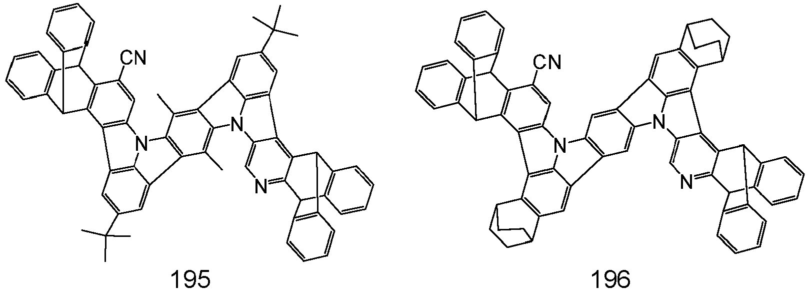

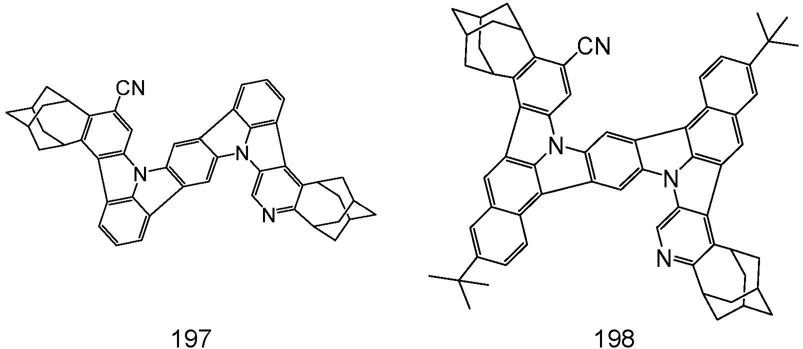

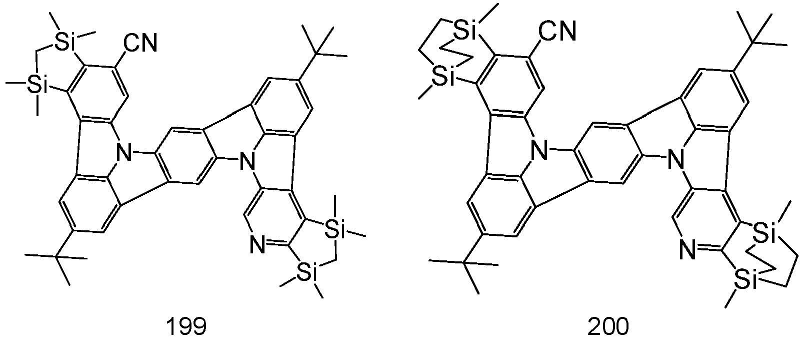

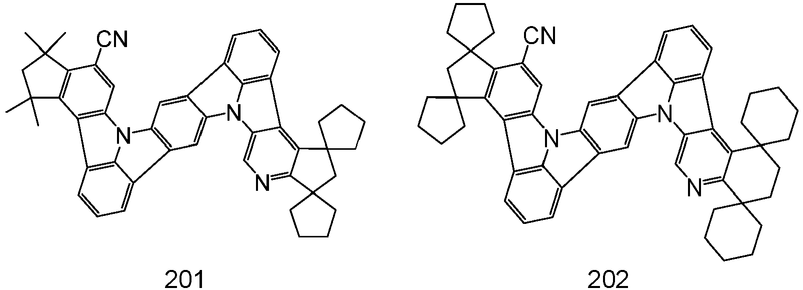

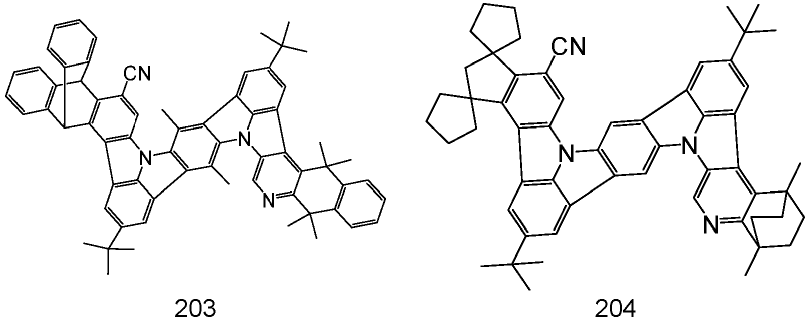

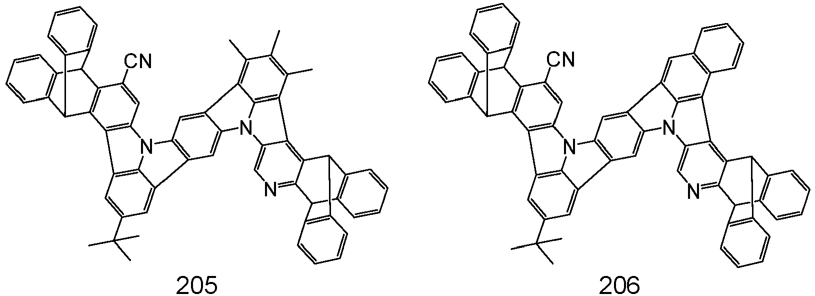

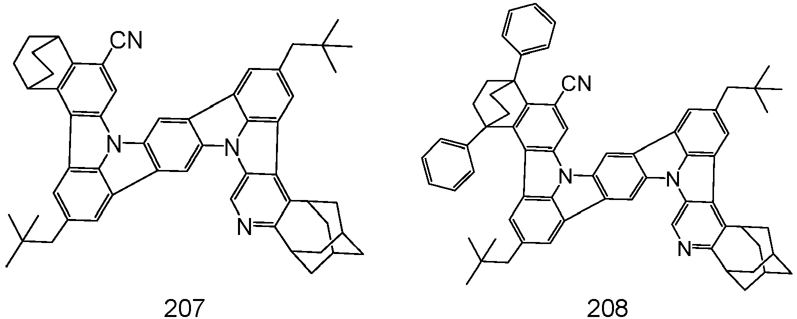

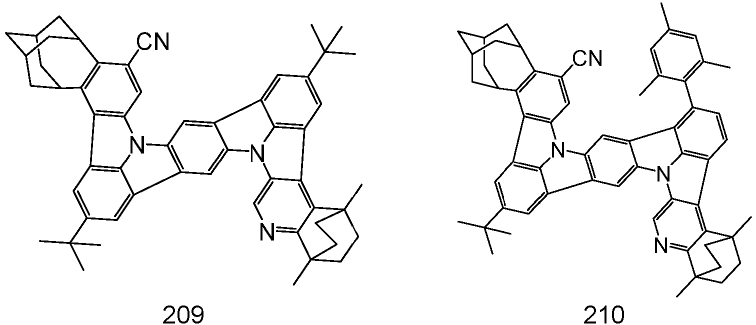

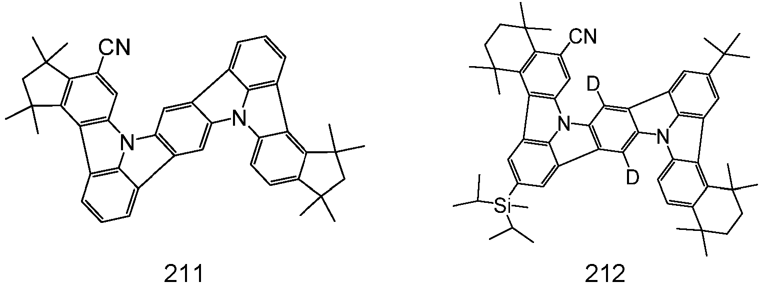

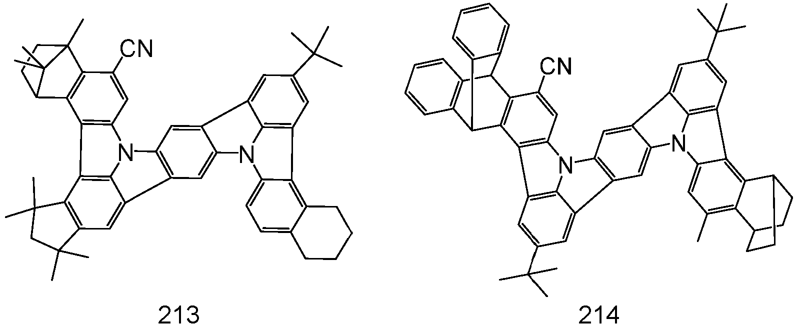

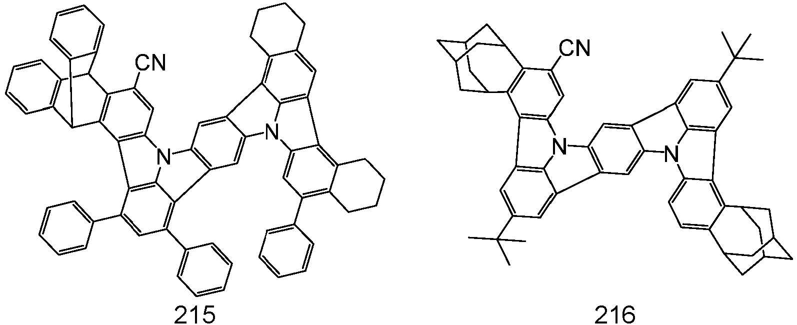

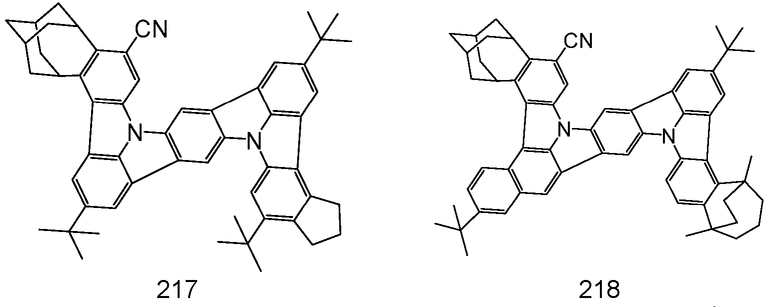

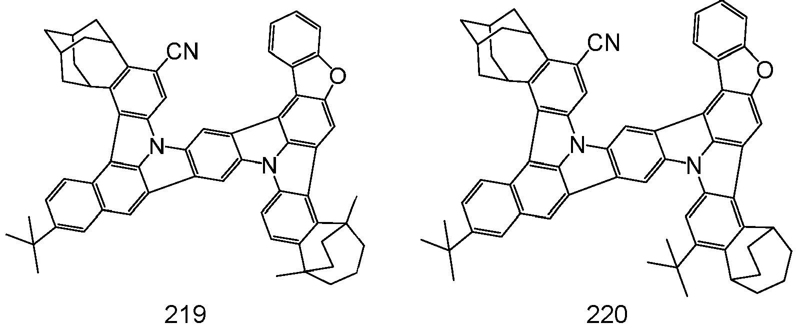

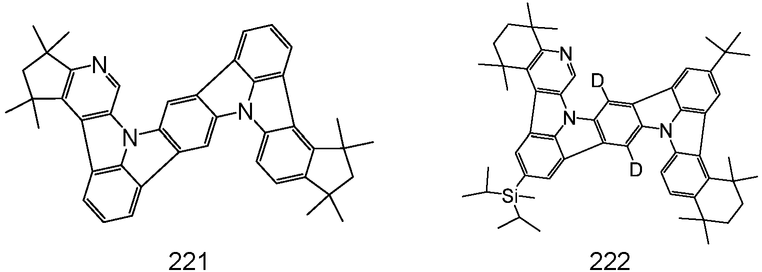

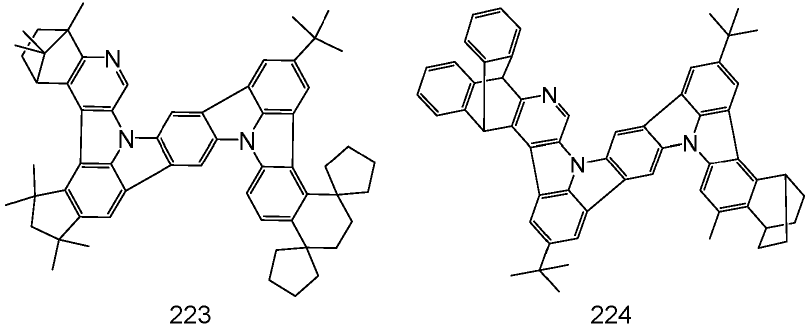

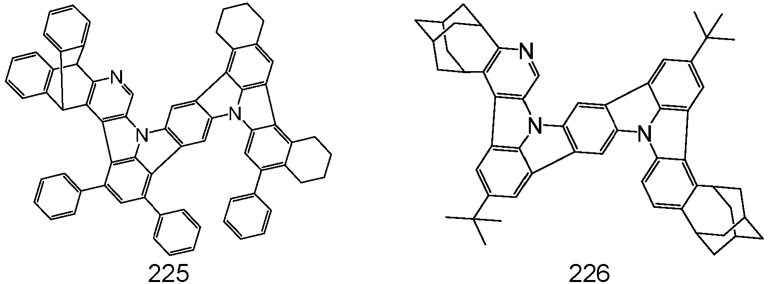

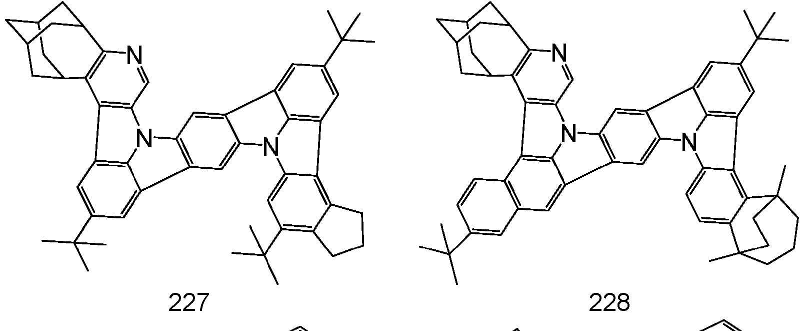

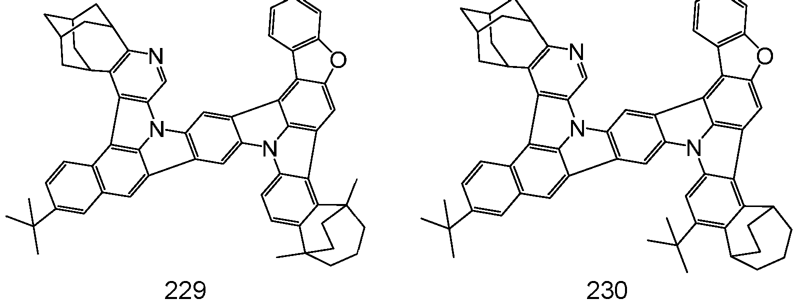

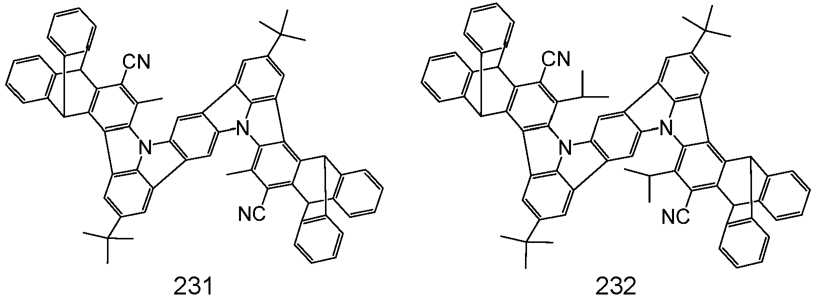

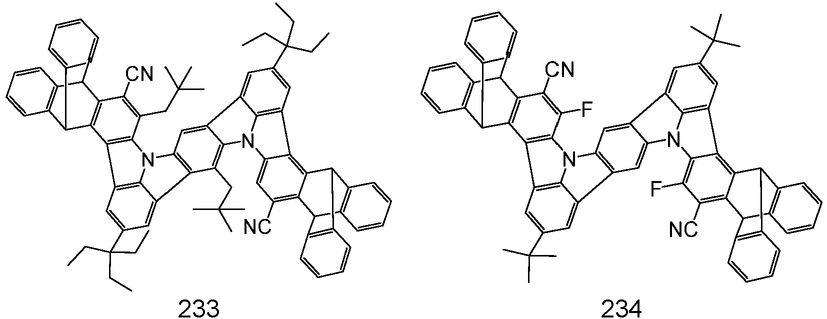

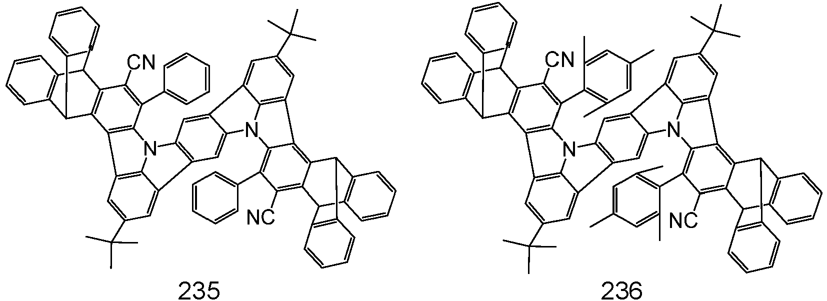

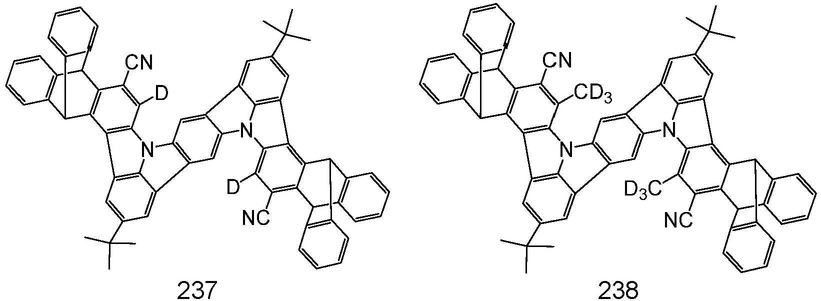

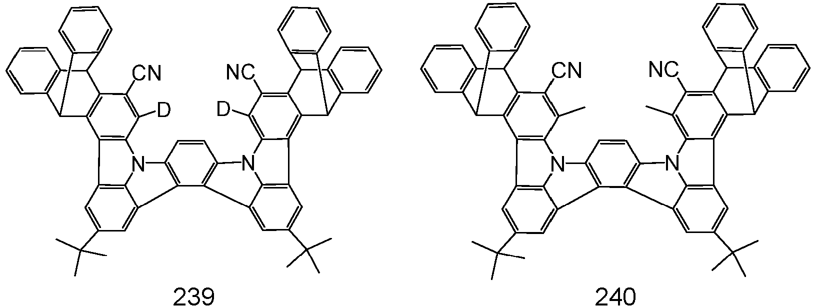

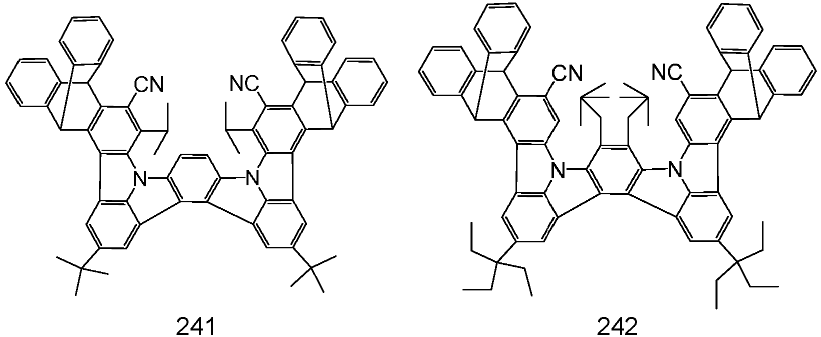

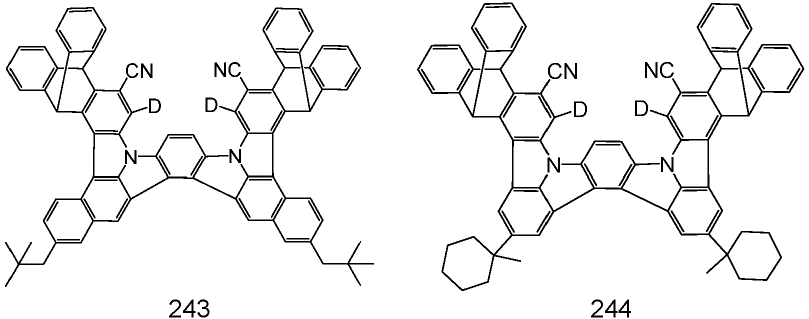

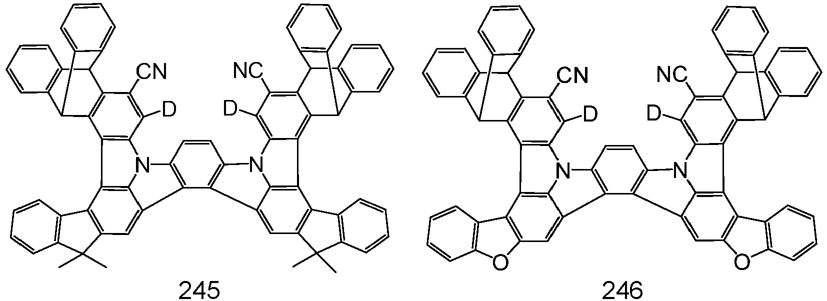

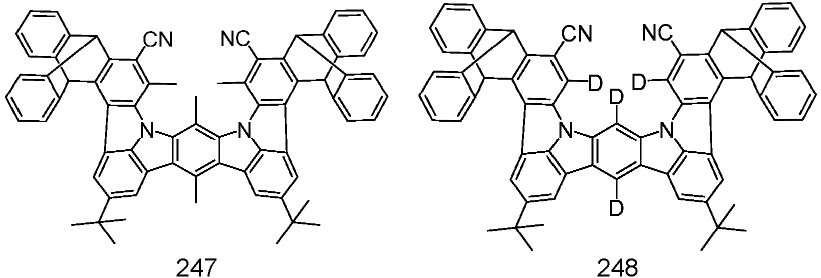

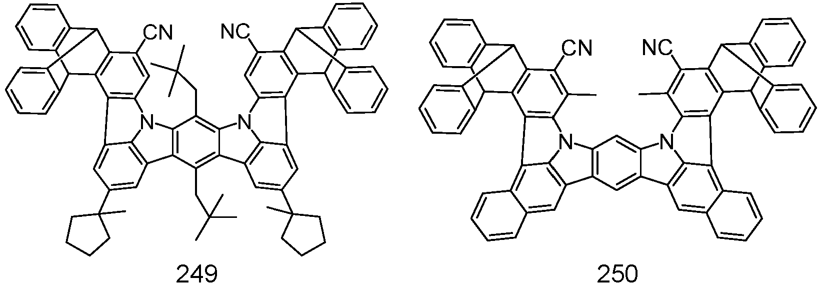

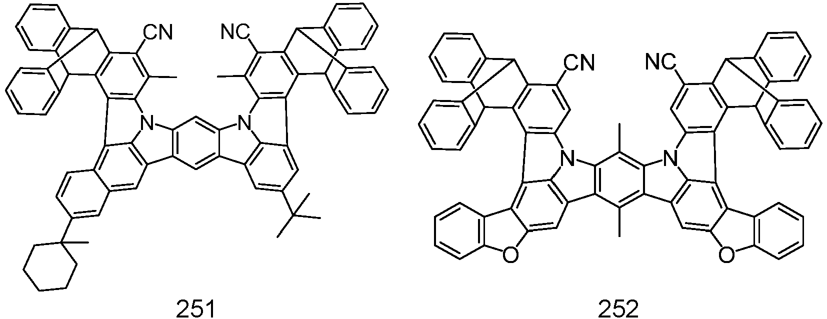

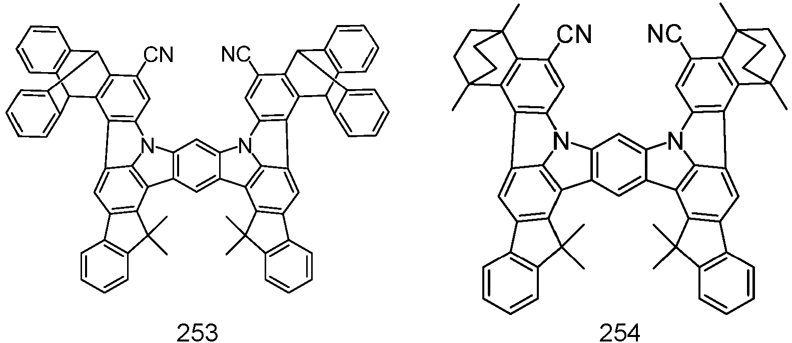

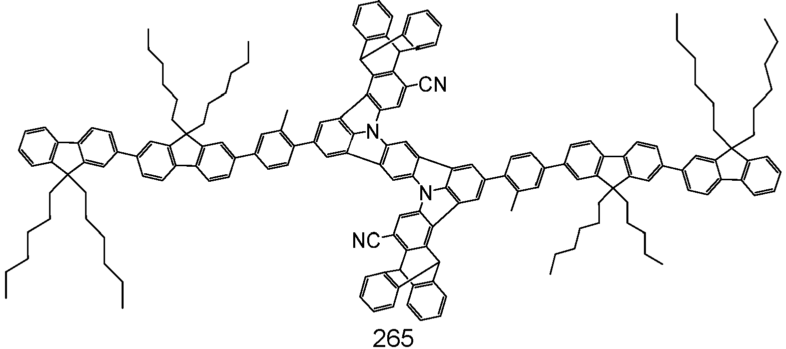

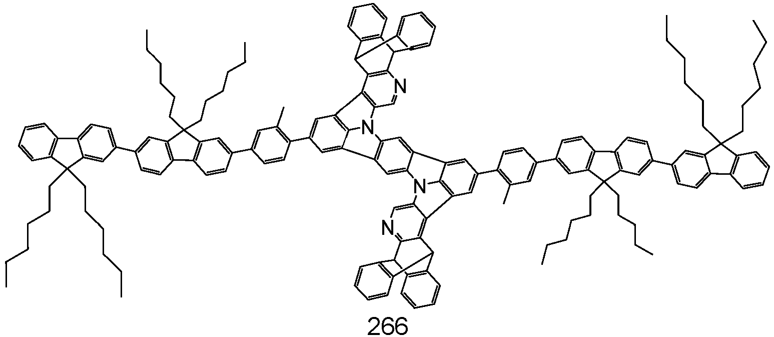

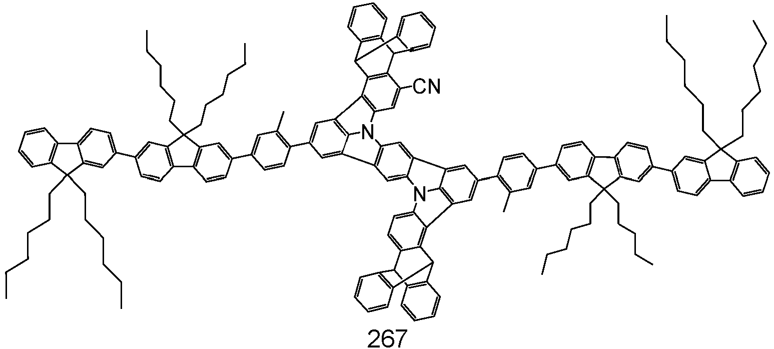

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem ein Grundgerüst mit einer aromatischen Aminogruppe synthetisiert wird und mindestens ein aromatischer oder heteroaromatischer Rest eingeführt wird, vorzugsweise mittels einer nukleophilen aromatischen Substitutionsreaktion oder einer Kupplungsreaktion.

Geeignete Verbindungen, umfassend ein Grundgerüst mit einer aromatischen Aminogruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Synthese der erfindungsgemäßen Verbindungen kann unter anderem gemäß den nachfolgenden Schemata 1, 2 und/oder 3 erfolgen.

Beispielsweise kann eine Synthese in drei Schritten durchgeführt werden, wie dies in Schema 1 dargestellt ist. Zunächst kann aus den bis Halogen (Br, I) bzw. Triflat funktionalisierten Bausteinen BS in einer Palladium-Phosphin-katalysierten C-N-Kupplung von Hartwig-Buchwald-Typ durch Umsetzung mit einem primaren Arylamin ein sekundäres o-Chlor-arylamin dargestellt werden (Stufe 1). Beispielhafte Bausteine BS sind in dem Beispielteil enthalten, auf welchen hier in allgemeiner Form Bezug genommen wird. Das Produkt der ersten Stufe kann in Stufe 2 in einer Palladium-Phosphin-katalysierten C-C-Kupplung zum Carbazol cyclisiert werden. Die so erhaltenen Carbazole können dann in einer S_{N}2_{Ar}-Reaktion mit 1,4-Dichlor-2,5-difluoraromaten/heteroaromaten umgesetzt werden (s. Stufe 3, Schema 1), das Kupplungsprodukt kann in Situ, durch Zugabe eine Pd-Quelle und eines Phosphins, in einer Palladium-Phosphin-katalysierten C-C-Kupplung zu den erfindungsgemäßen Verbindungen cyclisiert werden (s. Stufe 3, Schritt 2). Werden in Stufe 3 zwei verschiedene Carbazole - als Gemisch oder durch sequenzielle Zugabe - eingesetzt können gemischt funktionalisierte erfindungsgemäßen Verbindungen erhalten werden.

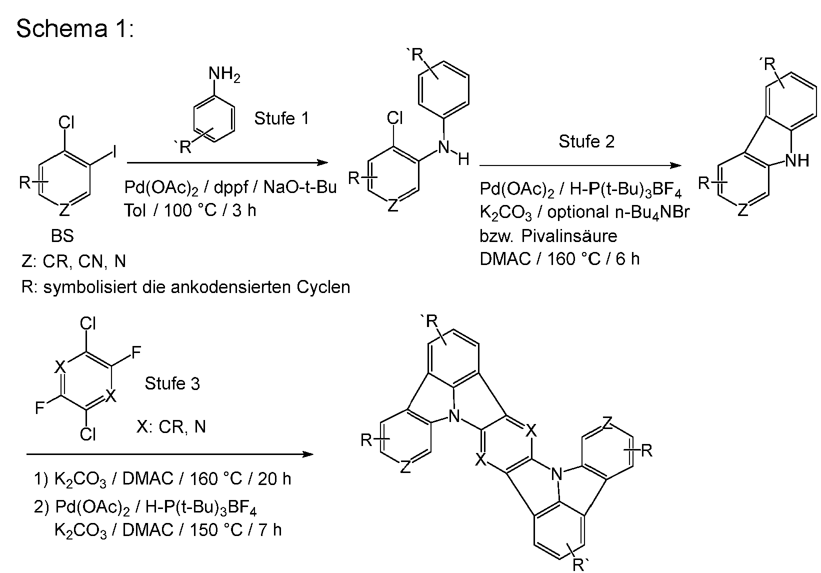

Alternativ können die erfindungsgemäßen Verbindungen ausgehend von den Carbazolen mit einem Substituenten R' in o-Stellung zu Z (Synthese siehe Experimentalteil) in vier Schritten dargestellt werden (s. Schema 2).

Zunächst kann eine regioselektive NBS-Bromierung von Carbazolen mit einem Substituenten R' in o-Stellung zu N (Synthese siehe Experimentalteil) in o-Position zum Carbazol-N-Atom erfolgen (Stufe 1). Die Bromfunktion kann in einer Palladium-Phosphin-katalysierten Borylierung mit B₂Pin₂ zum B-Pin-Ester umgesetzt werden (Stufe 2). Anschließend wird in einer Palladium-Phosphin-katalysierten C-C-Kupplung vom Suzuki-Typ die zentrale Ringeinheit gekuppelt (Stufe 3). Abschließend wird in einer Palladium-Phosphin-katalysierten C-C-Kupplung zu den erfindungsgemäßen Verbindungen cyclisiert (Stufe 4).

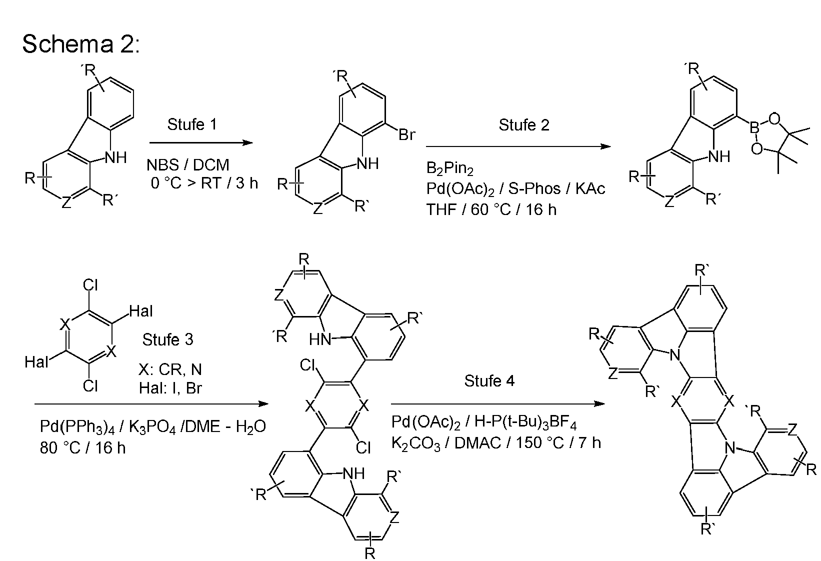

Alternativ können die erfindungsgemäßen Verbindungen ausgehend von den Bausteinen BS in drei Schritten dargestellt werden (s. Schema 3).

Zunächst kann aus den lod- bzw. Amino-funktionalisierten Bausteinen BS (Synthese siehe Experimentalteil) in einer Palladium-Phosphin-katalysierten C-N-Kupplung von Hartwig-Buchwald-Typ durch Umsetzung mit einem primaren o-Chlor-Arylamin bzw. o-Chlor-Brom/Iod-Aromaten ein sekundäres o-Bis-Chlor-arylamin dargestellt werden (Stufe 1a bzw. 1b). Dieses kann in Stufe 2 in einer Palladium-Phosphin-katalysierten C-C-Kupplung zum o-Chlor-Carbazol cyclisiert werden. Das Carbazol kann anschließend in einer Palladium-Phosphin-katalysierten C-N-Kupplung und einer anschließenden C-C-Kupplung zu den erfindungsgemäßen Verbindungen cyclisiert werden (s. Stufe 3). Die C-N- bzw. C-C-Kupplungen können nacheinander bzw. im Sinne einer Eintropfreaktion durchgeführt werden. Werden in Stufe 3 zwei verschiedene Carbazole - als Gemisch oder durch sequenzielle Zugabe - eingesetzt können gemischt funktionalisierte erfindungsgemäßen Verbindungen erhalten werden.

Diese Verfahrensweise hat den Vorteil, dass sie bezüglich der Kupplung und der Cyclisierung an den zentralen Baustein in Stufe 3 regioselektiv bezüglich des Carbazols erfolgt.

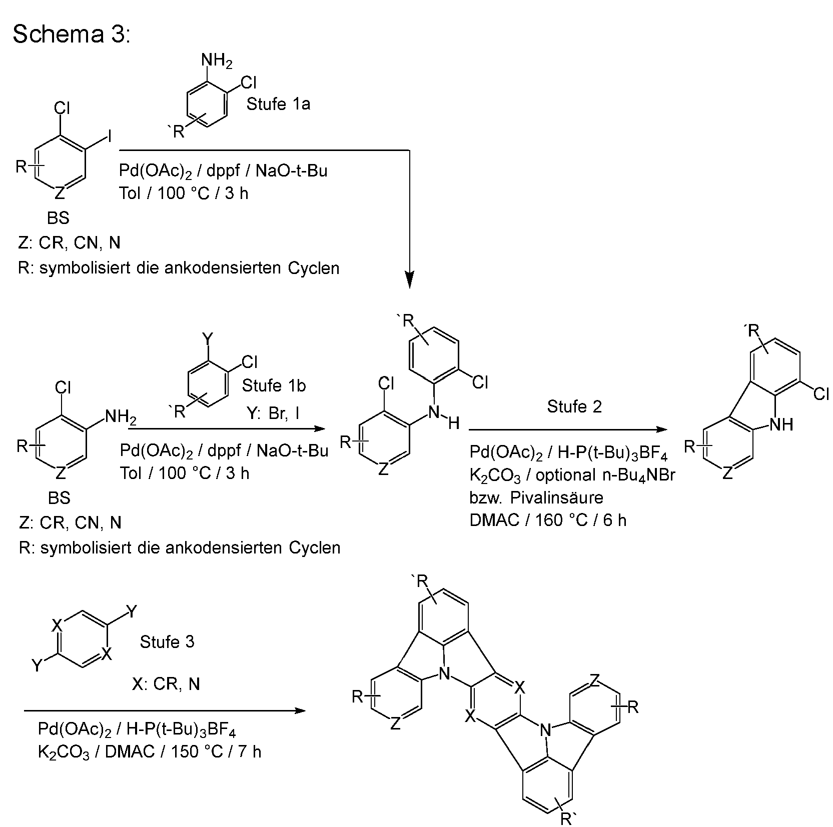

Die Bedeutung der in Schemata 1, 2 und 3 verwendeten Symbole entspricht im Wesentlichen denen, die für Formel (I) beziehungsweise bevorzugter Ausführungsformen dieser Strukturen definiert wurde, wobei aus Gründen der Übersichtlichkeit auf eine Nummerierung sowie auf eine vollständige Darstellung aller Symbole verzichtet wurde. Darüber hinaus wurde aus Gründen der Übersichtlichkeit vielfach auf die Verwendung von Symbolen zur Darstellung möglicher Stickstoffatome in den heteroaromatischen Ringen verzichtet, wie diese insbesondere in Formeln (I-1) und (I-2) durch die Symbole X, X^{a}, X^{b} und X^{c} dargelegt sind. Diese Angaben sind daher beispielhaft zu verstehen, wobei der Fachmann in der Lage ist die zuvor und nachfolgend, insbesondere in den Beispielen dargelegten Synthesen auf Verbindungen zu übertragen, bei denen ein oder mehrere der Symbole Symbole X, X^{a}, X^{b} und X^{c} für Stickstoff stehen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) und bevorzugten Ausführungsformen dieser Formel oder erfindungsgemäße Verbindungen, wobei ein oder mehrere Bindungen der erfindungsgemäßen Verbindungen oder der Strukturen der Formel (I) und bevorzugten Ausführungsformen dieser Formel zum Polymer, Oligomer oder Dendrimer vorhanden sind. Je nach Verknüpfung der Strukturen der Formel (I) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise ein Emitter und/oder ein Matrixmaterial, wobei sich diese Verbindungen von den erfindungsgemäßen Verbindungen unterscheiden. Geeignete Emitter und Matrixmaterialien sind hinten im Zusammenhang mit der organischen Elektrolumineszenzvorrichtung aufgeführt. Die weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, HostMaterialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden, vorzugsweise Hostmaterialien.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung, vorzugsweise als Emitter, besonders bevorzugt als grüner, roter oder blauer Emitter, speziell bevorzugt als blauer Emitter. Hierbei zeigen erfindungsgemäße Verbindungen bevorzugt fluorezierende Eigenschaften und stellen somit bevorzugt fluoreszierenden Emitter bereit.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung gemäß Formel (I) bzw. die oben ausgeführten bevorzugten Ausführungsformen in einer emittierenden Schicht als Emitter, vorzugsweise roter, grüner oder blauer Emitter, besonders bevorzugt als blauer Emitter.

Wenn die erfindungsgemäße Verbindung als Emitter in einer emittierenden Schicht eingesetzt wird, wird bevorzugt ein geeignetes Matrixmaterial eingesetzt, welches als solches bekannt ist.

Eine bevorzugte Mischung aus der erfindungsgemäßen Verbindung und einem Matrixmaterial enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% an Matrixmaterial bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-MatrixMaterial Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

In einer bevorzugten Ausgestaltung wird eine erfindungsgemäße Verbindung, die als Emitter verwendet wird, vorzugsweise in Kombination mit einem oder mehreren phosphoreszierenden Materialien (Triplettemitter) und/oder einer Verbindung eingesetzt, die ein TADF-Hostmaterial (thermally activated delayed fluorescence) darstellt. Hierbei wird vorzugsweise ein Hyperfluoreszenz- und/oder Hyperphosphoreszenz-System gebildet.

In WO 2015/091716 A1 und in WO 2016/193243 A1 werden OLEDs offenbart, die in der Emissionsschicht sowohl eine phosphoreszierende Verbindung als auch einen fluoreszierenden Emitter enthalten, wobei die Energie von der phosphoreszierenden Verbindung auf den fluoreszierenden Emitter übertragen wird (Hyperphosphoreszenz). Die phosphoreszierende Verbindung verhält sich in diesem Zusammenhang demnach wie ein Host-Material. Wie der Fachmann weiß, haben Hostmaterialien höhere Singulett und Triplett-Energien im Vergleich zu dem Emittern, damit die Energie des Host-Materials auch möglichst optimal auf den Emitter übertragen werden. Die im Stand der Technik offenbarten Systeme weisen genau solch eine Energierelation auf.

Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/001990, WO 2018/019687, WO 2018/019688, WO 2018/041769, WO 2018/054798, WO 2018/069196, WO 2018/069197, WO 2018/069273, WO 2018/178001, WO 2018/177981, WO 2019/020538, WO 2019/115423, WO 2019/158453 und WO 2019/179909 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine erfindungsgemäße Verbindung kann vorzugsweise in Kombination mit einem TADF-Hostmaterial und/oder einem TADF-Emitter eingesetzt werden, wie dies zuvor dargelegt ist.

Der als thermisch aktivierte verzögerte Fluoreszenz (TADF = "thermally activated delayed fluorescence") bezeichnete Vorgang wird beispielsweise von B. H. Uoyama et al., Nature 2012, Vol. 492, 234 beschrieben. Um diesen Prozess zu ermöglichen, ist im Emitter ein vergleichsweise kleiner Singulett-Triplett-Abstand ΔE(S₁ - T₁) von zum Beispiel weniger als etwa 2000 cm⁻¹ nötig. Um den an sich spin-verbotenen Übergang T₁ → S₁ zu öffnen, kann neben dem Emitter eine weitere Verbindung in der Matrix vorgesehen werden, die eine starke Spin-Bahn-Kopplung aufweist, sodass über die räumliche Nähe und die damit mögliche Wechselwirkung zwischen den Molekülen ein Inter-System-Crossing ermöglicht wird, oder die Spin-Bahn-Kopplung wird über ein im Emitter enthaltenes Metallatom erzeugt.

Weitere wertvolle Informationen zu Hyperfluoreszenz-Systemen sind unter anderem in WO2012/133188 (Idemitsu), WO2015/022974 (Kyushu Univ.), WO2015/098975 (Idemitsu), WO2020/053150 (Merck) und DE202019005189 (Merck) dargelegt.

Weitere wertvolle Informationen zu Hyperphosphoreszenz-Systemen sind unter anderem in WO2015/091716 A1, WO2016/193243 A1 (BASF), WO01/08230 A1 (Princeton Univ. (Mark Thompson)), US2005/0214575A1 (Fuji), WO2012/079673 (Merck), WO2020/053314 (Merck) und WO2020/053315 (Merck) dargelegt.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (I) oder deren oder deren zuvor dargelegten bevorzugten Ausführungsformen sind neu Ein weiterer Gegenstand der vorliegenden Erfindung ist daher eine Formulierungen, enthaltend mindestens ein Lösungsmittel und eine Verbindung gemäß Formel (I) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer und eine höhere Farbreinheit aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen als Emitter weisen sehr schmal Emissionsbanden mit geringen FWHM-Werten (Full Width Half Maximum) auf und führen zu besonders Farb-reiner Emission, erkennbar an den kleinen CIE-y-Werten. Besonders überraschend ist hierbei, dass sowohl blaue Emitter mit geringen FWHM-Werten als auch Emitter mit geringen FWHM-Werten bereitgestellt werden, die im grünen, gelben oder roten Bereich des Farbspektrums emittieren.
   Die Emissionsbanden weisen in der langwelligen Emissionsflanke eine Schulter oder ein Nebenmaximum auf, die jeweils weniger als 40 %, oftmals weniger als 30 %, der Intensität des Hauptmaximums aufweisen. Dies führt in Top-Emission OLED-Bauteilen zu einer günstig geringen Blickwinkelabhänigkeit des Farbeindrucks im Vergleich zu schmalbandigen Bor-enthaltenden Emittern nach Stand der Technik, die oftmals keine derartigen Schultern bzw. Nebenmaxima aufweisen und eine größere Blickwinkelabhänigkeit des Farbeindrucks zeigen.
2. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen, insbesondere als Emitter, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
3. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen als Emitter weisen eine hervorragende Effizienz auf. Hierbei bewirken erfindungsgemäße Verbindungen gemäß Formel (I) bzw. die zuvor und nachfolgend ausgeführten bevorzugten Ausführungsformen eine geringe Betriebsspannung bei Verwendung in elektronischen Vorrichtungen.
4. Die erfindungsgemäßen Verbindungen gemäß Formel (I) bzw. die zuvor und nachfolgend ausgeführten bevorzugten Ausführungsformen zeigen eine sehr hohe Stabilität und Lebensdauer.
5. Mit Verbindungen gemäß Formel (I) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
   Excitonenenergie wird von einem Matrix oder Host in der Emissionsschicht typischerweise entweder über den sogenannten Dexter- oder über den Förstertransfer auf den Emitter übertragen. Der Försterenergietransfer (FRET) von einem Host oder einer Matrix auf den erfindungsgemäßen Emitter ist dabei besonders bevorzugt, da dieser besonders effizient ist, was zu elektronischen Vorrichtungen mit besonders guten Leistungsdaten (bspw. Effizienz, Spannung und Lebensdauer) führt. Es zeigt sich, dass der Energieübertrag von einem Host oder einer Matrix auf die erfindungsgemäßen Verbindungen vorzugsweise über den Förstertransfer erfolgt.
6. Verbindungen gemäß Formel (I) bzw. die zuvor und nachfolgend ausgeführten bevorzugten Ausführungsformen weisen eine ausgezeichnete Glasfilmbildung auf.
7. Verbindungen gemäß Formel (I) bzw. die zuvor und nachfolgend ausgeführten bevorzugten Ausführungsformen bilden aus Lösungen sehr gute Filme und zeigen eine ausgezeichnete Löslichkeit.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbindungen, die mehrere Konfigurationsisomere, Enantiomere, Diastereomere oder tautomere Formen ausweisen können, wird eine Form stellvertretend gezeigt. Es werden folgende Abkürzungen für Lösemittel und Reagenzien verwendet: DCM - Dichlormethan, EE - Ethylacetat, THF - Tetrahydrofuran, EtOH - Ethanol, NCS - N-Chlorsuccinimid, NBS - N-Bromsuccinimid, NIS, N-Iod-Succinimid.

### 1) Darstellung der Synthone

### 1.1) Nitrile: Beispiel S1

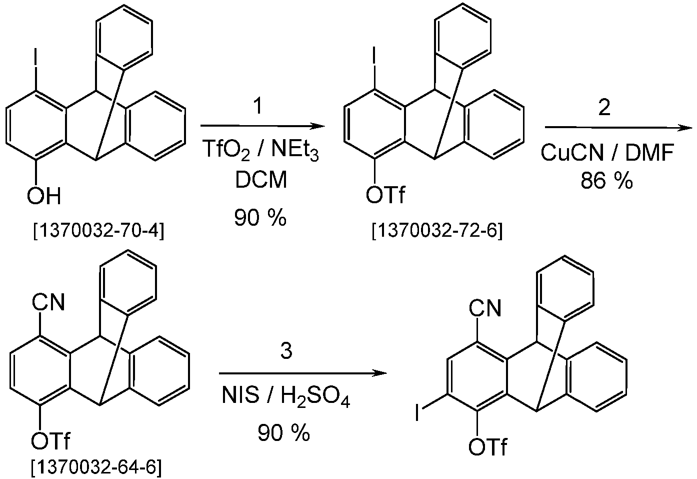

S1 kann auf der o.g. Route, gemäß der folgenden Literaturen, in 69 % Ausbeute dargestellt werden:
Stufe 1 und 2: W. S. Tan et al., J. Chin. Chem. Soc., 2012, 59, 399.
Stufe 3: J. M. Herbert et al., J. Label. Compd. Radiopharm., 2007, 50, 440.

Die Reinigung erfolgt via Flash-Chromatographie an einem Säulenautomaten (Combi-Flash Torrent, Fa. Axel Semrau).

Analog können folgende Synthone dargestellt werden.

| Bsp. | Edukt | Produkt | Ausbeute |
|---|---|---|---|
| S2 | 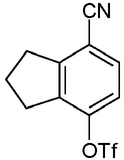 1403327-05-8, Stufe 3 | 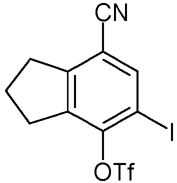 | 85 % |
| S3 | 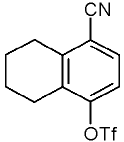 664364-61-8, Stufe 3 | 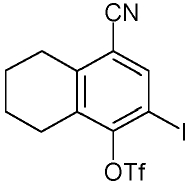 | 83 % |
| S4 | 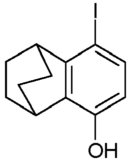 1560647-41-7, Stufe 1 - 3 | 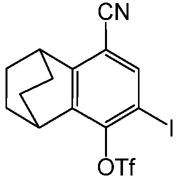 | 64 % |

### Alternative Darstellungsweise:

Alternativ kann S1 bis S 4 auf folgender Route in verbesserter Ausbeute dargestellt werden:

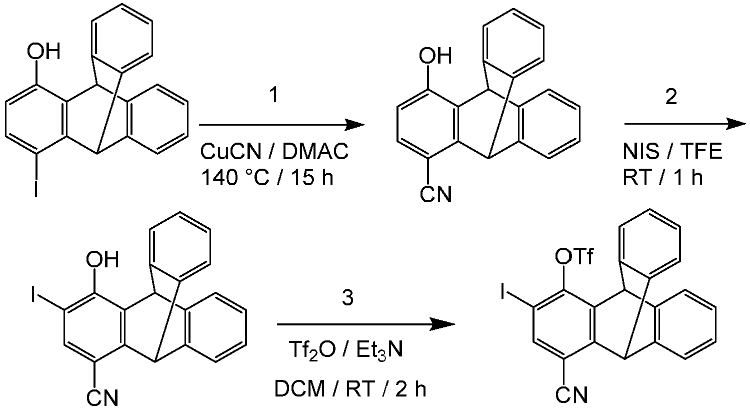

Stufe 1 und 3: Analog W. S. Tan et al., J. Chin. Chem. Soc., 2012, 59, 399. Ausbeute Stufe 1 ∼ 95 %, Ausbeute Stufe 3 quantitativ.

Stufe 2: lodierung mit N-Iodsuccinimid in Trifluorethanol (TFE) bzw. Hexafluor-iso-propanol analog R.-J. Tang et al. J. Org. Chem., 2018, 83, 930. Ausbeute 93 %.

### Beispiel S1b:

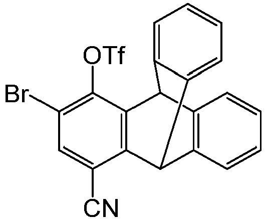

Analog können durch Einsatz von N-Bromsuccinimid die entsprechenden Brom-Triflate erhalten werden. Ausbeute über 3 Stufen 87 %.

### Beispiel S1c:

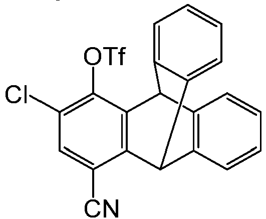

Analog können durch Einsatz von N-Chlorsuccinimid die entsprechenden Chlor-Triflate erhalten werden. Ausbeute über 3 Stufen 69 %.

### Beispiel S10:

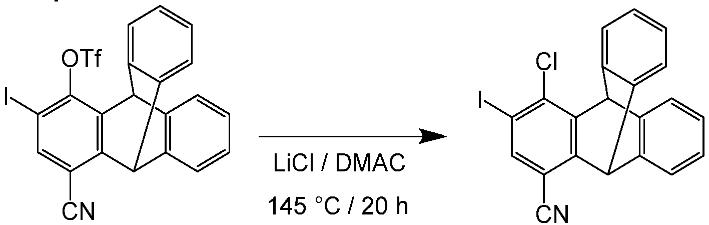

Durchführung analog W. S. Tan et al., J. Chin. Chem. Soc., 2012, 59, 399. Anstelle von DMF wird Dimethylacetamid (DMAC) verwendet, was zu verbesserten Ausbeuten führt. Ausbeute 66 %.

Analog zu S1 (alternative Darstellungsweise) und S10 können folgende Synthone dargestellt werden.

| Bsp. | Edukt | Produkt | Ausbeute |
|---|---|---|---|
| S10b | S1b | 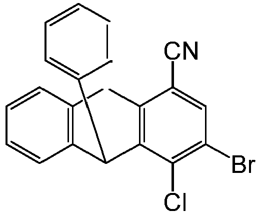 | 68 % |
| S11 | S2 | 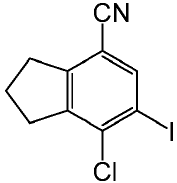 | 57 % |
| S12 | S3 | 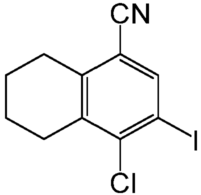 | 64 % |
| S13 | S4 | 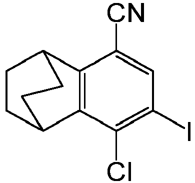 | 57 % |
| S14 | 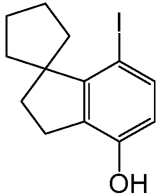 1588404-75-4 | 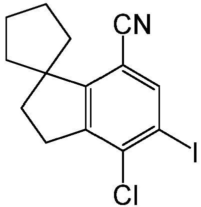 | 48 % |
| S15 | 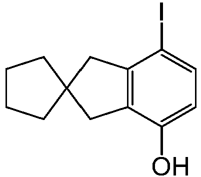 1419387-01-1 | 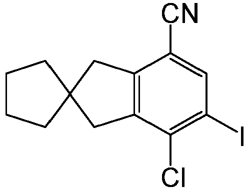 | 49 % |

Alternativ kann S10 wie folgt dargestellt werden: 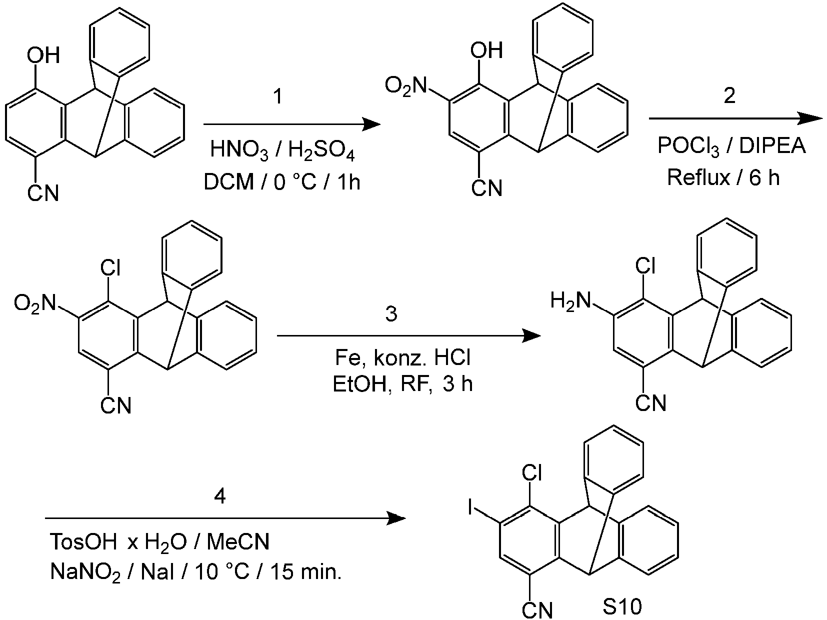
Stufe 1: Analog M. A. Zolfigol et al., Molecules 2001, 6, 614. Ausbeute: 93 %.
Stufe 2: G. Ralf et al. Journal fuer Praktische Chemie 1987, 329(6), 945. Ausbeute 89 %.
Stufe 3: Analog S. Chandrappa et al., Synlett 2010, 3019. Ausbeute: 87 %. Stufe 4: E. A. Krasnokutskaya, Synthesis 2007, (1), 81. Ausbeute 70 %

### Optimierte Synthese von S10:

### Stufe 1:

Eine auf 0 °C gekühlte Lösung von 29.5 g (100 mmol) 1-Cyano-4-hydroxytriptycen wird tropfenweise während 1 h mit einem Gemisch aus 19.0 g 65 Gew.-%iger Salpetersäure und 20.0 g 96 % Gew.-%iger Salpetersäure versetzt. Man rührt noch 30 min. nach und gießt dann unter sehr gutem Rühren vorsichtig (Schäumen!) auf ein Gemisch aus 37.8 g (450 mmol) Natriumhydrogencarbonat und 3 l Eiswasser. Man Trennt die org. Phase ab, extrahiert die wässrige Phase dreimal mit je 200 ml DCM, trocknet die vereinigten org. Phasen mit ges. Kochsalzlösung und stellt auf Magnesiumsulfat. Man Filtriert vom Trockenmittel ab, entfernt das DCM im Vakuum und chromatographiert den Rückstand (Kieselgel, n-Heptan/EE 5:1). Ausbeute: 31.5 g (93 mmol) 93 %; Reinheit ca. 98 % ig n. ¹H-NMR.

### Stufe 2:

Ein gut gerührtes Gemisch aus 34.0 g (100 mmol) 1-Cyano-3-nitro-4-hydroxytriptycen und 93.5ml (1 mol) Phosphorylchlorid wird mit 21.0 ml (120 mmol) Di-iso-propyl-ethylamin (DIPEA) versetzt und 4 h unter Rückfluss erhitzt. Man gießt die Reaktionsmischung unter sehr gutem Rühren langsam (exotherm, Induktionsperiode!) auf 2 l Eiswasser und rührt 30 min. nach. Man extrahiert die wässrige Phase fünf mal mit je 200 ml DCM trocknet die vereinigten org. Phasen mit ges. Kochsalzlösung und stellt auf Magnesiumsulfat. Man filtriert vom Trockenmittel ab, entfernt das DCM im Vakuum und chromatographiert den Rückstand (Kieselgel, n-Heptan/EE 5:1). Ausbeute: 40.1 g (89 mmol) 89 %; Reinheit ca. 97 % ig n. ¹H-NMR.

### Stufe 3:

Eine gut gerührte Suspension von 35.9 g (100 mmol) 1-Cyano-3-nitro-4-chlor-triptycen und 25.1 g (450 mmol) Eisenpulver in 700 ml EtOH wird unter Rückfluss tropfenweise während 30 min. mit 75.0 ml wässriger Salzsäure, 37 Gew.-%ig versetzt (Achtung: Wasserstoffentwicklung!). Man rührt noch 3 h am Rückfluss nach, lässt erkalten, verdünnt mit 2 l Wasser und 2 l DCM und stellt unter vorsichtiger Zugabe (Schäumen!) von festem Natriumcarbonat alkalisch (pH ∼ 9). Man saugt das Gemisch über Celite ab, trennt die org. Phase des Filtrats ab, extrahiert die wässrige Phase fünf mal mit je 100 ml DCM, trocknet die vereinigten org. Phasen durch zweimaliges waschen mit je 300 ml ges. Kochsalzlösung und stellt auf Magnesiumsulfat. Man filtriert vom Trockenmittel ab, entfernt das DCM im Vakuum, zieht das Rohprodukt auf Isolute auf und chromatographiert (Kieselgel, n-Heptan/DCM 1:1 > 1:2). Gegebenenfalls wird erneut chromatographiert, bis das Produkt weis bis leicht beige anfällt. Ausbeute: 28.5 g (87 mmol) 87 %; Reinheit ca. 98 % ig n. ¹H-NMR.

### Stufe 4:

Eine Lösung von 32.9 g (100 mmol) 1-Cyano-3-amino-4-chlor-triptycen in 500 ml Acetonitril (4l Vierhalskolben, Innenthermometer, Tropftichter, KPG-Rührer, Aronüberlagerung) wird portionsweise mit 57.1 g (300 mmol) p-Toluolsulfonsäure-Monohydrat [6192-52-5] versetzt und dann im Eisbad auf 10 °C gekühlt. Die Suspension wir unter sehr gutem Rühren und Eiskühlung tropfenweise mit einer Lösung von 13.9 g (200 mmol) Natriumnitrit und 37.5 g (250 mmol) Kaliumiodid in 60 ml Wasser versetzt und 15 min. bei 10 °C gerührt (Vorsicht: Stickstoffentwicklung - Schäumen). Anschließend lässt man auf Raumtemperatur erwärmen und rührt 70 min. nach. Man verdünnt dann mit 1500 ml Wasser, stelle durch Zugabe von gesättigter Natriumhydrogencarbonat-Lösung auf pH = 9.5 ein und versetzt mit 200 ml 2M Natrium-bisulfit-Lösung. Man saugt vom ausgefallenen Rohprodukt ab, wäscht dieses zweimal mit je 50 ml Wasser und saugt kurz trocken. Man löst das Rohprodukt in 500 ml DCM, trocknet die Lösung über Natriumsulfat, saugt vom Trockenmittel ab und zieht das Rohprodukt auf Isolute auf. Die Reinigung erfolgt durch Flash-chromatographie (Combi-Flash Torrent der Fa. A. Semrau). Ausbeute: 31.1 g (70 mmol), 70 %; Reinheit: ca. 97 % ig n. ¹H-NMR.

Analog können die Verbindungen S11-S15 dargestellt werden.

### 1.2) Synthese der substituieren lodo-Chloro-Pyridine Syntheseschema am Beispiel eines Homoadamtan-enamins:

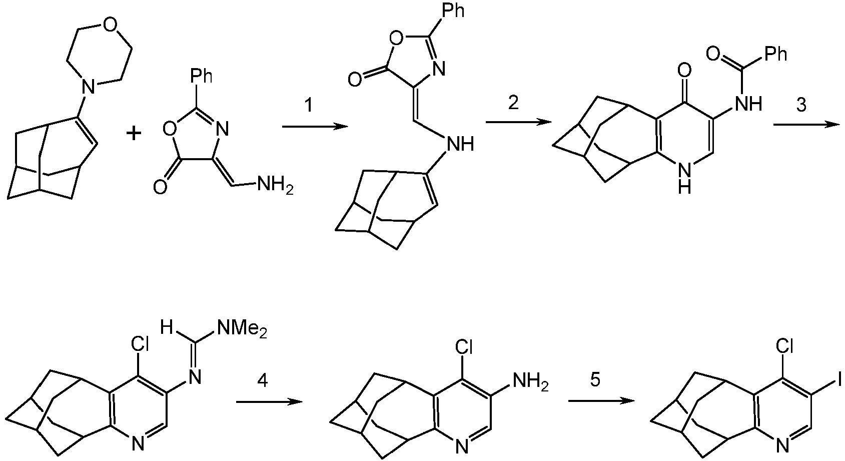

Die Stufe 1 bis 5 werden analog literaturbekannten Synthesen durchgeführt:

| | |
|---|---|
| Stufe 1 bis 4: | M. Adachi et al., Tetrahedron Letters, 37 (49), 8871, 1996; EP 0 556 008 B1. |
| Stufe 5: | J. D. Eckelbarger et al., US 8835409; E. A. Krasnokutskaya et al., Synthesis, 2007,1, 81. |

### A) Synthese der Enamine:

Die Enamine können nach dem in WO 2020/064662, Seite 108 ausgeführten Verfahren aus den gezeigten Ketonen und Morpholin in Ausbeuten von ca. 60 - 80 % dargestellt werden, bzw. sind literaturbekannt.

| Bsp. | Edukt Keton / Morpholin | Produkt Enamin |
|---|---|---|
| S100 | 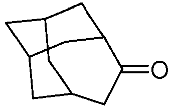 24669-56-5 | 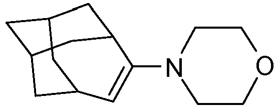 |
| S101 | 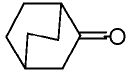 2716-23-6 | 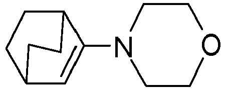 |
| S102 | 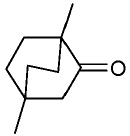 59117-09-8 | 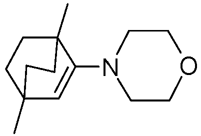 |
| S103 | 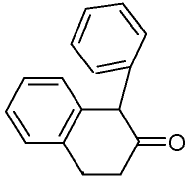 6372-63-0 | 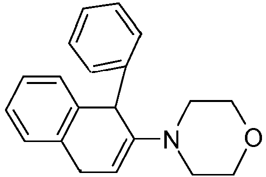 |
| S104 | 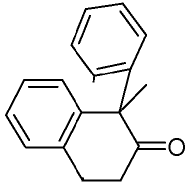 73164-06-4 | 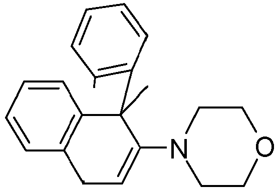 |
| S105 | 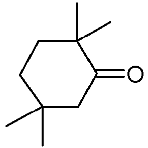 15189-14-7 | 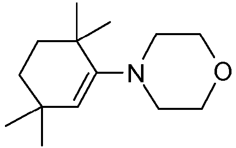 |
| S106 | 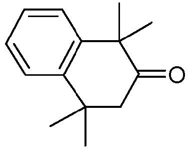 6308-02-7 | 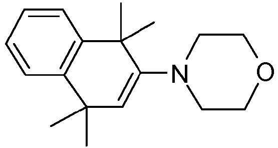 |
| S107 | 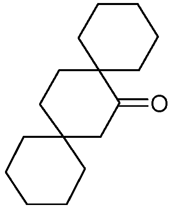 1781-82-4 | 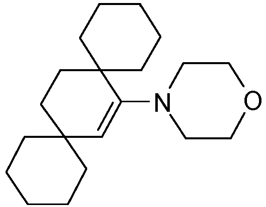 |
| S108 | 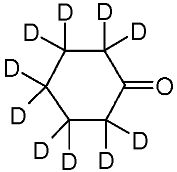 51209-49-5 | 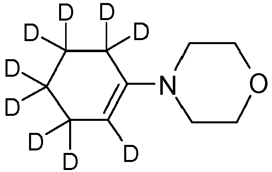 |
| S109 | 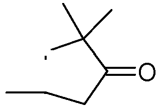 4694-115 | 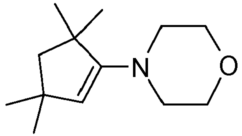 |
| S110 | 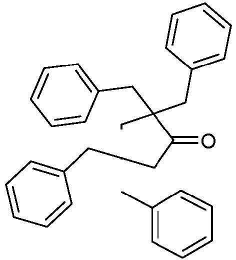 96676-35-6 | 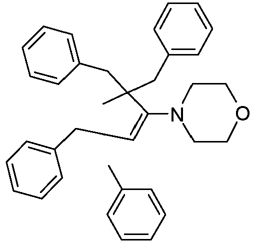 |
| S111 | 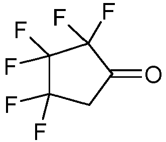 180690-80-6 | 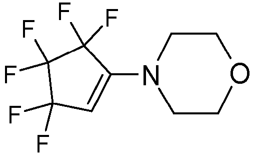 |

### B) Synthese der substituierten Pyridine:

### Beispiel S200

### Stufe 1: S200a

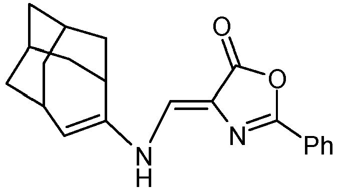

Ein Gemisch aus 23.3 g (100 mmol) S100 (analog für die anderen 6- und 7-Ring Enamine), 22.6 g (120 mmol) 4-(Aminomethylene)-2-phenyl-5(4H)-oxazolon [3674-51-9], 47.3 ml (500 mmol) Acetanhydrid [108-24-7] und 150 ml Toluol wird 4 h bei 100 °C gerührt (5-Ring-Enamine werden in o-Xylol bei 130 °C / 4 h im Autoklaven umgesetzt). Man engt im Vakuum komplett ein, versetzt das Öl mit 70 ml Methanol, rührt 3 h nach, saugt vom auskristallisierten Produkt ab, wäscht einmal mit 25 ml eiskaltem Methanol und trocknet im Vakuum. Das so erhaltene Rohprodukt wird ohne Reinigung weiter umgesetzt. Ausbeute: 26.2 g (78 mmol), 78 % E,Z-Isomerengemisch; Reinheit: ca. 95 % ig n. ¹H-NMR.

### Stufe 2: S200b

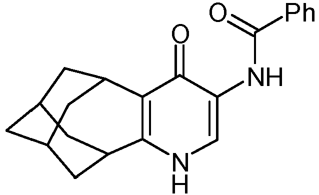

Ein Gemisch aus 33.4 g (100 mmol) S200a und 200 ml 1-Methyl-2-pyrrolidinon (NMP) wird 1.5 h bei 200 - 205 °C gerührt. Man lässt auf ca. 100 °C erkalten entfernt das NMP weitgehend im Vakuum, nimmt den glasartigen, zähen Rückstand in 100 ml warmem Acetonitril auf, rührt 12 h bei Raumtemperatur nach, saugt vom auskristallisierten Produkt ab und trocknet im Vakuum. Ausbeute: 25.1 g (75 mmol), 75 %; Reinheit: ca. 95 % ig n. ¹H-NMR.

### Stufe 3: S200c

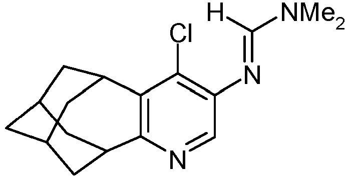

Eine Suspension von 33.4 g (100 mmol) S200b in einem Gemisch aus 150 ml N,N-Dimethylformamid (DMF) wird unter Eis-Kochsalz-Kühlung (ca. -10 °C) tropfenweise mit 14.0 ml (150 mmol) Phosphorylchlorid in 50 ml DMF versetzt und dann 16 h bei Raumtemperatur nachgerührt. Man gießt die Reaktionsmischung vorsichtig auf 1000 ml Eiswasser, rührt 10 min. nach, gibt 200 ml Dichlormethan (DCM) zu, rührt 10 min. nach und trennt die org. Phase ab. Man stellt die wässrige Phase unter vorsichtiger Zugabe von konz. wässriger Ammoniak-Lösung basisch (pH 8-9), extrahiert die wässrige Phase dreimal mit je 200 ml Ethylacetat, wäscht die vereinigten Ethylacetat-Extrakte zweimal mit je 200 ml Eiswasser, einmal mit 200 ml ges. Natriumhydrogencarbonat-Lösung und zweimal mit je 100 ml ges. Kochsalzlösung. Man trocknet über einem Gemisch aus Magnesiumsulfat und Natriumcarbonat, filtriert vom Trockenmittel ab, engt die org. Phase im Vakuum ein und kristallisiert den Rückstand einmal aus Acetonitril unter Zusatz von Ethylacetat (EE) um. Ausbeute: 24.7 g (81 mmol), 81 %; Reinheit: ca. 95 % ig n. ¹H-NMR.

### Stufe 4: S200d

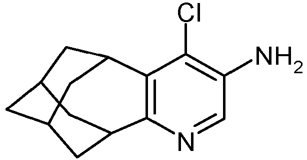

Ein Gemisch von 30.4 g (100 mmol) S200c, 100 ml 3 N Schwefelsäure und 200 ml Dioxan wird 1.5 h bei 100 °C gerührt. Nach Erkalten wird die Reaktionsmischung mit 1000 ml Eis-Wasser verdünnt und dann unter Eiskühlung mit 3 N NaOH auf pH ∼ 7.5 eingestellt. Man extrahiert die wässrige Phase dreimal mit je 200 ml DCM, wäscht die vereinigten org. Phasen zweimal mit je 200 ml Wasser, einmal mit 200 ml ges. Kochsalzlösung und trocknet über Magnesiumsulfat. Man filtriert von Trockenmittel ab, engt das Filtrat zur Trockene ein und kristallisiert aus Methanol um. Ausbeute: 23.1 g (93 mmol), 93 %; Reinheit: ca. 95 % ig n. ¹H-NMR.

### Stufe 5: S200

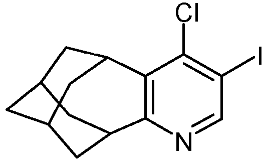

### Variante 1:

24.9 g (100 mmol) S200d werden unter gutem Rühren in 500 ml auf 3 - 5 °C gekühlte, konzentrierte, Salzsäure eingetragen. Die Suspension wird unter gutem Rühren tropfenweise während 15 min. mit einer kalten Lösung von 10.4 g (150 mmol) Natriumnitrit in 50 ml Wasser versetzt und anschließend ca. 20 min. bei 5 °C nachgerührt. Man gießt die so erhaltene Diazonium-Lösung in eine gut gerührte, auf 5 °C gekühlte Lösung von 90.0 g (600 mmol) Kaliumiodid in 5000 ml Wasser, das mit 1000 ml DCM versetzt ist (Achtung: Schäumen!). Nach beendeter Stickstoffentwicklung (ca. 25 min.) versetzt man bis zur Entfärbung mit Natriumbisulfit-Lösung und stellt mit 5 N NaOH unter sehr guter Kühlung vorsichtig auf pH ∼ 7.5 ein. Man verdünnt mit weiteren 1500 ml DCM, trennt die org. Phase ab, reextrahiert die wässrige zweimal mit je mit 500 ml DCM, wäscht die vereinigten org. Phasen zweimal mit je 500 ml Wasser und zweimal mit ja 500 ml ges. Kochsalzlösung und trocknet dann über Magnesiumsulfat. Nach Entfernen das DCM im Vakuum wird der Rückstand flashchromatographiert (Combi-Flash Torrent der Fa. A. Semrau). Ausbeute: 22.9 g (63 mmol), 63 %; Reinheit: ca. 97 % ig n. ¹H-NMR.

### Variante 2:

Eine Lösung von 24.9 g (100 mmol) S200d in 500 ml Acetonitril wird portionsweise mit 57.1 g (300 mmol) p-Toluolsulfonsäure-Monohydrat [6192-52-5] versetzt und dann im Eisbad auf 10 °C gekühlt. Die Suspension wir unter gutem Rühren und Eiskühlung portionsweise mit einer Lösung von 13.9 g (200 mmol) Natriumnitrit und 37.5 g (250 mmol) Kaliumiodid in 60 ml Wasser versetzt und 15 min. bei 10 °C gerührt. Anschließend lässt man auf Raumtemperatur erwärmen und rührt 70 min. nach. Man verdünnt dann mit 1500 ml Wasser, stelle durch Zugabe von gesättigter Natriumhydrogencarbonat-Lösung auf pH = 9.5 ein und versetzt mit 200 ml 2M Natrium-bisulfit-Lösung. Man saugt vom ausgefallenen Rohprodukt ab, wäscht dieses zweimal mit je 50 ml Wasser und saugt kurz trocken. Man löst das Rohprodukt in 500 ml DCM, trocknet die Lösung über Natriumsulfat, saugt vom Trockenmittel ab und zieht das Rohprodukt auf Isolute auf. Die Reinigung erfolgt durch Flash-chromatographie (Combi-Flash Torrent der Fa. A. Semrau). Ausbeute: 25.0 g (72 mmol), 72 %; Reinheit: ca. 97 % ig n. ¹H-NMR.

Analog zu den Stufen 1 bis 5 können folgende Pyridine erhalten werden. Ausbeute über fünf Stufe (Stufe 1-5):

| Bsp. | Enamin | Produkt | Ausbeute |
|---|---|---|---|
| S201 | S101 | 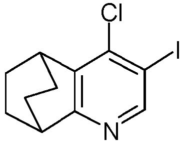 | 28 % |
| S202 | S102 | 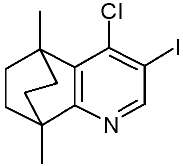 | 25 % |
| S203 | S103 | 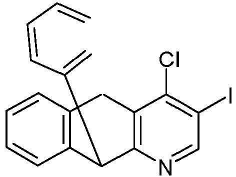 | 30 % |
| S204 | S104 | 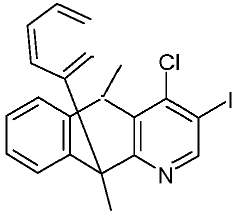 | 23 % |
| S205 | S105 | 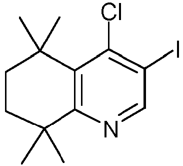 | 24 % |
| S206 | S106 | 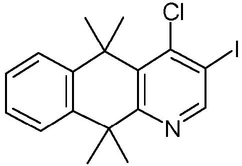 | 26 % |
| S207 | S107 | 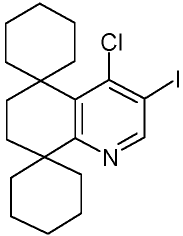 | 19 % |
| S208 | S108 | 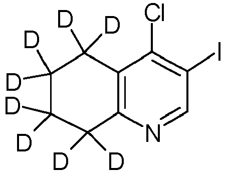 | 32 % |
| S209 | S109 | 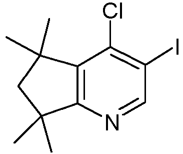 | 19 % |
| S210 | S110 | 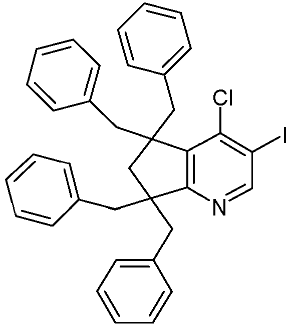 | 15 % |
| S211 | S111 | 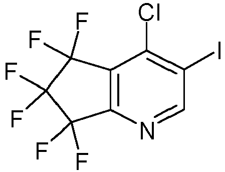 | 23 % |

### 1.3) Synthese der substituieren lodo-Chloro-Benzole

### Beispiel S300: Darstellung analog "Optimierte Synthese von S10".

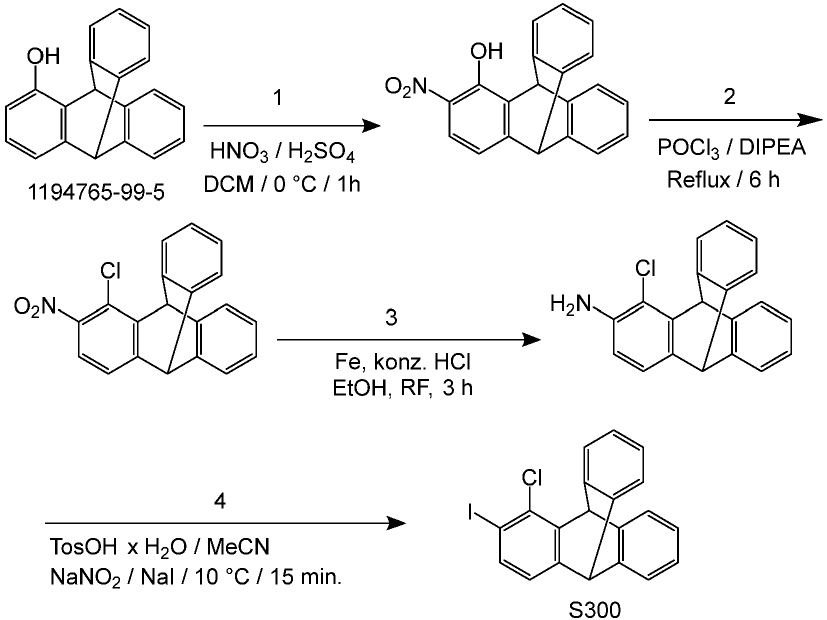

Stufe 1: Analog M. A. Zolfigol et al., Molecules 2001, 6, 614. Ausbeute: 96 %.

Stufe 2: G. Ralf et al. Journal fuer Praktische Chemie 1987, 329(6), 945. Ausbeute 91 %.

Stufe 3: Analog S. Chandrappa et al., Synlett 2010, 3019. Ausbeute: 90 %. Stufe 4: E. A. Krasnokutskaya, Synthesis 2007, (1), 81. Ausbeute: 78 %.

Analog können folgende Verbindungen dargestellt werden, Ausbeute über vier Stufen:

| Bsp. | Edukt | Produkt | Ausbeute |
|---|---|---|---|
| S301 | 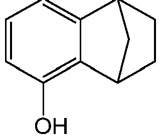 52960-96-0 | 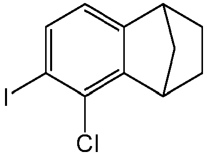 | 60 % |
| S302 | 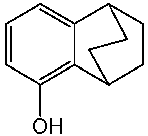 52960-97-1 | 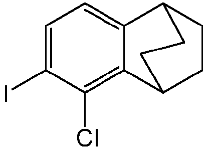 | 65 % |
| S303 | 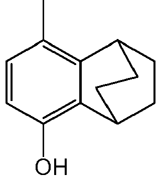 111221-21-7 | 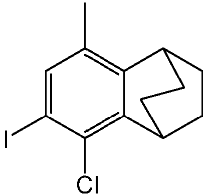 | 67 % |
| S304 | 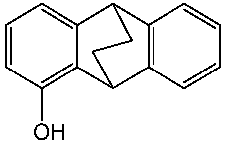 56301-19-0 | 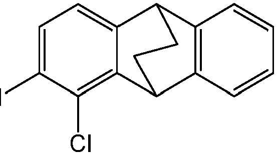 | 58 % |
| S305 | 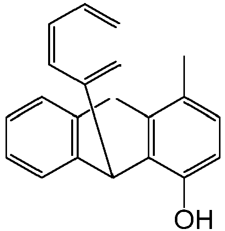 Darstellung aus 1370032-70-4 und 823-96-1 durch Suzuki-Kupplung analog S400 | 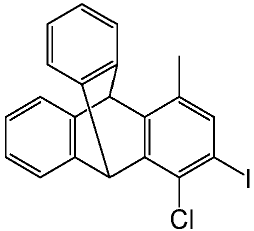 | 70 % |
| S306 | 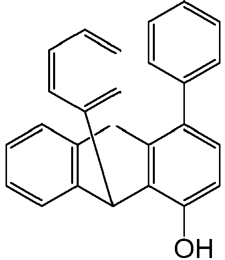 Darstellung aus 1370032-70-4 und 98-80-6 durch Suzuki-Kupplung Analog S400 | 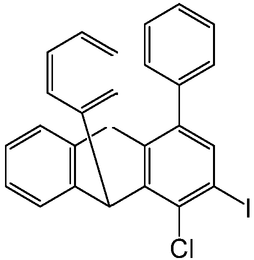 | 76 % |

### Beispiel S400:

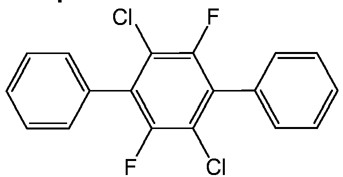

Suzkui-Kuppling: Ansatz 21.7 g (50 mmol) 1,4-Chlor-2,5-difluor-3,6-diiodbenzol [2410043-16-0], 13.4 g (110 mmol) Phenylboronsäure, 31.8 g (300 mmol) Natriumcarbonat, 702 mg (1 mmol) Bis(triphenylphosphino)-palladium(II)chlorid, 250 ml Acetonitril, 250 ml Methanol, 60 °C, 12 h. Aufarbeitung: Salze abfiltrieren, Filtrat einengen, Rückstand extraktiv DCM:Wasser aufarbeiten. Reinung flash-choromato-graphisch. Ausbeute: 12.9 g (38 mmol) 76 %; Reinheit: ca. 97 % ig n. ¹H-NMR.

Analog können folgende Verbindungen dargestellt werden:

| Bsp. | Edukt | Produkt | Ausbeute |
|---|---|---|---|
| S401 | 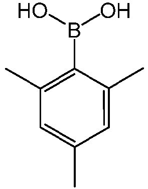 5980-97-2 | 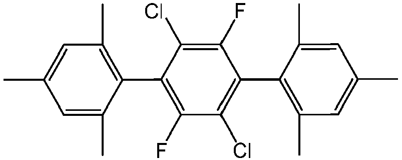 | 70 % |
| S402 | 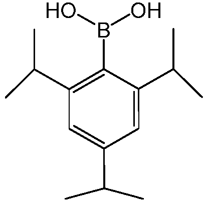 154549-38-9 | 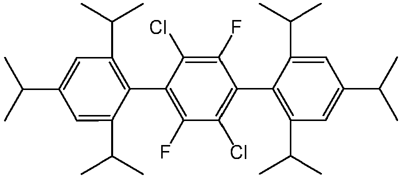 | 45 % |
| S403 | 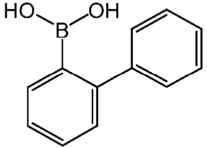 4688-76-0 | 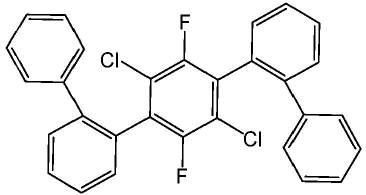 | 57 % |

### 2. Synthese der Carbazole C:

### Beispiel C1:

### Stufe 1:

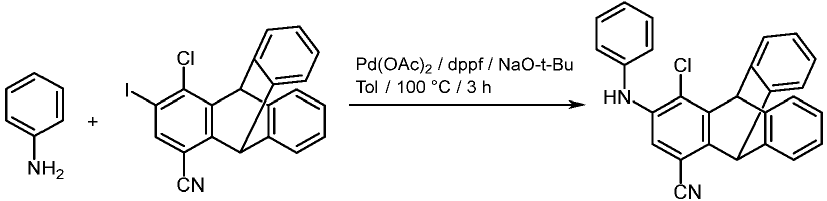

Ein gut gerührtes Gemisch aus 44.0 g (100 mmol) S10, 9.8 g (105 mmol) Anilin, 28.8 g (300 mmol) Natrium-tert-butylat, 1.11 g (2 mmol) dppf, 225 mg (1 mmol) Palladium(II)acetat in 500 ml Toluol wird 1 h unter Rückfluss erhitzt. Man lässt auf 70 °C erkalten, gibt 500 ml Wasser zu, rührt 10 min. nach, trennt die org. Phase ab, wäscht diese zweimal mit je 300 ml Wasser, einmal mit 300 ml ges. Kochsalzlösung und trocknet über Magnesiumsulfat. Man filtriert über ein mit Toluol vorgeschlämmtes Celite-Bett ab, engt das Filtrat im Vakuum ein, löst den Rückstand in 300 ml DCM und entfrent dieses im Vakuum, wobei das abdestillierte DCM durch simultane Zugabe von EtOH subsituiert wird. Man saugt von auskristallisierten Produkt ab, wäscht dieses dreimal mit je 50 ml EtOH und trocknet im Vakuum. Ausbeute: 36.7 g (91 mmol) 91 %; Reinheit: ca. 98 % ig nach ¹H-NMR. Bei Einsatz von Triflaten wird das Triflat langsam zudosiert, s, J. Louie et al., Journal of Organic Chemistry 1997, 62(5), 1268.

### Stufe 2:

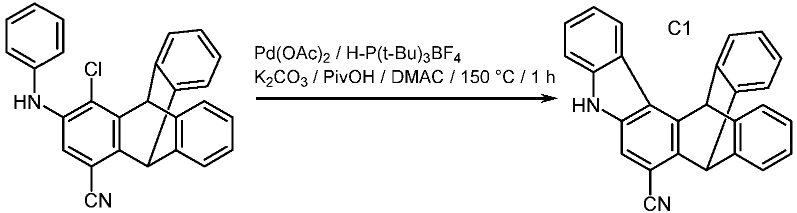

Ein gut gerührtes Gemisch aus 40.5 g (100 mmol) des Amins aus Stufe 1, 69.1 g (500 mmol) Kaliumcarbonat, 3,1 g (30 mmol) Pivalinsäure, 1.16 g (4 mmol) Tri-tert-butylphosphonium-tetrafluroborat, 449 mg (2 mmol) Palladium(II)acetat, 100 g Glaskugeln (3 mm Durchmesser) und 1000 ml Dimethylacetamid (DMAC) wird 1 h bei 150 °C gerührt. Man filtriert noch heiß über ein mit DMAC vorgeschlämmtes Clite-Bett ab, engt das Flitrat zur Trockene ein, löst den Rückstand in 500 ml DCM und entfrent dieses im Vakuum, wobei das abdestillierte DCM durch simultane Zugabe von 300 ml EtOH subsituiert wird. Man saugt von auskristallisierten Produkt ab, wäscht dieses dreimal mit je 50 ml EtOH und trocknet im Vakuum. Ausbeute: 29.5 g (80 mmol) 80 %; Reinheit: ca. 98 % ig nach ¹H-NMR.

Analog können folgende Verbindungen dargestellt werden, Ausbeute über zwei Stufen:

| Bsp. | Edukt | Produkt | Ausbeute |
|---|---|---|---|
| C2 | 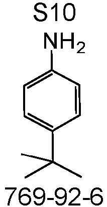 | 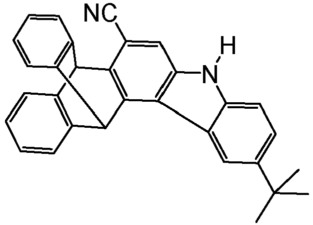 | 70 % |
| C3 | 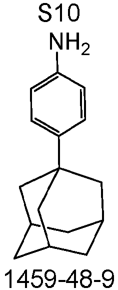 | 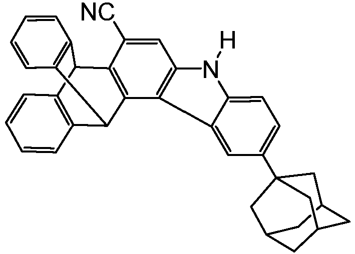 | 74 % |
| C4 | 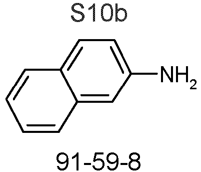 | 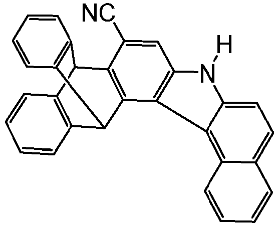 | 78 % |
| C5 | 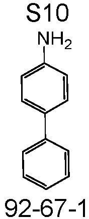 | 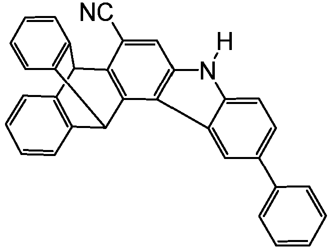 | 70 % |
| C6 | 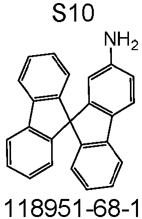 | 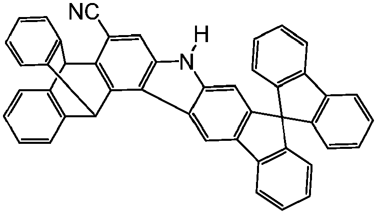 | 66 % |
| C7 | 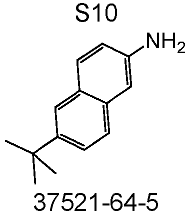 | 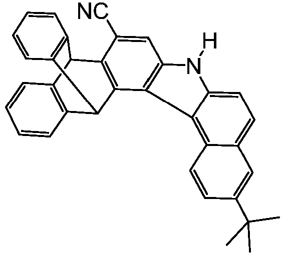 | 76 % |
| C8 | 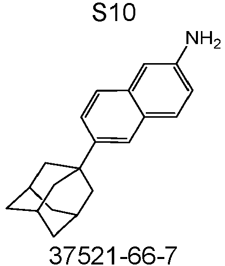 | 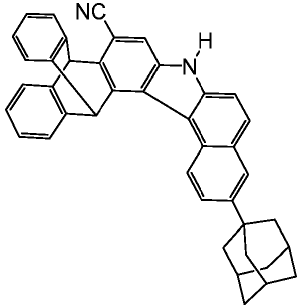 | 71 % |
| C9 | 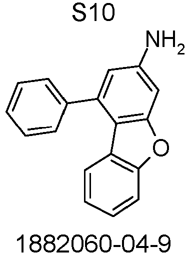 | 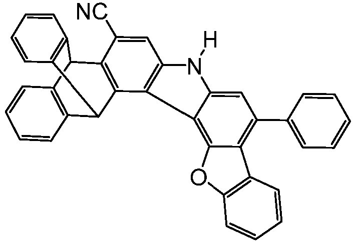 | 46 % |
| C10 | 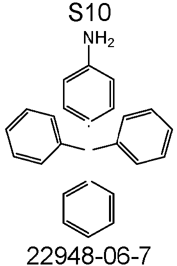 | 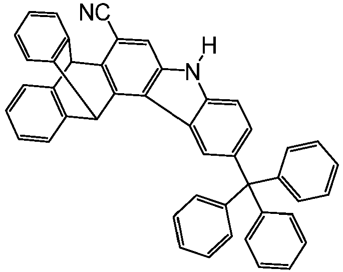 | 68 % |
| C11 | 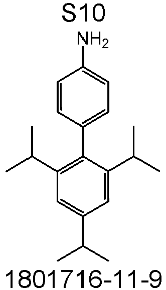 | 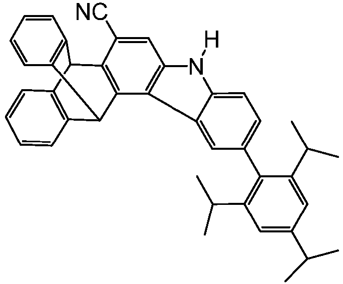 | 73 % |
| C12 | 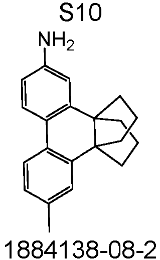 | 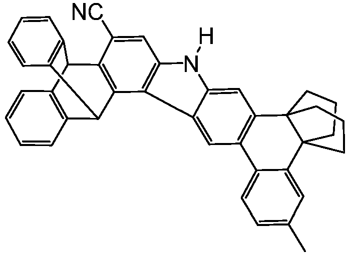 | 53 % |
| C13 | 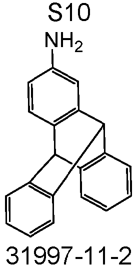 | 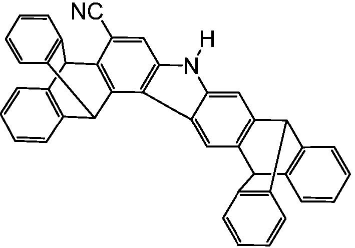 | 55 % |
| C14 | 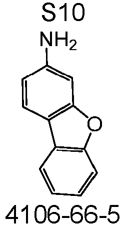 | 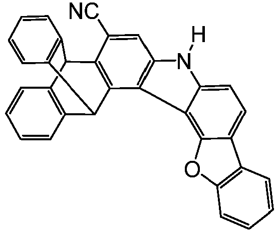 | 47 % |
| C15 | 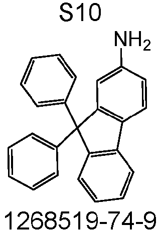 | 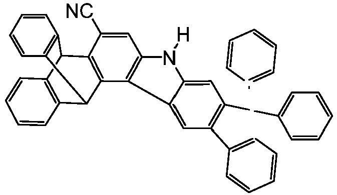 | 55 % |
| C16 | 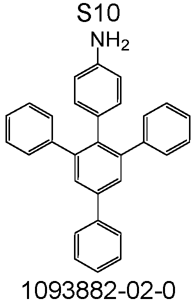 | 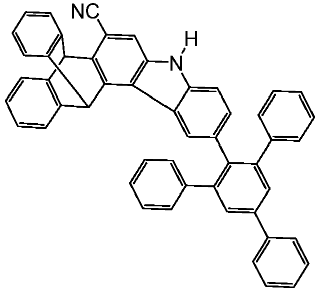 | 73 % |
| C17 | 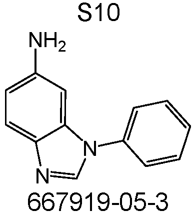 | 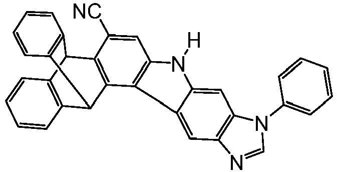 | 31 % |
| C18 | 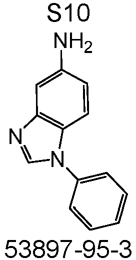 | 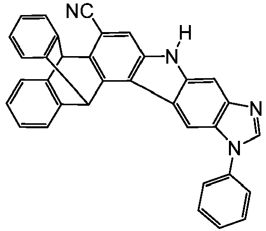 | 34 % |
| C19 | 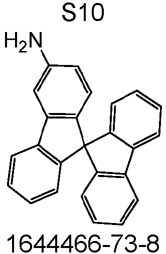 | 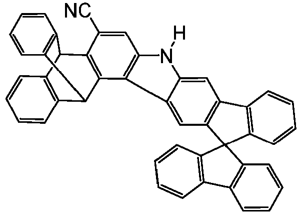 | 56 % |
| C20 | 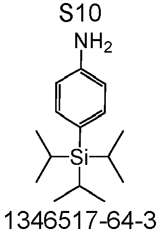 | 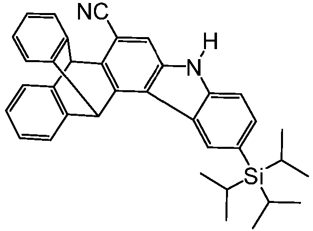 | 44 % |
| C21 | 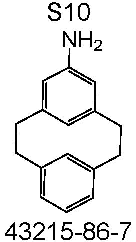 | 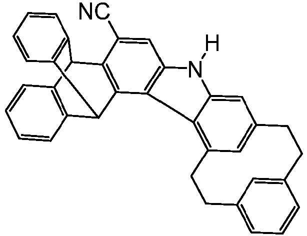 | 69 % |
| C22 | 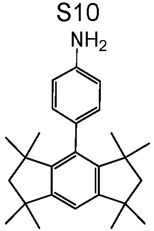 Darstellung nach Chem. Sci., 2019, 10, 6107 | 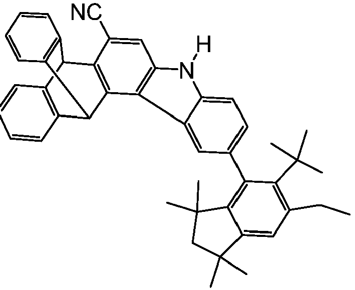 | 68 % |
| C23 | 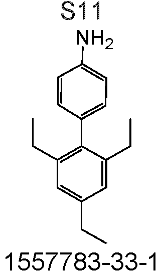 | 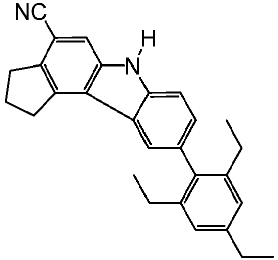 | 65 % |
| C24 | 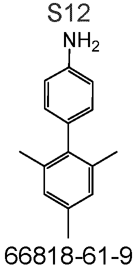 | 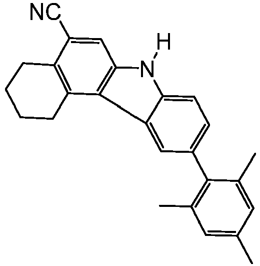 | 74 % |
| C25 | 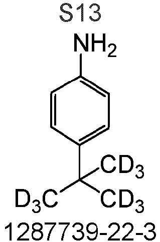 | 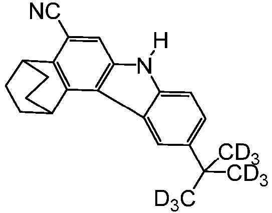 | 77 % |
| C26 | 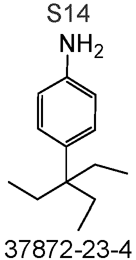 | 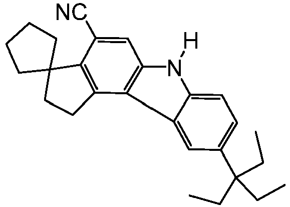 | 49 % |
| C27 | 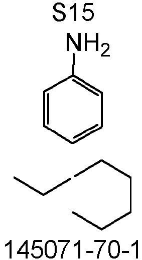 | 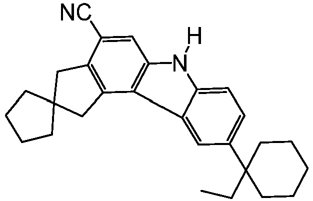 | 58 % |
| C28 | 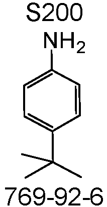 | 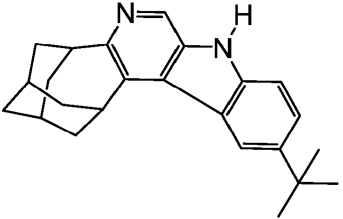 | 69 % |
| C29 | 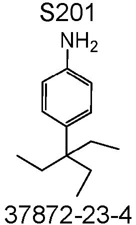 | 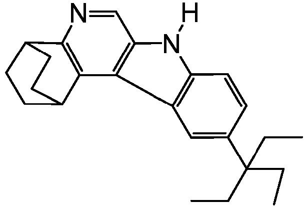 | 73 % |
| C30 | 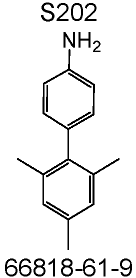 | 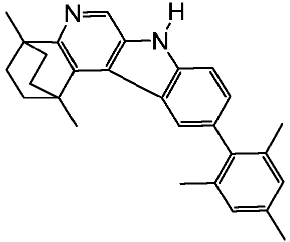 | 68 % |
| C31 | 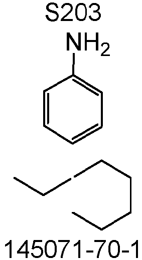 | 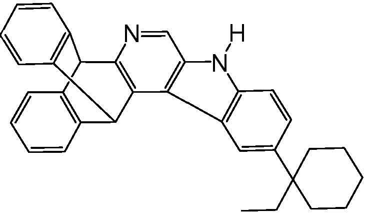 | 65 % |
| C32 | 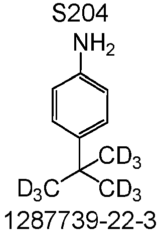 | 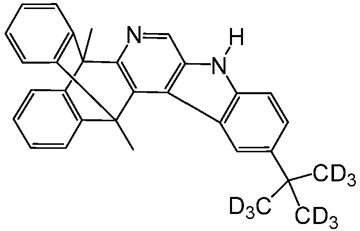 | 73 % |
| C33 | 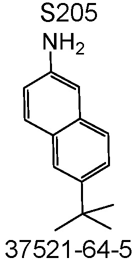 | 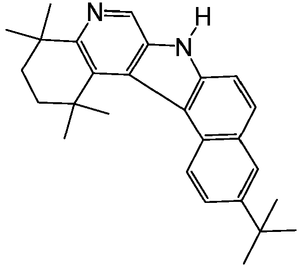 | 64 % |
| C34 | 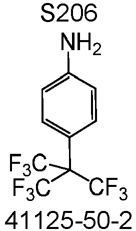 | 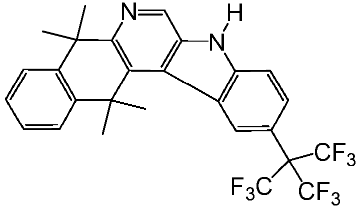 | 61 % |
| C35 | 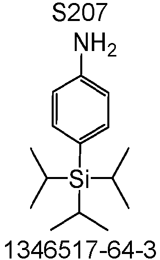 | 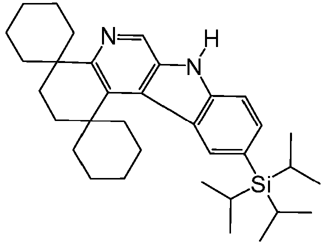 | 40 % |
| C36 | 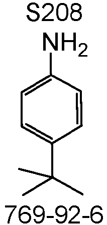 | 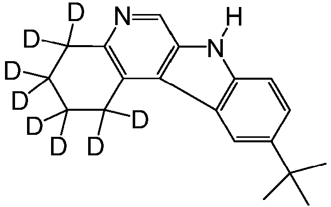 | 66 % |
| C37 | 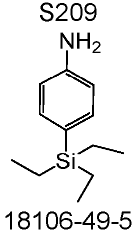 | 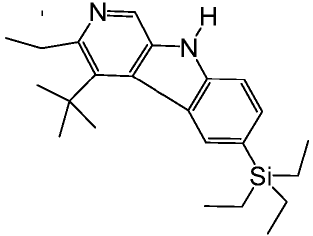 | 38 % |
| C38 | 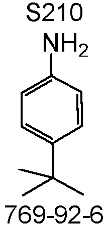 | 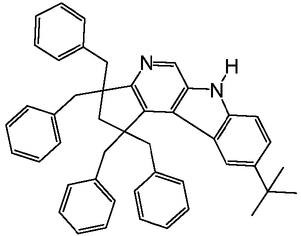 | 65 % |
| C39 | 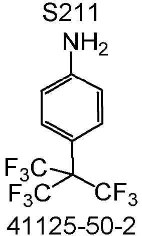 | 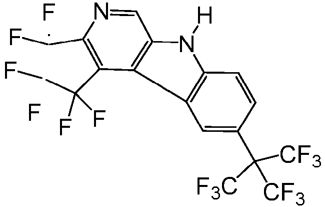 | 31 % |
| C40 | 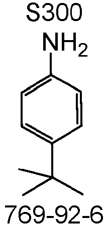 | 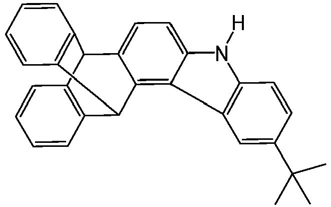 | 74 % |
| C41 | 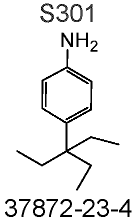 | 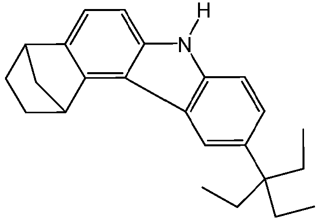 | 69 % |
| C42 | 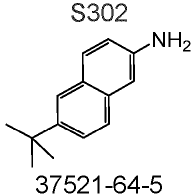 | 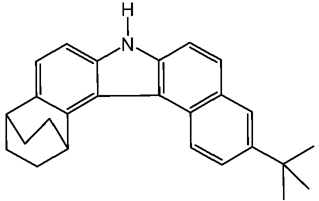 | 68 % |
| C43 | 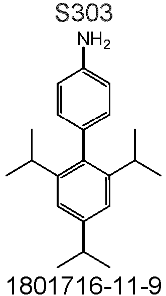 | 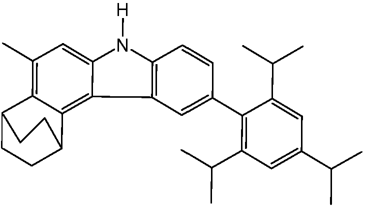 | 70 % |
| C44 | 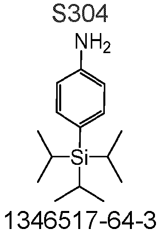 | 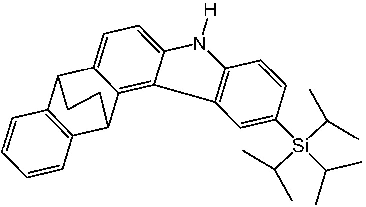 | 55 % |
| C45 | 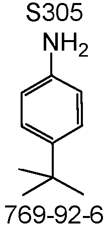 | 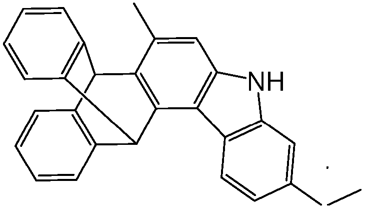 | 68 % |
| C46 | 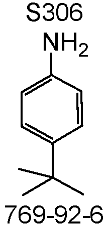 | 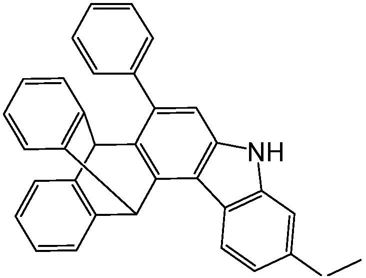 | 73 % |
| C200 | 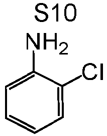 | 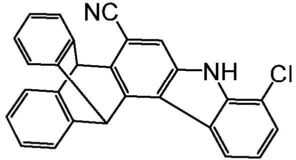 | 44 % |
| C201 | 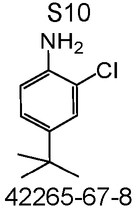 | 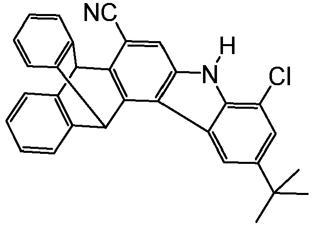 | 46 % |
| C202 | 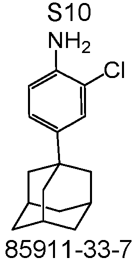 | 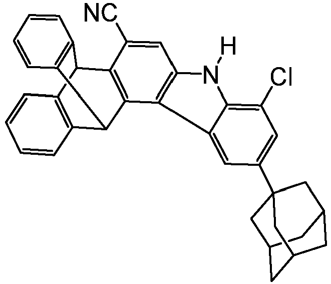 | 43 % |
| C203 | 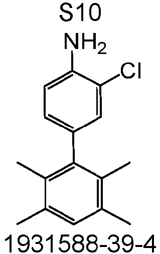 | 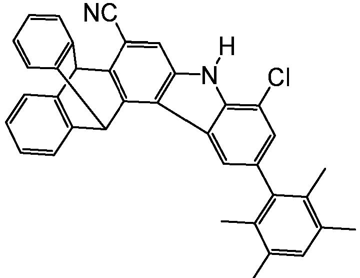 | 47 % |
| C204 | 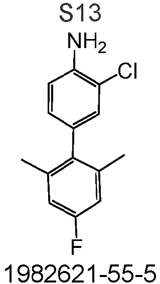 | 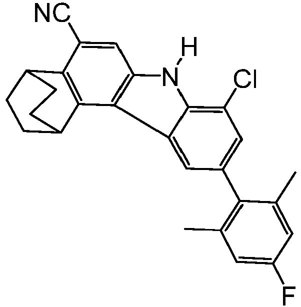 | 49 % |
| C205 | 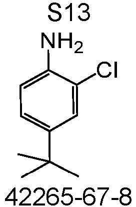 | 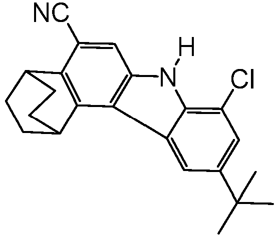 | 45 % |
| C206 | 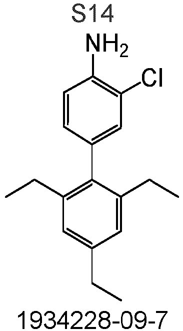 | 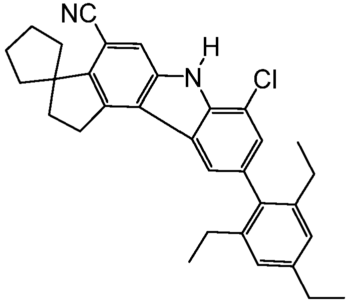 | 42 % |
| C207 | 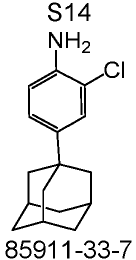 | 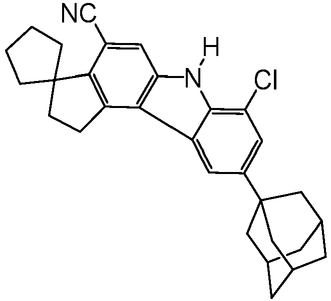 | 40 % |
| C208 | 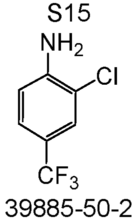 | 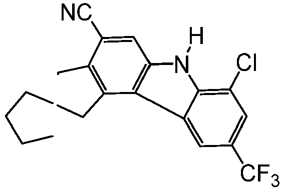 | 44 % |

### Beispiel C100:

### Stufe 1:

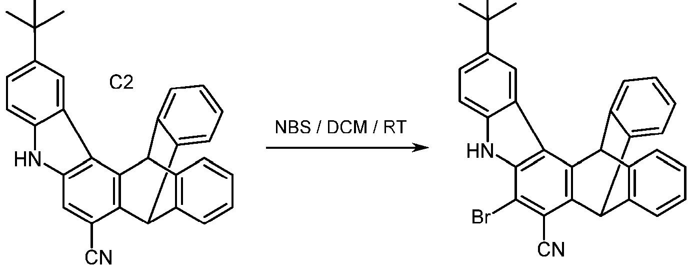

Eine gut gerührte Lösung von 42.5 g (100 mmol) C2 in 1000 ml DCM wird Portionsweise mit 19.8 g (100 mmol) N-Bromsuccinimid (NBS) versetzt und anschließend 5 h bei RT nachgerührt. Man entfernt das DCM im Vakuum, wobei das abdestillierte DCM durch simultane Zugabe von MeOH subsituiert wird, Endvolumen ca. 300 ml. Man saugt von auskristallisierten Produkt ab, wäscht dieses zweimal mit je 50 ml MeOH und trocknet im Vakuum. Ausbeute: 48.0 g (95 mmol) 95 %; Reinheit: ca. 98 % ig nach ¹H-NMR.

### Stufe 2:

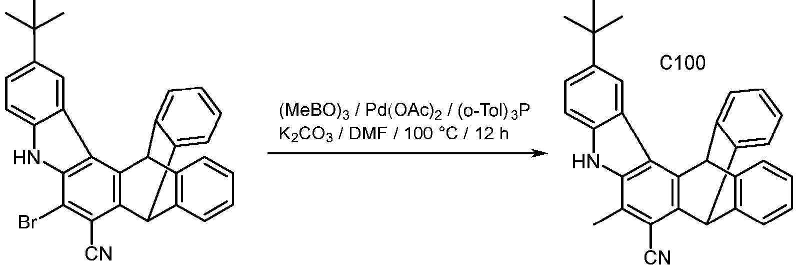

Ein gut gerührtes Gemisch aus 44.7 g (100 mmol) des Br-Carbazols aus Stufe 1, 7.0 ml (50 mmol) Trimetylboroxin [823-96-1], 41.5 g (300 mmol) Kaliumcarbonat, 1.83 g (6 mmol) Tri-o-tolylphosphin, 449 mg (2 mmol) Palladium(II)acetat, 100 g Glaskugeln (3 mm Durchmesser) und 800 ml Dimethylacetamid (DMAC) wird 12 h bei 120 °C gerührt. Man filtriert noch heiß über ein mit DMAC vorgeschlämmtes Clite-Bett ab, engt das Flitrat zur Trockene ein, löst den Rückstand in 500 ml DCM und entfernt dieses im Vakuum, wobei das abdestillierte DCM durch simultane Zugabe von 300 ml EtOH subsituiert wird. Man saugt von auskristallisierten Produkt ab, wäscht dieses dreimal mit je 50 ml EtOH und trocknet im Vakuum. Ausbeute:31.7 g (83 mmol) 83 %; Reinheit: ca. 98 % ig nach ¹H-NMR.

Analog können folgende Verbindungen dargestellt werden, Ausbeute über zwei Stufen:

| Bsp. | Edukt | Produkt | AusBeute |
|---|---|---|---|
| C101 | 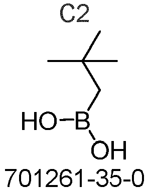 | 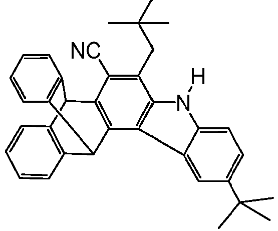 | 56 % |
| C102 | 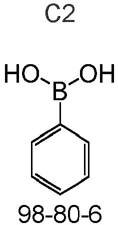 | 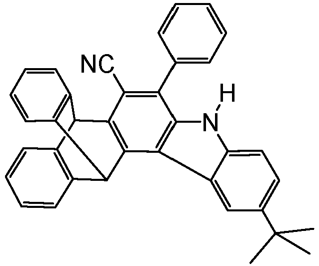 | 88 % |
| C103 | 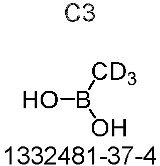 | 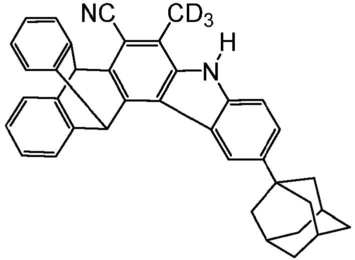 | 79 % |
| C104 | 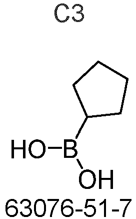 | 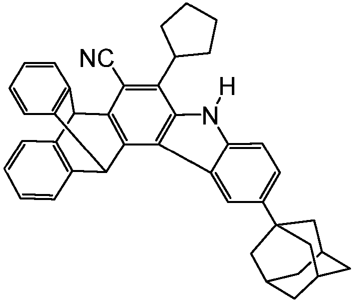 | 45 % |
| C105 | 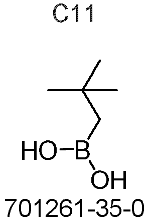 | 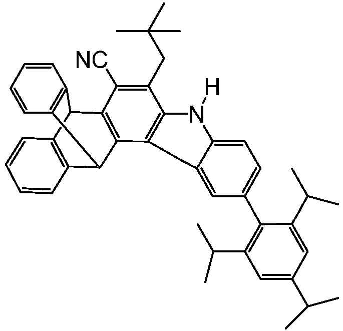 | 51 % |
| C106 | 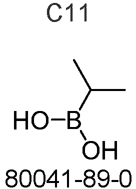 | 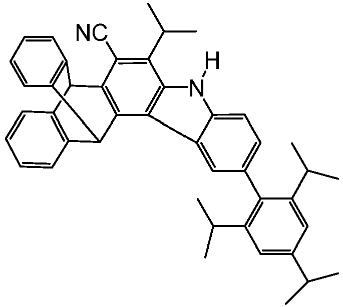 | 55 % |
| C107 | 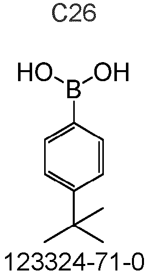 | 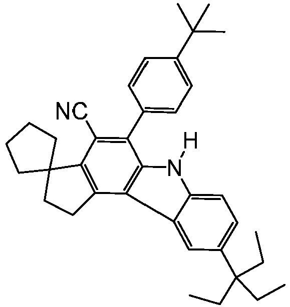 | 79 % |
| C108 | 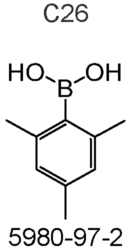 | 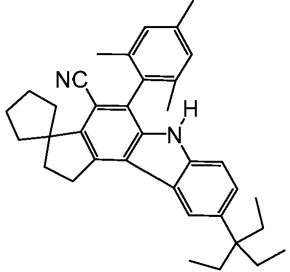 | 63 % |

### 3. Erfindungsgemäße Verbindungen:

### Beispiel D1:

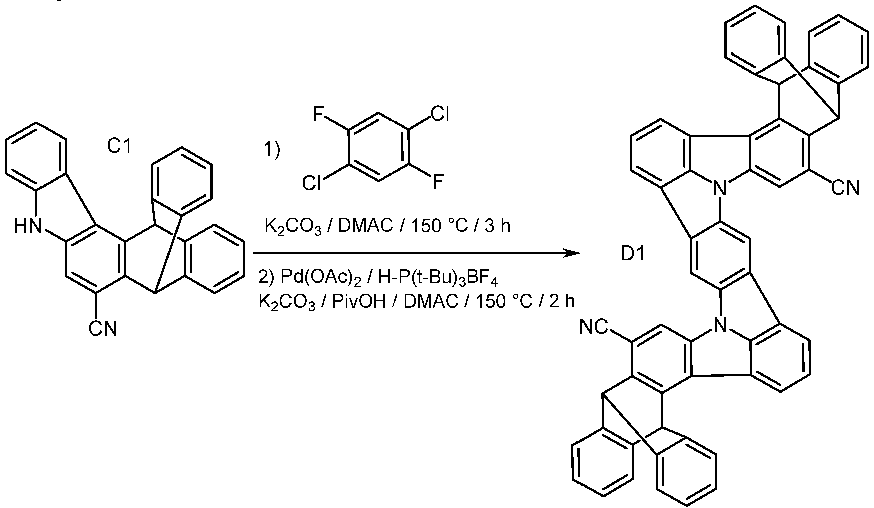

Ein gut gerührtes Gemisch aus 36.8 g (100 mmol) des Carbazols C1, 9.1 g (50 mmol) 1,4-Dichlor-2,5-difluorbenzol [400-05-5, ], 69.1 g (500 mmol) Kaliumcarbonat, 100 g Glaskugeln (3 mm Durchmesser) und 1000 ml Dimethylacetamid (DMAC) wird 3 h bei 150 °C gerührt. Man lässt auf 80 °C erkalten, gibt 3,1 g (30 mmol) Pivalinsäure, 1.16 g (4 mmol) Tri-tert-butylphosphonium-tetrafluroborat und 449 mg (2 mmol) Palladium(II)acetat zu und rührt weitere 2 h bei 150 °C. Man lässt auf 80 °C abkühlen, tropft 2000 ml Wasser zu, saugt vom ausgefallenen Rohrodukt ab, wäscht dieses dreimal mit je 200 ml Wasser und dreimal mit je 200 ml Ethanol und trocknet im Vakuum. Man löst das Rohprodukt in 500 - 1000 ml DCM (bei Pyridinen setzt man 10 Gew.-% Ethylacetat zu), filtriert über ein mit DCM vorschlämmtes Kieselgelbett und entfrent dieses im Vakuum, wobei das abdestillierte DCM gegen Ende durch simultane Zugabe von 300 ml EtOH subsituiert wird. Man saugt von auskristallisierten Produkt ab, wäscht dieses dreimal mit je 50 ml EtOH und trocknet im Vakuum. Die weitere Reinigung erfolgt durch kontinuierliche Heißextraktion (übliche org. Lösungsmittel bzw. deren Kombination, bevorzugt DCM bzw. Acetonitril/DCM 3:1 bis 1:3) bzw. durch Flashchromatographie (CombiFlash Torrent Säulenautomat der Fa. A. Semrau, Kieselgel, RP-Kieselgele, Aluminiumoxid, Laufmittel: Toluol/n-Heptan/Triethylamin, Acetonitril/THF bzw. DCM) und abschließende fraktionierte Sublimation bzw. Tempern im Hochvakuum (typischerweise T ca. 200-400 °C, p ca. 10⁻⁵ bis 10⁻⁶ mbar). Ausbeute: 30.0 g (26 mmol) 52 %; Reinheit: ca. 99.9 % ig nach HPLC.

Werden zwei verschiedene Carbazole C - als Gemisch oder bevorzugt durch sequenzielle Zugabe, d.h. zunächst 50 mmol der ersten Carbazols dann nach einer Reaktionszeit von ca. 2 h 50 mmol des zweiten Cabazols - eingesetzt, können nach chreomatographischer Trennung der möglichen Kupplungs- und Cyclisierungsprodukte, gemischt funktionalisierte erfindungsgemäßen Verbindungen erhalten werden.

Analog können folgende Verbindungen dargestellt werden:

| Bsp. | Edukt | Produkt | Ausbeute |
|---|---|---|---|
| D2 | 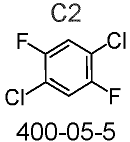 | 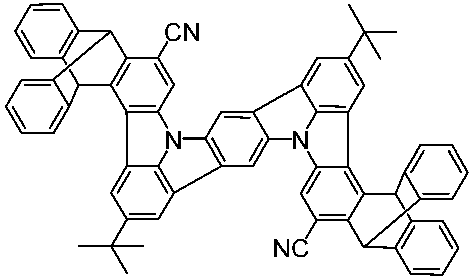 | 50 % |
| D3 | 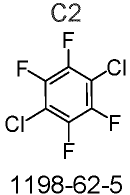 | 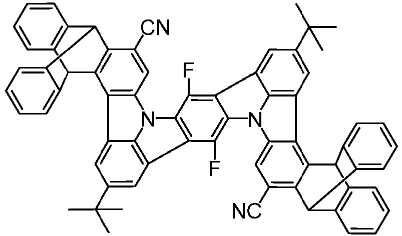 | 23 % |
| D4 | C3 | 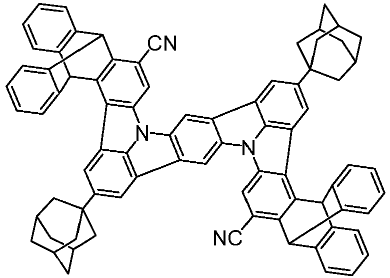 | 55 % |
| | 400-05-5 | | |
| D5 | C4 | 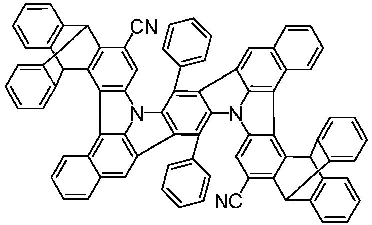 | 39 % |
| | S400 | | |
| D6 | C5 | 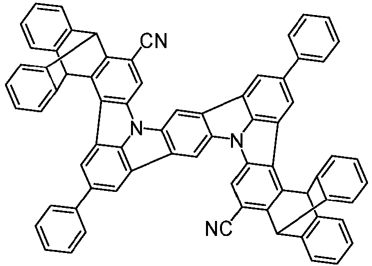 | 61 % |
| | 400-05-5 | | |
| D7 | C6 | 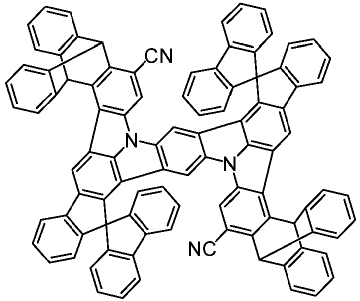 | 21 % |
| | 400-05-5 | | |
| D8 | C7 | 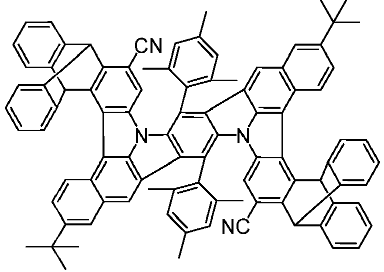 | 26 % |
| | S401 | | |
| D9 | C8 | 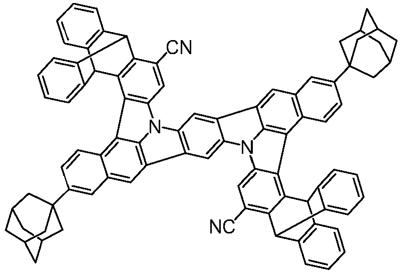 | 57 % |
| | 400-05-5 | | |
| D10 | C9 | 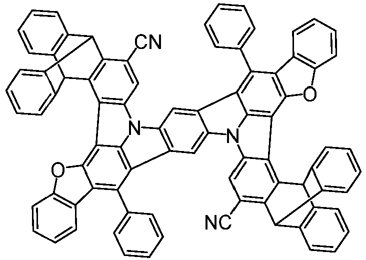 | 30 % |
| | 400-05-5 | | |
| D11 | C10 | 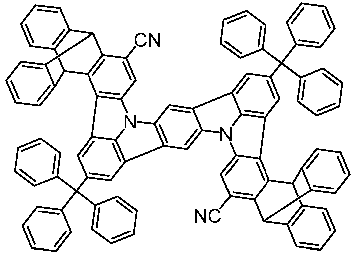 | 54 % |
| | 400-05-5 | | |
| D12 | C11 | 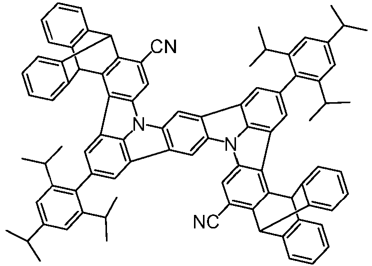 | 58 % |
| | 400-05-5 | | |
| D13 | C12 | 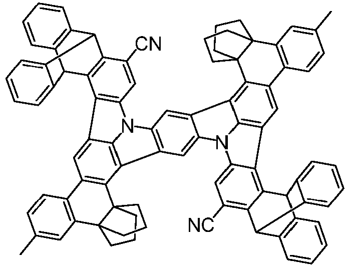 | 27 % |
| | 400-05-5 | | |
| D14 | C13 | 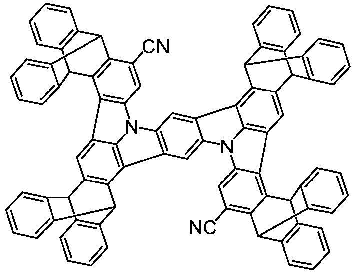 | 32 % |
| | 400-05-5 | | |
| D15 | C14 | 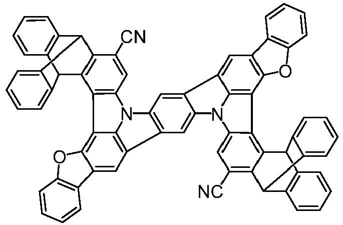 | 56 % |
| | 400-05-5 | | |
| D16 | C15 | 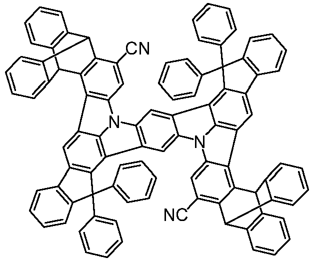 | 30 % |
| | 400-05-5 | | |
| D17 | C16 | 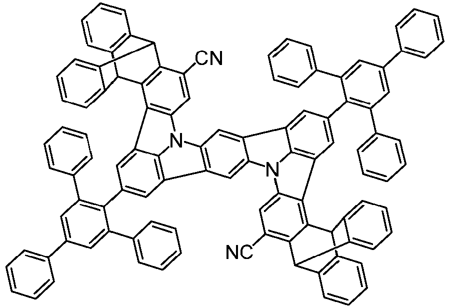 | 48 % |
| | 400-05-5 | | |
| D18 | C17 | 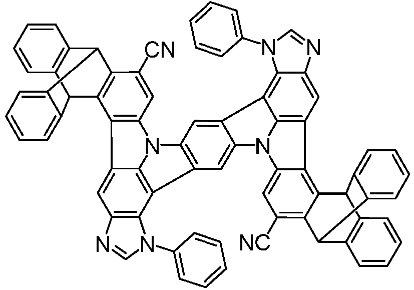 | 19 % |
| | 400-05-5 | | |
| D19 | C18 | 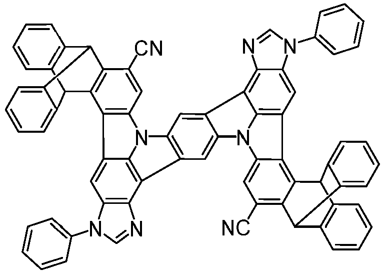 | 21 % |
| | 400-05-5 | | |
| D20 | C19 | 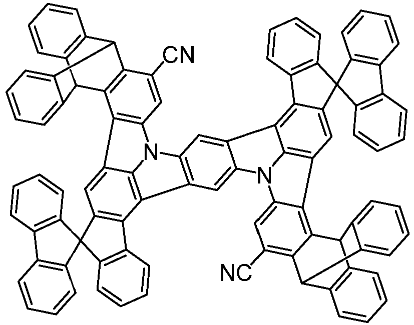 | 20 % |
| | 400-05-5 | | |
| D21 | C20 | 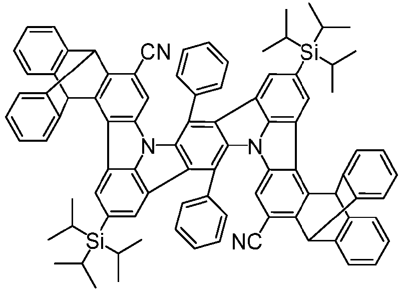 | 17 % |
| | S400 | | |
| D22 | C21 | 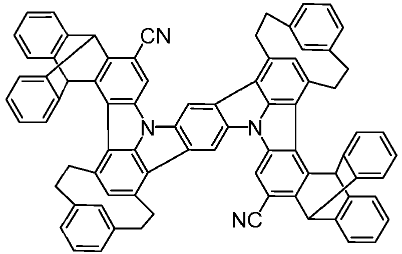 Isomerengemisch | 44 % |
| | 400-05-5 | | |
| D23 | C22 | 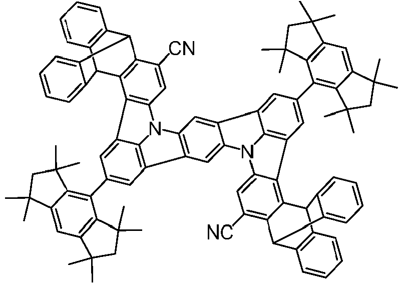 | 49 % |
| | 400-05-5 | | |
| D24 | C23 | 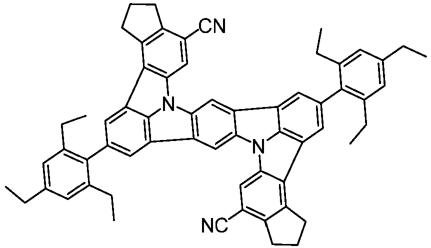 | 57 % |
| | 400-05-5 | | |
| D25 | C24 | 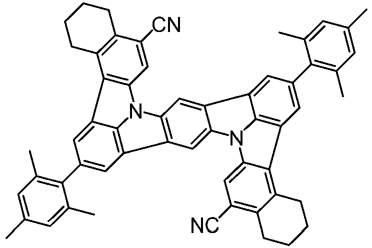 | 59 % |
| | 400-05-5 | | |
| D26 | C25 | 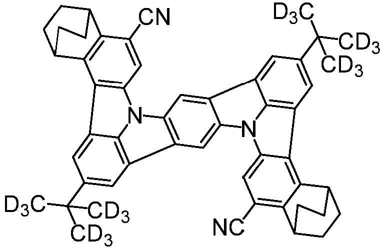 | 58 % |
| | 400-05-5 | | |
| D27 | C26 | 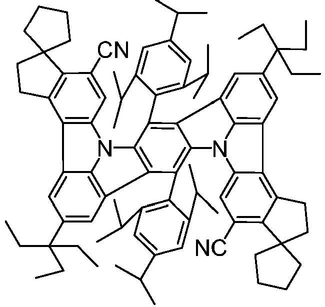 | 13 % |
| | S402 | | |
| D28 | C27 | 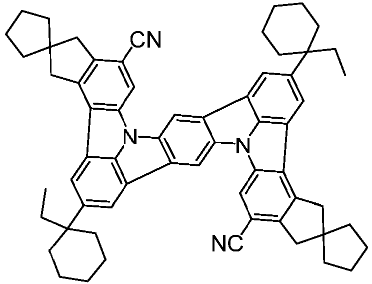 | 49 % |
| | 400-05-5 | | |
| D29 | C28 | 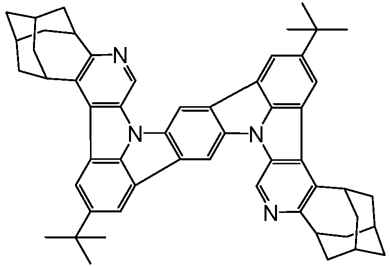 | 55 % |
| | 400-05-5 | | |
| D30 | C28 | 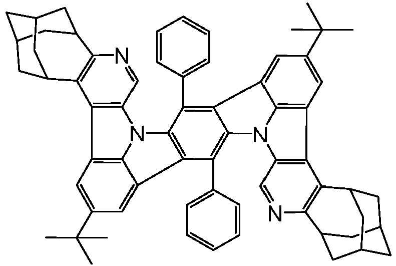 | 59 % |
| | S400 | | |
| D31 | C28 | 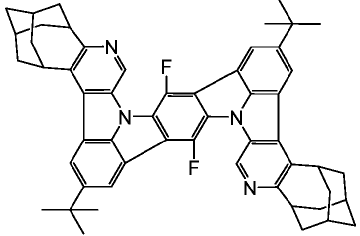 | 19 % |
| | 1198-62-5 | | |
| D32 | C29 | 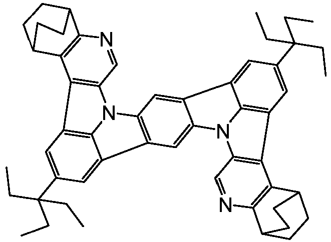 | 60 % |
| | 400-05-5 | | |
| D33 | C30 | 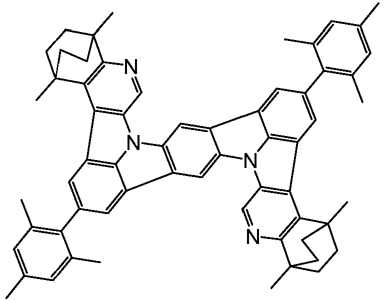 | 62 % |
| | 400-05-5 | | |
| D34 | C31 | 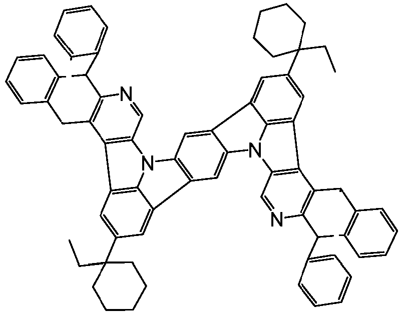 | 57 % |
| | 400-05-5 | | |
| D35 | C32 | 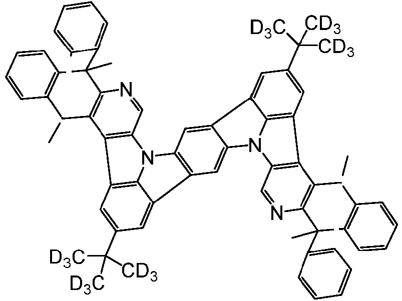 | 55 % |
| | 400-05-5 | | |
| D36 | C33 | 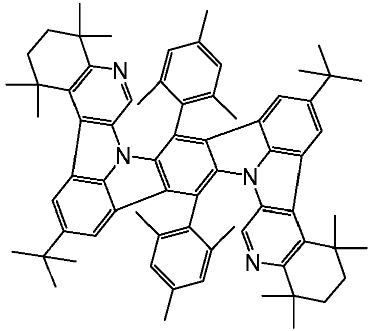 | 43 % |
| | S401 | | |
| D37 | C34 | 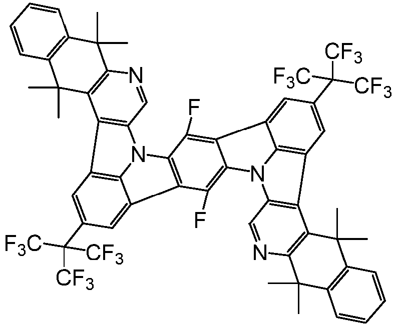 | 46 % |
| | 1198-62-5 | | |
| D38 | C35 | 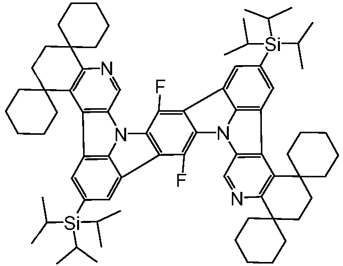 | 40 % |
| | 1198-62-5 | | |
| D39 | C36 | 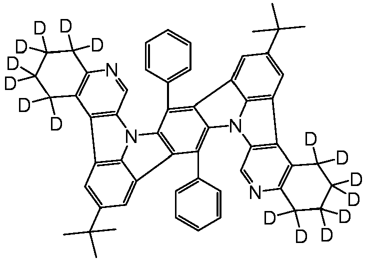 | 57 % |
| | S400 | | |
| D40 | C37 | 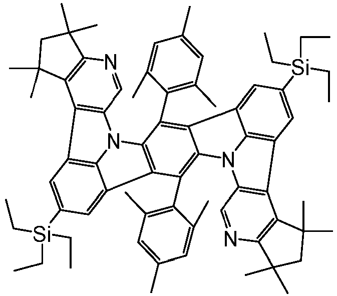 | 38 % |
| | S401 | | |
| D41 | C38 | 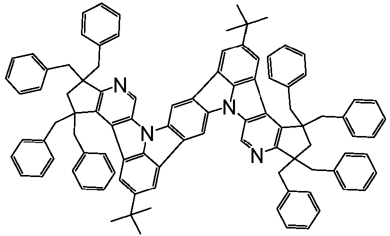 | 50 % |
| | 400-05-5 | | |
| D42 | C39 | 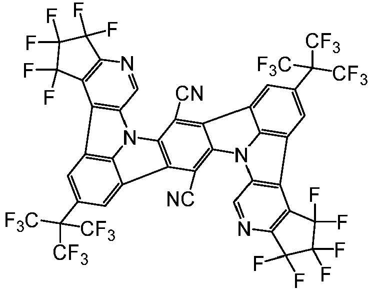 | 17 % |
| | 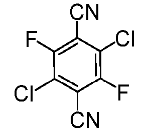 | | |
| | 60341-41-5 | | |
| D100 | C2 | 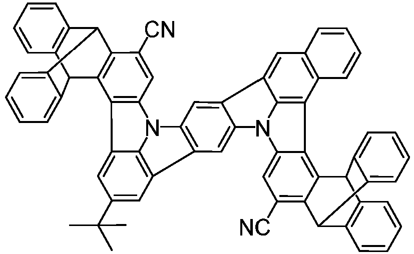 | 27 % |
| | C4 | | |
| | 400-05-5 | | |
| D101 | C2 | 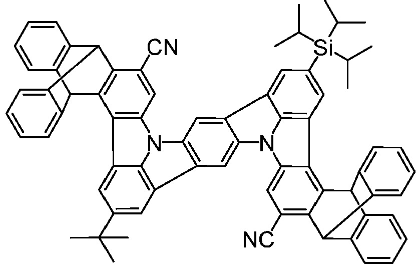 | 24 % |
| | C20 | | |
| | 400-05-5 | | |
| D102 | C14 | 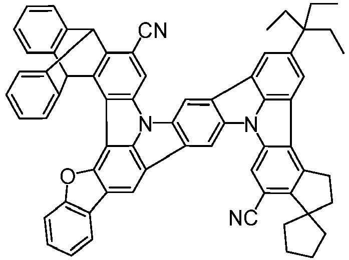 | 25 % |
| | C26 | | |
| | 400-05-5 | | |
| D103 | C13 | 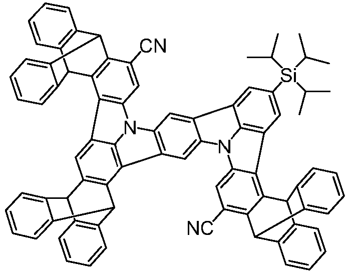 | 20 % |
| | C20 | | |
| | 400-05-5 | | |
| D104 | C25 | 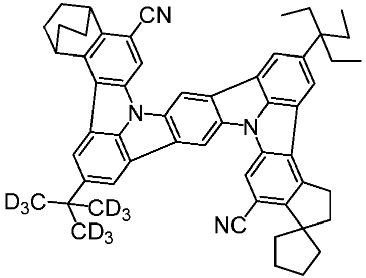 | 22 % |
| | C26 | | |
| | 400-05-5 | | |
| D105 | C26 | 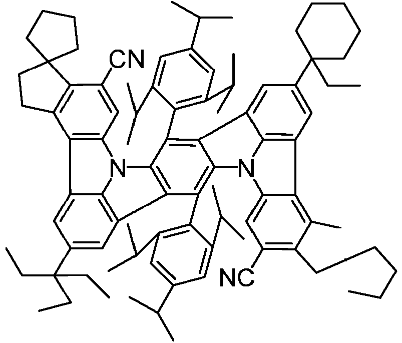 | 24 % |
| | C27 | | |
| | S402 | | |
| D200 | C28 | 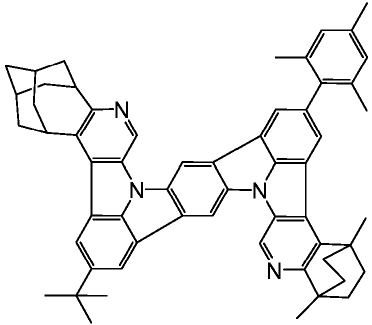 | 30 % |
| | C30 | | |
| | 400-05-5 | | |
| D201 | C28 | 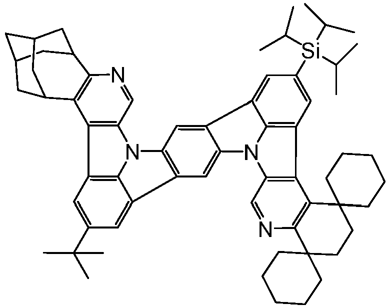 | 18 % |
| | C35 | | |
| | 400-05-5 | | |
| D202 | C30 | 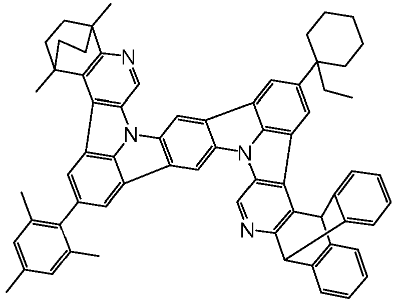 | 27 % |
| | C31 | | |
| | 400-05-5 | | |
| D203 | C32 | 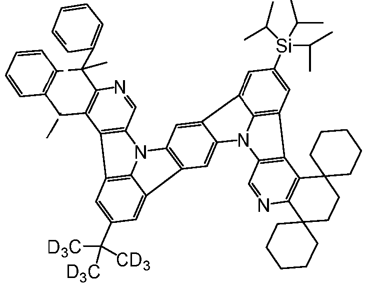 | 21 % |
| | C35 | | |
| | 400-05-5 | | |
| D204 | C37 | 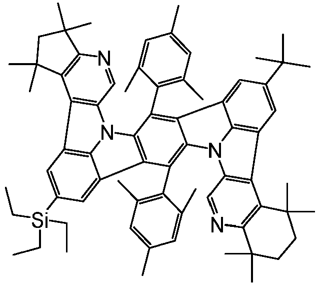 | 25 % |
| | C33 | | |
| | S401 | | |
| D300 | C2 | 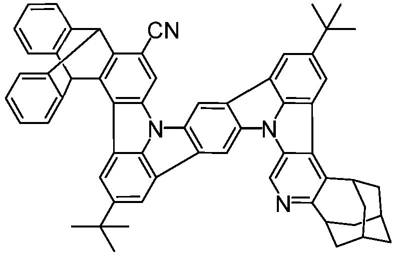 | 23 % |
| | C28 | | |
| | 400-05-5 | | |
| D301 | C3 | 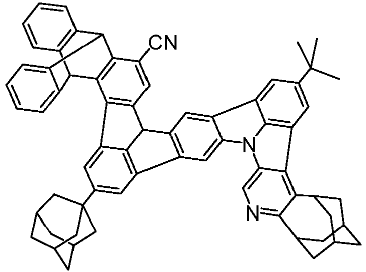 | 24 % |
| | C28 | | |
| | 400-05-5 | | |
| D302 | C5 | 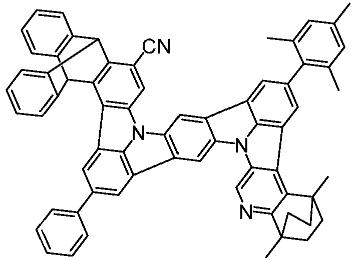 | 26 % |
| | C30 | | |
| | 400-05-5 | | |
| D302 | C7 | 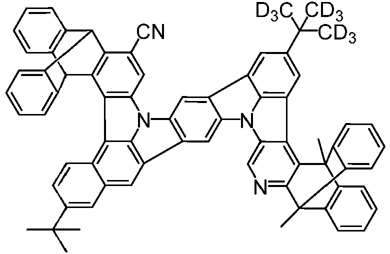 | 23 % |
| | C32 | | |
| | 400-05-5 | | |
| D304 | C25 | 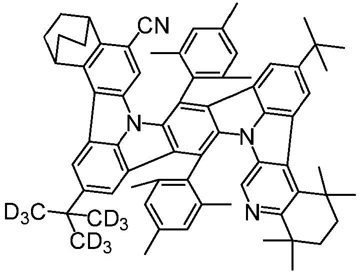 | 26 % |
| | C33 | | |
| | S401 | | |
| D400 | C2 | 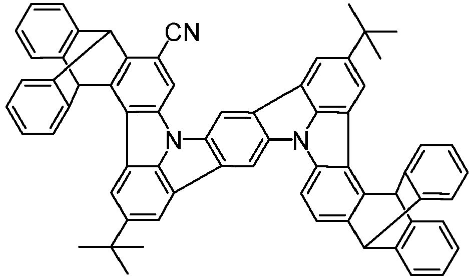 | 27 % |
| | C40 | | |
| | 400-05-5 | | |
| D401 | C2 | 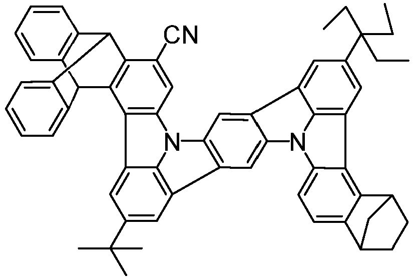 | 31 % |
| | C41 | | |
| | 400-05-5 | | |
| D402 | C20 | 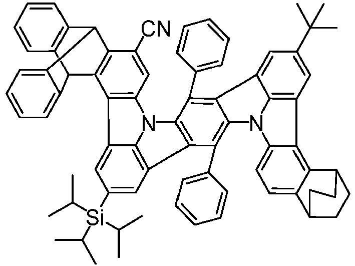 | 25 % |
| | C42 | | |
| | S400 | | |
| D403 | C25 | 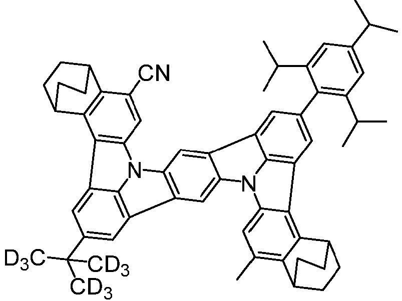 | 25 % |
| | C43 | | |
| | 400-05-5 | | |
| C404 | C26 | 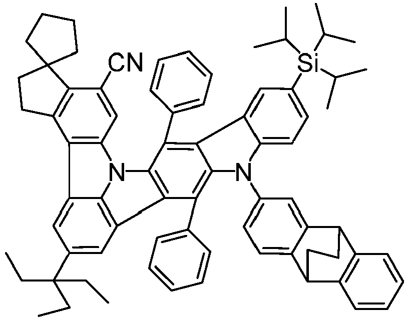 | 19 % |
| | C44 | | |
| | S400 | | |
| D405 | C27 | 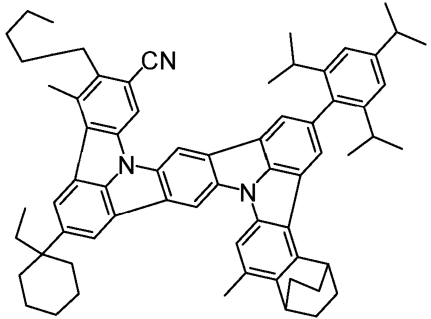 | 26 % |
| | C43 | | |
| | 400-05-5 | | |
| D500 | C28 | 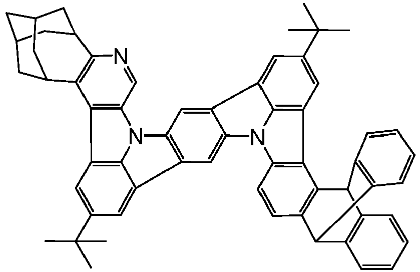 | 25 % |
| | C40 | | |
| | 400-05-5 | | |
| D501 | C30 | 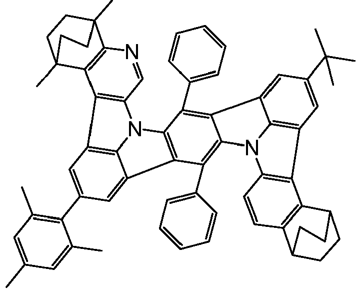 | 28 % |
| | C42 | | |
| | S400 | | |
| D502 | C32 | 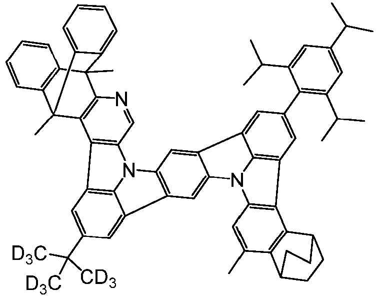 | 27 % |
| | C43 | | |
| | 400-05-5 | | |
| D503 | C35 | 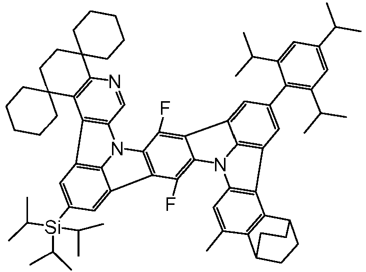 | 19 % |
| | C43 | | |
| | 1198-62-5 | | |
| D600 | C100 | 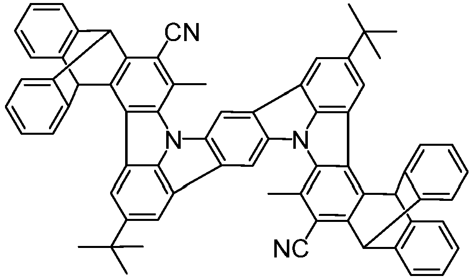 | 41 % |
| | 400-05-5 | | |
| D601 | C101 | 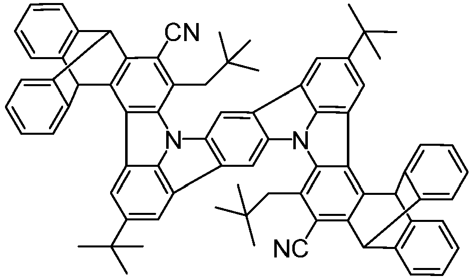 | 36 % |
| | 400-05-5 | | |
| D602 | C102 | 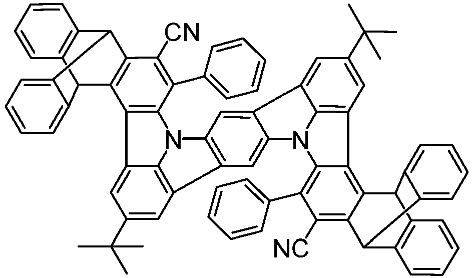 | 31 % |
| | 400-05-5 | | |
| D603 | C103 | 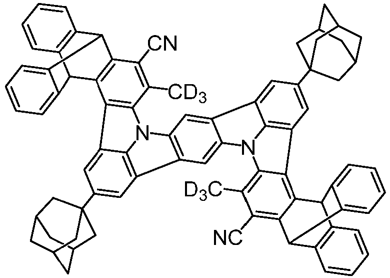 | 44 % |
| | 400-05-5 | | |
| D604 | C104 | 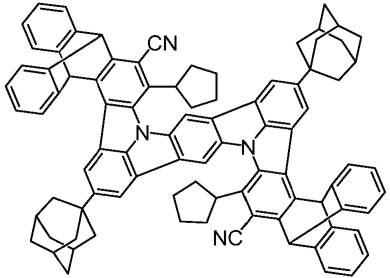 | 30 % |
| | 400-05-5 | | |
| D605 | C105 | 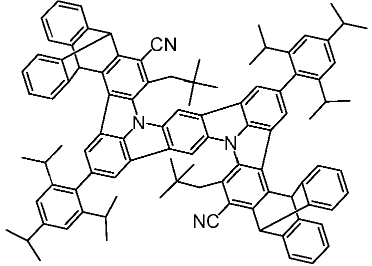 | 31 % |
| | 400-05-5 | | |
| D606 | C106 | 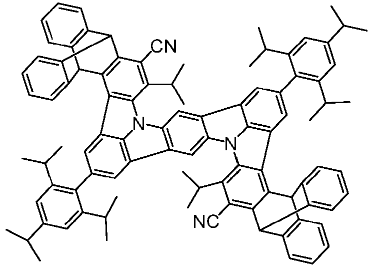 | 37 % |
| | 400-05-5 | | |
| D607 | C107 | 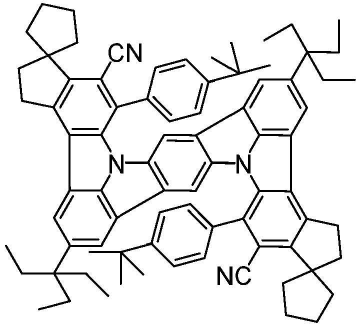 | 28 % |
| | 400-05-5 | | |
| D608 | C108 | 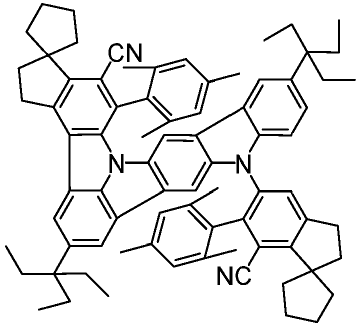 | 18 % |
| | 400-05-5 | | |
| D610 | C100 | 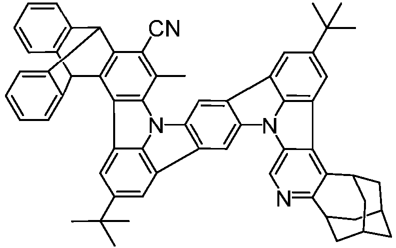 | 22 % |
| | C28 | | |
| | 400-05-5 | | |
| D611 | C101 | 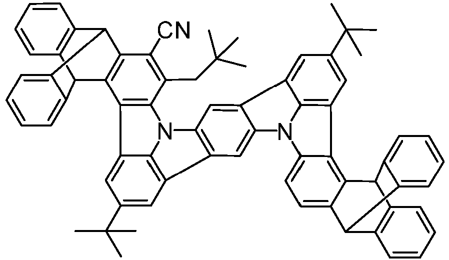 | 19 % |
| | C40 | | |
| | 400-05-5 | | |
| E1 | 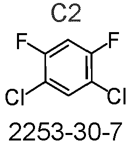 | 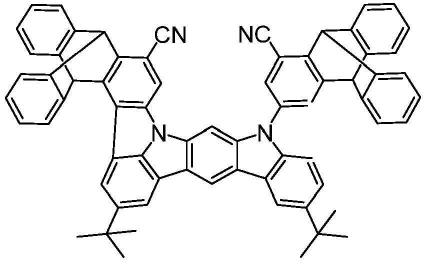 | 48 % |
| E2 | 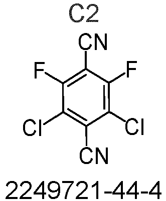 | 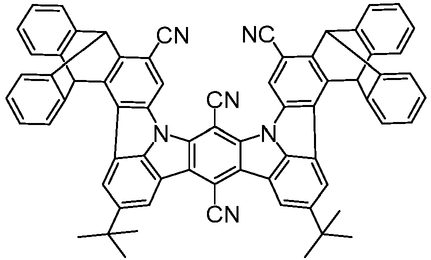 | 40 % |
| E3 | 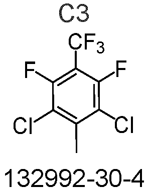 | 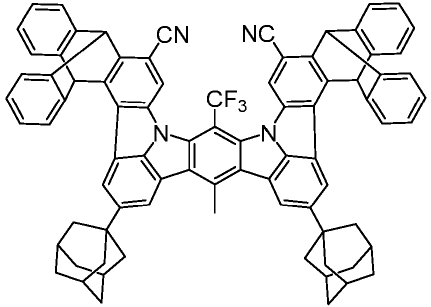 | 27 % |
| E4 | 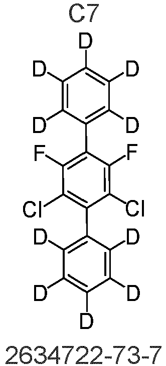 | 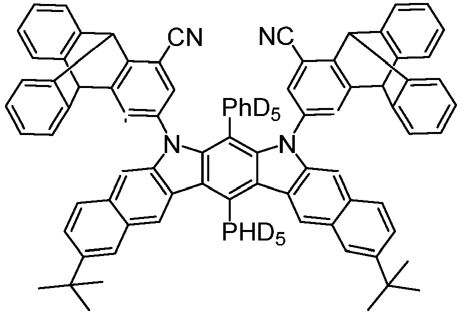 | 33 % |
| E5 | 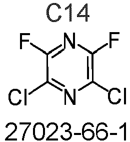 | 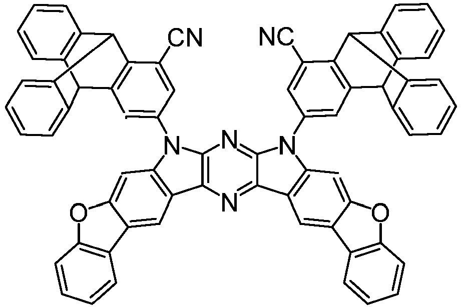 | 67 % |
| E6 | 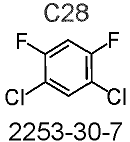 | 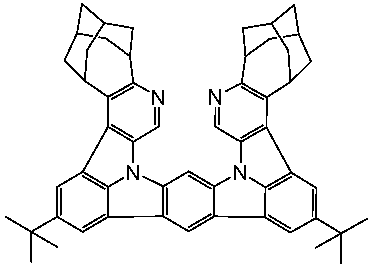 | 65 % |
| F1 | 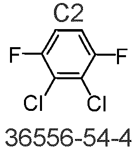 | 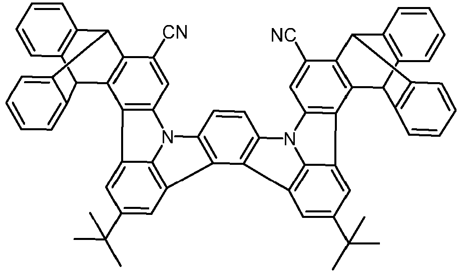 | 56 % |
| F2 | 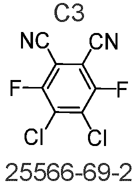 | 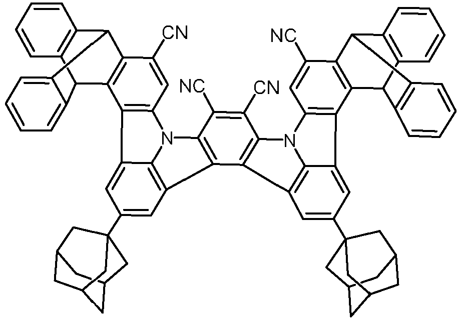 | 53 % |
| F3 | 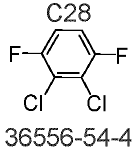 | 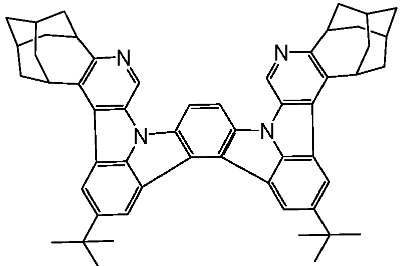 | 57 % |

### Beispiel D700:

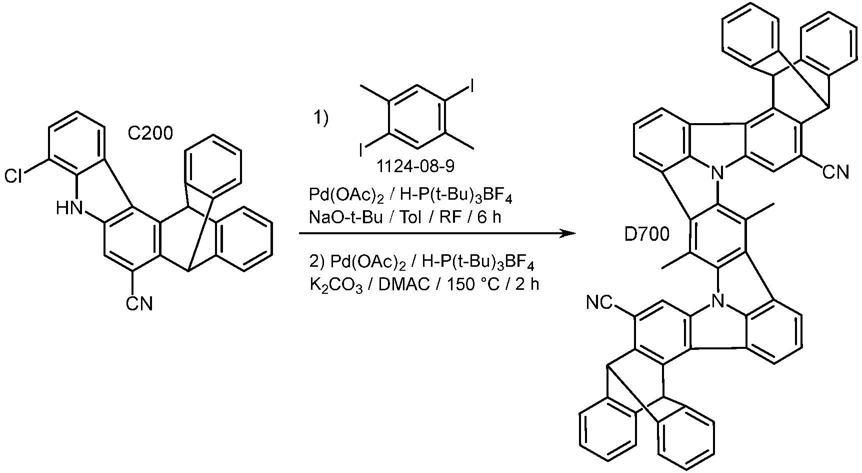

Stufe 1: Doppelte Buchwald-Hartwig-Kupplung, Durchführung analog EP3723149A1, Example 2-5, Intermediate 12. Das Bis-(chlor-carbazol) wird isoliert. Ausbeute: 63 %
Stufe 2: Doppelte Cyclisierung, Durchführung analog Beispiel C1, Stufe 2, Ausbeute 57 %. Anstelle von H-P(t-Bu₃)BF₄ kann H-P(t-Cy₃)BF₄ eingesetzt werden, ein Zusatz von 30 mol % Pivalinsäure wirkt typischerweise ausbeutesteigernd. Alternativ kann die Cyclisierung mit NHC-Pd-Komplexen wie z.B. Allyl-[1,3-bis-(2,6-diisopropylphenyl)-imidazol-2-yliden]-chloropalladium(II) erfolgen, s. z.B. analog T. Kader et al., Chem. Europ. J., 2019, 25(17), 4412 oder analog US 9,000,421 B1, typische Ausbeuten 30 - 80 %.

Analog können folgende Verbindungen dargestellt werden:

| Bsp. | Edukt | Produkt | Ausbeute |
|---|---|---|---|
| D701 | 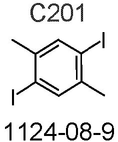 | 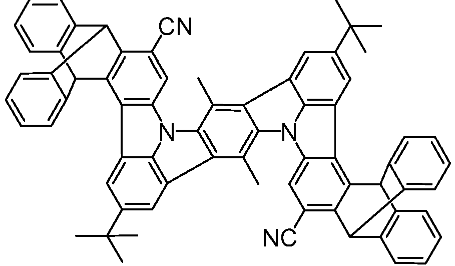 | 38 % |
| D702 | 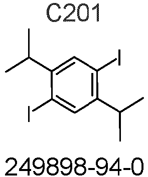 | 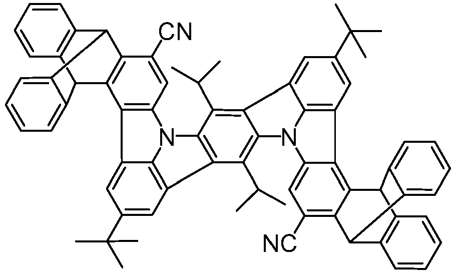 | 27 % |
| D703 | 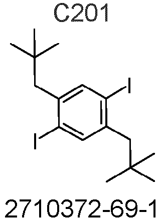 | 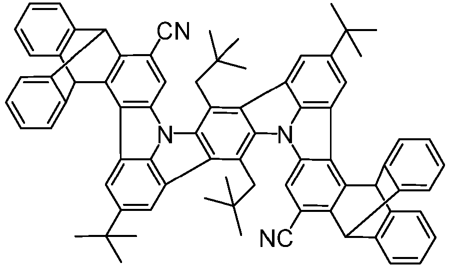 | 33 % |
| D704 | 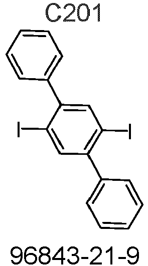 | 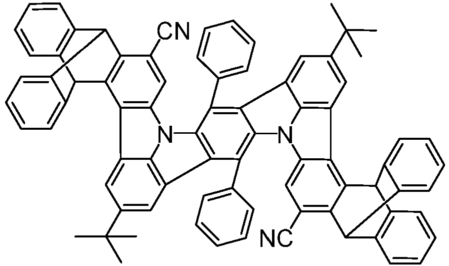 | 20 % |
| D705 | 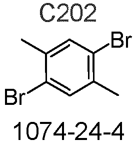 | 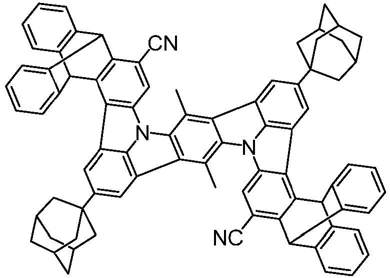 | 32 % |
| D706 | C203 | 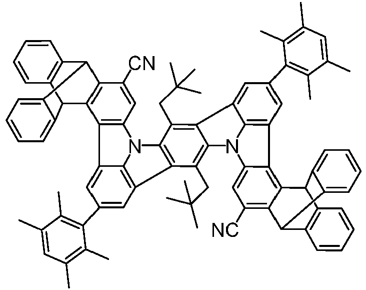 | 34 % |
| | 2710372-69-1 | | |
| D707 | C204 | 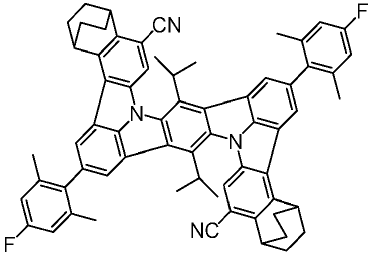 | 29 % |
| | 249898-94-0 | | |
| D708 | C205 | 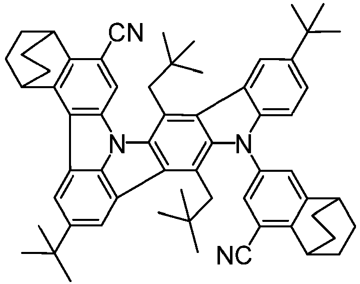 | |
| | 2710372-69-1 | | |
| D709 | C206 | 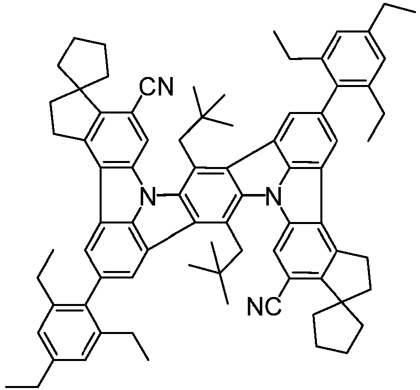 | 38 % |
| | 2710372-69-1 | | |
| D710 | C207 | 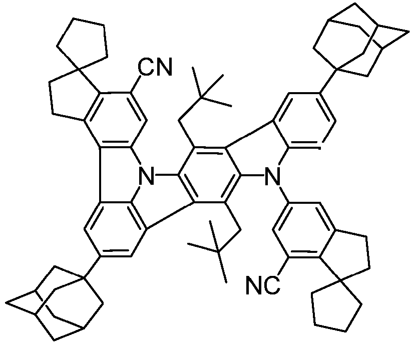 | 31 % |
| | 2710372-69-1 | | |
| D711 | C208 | 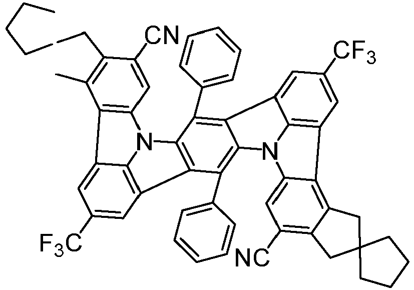 | 19 % |
| | 96843-21-9 | | |

### Beispiel D800:

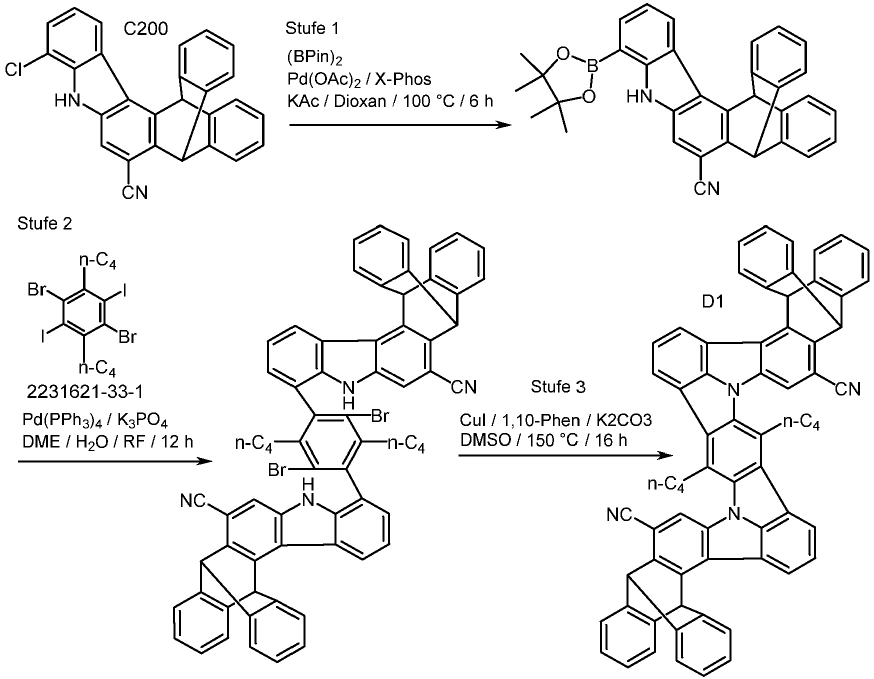

Stufe 1: Pd-katalysierte Borylierung, Durchführung analog EP3723149A1, Example 2-4, Intermediate 10. Ausbeute: 80 %
Stufe 2: Doppelte Suzukikupplung, Durchführung analog EP3723149A1, Example 2-4, Intermediate 11. Ausbeute: 78 %
Stufe 3: Doppelte Ullmann-Reaktion unter Cyclisierung, Durchführung analog EP3723149A1, Example 2-4, BD-6. Ausbeute: 48 %.

Analog können folgende Verbindungen dargestellt werden:

| Bsp. | Edukt | Produkt | Ausbeute |
|---|---|---|---|
| D801 | 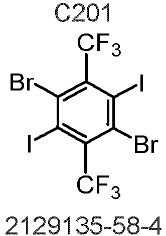 | 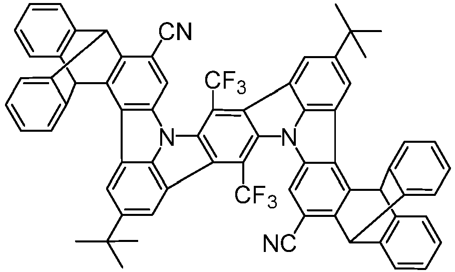 | 35 % |
| D802 | 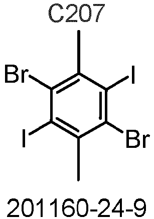 | 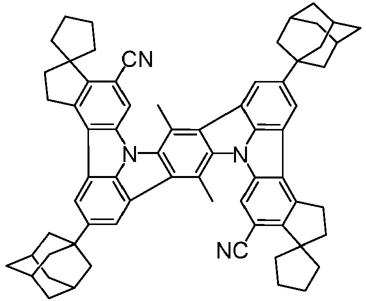 | 23 % |
| D803 | C205 | 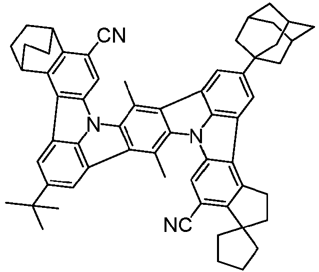 | 21 % |
| | C207 | | |
| | 201160-24-9 | | |

### Beispiel D900:

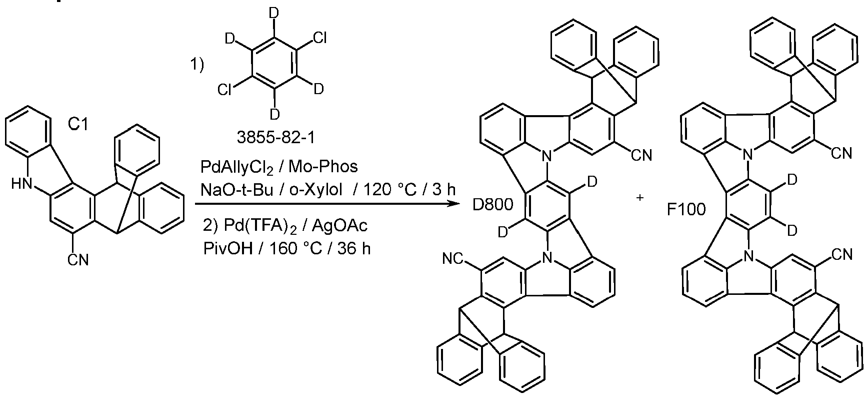

Stufe 1 & 2: Durchführung analog Taisei Taniguchi et al., Chem. Lett. 2019, 48, 1160. Ausbeute: 21 % D800; 8 % F100.

Analog können folaende Verbindunaen daraestellt werden:

| Bsp. | Edukt | Produkt | Ausbeute |
|---|---|---|---|
| D901 | C2 | 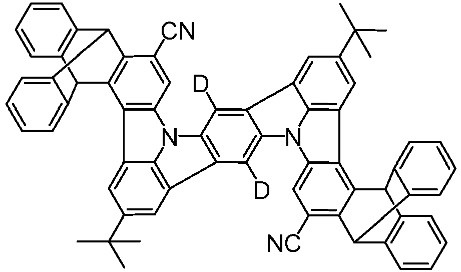 | 26 % |
| | 3855-82-1 | | |
| F101 | C2 | 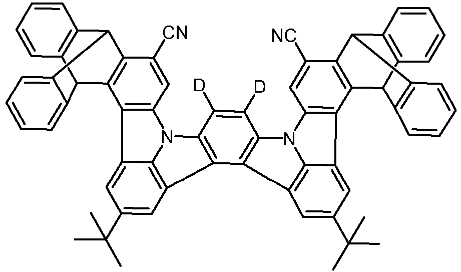 | 16 % |
| | 3855-82-1 | | |
| D902 | C11 | 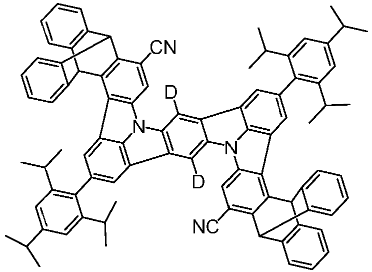 | 22 % |
| | 3855-82-1 | | |
| F102 | C11 | 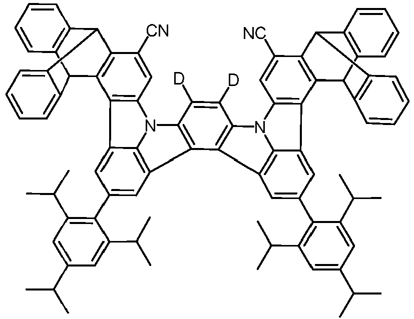 | 14 % |
| | 3855-82-1 | | |
| D903 | C28 | 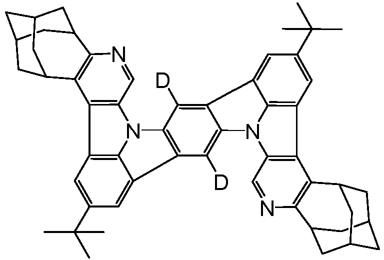 | 26 % |
| | 3855-82-1 | | |
| F103 | C28 | 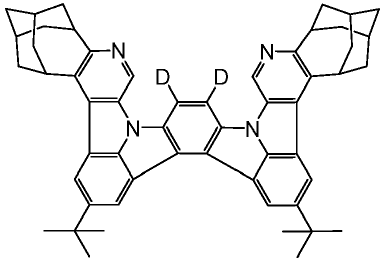 | 17 % |
| | 3855-82-1 | | |

### Messung von Photolumineszenzspektren (PL-Spektren):

Abbildung 1 zeigt das PL-Spektrum der erfindungsgemäßen Verbindungen D2, gemessen mit einem PL-Spektrometer der Fa. Hitachi, F-4500 PL, in ca. 10⁻⁵ molarer, entgaster Toluol-Lösung bei Raumtemperatur (ca. 25 °C).

Das in Abbildung 1 gezeigte Spektrum weist folgende Daten auf:
PLmax: 453 nm, FWHM: 16.3 nm, 0.098 eV

Die PL-Spektren weisen sehr schmale Emissionsbanden mit geringen FWHM Werten (typischerweise < 0.15 eV) auf und führen zu besonders farbreiner Emission. Außerdem zeigen sie in der langwelligen Emissionsflanke eine Schulter bzw. ein Nebenmaximum, die weniger als 30 %, der Intensität des Hauptmaximums aufweisen. Dies führt in Top-Emission OLED-Bauteilen zu einer günstig geringen Blickwinkelabhängigkeit des Farbeindrucks im Vergleich zu schmalbandigen Bor-enthaltenden Emittern nach Stand der Technik, die oftmals keine derartigen Schultern bzw. Nebenmaxima aufweisen und eine größere Blickwinkelabhängigkeit des Farbeindrucks zeigen.

### Herstellung von OLED-Bauteilen

### 1) Vakuum-prozessierte Bauteile

Die erfindungsgemäßen Verbindungen lassen sich unter anderem als Dotand in der Emissionsschicht in Fluoreszenz- und in Hyperphosphoreszenz-OLED-Bauteilen einsetzen.

Die Herstellung von erfindungsgemäßen OLEDs *(organic light emitting* diodes) sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylene-dioxythiophene)poly(styrenesulfonate), bezogen als CLEVIOS^{™} P VP AI 4083 von Heraeus Precious Metals GmbH Deutschland, aus wässriger Lösung aufgeschleudert) und anschließend bei 180°C 10 min. lang ausgeheizt. Diese beschichteten Glasplättchen bilden die Substrate, auf welche die OLEDs aufgebracht werden. Nach der Herstellung werden die OLEDs zum Schutz gegen Sauerstoff und Wasserdampf verkapselt. Der genaue Schichtaufbau der elektrolumineszierenden OLEDs ist den Beispielen zu entnehmen. Die zur Herstellung der OLEDs benötigten Materialien sind in Tabelle 8 gezeigt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 100 bzw. 1000 cd/m² bestimmt und daraus die Emissionsfarbe und die EL-FWHM-Werte (**E**Lectroluminescence - **F**ull **W**idth **H**alf **M**aximum - Breite der EL-Emissionsspektren auf halber Peakhöhe in eV, zur besseren Vergleichbarkeit über den gesamten Spektralbereich) entnommen.

### Fluoreszenz-OLED- Bauteile:

Alle Materialien werden in einer Vakuumkammer thermisch aufgedampft. Dabei besteht die Emissionsschicht (EML) immer aus mindestens einem Matrixmaterial (Hostmaterial, Wirtsmaterial) SMB und einem emittierenden Dotierstoff (Dotand, Emitter) ES bzw. EAS, der dem Matrixmaterial bzw. den Matrixmaterialien durch Co-Verdampfung in einem bestimmten Volumenanteil beigemischt wird. Eine Angabe wie SMB:ES bzw. EAS (97:3%) bedeutet hierbei, dass das Material SMB in einem Volumenanteil von 97% und der Dotand ES bzw. EAS in einem Anteil von 3% in der Schicht vorliegt. Analog kann auch die Elektronentransportschicht aus einer Mischung zweier Materialien bestehen, z.B. wie hier aus ETM1 (50%) und ETM2 (50%), s. Tabelle 1. Die zur Herstellung der OLEDs verwendeten Materialien sind in Tabelle 8 gezeigt. Als Vergleich wird die Verbindungen Ref.-D1, s. Tabelle 8 verwendet.

### Blaue Fluoreszenz-OLED- Bauteile BF:

Die OLEDs haben prinzipiell folgenden Schichtaufbau:
Substrat
- Lochinjektionsschicht 1 (HIL1) aus HTM1 dotiert mit 5 % NDP-9 (kommerziell erhältlich von der Fa. Novaled), 20 nm
- Lochtransportschicht 1 (HTL1) aus HTM1, 160 nm
- Lochtransportschicht 2 (HTL2), s. Tabelle 1
- Emissionsschicht (EML), s. Tabelle 1
- Elektronentransportschicht (ETL2), s. Tabelle 1
- Elektronentransportschicht (ETL1) aus ETM1 (50%) und ETM2 (50%), 30 nm Elektroneninjektionsschicht (EIL) aus ETM2, 1 nm
- Kathode aus Aluminium, 100 nm

**Tabelle 1: Aufbau Blaue Fluoreszenz-OLED- Bauteile**

| **Bsp.** | **HTL2** | **EML** | **ETL2** |
|---|---|---|---|
| **Ref-BF1** | HTM2 | SMB1:Ref.-D1 (97:3%) | ETM1 |
| | 10 nm | 20 nm | 10 nm |
| **BF1** | HTM2 | SMB1:D2 (97:3%) | ETM1 |
| | 10 nm | 20 nm | 10 nm |
| **BF2** | HTM2 | SMB2:D2 (95:5%) | ETM1 |
| | 10 nm | 20 nm | 10 nm |
| **BF3** | HTM2 | SMB3:D2 (97:3%) | ETM1 |
| | 10 nm | 20 nm | 10 nm |
| **BF4** | HTM2 | SMB1:D3 (97:3%) | ETM1 |
| | 10 nm | 20 nm | 10 nm |
| **BF5** | HTM2 | SMB1:D4 (95:5%) | ETM1 |
| | 10 nm | 20 nm | 10 nm |
| **BF6** | HTM2 | SMB1:D6 (97:3%) | ETM1 |
| | 10 nm | 20 nm | 10 nm |
| **BF7** | HTM2 | SMB1:D7 (98:2%) | ETM1 |
| | 10 nm | 20 nm | 10 nm |
| **BF8** | HTM2 | SMB1:D12 (97:3%) | ETM1 |
| | 10 nm | 20 nm | 10 nm |
| **BF9** | HTM2 | SMB1:D15 (97:3%) | ETM1 |
| | 10 nm | 20 nm | 10 nm |
| **BF10** | HTM2 | SMB1:D18 (97:3%) | ETM1 |
| | 10 nm | 20 nm | 10 nm |
| **BF11** | HTM2 | SMB1:D21 (97:3%) | ETM1 |
| | 10 nm | 20 nm | 10 nm |
| **BF12** | HTM2 | SMB1:D25 (96:4%) | ETM1 |
| | 10 nm | 20 nm | 10 nm |
| **BF13** | HTM2 | SMB1:D26 (96:4%) | ETM1 |
| | 10 nm | 20 nm | 10 nm |
| **BF14** | HTM2 | SMB1:D28 (97:3%) | ETM1 |
| | 10 nm | 20 nm | 10 nm |
| **BF15** | HTM2 | SMB1:D29 (97:3%) | ETM1 |
| | 10 nm | 20 nm | 10 nm |
| **BF16** | HTM2 | SMB1:D30 (97:3%) | ETM1 |
| | 10 nm | 20 nm | 10 nm |
| **BF17** | HTM2 | SMB1:D100 (97:3%) | ETM1 |
| | 10 nm | 20 nm | 10 nm |
| **BF18** | HTM2 | SMB1:D101 (95:5%) | ETM1 |
| | 10 nm | 20 nm | 10 nm |
| **BF19** | HTM2 | SMB1:D102 (97:3%) | ETM1 |
| | 10 nm | 20 nm | 10 nm |
| **BF20** | HTM2 | SMB1:D200 (97:3%) | ETM1 |
| | 10 nm | 20 nm | 10 nm |
| **BF21** | HTM2 | SMB1:D300 (97:3%) | ETM1 |
| | 10 nm | 20 nm | 10 nm |
| **BF22** | HTM2 | SMB1:D400 (97:3%) | ETM1 |
| | 10 nm | 20 nm | 10 nm |
| **BF23** | HTM2 | SMB1:D500 (97:3%) | ETM1 |
| | 10 nm | 20 nm | 10 nm |
| **BF24** | HTM2 | SMB1:D600 (97:3%) | ETM1 |
| | 10 nm | 20 nm | 10 nm |
| **BF25** | HTM2 | SMB1:D601 (97:3%) | ETM1 |
| | 10 nm | 20 nm | 10 nm |
| **BF26** | HTM2 | SMB1:D604 (97:3%) | ETM1 |
| | 10 nm | 20 nm | 10 nm |
| **BF27** | HTM2 | SMB1:D701 (97:3%) | ETM1 |
| | 10 nm | 20 nm | 10 nm |
| **BF28** | HTM2 | SMB1:D703 (97:3%) | ETM1 |
| | 10 nm | 20 nm | 10 nm |
| **BF29** | HTM2 | SMB1:D704 (97:3%) | ETM1 |
| | 10 nm | 20 nm | 10 nm |
| **BF30** | HTM2 | SMB1:D43 (97:3%) | ETM1 |
| | 10 nm | 20 nm | 10 nm |
| **BF31** | HTM2 | SMB1:D46 (97:3%) | ETM1 |
| | 10 nm | 20 nm | 10 nm |
| **BF32** | HTM2 | SMB1:D48 (97:3%) | ETM1 |
| | 10 nm | 20 nm | 10 nm |
| **BF33** | HTM2 | SMB1:D749 (97:3%) | ETM1 |
| | 10 nm | 20 nm | 10 nm |

**Tabelle 2: Ergebnisse**

| **Bsp.** | **EQE (%)** | **Spannung (V)** | **Farbe** | **EL-FWHM** |
|---|---|---|---|---|
| | **1000 cd/m²** | **1000 cd/m²** | | **[eV]** |
| **Ref-BF1** | 3.3 | 5.2 | Tiefblau | 0.18 |
| **BF1** | 8.7 | 4.3 | Blau | 0.13 |
| **BF2** | 8.5 | 4.3 | Blau | 0.14 |
| **BF3** | 8.6 | 4.2 | Blau | 0.14 |
| **BF4** | 8.1 | 4.2 | Blau | 0.13 |
| **BF5** | 7.9 | 4.1 | Blau | 0.14 |
| **BF6** | 8.0 | 4.2 | Blau | 0.14 |
| **BF7** | 8.3 | 4.4 | Blau | 0.13 |
| **BF8** | 8.2 | 4.2 | Blau | 0.14 |
| **BF9** | 8.3 | 4.2 | Blau | 0.13 |
| **BF10** | 7.4 | 4.4 | Blau | 0.15 |
| **BF11** | 8.5 | 4.3 | Blau | 0.14 |
| **BF12** | 7.9 | 4.2 | Blau | 0.15 |
| **BF13** | 7.8 | 4.3 | Blau | 0.15 |
| **BF14** | 8.3 | 4.2 | Blau | 0.15 |
| **BF15** | 7.3 | 4.3 | Blau | 0.15 |
| **BF16** | 7.1 | 4.4 | Blau | 0.14 |
| **BF17** | 8.8 | 4.1 | Hellblau | 0.15 |
| **BF18** | 8.1 | 4.3 | Blau | 0.14 |
| **BF19** | 8.0 | 4.3 | Blau | 0.14 |
| **BF20** | 7.0 | 4.4 | Blau | 0.15 |
| **BF21** | 7.3 | 4.3 | Blau | 0.13 |
| **BF22** | 7.8 | 4.2 | Blau | 0.15 |
| **BF23** | 7.1 | 4.3 | Blau | 0.15 |
| **BF24** | 8.4 | 4.2 | Blau | 0.14 |
| **BF25** | 8.1 | 4.3 | Blau | 0.15 |
| **BF26** | 8.5 | 4.3 | Blau | 0.14 |
| **BF27** | 8.7 | 4.2 | Blau | 0.15 |
| **BF28** | 8.2 | 4.3 | Blau | 0.15 |
| **BF29** | 8.0 | 4.3 | Blau | 0.14 |
| **BF30** | 8.4 | 4.2 | Blau | 0.15 |
| **BF31** | 8.9 | 4.1 | Blau | 0.14 |
| **BF32** | 8.7 | 4.2 | Blau | 0.13 |
| **BF33** | 9.0 | 4.2 | Blau | 0.14 |

### Hyperphosphoreszenz-OLED-Bauteile:

Alle Materialien werden in einer Vakuumkammer thermisch aufgedampft. Dabei besteht die Emissionsschicht (EML) bzw. die Emissionsschichten immer aus mindestens einem Matrixmaterial (Hostmaterial, Wirtsmaterial) TMM, einem (phosphoreszierenden) Sensitizer PS und einem fluoreszierenden Emitter ES bzw. EAS. Das Matrixmaterial (Hostmaterial, Wirtsmaterial) TMM kann aus zwei Komponenten bestehen, die als Mischung verdampft werden (premixed Host, z.B. TMM2), die Komponenten und die Zusammensetzung wird ebenfalls in Tabelle 8 gezeigt. Sensitizer PS und fluoreszierender Emitter ES bzw. EAS werden dem Hostmaterial TMM durch Coverdampfung in einem bestimmten Volumenanteil beigemischt. Eine Angabe wie TMM:PS(5%):ES bzw. EAS(3%) bedeutet hierbei, dass das Material TMM in einem Volumenanteil von 92%, PS in einem Anteil von 5% und ES bzw. EAS in einem Anteil von 3% in der Schicht vorliegt.

### Blaue Hyperphosphoreszenz-OLED-Bauteile BH:

Die OLEDs haben prinzipiell folgenden Schichtaufbau:
- Substrat
- Lochinjektionsschicht 1 (HIL1) aus HTM2 dotiert mit 5 % NDP-9 (kommerziell erhältlich von der Fa. Novaled), 20 nm
- Lochtransportschicht 1 (HTL1) aus HTM2, 30 nm
- Lochtransportschicht 2 (HTL2), s. Tabelle 3
- Emissionsschicht (EML), s. Tabelle 3
- Elektronentransportschicht (ETL2), s. Tabelle 3
- Elektronentransportschicht (ETL1) aus ETM1 (50%) und ETM2 (50%), 20 nm
- Elektroneninjektionsschicht (EIL) aus ETM2, 1 nm
- Kathode aus Aluminium, 100 nm

**Tabelle 3: Aufbau Blaue Hyperphosphoreszenz-OLED-Bauteile**

| **Bsp.** | **HTL2** | **EML** | **ETL2** |
|---|---|---|---|
| **BH1** | HTM3 | TMM1:PS1(7%):D4 (2%) | ETM3 |
| | 10 nm | 25 nm | 10 nm |
| **BH2** | HTM3 | TMM1:PS1(7%):D21 (2%) | ETM3 |
| | 10 nm | 25 nm | 10 nm |
| **BH3** | HTM3 | TMM1:PS1(7%):D25 (2%) | ETM3 |
| | 10 nm | 25 nm | 10 nm |
| **BH4** | HTM3 | TMM1:PS1(7%):D35 (2%) | ETM3 |
| | 10 nm | 25 nm | 10 nm |
| **BH5** | HTM3 | TMM1:PS1(7%):D101 (2%) | ETM3 |
| | 10 nm | 25 nm | 10 nm |
| **BH6** | HTM3 | TMM1:PS1(7%):D49 (2%) | ETM3 |
| | 10 nm | 25 nm | 10 nm |

**Tabelle 4: Eraebnisse**

| **Bsp.** | **EQE (%) 100 cd/m²** | **Spannung (V) 100 cd/m²** | **Farbe** | **EL-FWHM [eV]** |
|---|---|---|---|---|
| **BH1** | 19.2 | 3.4 | Blau | 0.15 |
| **BH2** | 19.9 | 3.5 | Blau | 0.15 |
| **BH3** | 19.8 | 3.3 | Blau | 0.15 |
| **BH4** | 16.2 | 3.4 | Blau | 0.16 |
| **BH5** | 20.3 | 3.3 | Blau | 0.15 |
| **BH6** | 19.0 | 3.4 | Blau | 0.16 |

### Grüne Hyperphosphoreszenz-OLED-Bauteile GH:

Die OLEDs haben prinzipiell folgenden Schichtaufbau:
- Substrat
- Lochinjektionsschicht 1 (HIL1) aus HTM2 dotiert mit 5 % NDP-9 (kommerziell erhältlich von der Fa. Novaled), 20 nm
- Lochtransportschicht 1 (HTL1) aus HTM2, 30 nm
- Lochtransportschicht 2 (HTL2), s. Tabelle 5
- Emissionsschicht (EML), s. Tabelle 5
- Elektronentransportschicht (ETL2), s. Tabelle 5
- Elektronentransportschicht (ETL1) aus ETM1 (50%) und ETM2 (50%), 30 nm
- Elektroneninjektionsschicht (EIL) aus ETM2, 1 nm
- Kathode aus Aluminium, 100 nm

**Tabelle 5: Aufbau Grüne Hyperphosphoreszenz-OLED-Bauteile**

| **Bsp.** | **HTL2** | **EML** | **ETL2** |
|---|---|---|---|
| **GH1** | HTM3 | TMM1:PS1(8%):D9 (2%) | ETM3 |
| | 10 nm | 25 nm | 10 nm |
| **GH2** | HTM3 | TMM1:PS1(8%):D19 (3%) | ETM3 |
| | 10 nm | 25 nm | 10 nm |

**Tabelle 6: Eraebnisse**

| **Bsp.** | **EQE (%) 100 cd/m²** | **Spannung (V) 100 cd/m²** | **Farbe** | **EL-FWHM [eV]** |
|---|---|---|---|---|
| **GH1** | 20.5 | 3.2 | Grün | 0.15 |
| **GH2** | 23.7 | 3.1 | Grün | 0.15 |

### 2) Lösungs-prozessierte Bauteile:

Die Herstellung lösungsbasierter OLEDs ist in der Literatur grundsätzlich beschrieben, z.B. in der WO 2004/037887 und der WO 2010/097155. Bei den folgenden Beispielen wurden beide Herstellungsverfahren (Aufbringung aus Gasphase und Lösungsprozessierung) kombiniert, so dass bis einschließlich Emissionsschicht aus Lösung prozessiert wurde und die darauffolgenden Schichten (Lochblockierschicht / Elektronentransportschicht) im Vakuum aufgedampft wurden. Die vorbeschriebenen allgemeinen Verfahren werden dafür wie folgt auf die hier beschriebenen Gegebenheiten (Schichtdickenvariation, Materialien) angepasst und kombiniert.

Der verwendete Aufbau ist damit wie folgt:
- Substrat
- ITO, 50 nm
- PEDOT, 20 nm
- Lochtransportschicht HIL-Sol, aus HTM-Sol, 20 nm
- Emissionsschicht aus SMB4(97%) und ES(3%) bzw. EAS(3%), 50 nm
- Elektronentransportschicht (ETL1) aus ETM1 (50%) und ETM2 (50%), 25 nm
- Kathode aus Aluminium, 100 nm

Als Substrat werden Glasplättchen, die mit strukturiertem ITO (Indium-Zinn-Oxid) der Dicke 50 nm beschichtet sind, verwendet. Zur besseren Prozessierung werden diese mit dem Buffer (PEDOT) Clevios P VP Al 4083 (Heraeus Clevios GmbH, Leverkusen) beschichtet oben steht PEDOT. Das Aufschleudern erfolgt an Luft aus Wasser. Die Schicht wird anschließend für 10 Minuten bei 180°C ausgeheizt. Auf die so beschichteten Glasplättchen werden die Lochtransportschicht sowie die Emissionsschicht aufgebracht. Bei der Lochtransportschicht handelt es sich um das Polymer HTM-Sol der in Tabelle 8 gezeigten Struktur, das gemäß WO2010/097155 synthetisiert wurde. Das Polymer wird in Toluol gelöst, so dass die Lösung typischerweise einen Feststoffgehalt von ca. 5 g/l besitzt, wenn, wie hier, die für ein Device typische Schichtdicke von 20 nm mittels Spincoating erzielt werden soll. Die Schichten werden in einer Inertgasatmosphäre, im vorliegenden Fall Argon, aufgeschleudert und 60 min bei 180°C ausgeheizt.

Die Emissionsschicht setzt sich immer aus mindestens einem Matrixmaterial (Hostmaterial, Wirtsmaterial) und einem emittierenden Dotierstoff (Dotand, Emitter) zusammen. Eine Angabe wie SMB4 (97%) und ES bzw. EAS (3%) bedeutet hierbei, dass das Material SMB4 in einem Gewichtsanteil von 97% und der Dotand ES bzw. EAS in einem Gewichtsanteil von 3% in der Emissionsschicht vorliegt. Die Mischung für die Emissionsschicht wird in Toluol oder Chlorbenzol gelöst. Der typische Feststoffgehalt solcher Lösungen liegt bei ca. 18 g/l, wenn, wie hier, die für ein Device typische Schichtdicke von 50 nm mittels Spincoating erzielt werden soll. Die Schichten werden in einer Inertgasatmosphäre, im vorliegenden Fall Argon, aufgeschleudert und 10 Minuten bei 140° bis 160 °C ausgeheizt. Die verwendeten Materialien sind in Tabelle 8 gezeigt.

Die Materialien für die Elektronentransportschicht sowie für die Kathode werden in einer Vakuumkammer thermisch aufgedampft. Dabei kann z.B. die Elektronentransportschicht aus mehr als einem Material bestehen, die einander durch Co-Verdampfung in einem bestimmten Volumenanteil beigemischt werden. Eine Angabe wie ETM1 (50%) und ETM2 (50%) bedeutet hierbei, dass die Materialien ETM1 und ETM2 in einem Volumenanteil von je 50% in der Schicht vorliegen. Die im vorliegenden Fall verwendeten Materialien sind in Tabelle 8 gezeigt.

**Tabelle 7: Ergebnisse der Lösungs-prozessierten OLEDs bei 1000 cd/m²**

| **Bsp.** | **Dotand** | **EQE (%)** | **Spannung (V)** | **Farbe** | **EL-FWHM [eV]** |
|---|---|---|---|---|---|
| Sol-BF1 | D11 | 6.8 | 4.5 | Blau | 0.14 |
| Sol-BF2 | D17 | 7.3 | 4.4 | Blau | 0.15 |
| Sol-BF3 | D22 | 7.1 | 4.3 | Blau | 0.15 |
| Sol-BF4 | D24 | 6.7 | 4.4 | Blau | 0.14 |
| Sol-BF5 | D103 | 7.0 | 4.4 | Blau | 0.14 |
| Sol-BF6 | D402 | 7.5 | 4.5 | Blau | 0.15 |
| Sol-BF7 | D402 | 7.0 | 4.4 | Blau | 0.15 |

**Tabelle 8: Strukturformeln der verwendeten Materialien**

| | |
|---|---|
| 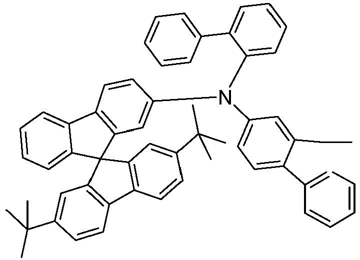 HTM1 [1365840-52-3] | 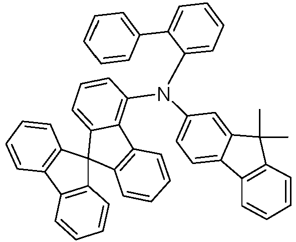 HTM2 [1450933-44-4] |
| 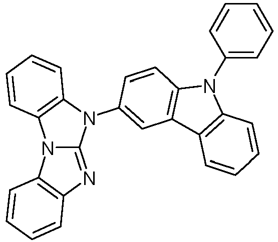 HTM3 [1401068-29-8] | 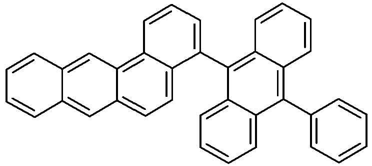 SMB1 [1087346-88-0] |
| 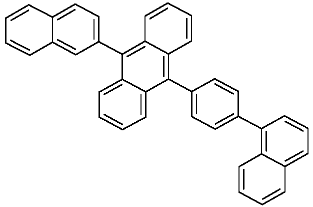 SMB2 [667940-34-3] | 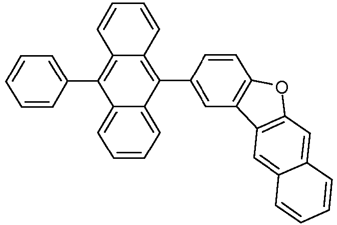 SMB3 [1627916-48-6] |
| 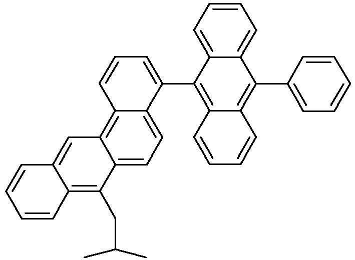 SMB4 [1818872-85-3] | 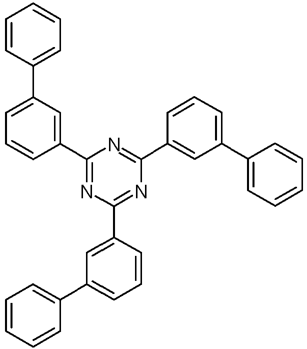 TMM1 / ETM3 [1201800-83-0] |
| 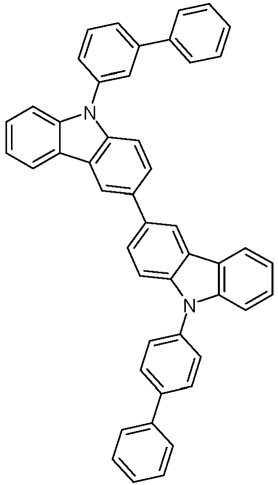 [1643476-29-2] (40%) | 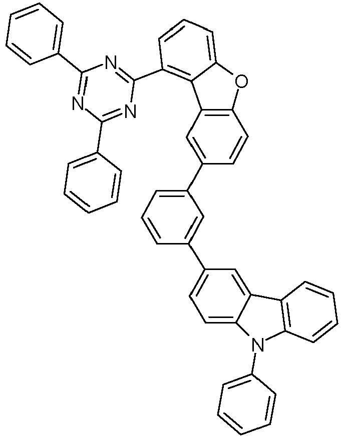 [1822310-86-0] (60%) |

| TMM2 | |
|---|---|
| 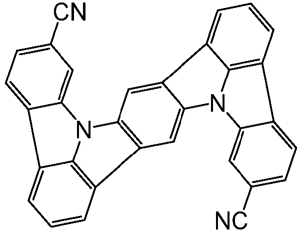 2286203-95-8 Ref.-D1 | 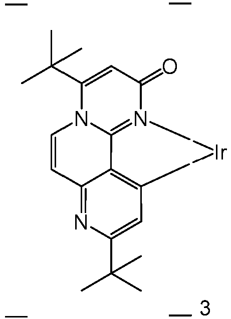 PS1 [1615218-73-9] |
| 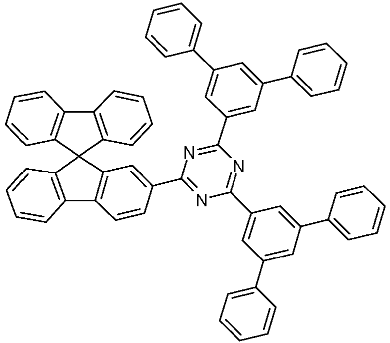 ETM1 [1233200-52-6] | 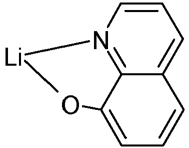 ETM2 [25387-93-3] |
| 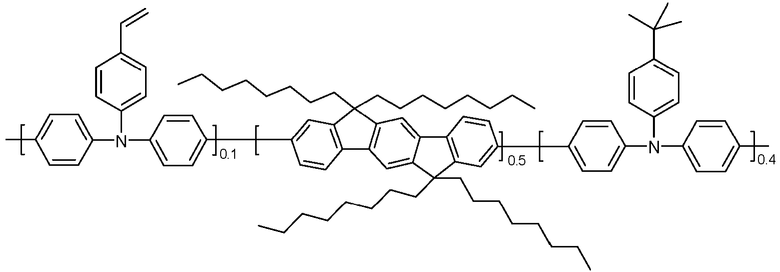 HTM-Sol | |

Die Abkürzungen der erfindungsgemäßen Verbindungen, die in den zuvor in Bezug auf die OLED-Bauteile dargelegten Tabellen verwendet werden, beziehen sich auf die in den obigen Synthesebeispielen bereitgestellten Abkürzungen.

Die erfindungsgemäßen Verbindungen zeigen im Vgl. zur Referenz schmalere Elektrolumineszenzspektren, erkennbar an den geringeren bzw. gleichgroßen EL-FWHM-Werten (**EL**ectroluminescence - **F**ull **W**idth **H**alf **M**aximum - Breite der EL-Emissionsspektren in eV auf halber Peakhöhe). Schmalere Elektrolumineszenzspektren führen zu deutlich verbesserter Farbreinheit (kleinere CIE y Werte). Außerdem sind die EQE-Werte (**E**xterne **Q**uanten **E**ffizienen) deutlich größer und die Betriebsspannungen geringer im Vgl. zur Referenz, was zu deutlich verbesserten Leistungseffizienzen des Devices und somit zu geringerem Stromverbrauch führt.

### Herstellung von Bauteilen zur Farbkonversion

Die erfindungsgemäßen Verbindungen können zur Farbkonversion eingesetzt werden. Dazu werden die Verbindungen in eine Komposition eingearbeitet, die dann durch bekannte Verfahren (Spin-coating, Slitcoating, Raklen, Siebdruck, Nozzle-Printing, Ink-Jet-Printing, etc.) zu Pixeln oder flächigen Schichten verarbeitet werden. Die Kompositionen bestehen typischerweise aus vernetzbaren Komponenten (Monomeren, Oligomeren, Polymeren), z. B. auf Basis von Acrylaten, Acrylamiden, Polyestern, Siliconen, etc. und einem oder mehreren thermisch oder photochemisch aktivierbaren Starterkomponenten. Daneben können weitere Komponenten wie org. Hilfsstoffe (Antioxidantien, Stabilisatoren, Verlaufshilfsmittel, Viskositätsmoderatoren, etc.) oder anorg. Füllstoffe (SiO₂, TiO₂, Al₂O₃, etc.) eingebracht werden.

### Allgemeine Herstellungsprozedur der Komposition und abgeleiteter Schichten:

0.5 g der erfindungsgemäßen Verbindung ES bzw. EAS, 0.2 g Titandioxid (TiO₂ ToyoColor, Fa. Toyo Ink Group) und 10 g OE-6550 Optical Encapsulant (Fa. Dow Corning) werden unter sehr gutem Rühren (Magnetrührer) unter Einwirkung von Ultraschall (Ultraschallbad) bei 40 °C homogenisiert. Schichten mit ca. 15 µm Schichtdicke werden durch Rakeln erzeugt und dann durch Ausheizen unter Stickstoffatmosphäre (150 °C, 1 Stunde) gehärtet.

### Spektrale Messung der Schichten:

Fluoreszenspektren und EQE-Werte (Externe Quanten Effizienz, EQE = Emittierte Photonen / Absorbierte Photonen) der Schichten werden in einem Fluoreszenspektometer (C9920, Hamamatsu photonics) mit Ulbricht-Kugel und Faseroptik (Anregungswellenlänge CWL: 420 - 440 nm für Blaue, 450 nm für Grüne Emitter, Referenzmessung an Luft bei Raumtemperatur) ermittelt.

### Ergebnisse

**Tabelle 9 fasst die Ergebnisse zusammen:**

| Bsp. | Material | Farbe | FWHM [eV] | EQE [%] |
|---|---|---|---|---|
| CCB1 | D10 | Blau | 0.14 | 24.9 |
| CCB2 | D11 | Blau | 0.13 | 25.5 |
| CCB3 | D14 | Blau | 0.14 | 26,7 |
| CCB4 | D23 | Blau | 0.15 | 25.8 |
| CCB5 | D30 | Blau | 0.14 | 21.4 |
| CCB6 | D44 | Blau | 0.15 | 20.0 |
| CCB7 | D45 | Blau | 0.14 | 21.9 |
| CCB8 | D47 | Blau | 0.15 | 23.6 |
| CCB6 | D102 | Blau | 0.13 | 26.9 |
| CCG1 | D5 | Grün | 0.15 | 27.4 |
| CCG2 | D7 | Grün | 0.15 | 28.5 |

## Patentansprüche

1. Verbindung umfassend mindestens eine Struktur der Formel (I), 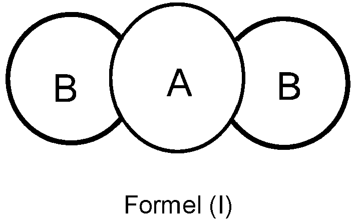 wobei A bei jedem Auftreten gleich oder verschieden für eine Teilstruktur der Formel (A1) oder (A2), 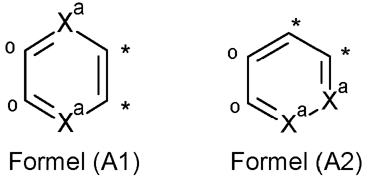 an die zwei Teilstrukturen B kondensiert sind, steht und die Symbole o und * die zwei Kondensationsstellen der jeweiligen Teilstruktur B darstellen, wobei eine Teilstruktur B über die mit o gekennzeichneten Positionen an A kondensiert ist und eine Teilstruktur B über die mit * gekennzeichneten Positionen an A kondensiert ist und B bei jedem Auftreten gleich oder verschieden für eine Teilstruktur der Formel (B) steht 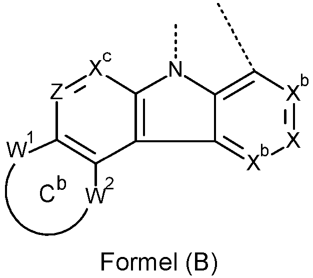
wobei die gestrichelten Bindungen die Kondensationsstellen der Teilstruktur B an A darstellen,
der Ring C^{b} bei jedem Auftreten gleich oder verschieden für einen kondensierten aliphatischen oder heteroaliphatischen Ring mit 5 bis 60 Ringatomen steht, der mit einem oder mehreren Resten R substituiert sein kann,
und für die weiteren Symbole gilt:
Z steht bei jedem Auftreten gleich oder verschieden für N, C-CN oder CR^{c};
W¹, W² steht bei jedem Auftreten gleich oder verschieden für C(R)₂, O, S, Si(R)₂;
X steht bei jedem Auftreten gleich oder verschieden für N oder CR mit der Maßgabe, dass nicht mehr als zwei der Gruppen X, X^{b} in einem Cyclus für N stehen;
X^{a} steht bei jedem Auftreten gleich oder verschieden für N oder CR^{a};
X^{b} steht bei jedem Auftreten gleich oder verschieden für N oder CR^{b} mit der Maßgabe, dass nicht mehr als zwei der Gruppen X, X^{b} in einem Cyclus für N stehen;
X^{c} steht bei jedem Auftreten gleich oder verschieden für N oder CR^{c};
R ist bei jedem Auftreten gleich oder verschieden H, D, OH, F, Cl, Br, I, CN, NO₂, N(Ar)₂, N(R^{d})₂, C(=O)N(Ar)₂, C(=O)N(R^{d})₂, C(Ar)₃, C(R^{d})₃, Si(Ar)₃, Si(R^{d})₃, B(Ar)₂, B(R^{d})₂, C(=O)Ar, C(=O)R^{d}, P(=O)(Ar)₂, P(=O)( R^{d})₂, P(Ar)₂, P(R^{d})₂, S(=O)Ar, S(=O)R^{d}, S(=O)₂Ar, S(=O)₂R^{d}, OSO₂Ar, OSO₂R^{d}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR^{d}, -C(=O)O-, -C(=O)NR^{d}-, NR^{d}, P(=O)( R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Arylthio- oder Heteroarylthiogruppe mit 5 bis 60 aromatischen Ringatomen, die durch einen oder mehrere Reste R^{d} substituiert sein kann, oder eine Diarylamino-, Arylheteroarylamino-, Diheteroarylaminogruppe mit 5 bis 60 aromatischen Ringatomen, die durch einen oder mehrere Reste R^{d} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{d} substituiert sein kann; dabei kann ein Rest R mit einer weiteren Gruppe ein Ringsystem bilden;
Ar ist bei jedem Auftreten gleich oder verschieden ein aromatisches oder heteroaromatisches Ringsystem mit 5 bis 60 aromatischen Ringatomen, das mit einem oder mehreren Resten R^{d} substituiert sein kann, dabei können zwei Reste Ar, welche an dasselbe C-Atom, Si-Atom, N-Atom, P-Atom oder B-Atom binden, auch durch eine Einfachbindung oder eine Brücke, ausgewählt aus B(R^{d}), C(R^{d})₂, Si(R^{d})₂, C=O, C=NR^{d}, C=C(R^{d})₂, O, S, S=O, SO₂, N(R^{d}), P(R^{d}) und P(=O)R^{d}, miteinander verbrückt sein;
R^{a}, R^{b}, R^{c}, R^{d} ist bei jedem Auftreten gleich oder verschieden H, D, OH, F, Cl, Br, I, CN, NO₂, N(Ar')₂, N(R')₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d} auch miteinander oder einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch - R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere Reste R¹ miteinander ein Ringsystem bilden, dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere Substituenten R² miteinander ein Ringsystem bilden;
mit der Maßgabe, dass die Struktur der Formel (I) mindestens eine Teilstruktur B umfasst, in der die Gruppe Z für N oder C-CN steht.

2. Verbindung nach Anspruch 1, umfassend mindestens eine Struktur der Formeln (I-1) und/oder (I-2), 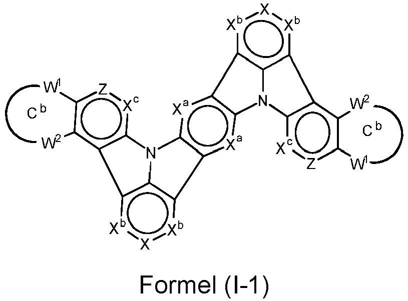 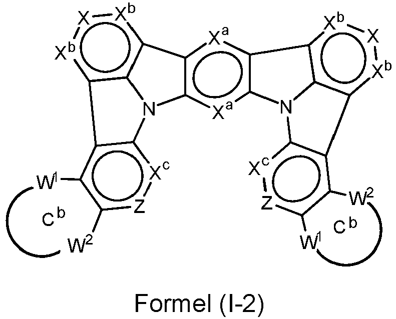 wobei die Symbole C^{b}, W¹, W², Z, X, X^{a}, X^{b} und X^{c} die in Anspruch 1 genannten Bedeutungen aufweisen.

3. Verbindung nach Anspruch 1 oder 2, **dadurch gekennzeichnet, dass** die Verbindung mindestens eine Teilstruktur der Formeln (B1-1) bis (B1-30) umfasst, 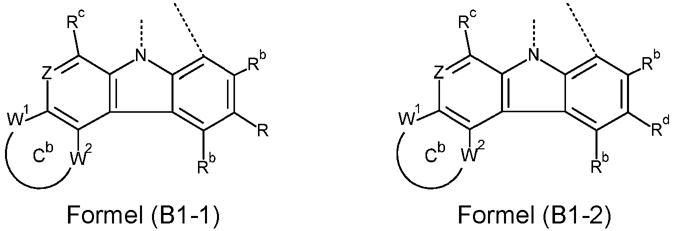 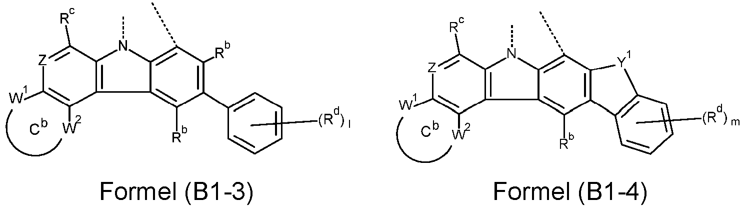 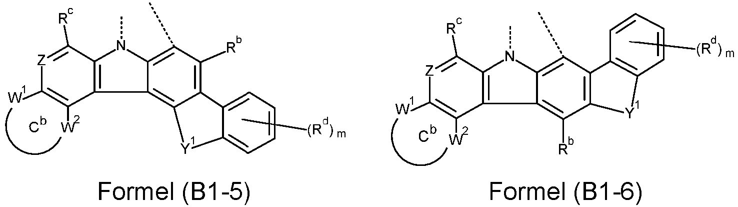 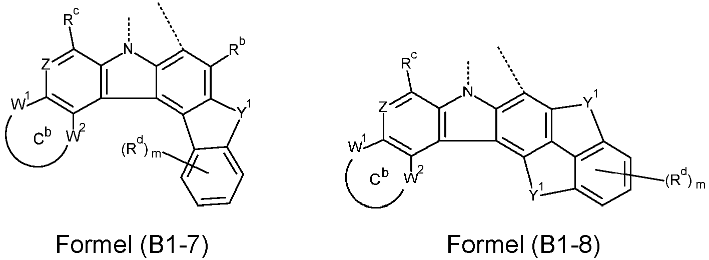 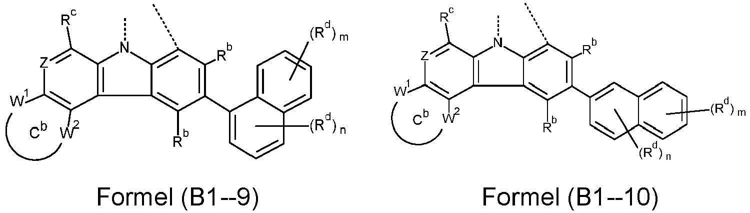 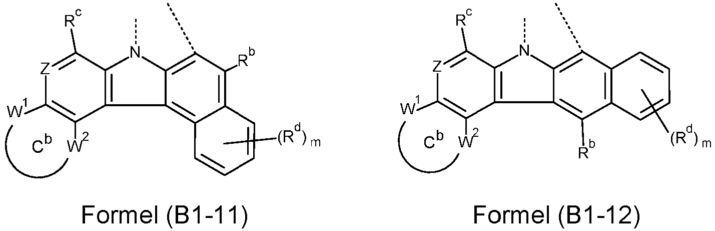 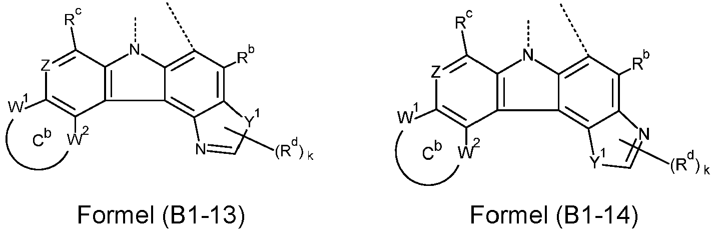 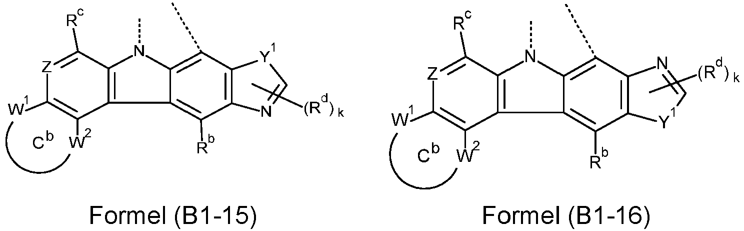 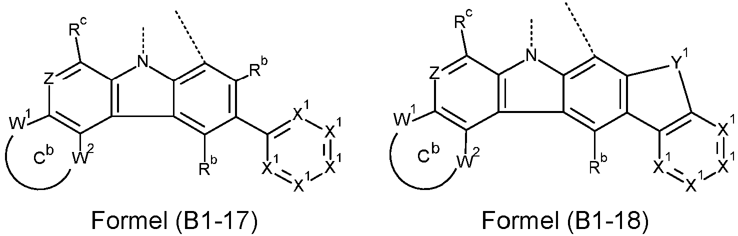 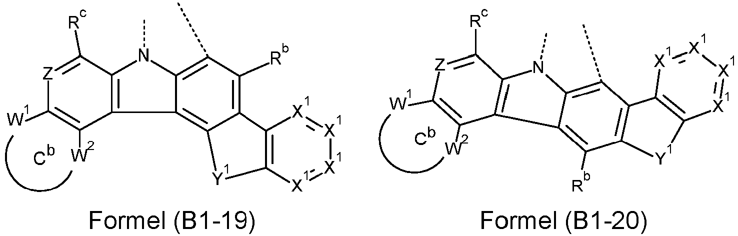 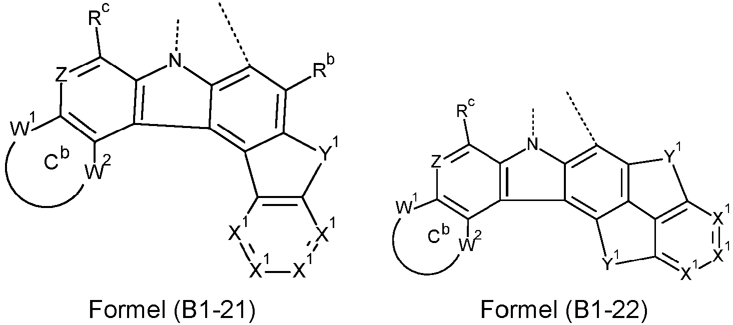 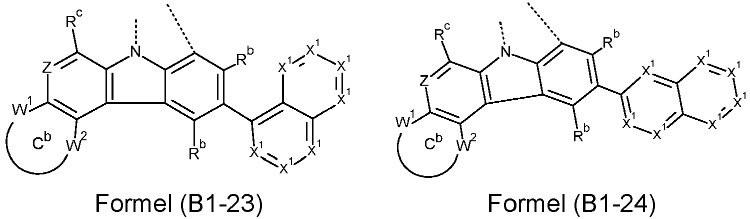 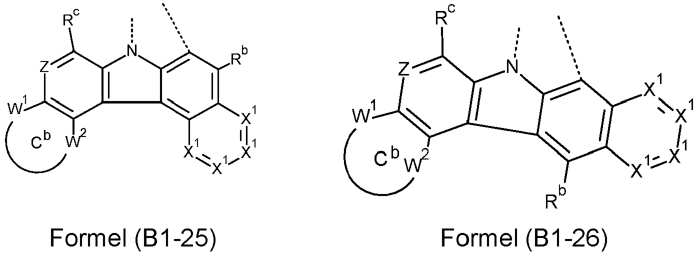 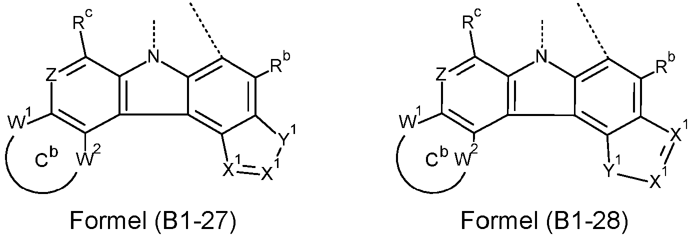 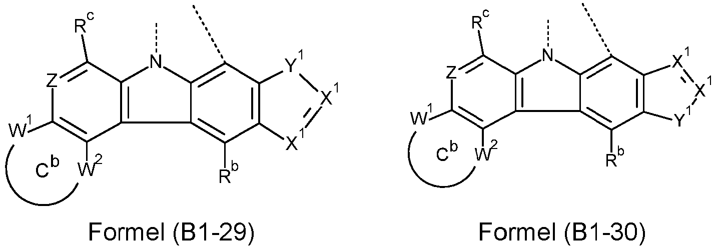 wobei die Symbole C^{b}, W¹, W², Z, R, R^{b}, R^{c} und R^{d} die in Anspruch 1 genannten Bedeutungen aufweisen, die gestrichelten Bindungen die Kondensationsstellen der Teilstruktur an A darstellen und für die weiteren verwendeten Symbole und Indices gilt:
X¹ steht bei jedem Auftreten gleich oder verschieden für N oder CR^{d} mit der Maßgabe, dass nicht mehr als zwei der Gruppen X¹ in einem Cyclus für N stehen;
Y¹ ist bei jedem Auftreten gleich oder verschieden C(R^{d})₂, (R^{d})₂C-C(R^{d})₂, (R^{d})C=C(R^{d}), NR^{d}, NAr', O, S, SO, SO₂, Se, P(O)R^{d}, BR^{d} oder Si(R^{d})₂;
k ist 0 oder 1;
n ist 0, 1, 2 oder 3;
m ist 0, 1, 2, 3 oder 4;
l ist 0, 1, 2, 3, 4 oder 5.

4. Verbindung nach einem oder mehreren der Ansprüche 1 bis 3, umfassend mindestens eine Struktur der Formeln (II-1) bis (II-15), 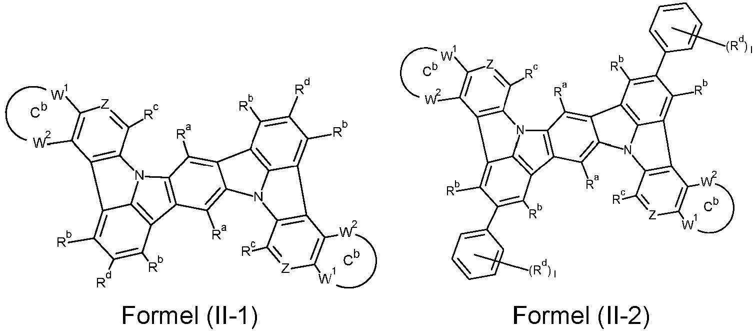 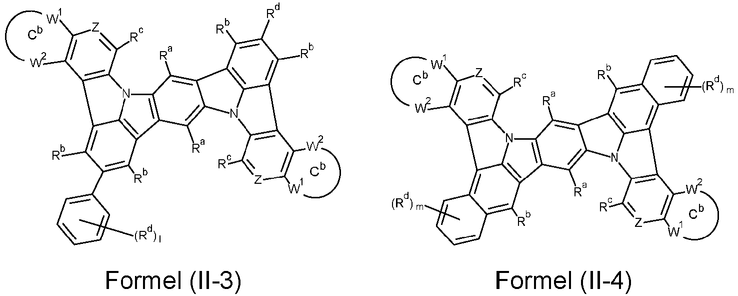 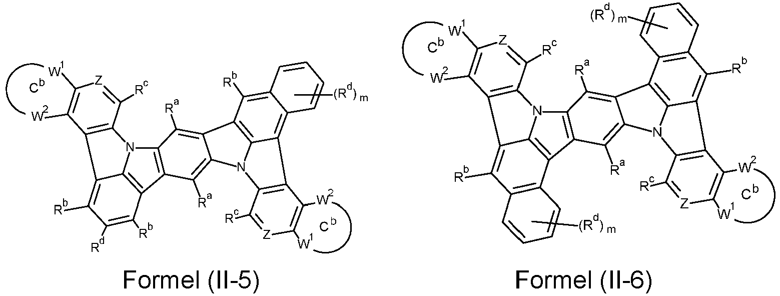 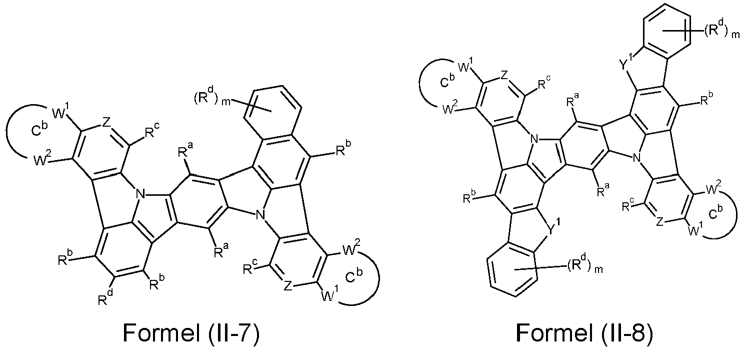 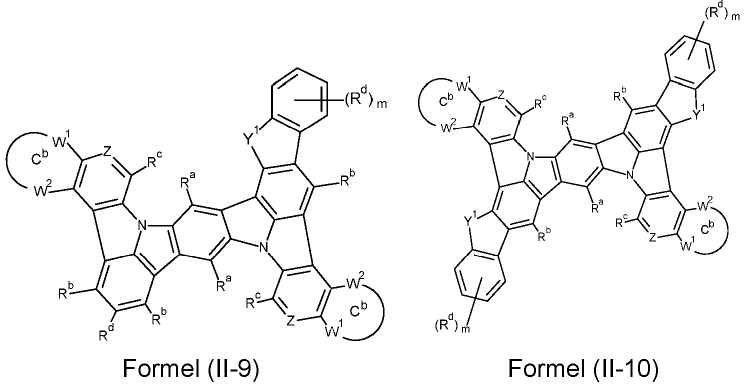 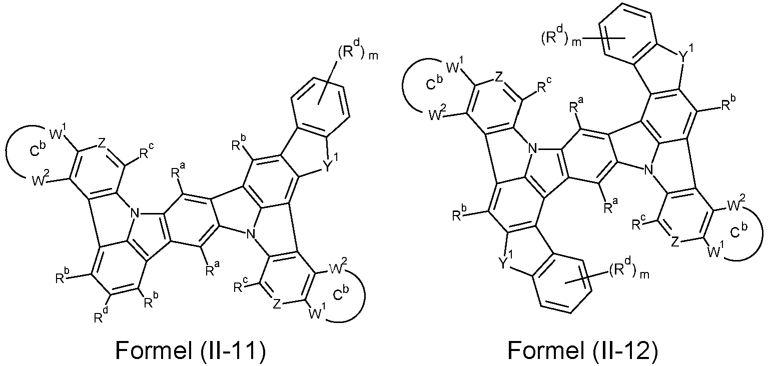 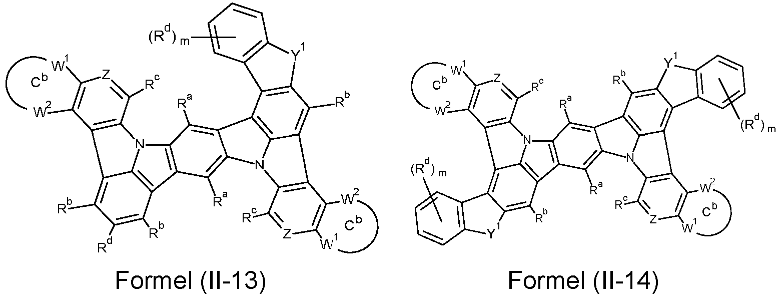 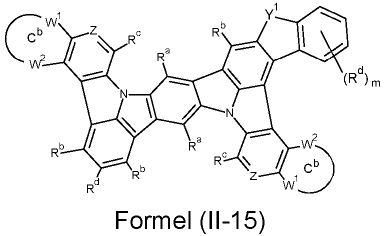 wobei die Symbole C^{b}, W¹, W², Z, R, R^{a}, R^{b}, R^{c} und R^{d} die in Anspruch 1 genannten Bedeutungen aufweisen, das Symbol Y¹ die in Anspruch 3 genannte Bedeutung aufweist und für die weiteren verwendeten Indices gilt:
m ist 0, 1, 2, 3 oder 4;
l ist 0, 1, 2, 3, 4 oder 5.

5. Verbindung nach einem oder mehreren der Ansprüche 1 bis 4, **dadurch gekennzeichnet, dass** der kondensierte Ring C^{b} ausgewählt ist aus einer Struktur der Formeln (BCY-1) bis (BCY-10), 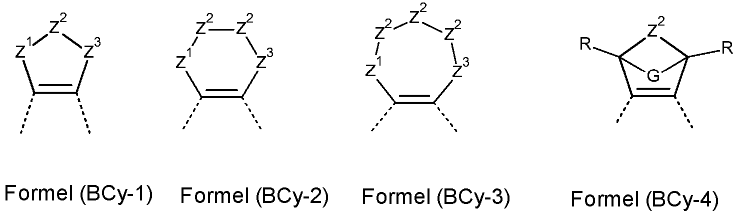 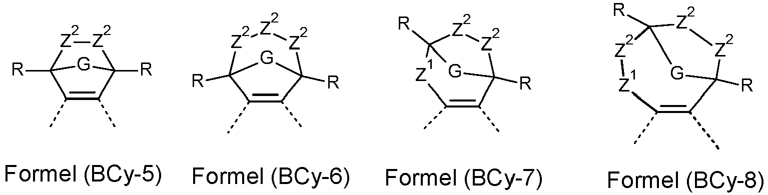 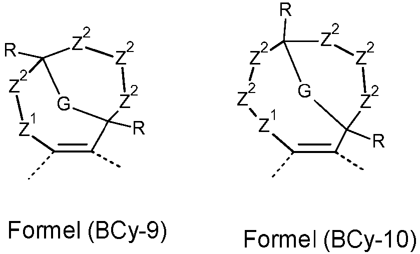 wobei R die in Anspruch 1 genannte Bedeutung aufweist, die gestrichelten Bindungen die Bindungsstellen des kondensierten Rings an die weiteren Gruppen darstellen und weiterhin gilt:
Z¹, Z³ ist gleich oder verschieden bei jedem Auftreten C(R³)₂, O, S oder Si(R³)₂;
Z² ist C(R)₂, O, S, NR oder C(=O), wobei zwei benachbarte Gruppen Z² für -CR=CR- oder eine ortho-verknüpfte Arylen- oder Heteroarylengruppe mit 5 bis 14 aromatischen Ringatomen, welche durch einen oder mehrere Reste R substituiert sein kann, stehen können;
G ist eine Alkylengruppe mit 1, 2 oder 3 C-Atomen, welche mit einem oder mehreren Resten R substituiert sein kann, -CR=CR- oder eine ortho-verknüpfte Arylen- oder Heteroarylengruppe mit 5 bis 14 aromatischen Ringatomen, welche durch einen oder mehrere Reste R substituiert sein kann;
R³ ist bei jedem Auftreten gleich oder verschieden H, D, F, Cl, Br, I, CN, NO₂, N(Ar')₂, N(R^{d})₂, C(=O)Ar', C(=O)R^{d}, P(=O)(Ar')₂, P(Ar')₂, B(Ar')₂, B(R^{d})₂, C(Ar')₃, C(R^{d})₃, Si(Ar')₃, Si(R^{d})₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R^{d} substituiert sein kann, wobei eine oder mehrere nicht benachbarte CH₂-Gruppen durch -R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann, oder eine Aralkyl- oder Heteroaralkylgruppe mit 5 bis 60 aromatischen Ringatomen, die mit einem oder mehreren Resten R^{d} substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem Rest R, R^{a}, R^{c} oder R³ ein aliphatisches Ringsystem bilden, wobei Ar' und R^{d} die in Anspruch 1 genannten Bedeutungen aufweisen;
mit der Maßgabe, dass in diesen Gruppen nicht zwei Heteroatome direkt aneinander gebunden sind und nicht zwei Gruppen C=O direkt aneinander gebunden sind.

6. Verbindung nach einem oder mehreren der Ansprüche 1 bis 5, **dadurch gekennzeichnet, dass** der kondensierte Ring C^{b} ausgewählt ist aus einer Struktur der Formeln (BRA-1) bis (BRA-12) 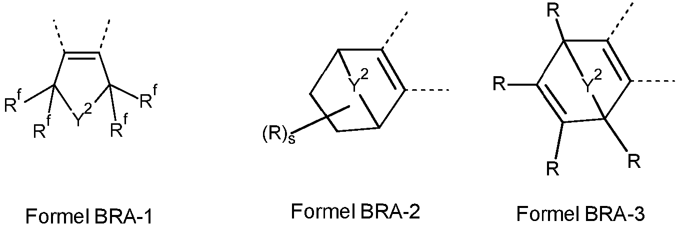 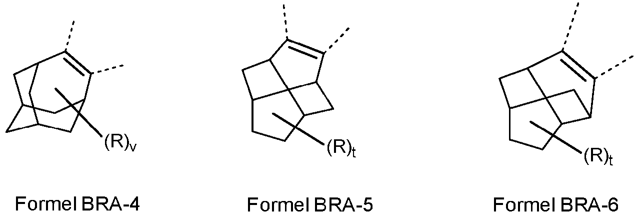 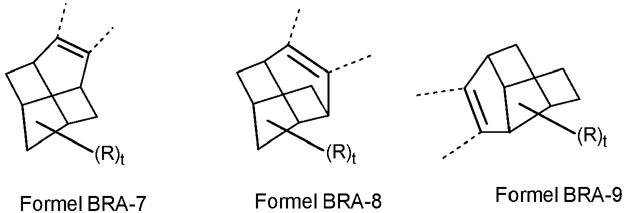 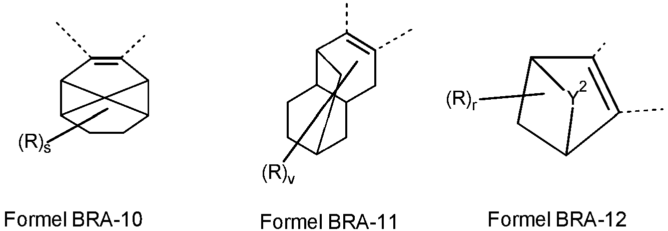 wobei R die in Anspruch 1 genannte Bedeutung aufweist, die gestrichelten Bindungen die Anbindungsstellen des kondensierten Rings an die weiteren Gruppen darstellen und die weiteren Symbole die folgende Bedeutung aufweisen:
Y² ist bei jedem Auftreten gleich oder verschieden C(R)₂, (R)₂C-C(R)₂, (R)C=C(R), NR, NAr', O oder S;
R^{f} ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR^{d}, -C(=O)O-, -C(=O)NR^{d}-, NR^{d}, P(=O)(R^{d}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann, oder eine Aryloxy- oder Heteroaryloxygruppe mit 5 bis 60 aromatischen Ringatomen, die durch einen oder mehrere Reste R^{d} substituiert sein kann; dabei können zwei Reste R^{f} auch miteinander oder ein Rest R^{f} mit einem Rest R oder mit einer weiteren Gruppe ein Ringsystem bilden, wobei R^{d} die in Anspruch 1 genannte Bedeutung aufweist;
r ist 0, 1, 2, 3 oder 4;
s ist 0, 1, 2, 3, 4, 5 oder 6;
t ist 0, 1, 2, 3, 4, 5, 6, 7 oder 8;
v ist 0, 1, 2, 3, 4, 5, 6, 7, 8 oder 9.

7. Verbindung nach einem oder mehreren der Ansprüche 1 bis 6, dass der kondensierte Ring C^{b} ausgewählt ist aus einer Struktur der Formeln (BRA-1a) bis (BRA-3f) 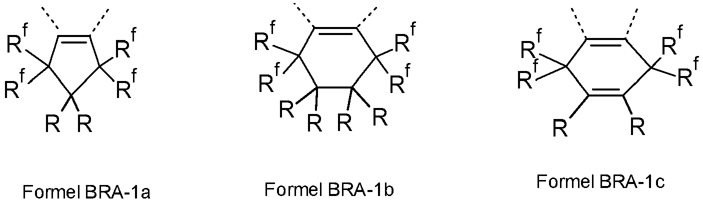 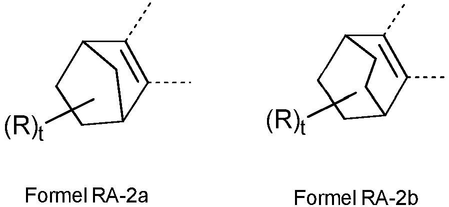 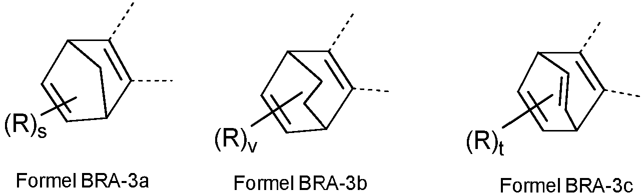 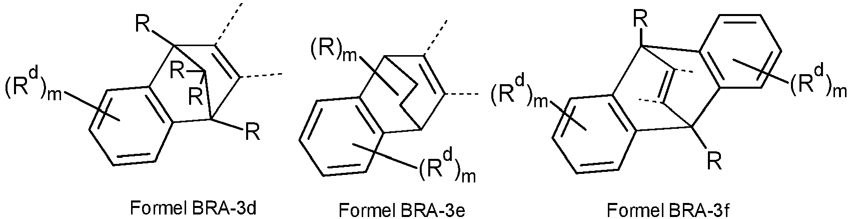 wobei die gestrichelten Bindungen die Anbindungsstellen des kondensierten Rings an die weiteren Gruppen darstellen, der Index m 0, 1, 2, 3 oder 4 ist und die Symbole R, R^{d}, R^{f} und die Indices s, t und v die zuvor, insbesondere in Anspruch 1 und 6 dargelegte Bedeutung aufweisen.

8. Verbindung nach einem oder mehreren der Ansprüche 1 bis 7, **dadurch gekennzeichnet, dass** mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} mindestens eine Struktur der folgenden Formeln (Cy-1) bis (Cy-10) formen, 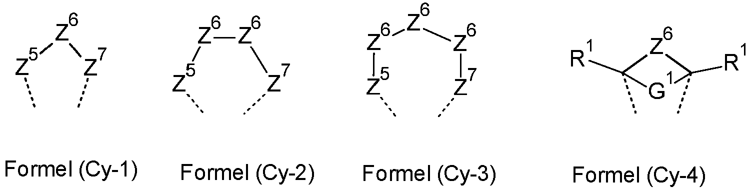 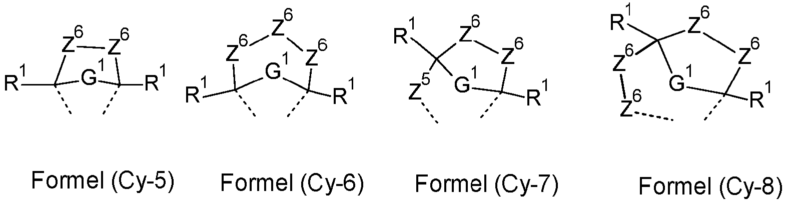 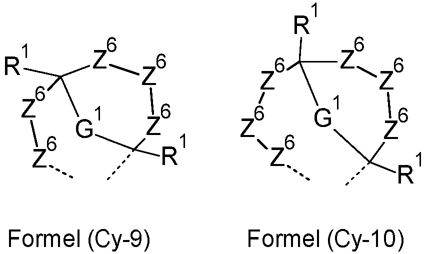 wobei R¹ die in Anspruch 1 genannte Bedeutung aufweist, die gestrichelten Bindungen die Anbindungsstellen an die Atome der Gruppen, an die die zwei Reste R, R^{a}, R^{b}, R^{c}, R^{d} binden, darstellen und weiterhin gilt:
Z⁵, Z⁷ ist gleich oder verschieden bei jedem Auftreten C(R⁴)₂, O, S, NR⁴ oder C(=O),
Z⁶ ist C(R¹)₂, O, S, NR¹ oder C(=O), wobei zwei benachbarte Gruppen Z² für -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann, stehen können;
G¹ ist eine Alkylengruppe mit 1, 2 oder 3 C-Atomen, welche mit einem oder mehreren Resten R¹ substituiert sein kann, -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann;
R⁴ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R⁴, welche an dasselbe Kohlenstoffatom gebunden sind, miteinander ein aliphatisches oder aromatisches Ringsystem bilden und so ein Spirosystem aufspannen; weiterhin kann R⁴ mit einem Rest R, R^{a}, R^{b}, R^{c}, R^{d} oder R¹ ein aliphatisches Ringsystem bilden, die Symbole R¹ und Ar" die in Anspruch 1 genannten Bedeutungen aufweisen,
mit der Maßgabe, dass in diesen Gruppen nicht zwei Heteroatome direkt aneinander gebunden sind und nicht zwei Gruppen C=O direkt aneinander gebunden sind.

9. Verbindung nach einem oder mehreren der Ansprüche 1 bis 8, **dadurch gekennzeichnet, dass** mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} mindestens eine Struktur der Formeln (RA-1) bis (RA-13) formen 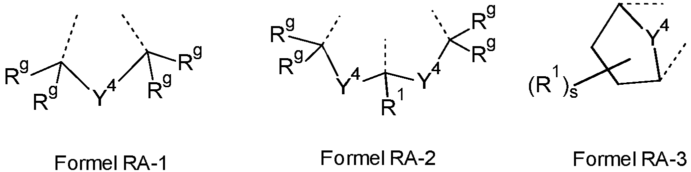 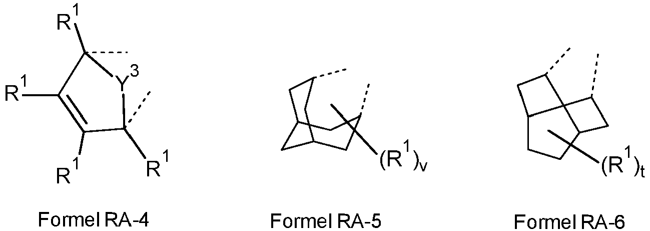 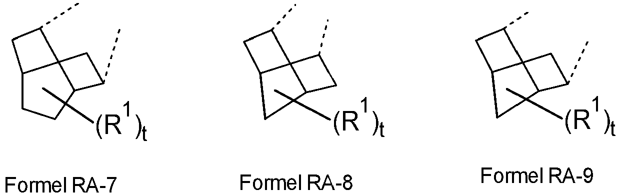 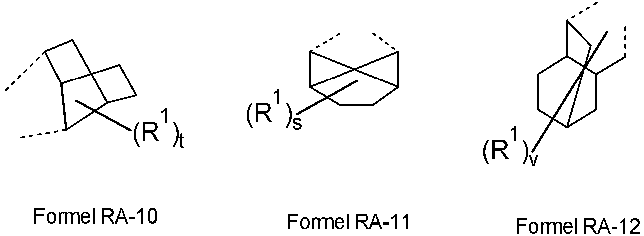 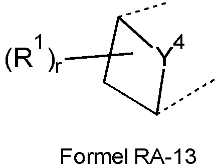 wobei R¹ die zuvor dargelegte Bedeutung hat, die gestrichelten Bindungen die Anbindungsstellen an die Atome der Gruppen, an die die zwei Reste R, R^{a}, R^{b}, R^{c}, R^{d} binden, darstellen, und die weiteren Symbole die folgende Bedeutung aufweisen:
Y⁴ ist bei jedem Auftreten gleich oder verschieden C(R¹)₂, (R¹)₂C-C(R¹)₂, (R¹)C=C(R¹), NR¹, NAr', O oder S;
R^{g} ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R²), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{g} auch miteinander oder ein Rest R^{g} mit einem Rest R¹ oder mit einer weiteren Gruppe ein Ringsystem bilden, wobei R² die in Anspruch 1 genannte Bedeutung aufweist;
r ist 0, 1, 2, 3 oder 4;
s ist 0, 1, 2, 3, 4, 5 oder 6;
t ist 0, 1, 2, 3, 4, 5, 6, 7 oder 8;
v ist 0, 1, 2, 3, 4, 5, 6, 7, 8 oder 9.

10. Verbindung nach einem oder mehreren der Ansprüche 1 bis 9, **dadurch gekennzeichnet, dass** mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} Strukturen der Formel (RB) formen, 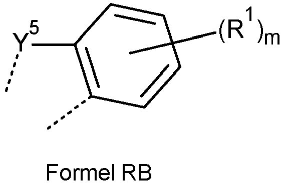 wobei R¹ die in Anspruch 1 dargelegte Bedeutung aufweist, die gestrichelten Bindungen die Anbindungsstellen darstellen, über die die zwei Reste R, R^{a}, R^{b}, R^{c}, R^{d} binden, der Index m 0, 1, 2, 3 oder 4 ist, und Y⁵ C(R¹)₂, NR¹, NAr', BR¹, BAr', O oder S ist.

11. Verbindung nach einem oder mehreren der Ansprüche 1 bis 10, **dadurch gekennzeichnet, dass** R oder Ar gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{d} substituiert sein können.

12. Verbindung gemäß mindestens einem der vorhergehenden Ansprüche, **dadurch gekennzeichnet, dass** der Rest R^{b} und/oder R^{d} mindestens eine Gruppe ausgewählt aus C(Ar')₃, C(R¹)₃, Si(Ar')₃, Si(R¹)₃, B(R¹)₂ darstellt oder eine Fluorengruppe, die mit einem oder mehreren Resten R¹ substituiert sein kann, umfasst, darstellt oder mit einem Rest R^{b} beziehungsweise R^{d} bildet.

13. Verbindung gemäß mindestens einem der vorhergehenden Ansprüche, **dadurch gekennzeichnet, dass** die Verbindung genau zwei oder genau drei Strukturen gemäß Formel (I), (I-1), (I-2) und/oder (II-1) bis (II-15) umfasst.

14. Oligomer, Polymer oder Dendrimer enthaltend eine oder mehrere Verbindungen nach einem der Ansprüche 1 bis 13, wobei statt eines Wasserstoffatoms oder eines Substituenten eine oder mehrere Bindungen der Verbindungen zum Polymer, Oligomer oder Dendrimer vorhanden sind.

15. Formulierung, enthaltend mindestens eine Verbindung nach einem oder mehreren der Ansprüche 1 bis 13 oder ein Oligomer, Polymer oder Dendrimer nach Anspruch 14 und mindestens eine weitere Verbindung, wobei die weitere Verbindung bevorzugt ausgewählt ist aus einem oder mehreren Lösemitteln.

16. Zusammensetzung, enthaltend mindestens eine Verbindung nach einem oder mehreren der Ansprüche 1 bis 13 oder ein Oligomer, Polymer oder Dendrimer nach Anspruch 14 und mindestens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien, vorzugsweise Hostmaterialien.

17. Zusammensetzung nach Anspruch 16, **dadurch gekennzeichnet, dass** die Zusammensetzung mindestens eine weitere Verbindung ein TADF-Hostmaterial darstellt und/oder mindestens eine weitere Verbindung ein phosphoreszierender Emitter (Triplettemitter) darstellt, wobei die weiteren Verbindungen mit einer Verbindung nach einem oder mehreren der Ansprüche 1 bis 13 oder einem Oligomer, Polymer oder Dendrimer nach Anspruch 14 vorzugsweise ein Hyperfluoreszenz- und/oder Hyperphosphoreszenz-System bilden.

18. Verfahren zur Herstellung einer Verbindung nach einem oder mehreren der Ansprüche 1 bis 13, **dadurch gekennzeichnet, dass** ein Grundgerüst mit einer aromatischen Aminogruppe synthetisiert wird und mindestens ein aromatischer oder heteroaromatischer Rest eingeführt wird, vorzugsweise mittels einer nukleophilen aromatischen Substitutionsreaktion oder einer Kupplungsreaktion.

19. Verwendung einer Verbindung nach einem oder mehreren der Ansprüche 1 bis 13 oder ein Oligomer, Polymer oder Dendrimer nach Anspruch 14 in einer elektronischen Vorrichtung.

20. Elektronische Vorrichtung enthaltend mindestens eine Verbindung nach einem oder mehreren der Ansprüche 1 bis 13 oder ein Oligomer, Polymer oder Dendrimer nach Anspruch 14.

## Claims

1. Compound comprising at least one structure of the formula (I) 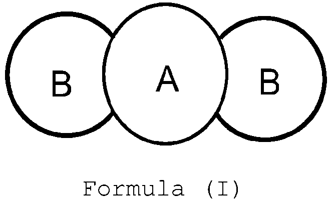 where A is the same or different at each instance and is a substructure of the formula (A1) or (A2): 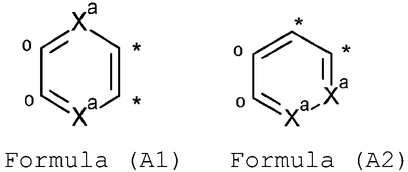 to which two substructures B are fused, and the symbols o and * represent the two fusion sites of the respective substructure B, where one substructure B is fused to A via the positions labelled o and one substructure B is fused to A via the positions labelled *, and B is the same or different at each instance and is a substructure of the formula (B): 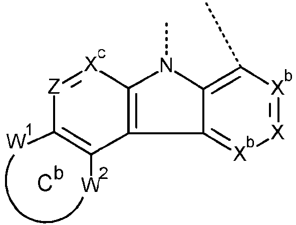 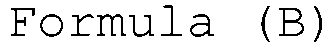
where the dotted bonds represent the fusion sites of the substructure B to A,
the ring C^{b} is the same or different at each instance and is a fused aliphatic or heteroaliphatic ring which has 5 to 60 ring atoms and may be substituted by one or more R radicals,
and the further symbols are as follows:
Z is the same or different at each instance and is N, C-CN or CR^{c};
W¹, W² is the same or different at each instance and is C(R)₂, O, S, Si(R)₂;
X is the same or different at each instance and is N or CR, with the proviso that not more than two of the X, X^{b} groups in one cycle are N;
X^{a} is the same or different at each instance and is N or CR^{a};
X^{b} is the same or different at each instance and is N or CR^{b}, with the proviso that not more than two of the X, X^{b} groups in one cycle are N;
X^{c} is the same or different at each instance and is N or CR^{c};
R is the same or different at each instance and is H, D, OH, F, Cl, Br, I, CN, NO₂, N(Ar)₂, N(R^{d})₂, C (=O)N(Ar)₂, C(=O)N(R^{d})₂, C(Ar)₃, C(R^{d})₃, Si(Ar)₃, Si(R^{d})₃, B(Ar)₂, B(R^{d})₂, C(=O)Ar, C(=O)R^{d} , P(=O)(Ar)₂, P(=O)(R^{d})₂, P(Ar)₂, P(R^{d})₂, S(=O)Ar, S(=O)R^{d}, S(=O)₂Ar, S(=O)₂R^{d}, OSO₂Ar, OSO₂R^{d}, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R^{d} radicals, where one or more nonadjacent CH₂ groups may be replaced by R^{d}C=CR^{d}, C=C, Si(R^{d})₂, C=O, C=S, C=Se, C=NR^{d}, -C(=O)O-, -C(=O)NR^{d}-, NR^{d}, P(=O)(R^{d}), -O-, -S-, SO or SO₂, or an aromatic or heteroaromatic ring system which has 5 to 60 aromatic ring atoms and may be substituted in each case by one or more R^{d} radicals, or an aryloxy or heteroaryloxy group which has 5 to 60 aromatic ring atoms and may be substituted by one or more R^{d} radicals, or an arylthio- or heteroarylthio group which has 5 to 60 aromatic ring atoms and may be substituted by one or more R^{d} radicals, or a diarylamino, arylheteroarylamino, diheteroarylamino group which has 5 to 60 aromatic ring atoms and may be substituted by one or more R^{d} radicals, or an aralkyl or heteroarylalkyl group which has 5 to 60 aromatic ring atoms and 1 to 10 carbon atoms in the alkyl radical and may be substituted by one or more R^{d} radicals; at the same time, one R radical together with a further group may form a ring system;
Ar is the same or different at each instance and is an aromatic or heteroaromatic ring system which has 5 to 60 aromatic ring atoms and may be substituted by one or more R^{d} radicals; at the same time, it is possible for two Ar radicals bonded to the same carbon atom, silicon atom, nitrogen atom, phosphorus atom or boron atom also to be joined together via a bridge by a single bond or a bridge selected from B(R^{d}), C(R^{d})₂, Si(R^{d})₂, C=O, C=NR^{d}, C=C(R^{d})2, O, S, S=O, SO₂, N(R^{d}), P(R^{d}) and P(=O)R^{d};
R^{a}, R^{b}, R^{c}, R^{d} is the same or different at each instance and is H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R¹ radicals, where one or more nonadjacent CH₂ groups may be replaced by R¹C=CR¹, C=C, Si(R¹)₂, C=O, C=S, C=Se,
C=NR¹, -C(=O)O-, -C(=O)NR¹-, NR¹, P(=O)(R¹), -O-, -S-, SO or SO₂, or an aromatic or heteroaromatic ring system which has 5 to 60 aromatic ring atoms and may be substituted in each case by one or more R¹ radicals, or an aryloxy or heteroaryloxy group which has 5 to 60 aromatic ring atoms and may be substituted by one or more R¹ radicals; at the same time, two R^{a}, R^{b}, R^{c}, R^{d} radicals may also form a ring system together or with a further group;
Ar' is the same or different at each instance and is an aromatic or heteroaromatic ring system which has 5 to 60 aromatic ring atoms and may be substituted by one or more R¹ radicals; at the same time, it is possible for two Ar' radicals bonded to the same carbon atom, silicon atom, nitrogen atom, phosphorus atom or boron atom also to be joined together via a bridge by a single bond or a bridge selected from B(R¹), C(R¹)₂, Si(R¹)₂, C=O, C=NR¹, C=C(R¹)₂, O, S, S=O, SO₂, N(R¹), P(R¹) and P(=O)R¹;
R¹ is the same or different at each instance and is H, D, F, Cl, Br, I, CN, NO₂, N(Ar'')₂, N(R²)₂, C(=O)Ar", C(=O)R², P(=O)(Ar")₂, P(Ar'')₂, B(Ar")₂, B(R²)₂, C(Ar")₃, C(R²)₃, Si(Ar'')₃, Si(R²)₃, a straight-chain alkyl, alkoxy or thioalkoxy group having 1 to 40 carbon atoms or a branched or cyclic alkyl, alkoxy or thioalkoxy group having 3 to 40 carbon atoms or an alkenyl group having 2 to 40 carbon atoms, each of which may be substituted by one or more R² radicals, where one or more nonadjacent CH₂ groups may be replaced by -R²C=CR²-, -C≡C-, Si(R²)₂, C=O, C=S, C=Se, C=NR², -C(=O)O-, -C(=O)NR²-, NR², P(=O)(R²), -O-, -S-, SO or SO₂ and where one or more hydrogen atoms may be replaced by D, F, Cl, Br, I, CN or NO₂, or an aromatic or heteroaromatic ring system which has 5 to 60 aromatic ring atoms and may be substituted in each case by one or more R² radicals, or an aryloxy or heteroaryloxy group which has 5 to 60 aromatic ring atoms and may be substituted by one or more R² radicals, or an aralkyl or heteroaralkyl group which has 5 to 60 aromatic ring atoms and may be substituted by one or more R² radicals, or a combination of these systems; at the same time, two or more R¹ radicals together may form a ring system; at the same time, one or more R¹ radicals may form a ring system with a further part of the compound; Ar" is the same or different at each instance and is an aromatic or heteroaromatic ring system which has 5 to 30 aromatic ring atoms and may be substituted by one or more R² radicals; at the same time, it is possible for two Ar" radicals bonded to the same carbon atom, silicon atom, nitrogen atom, phosphorus atom or boron atom also to be joined together via a bridge by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is the same or different at each instance and is selected from the group consisting of H, D, F, CN, an aliphatic hydrocarbyl radical having 1 to 20 carbon atoms or an aromatic or heteroaromatic ring system which has 5 to 30 aromatic ring atoms and in which one or more hydrogen atoms may be replaced by D, F, Cl, Br, I or CN and which may be substituted by one or more alkyl groups each having 1 to 4 carbon atoms; at the same time, two or more substituents R² together may form a ring system; with the proviso that the structure of the formula (I) comprises at least one substructure B in which the Z group is N or C-CN.

2. Compound according to Claim 1, comprising at least one structure of the formulae (I-1) and/or (I-2): 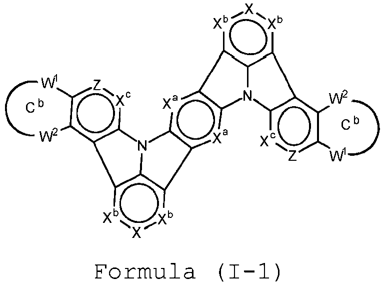 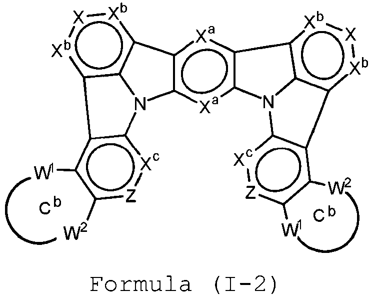 where the symbols C^{b}, W¹, W², Z, X, X^{a}, X^{b} and X^{c} have the definitions given in Claim 1.

3. Compound according to Claim 1 or 2, **characterized in that** the compound comprises at least one substructure of the formulae (B1-1) to (B1-30): 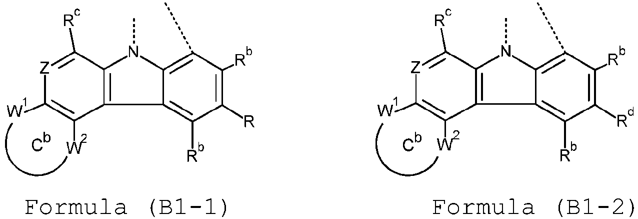 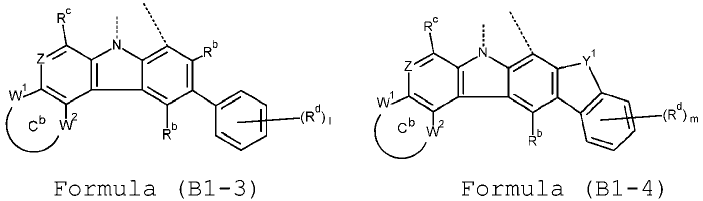 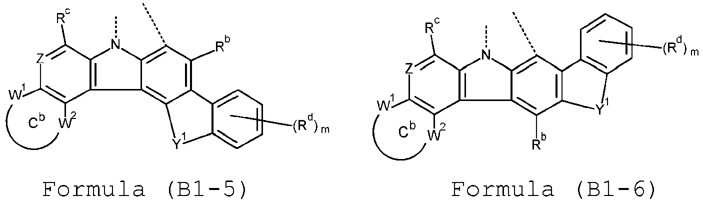 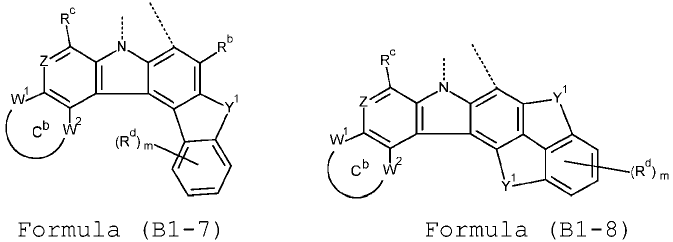 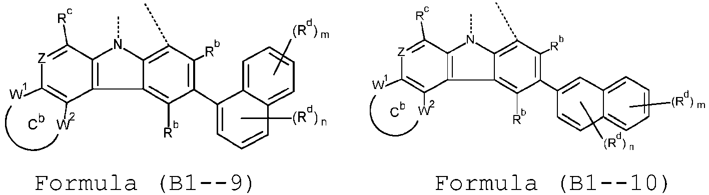 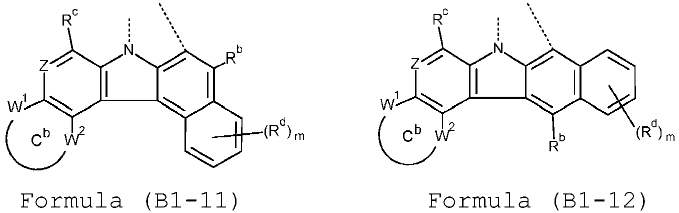 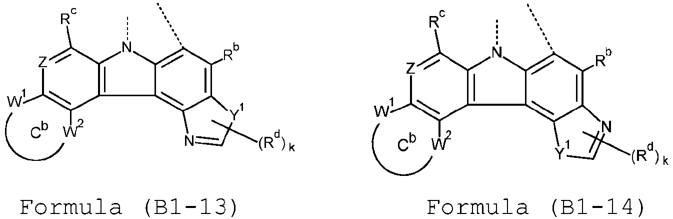 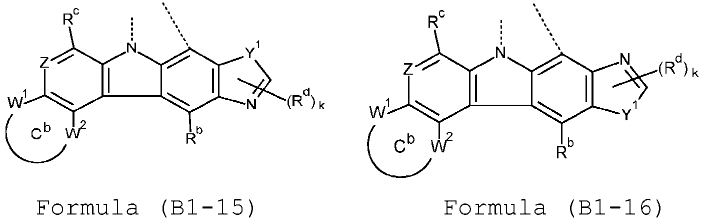 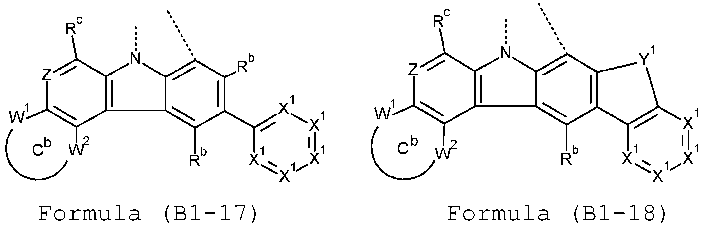 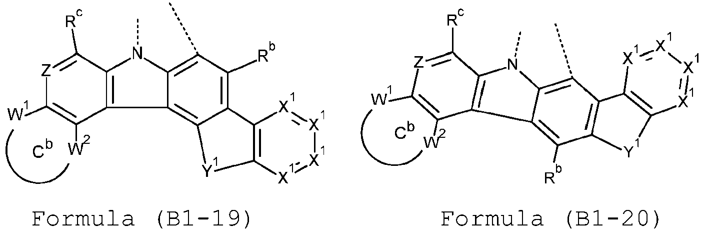 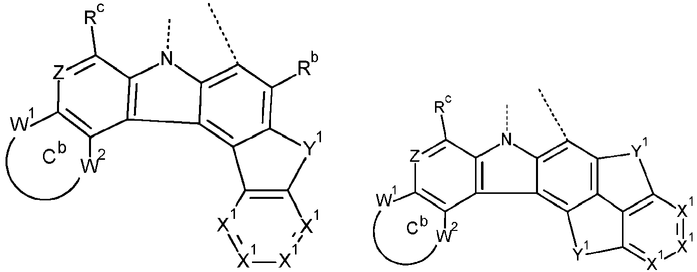 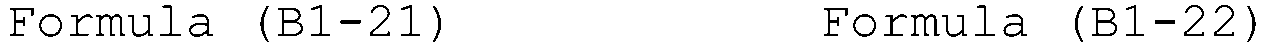 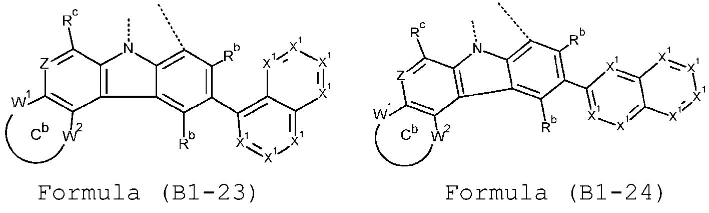 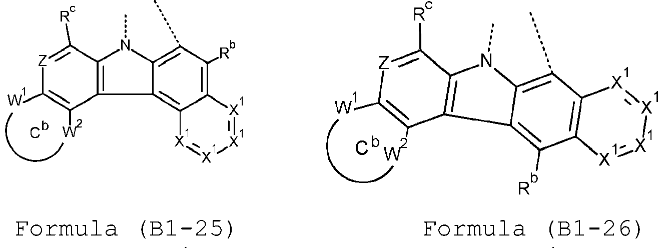 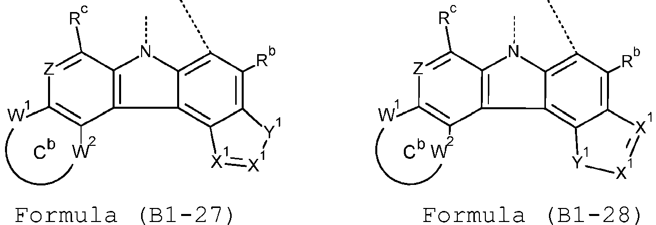 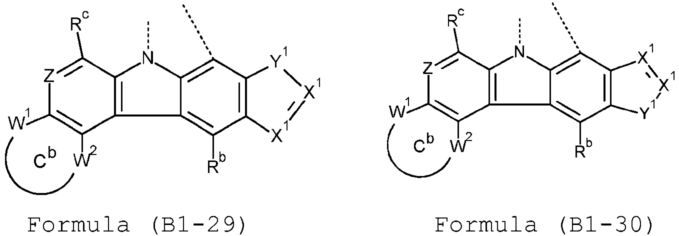 where the symbols C^{b}, W¹, W², Z, R, R^{d}, R^{c} and R^{d} have the definitions given in Claim 1, the dotted bonds represent the fusion sites of the substructure to A, and the further symbols and indices used are as follows:
X¹ is the same or different at each instance and is N or CR^{d}, with the proviso that not more than two of the X¹ groups in one cycle are N;
Y¹ is the same or different at each instance and is C(R^{d})₂, (R^{d})₂C-C(R^{d})₂, (R^{d})C=C(R^{d}), NR^{d}, NAr', O, S, SO, SO₂, Se, P(O)R^{d}, BR^{d} or Si(R^{d})₂;
k is 0 or 1;
n is 0, 1, 2 or 3;
m is 0, 1, 2, 3 or 4;
l is 0, 1, 2, 3, 4 or 5.

4. Compound according to one or more of Claims 1 to 3, comprising at least one structure of the formulae (II-1) to (II-15): 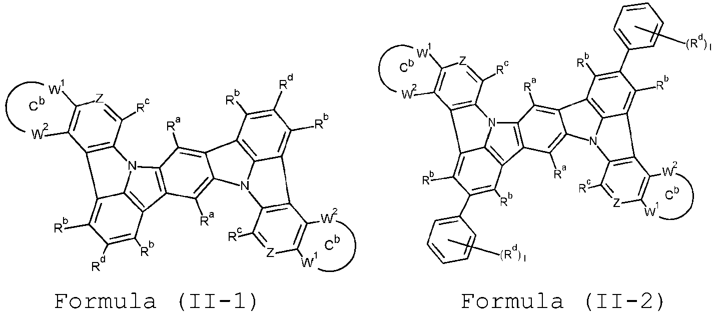 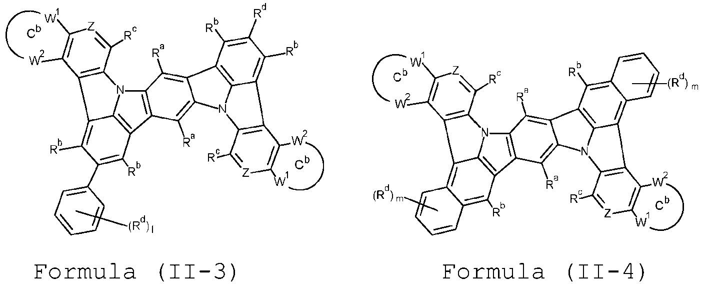 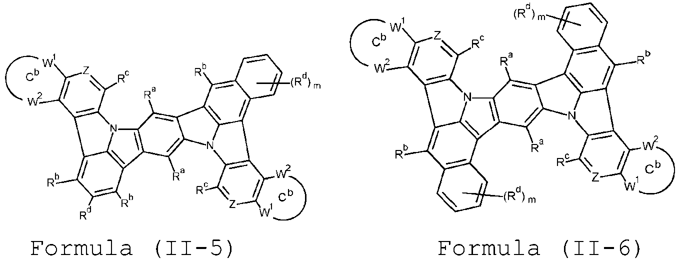 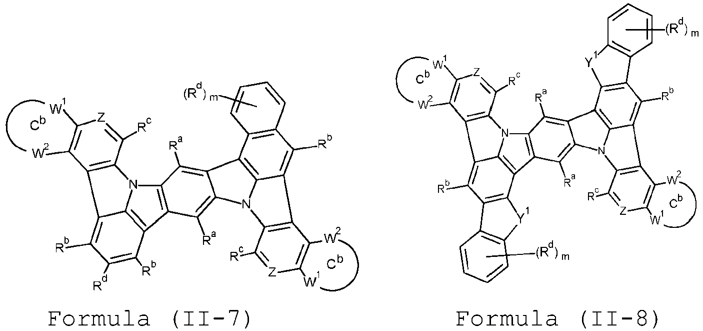 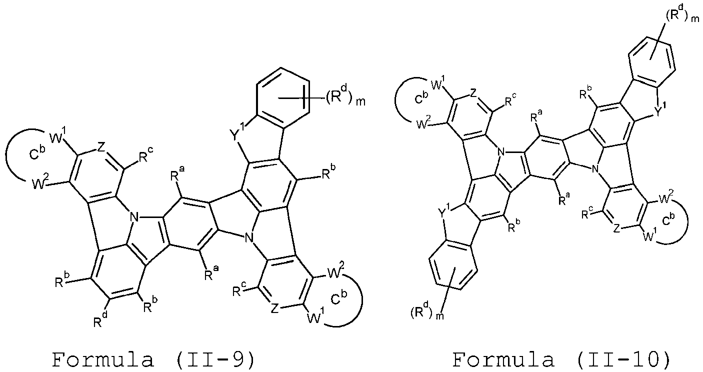 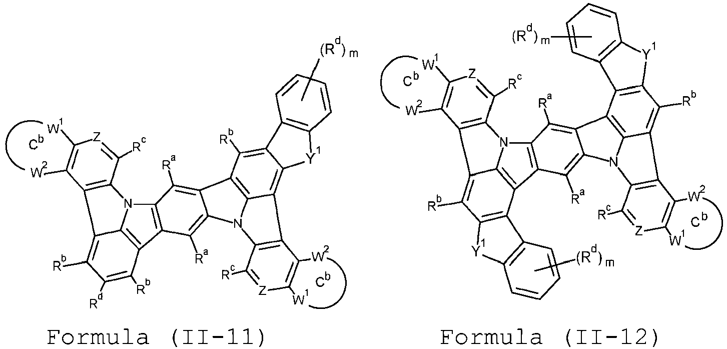 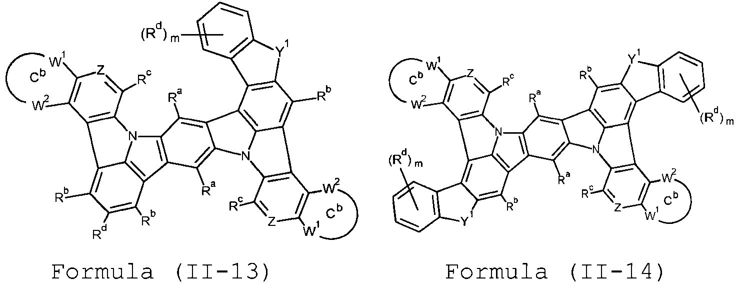 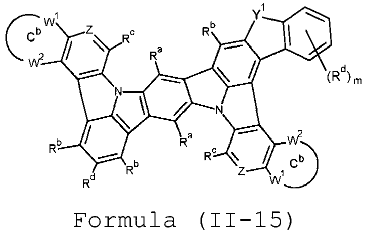 where the symbols C^{b}, W¹, W², Z, R, R^{a}, R^{b}, R^{c} and R^{d} have the definitions given in Claim 1, the symbol Y¹ has the definition given in Claim 3, and the further indices used are as follows:
m is 0, 1, 2, 3 or 4;
l is 0, 1, 2, 3, 4 or 5.

5. Compound according to one or more of Claims 1 to 4, **characterized in that** the fused ring C^{b} is selected from a structure of the formulae (BCY-1) to (BCY-10): 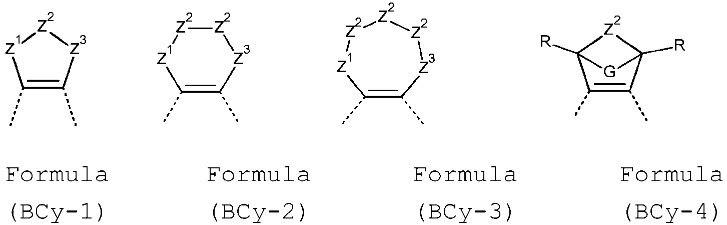 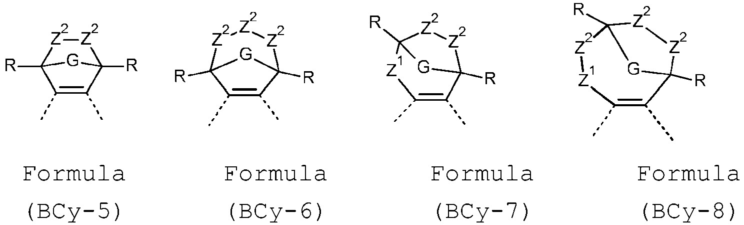 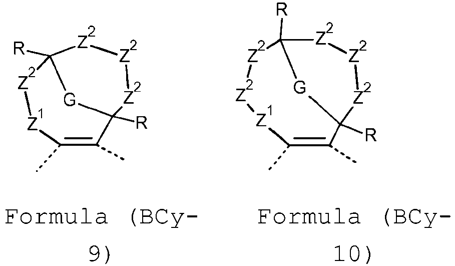 where R has the definition given in Claim 1, the dotted bonds represent the bonding sites of the fused ring to the further groups, and in addition:
Z¹, Z³ is the same or different at each instance and is C(R³)₂, O, S or Si(R³)₂;
Z² is C(R)₂, O, S, NR or C(=O), where two adjacent Z² groups may be -CR=CR- or an ortho-bonded arylene or heteroarylene group which has 5 to 14 aromatic ring atoms and may be substituted by one or more R radicals;
G is an alkylene group which has 1, 2 or 3 carbon atoms and may be substituted by one or more R radicals, -CR=CR- or an ortho-bonded arylene or heteroarylene group which has 5 to 14 aromatic ring atoms and may be substituted by one or more R radicals;
R³ is the same or different at each instance and is H, D, F, Cl, Br, I, CN, NO₂, N(Ar')₂, N(R^{d})₂, C(=O)Ar', C(=O)R^{d}, P(=O)(Ar')₂, P(Ar')₂, B(Ar')₂, B(R^{d})₂, C(Ar')₃, C(R^{d})₃, Si(Ar')₃, Si(R^{d})₃, a straight-chain alkyl, alkoxy or thioalkoxy group having 1 to 40 carbon atoms or a branched or cyclic alkyl, alkoxy or thioalkoxy group having 3 to 40 carbon atoms or an alkenyl group having 2 to 40 carbon atoms, each of which may be substituted by one or more R^{d} radicals, where one or more nonadjacent CH₂ groups may be replaced by -R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO or SO₂ and where one or more hydrogen atoms may be replaced by D, F, Cl, Br, I, CN or NO₂, or an aromatic or heteroaromatic ring system which has 5 to 60 aromatic ring atoms, each of which may be substituted by one or more R^{d} radicals, or an aryloxy or heteroaryloxy group which has 5 to 60 aromatic ring atoms and may be substituted by one or more R^{d} radicals, or an aralkyl or heteroaralkyl group which has 5 to 60 aromatic ring atoms and may be substituted by one or more R^{d} radicals, or a combination of these systems; at the same time, two R³ radicals bonded to the same carbon atom may together form an aliphatic or aromatic ring system and thus form a spiro system; in addition, R³ may form an aliphatic ring system with an R, R^{a}, R^{c} or R³ radical, where Ar' and R^{d} have the definitions given in Claim 1;
with the proviso that no two heteroatoms in these groups are bonded directly to one another and no two C=O groups are bonded directly to one another.

6. Compound according to one or more of Claims 1 to 5, **characterized in that** the fused ring C^{b} is selected from a structure of the formulae (BRA-1) to (BRA-12): 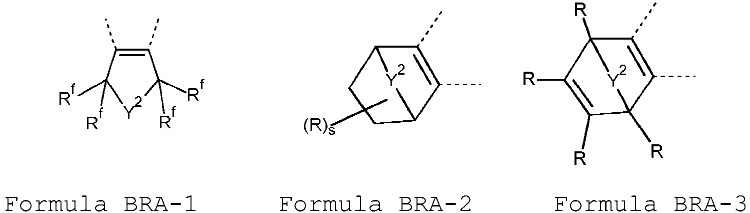 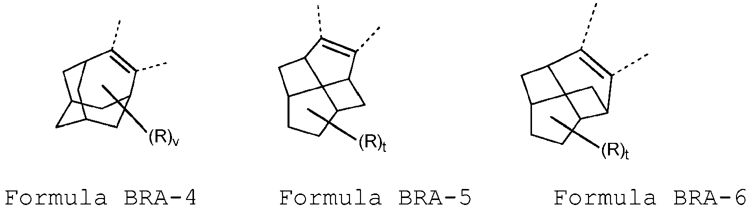 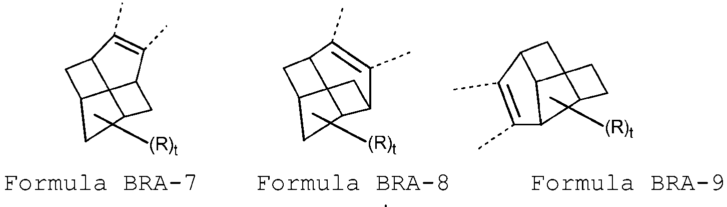 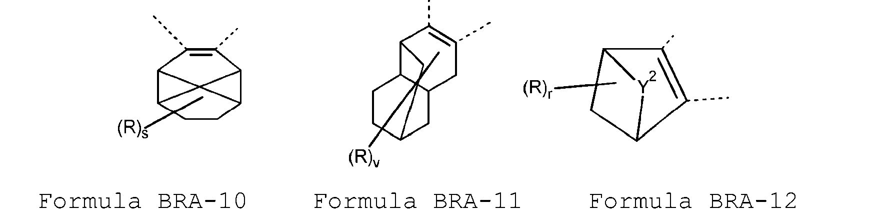 where R has the definition given in Claim 1, the dotted bonds represent the sites of attachment of the fused ring to the further groups, and the further symbols are defined as follows:
Y² is the same or different at each instance and is C(R)₂, (R)₂C-C(R)₂, (R)C=C(R), NR, NAr', O or S;
R^{f} is the same or different at each instance and is F, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R^{d} radicals, where one or more nonadjacent CH₂ groups may be replaced by R^{d}C=CR^{d}, C=C, Si(R^{d})₂, C=O, C=S, C=Se, C=NR^{d}, -C(=O)O-, -C(=O)NR^{d}-, NR^{d}, P(=O)(R^{d}), -O-, -S-, SO or SO₂, or an aromatic or heteroaromatic ring system which has 5 to 60 aromatic ring atoms and may be substituted in each case by one or more R^{d} radicals, or an aryloxy or heteroaryloxy group which has 5 to 60 aromatic ring atoms and may be substituted by one or more R^{d} radicals; at the same time, it is also possible for two R^{f} radicals together or one R^{f} radical together with an R radical or together with a further group to form a ring system, where R^{d} has the definition given in Claim 1;
r is 0, 1, 2, 3 or 4;
s is 0, 1, 2, 3, 4, 5 or 6;
t is 0, 1, 2, 3, 4, 5, 6, 7 or 8;
v is 0, 1, 2, 3, 4, 5, 6, 7, 8 or 9.

7. Compound according to one or more of Claims 1 to 6, in that the fused ring C^{b} is selected from a structure of the formulae (BRA-1a) to (BRA-3f): 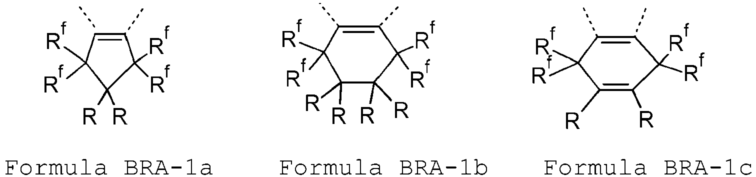 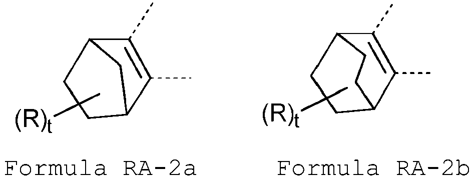 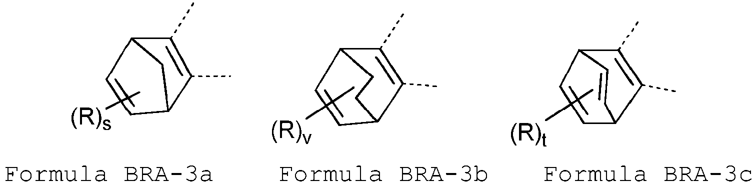 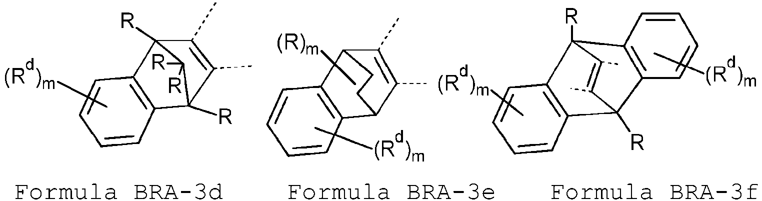 where the dotted bonds represent the sites of attachment of the fused ring to the further groups, the index m is 0, 1, 2, 3 or 4, and the symbols R, R^{d}, R^{f} and the indices s, t and v have the definition set out above, especially in Claims 1 and 6.

8. Compound according to one or more of Claims 1 to 7, **characterized in that** at least two R, R^{a}, R^{b}, R^{c}, R^{d} radicals together with the further groups to which the two R, R^{a}, R^{b}, R^{c}, R^{d} radicals bind form a fused ring, where the two R, R^{a}, R^{b}, R^{c}, R^{d} radicals form at least one structure of the following formulae (Cy-1) to (Cy-10): 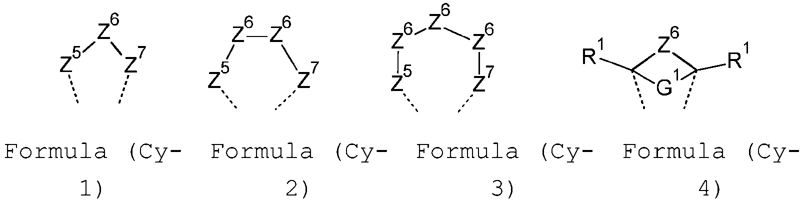 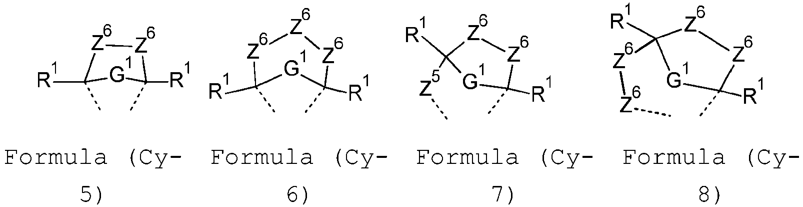 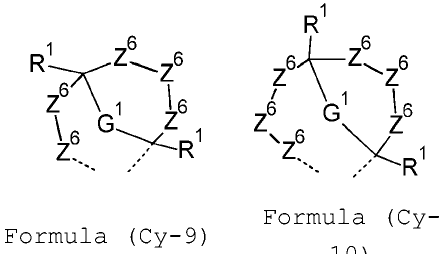 where R¹ has the definition given in Claim 1, the dotted bonds represent the sites of attachment to the atoms of the groups to which the two R, R^{a}, R^{b}, R^{c}, R^{d} radicals bind, and in addition:
Z⁵, Z⁷ is the same or different at each instance and is C(R⁴)₂, O, S, NR⁴ or C(=O);
Z⁶ is C(R¹)₂, O, S, NR¹ or C(=O), where two adjacent groups Z² may represent -CR¹=CR¹- or an ortho-bonded arylene or heteroarylene group having 5 to 14 aromatic ring atoms which may be substituted by one or more R¹ radicals;
G¹ is an alkylene group which has 1, 2 or 3 carbon atoms and may be substituted by one or more R¹ radicals, -CR¹=CR¹- or an ortho-bonded arylene or heteroarylene group which has 5 to 14 aromatic ring atoms and may be substituted by one or more R¹ radicals;
R⁴ is the same or different at each instance and is H, D, F, Cl, Br, I, CN, NO₂, N(Ar")₂, N(R²)₂, C(=O)Ar", C(=O)R², P(=O)(Ar")₂, P(Ar'')₂, B(Ar'')₂, B(R²)₂, C(Ar'')₃, C(R²)₃, Si(Ar'')₃, Si(R²)₃, a straight-chain alkyl, alkoxy or thioalkoxy group having 1 to 40 carbon atoms or a branched or cyclic alkyl, alkoxy or thioalkoxy group having 3 to 40 carbon atoms or an alkenyl group having 2 to 40 carbon atoms, each of which may be substituted by one or more R² radicals, where one or more nonadjacent CH₂ groups may be replaced by -R²C=CR²-, -C≡C-, Si(R²)₂, C=O, C=S, C=Se, C=NR², -C(=O)O-, -C(=O)NR²-, NR², P(=O)(R²), -O-, -S-, SO or SO₂ and where one or more hydrogen atoms may be replaced by D, F, Cl, Br, I, CN or NO₂, or an aromatic or heteroaromatic ring system which has 5 to 60 aromatic ring atoms, each of which may be substituted by one or more R² radicals, or an aryloxy or heteroaryloxy group which has 5 to 60 aromatic ring atoms and may be substituted by one or more R² radicals, or an aralkyl or heteroaralkyl group which has 5 to 60 aromatic ring atoms and may be substituted by one or more R² radicals, or a combination of these systems; at the same time, two R⁴ radicals bonded to the same carbon atom may together form an aliphatic or aromatic ring system and thus form a spiro system; in addition, R⁴ may form an aliphatic ring system with an R, R^{a}, R^{b}, R^{c}, R^{d} or R¹ radical; the symbols R¹ and Ar" have the definitions given in Claim 1;
with the proviso that no two heteroatoms in these groups are bonded directly to one another and no two C=O groups are bonded directly to one another.

9. Compound according to one or more of Claims 1 to 8, **characterized in that** at least two R, R^{a}, R^{b}, R^{c}, R^{d} radicals together with the further groups to which the two R, R^{a}, R^{b}, R^{c}, R^{d} radicals bind form a fused ring, where the two R, R^{a}, R^{b}, R^{c}, R^{d} radicals form at least one structure of the formulae (RA-1) to (RA-13): 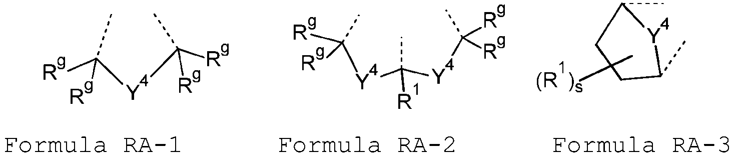 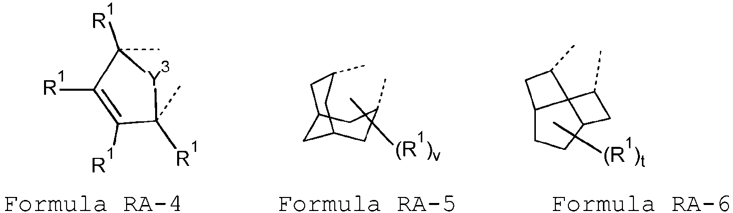 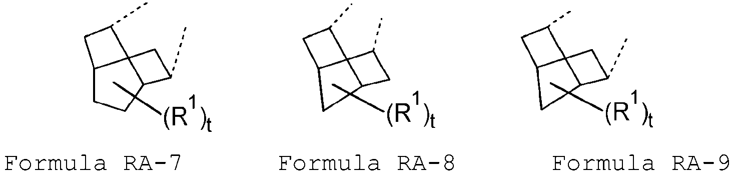 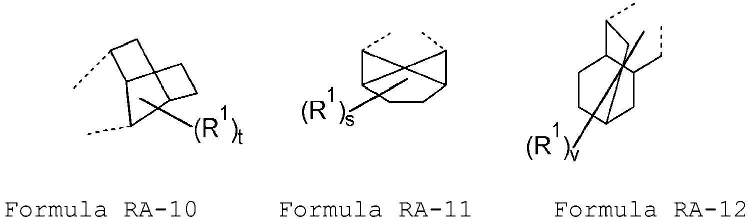 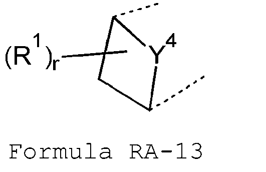 where R¹ has the definition detailed above, the dotted bonds represent the sites of attachment to the atoms of the groups to which the two R, R^{a}, R^{b}, R^{c}, R^{d} radicals bind, and the further symbols are defined as follows:
Y⁴ is the same or different at each instance and is C(R¹)₂, (R¹)₂C-C(R¹)₂, (R¹)C=C(R¹), NR¹, NAr', O or S;
R^{g} is the same or different at each instance and is F, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R² radicals, where one or more nonadjacent CH₂ groups may be replaced by R²C=CR², C=C, Si(R²)₂, C=O, C=S, C=Se, C=NR², -C(=O)O-, -C(=O)NR²-, NR², P(=O) (R²), -O-, -S-, SO or SO₂, or an aromatic or heteroaromatic ring system which has 5 to 60 aromatic ring atoms and may be substituted in each case by one or more R² radicals, or an aryloxy or heteroaryloxy group which has 5 to 60 aromatic ring atoms and may be substituted by one or more R² radicals; at the same time, it is also possible for two R^{g} radicals together or one R^{g} radical together with an R¹ radical or together with a further group to form a ring system, where R² has the definition given in Claim 1;
r is 0, 1, 2, 3 or 4;
s is 0, 1, 2, 3, 4, 5 or 6;
t is 0, 1, 2, 3, 4, 5, 6, 7 or 8;
v is 0, 1, 2, 3, 4, 5, 6, 7, 8 or 9.

10. Compound according to one or more of Claims 1 to 9, **characterized in that** at least two R, R^{a}, R^{b}, R^{c}, R^{d} radicals together with the further groups to which the two R, R^{a}, R^{b}, R^{c}, R^{d} radicals bind form a fused ring, where the two R, R^{a}, R^{b}, R^{c}, R^{d} radicals form structures of the formula (RB): 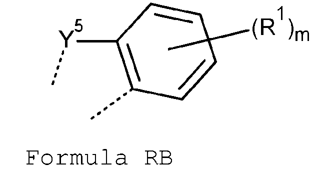 where R¹ has the definition set out in Claim 1, the dotted bonds represent the sites of attachment via which the two R, R^{a}, R^{b}, R^{c}, R^{d} radicals bind, the index m is 0, 1, 2, 3 or 4, and Y⁵ is C(R¹)₂, NR¹, NAr', BR¹, BAr', O or S.

11. Compound according to one or more of Claims 1 to 10, **characterized in that** R or Ar is the same or different at each instance and is selected from phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, quinoline, isoquinoline, quinazoline, quinoxaline, phenanthrene or triphenylene, each of which may be substituted by one or more R^{d} radicals.

12. Compound according to at least one of the preceding claims, **characterized in that** the R^{d} and/or R^{d} radical represents at least one group selected from C(Ar')₃, C(R¹)₃, Si(Ar')₃, Si(R¹)₃, B(R¹)₂, or represents or, together with an R^{b} or R^{d} radical, forms a fluorene group that may be substituted by one or more R¹ radicals.

13. Compound according to at least one of the preceding claims, **characterized in that** the compound comprises exactly two or exactly three structures of formula (I), (I-1), (I-2) and/or (II-1) to (II-15).

14. Oligomer, polymer or dendrimer containing one or more compounds according to any of Claims 1 to 13, wherein, in place of a hydrogen atom or a substituent, there are one or more bonds of the compounds to the polymer, oligomer or dendrimer.

15. Formulation comprising at least one compound according to one or more of Claims 1 to 13 or an oligomer, polymer or dendrimer according to Claim 14 and at least one further compound, where the further compound is preferably selected from one or more solvents.

16. Composition comprising at least one compound according to one or more of Claims 1 to 13 or an oligomer, polymer or dendrimer according to Claim 14 and at least one further compound selected from the group consisting of fluorescent emitters, phosphorescent emitters, emitters that exhibit TADF, host materials, electron transport materials, electron injection materials, hole conductor materials, hole injection materials, electron blocker materials and hole blocker materials, preferably host materials.

17. Composition according to Claim 16, **characterized in that** the composition at least one further compound is a TADF host material and/or at least one further compound is a phosphorescent emitter (triplet emitter), where the further compounds preferably form a hyperfluorescence system and/or hyperphosphorescence system with a compound according to one or more of Claims 1 to 13 or an oligomer, polymer or dendrimer according to Claim 14.

18. Process for preparing a compound according to one or more of Claims 1 to 13, **characterized in that** a base skeleton having an aromatic amino group is synthesized and at least one aromatic or heteroaromatic radical is introduced, preferably by means of a nucleophilic aromatic substitution reaction or a coupling reaction.

19. Use of a compound according to one or more of Claims 1 to 13 or an oligomer, polymer or dendrimer according to Claim 14 in an electronic device.

20. Electronic device comprising at least one compound according to one or more of Claims 1 to 13 or an oligomer, polymer or dendrimer according to Claim 14.

## Revendications

1. Composé comprenant au moins une structure de formule (I), 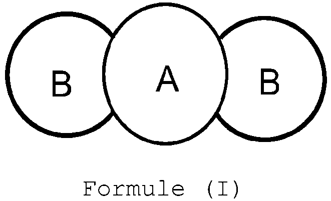
dans laquelle A est identique ou différent à chaque occurrence pour une structure partielle de formule (A1) ou (A2), 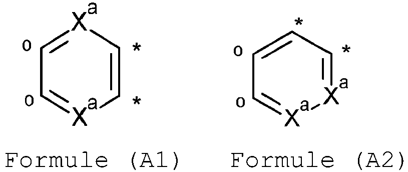
sur laquelle deux structures partielles B sont condensées, et les symboles o et * représentent les deux positions de condensation de la structure partielle B respective, une structure partielle B étant condensée sur A par l'intermédiaire des positions marquées par o et une structure partielle B étant condensée sur A par l'intermédiaire des positions marquées par * et B représentant en chaque occurrence, de manière identique ou différente, une structure partielle de formule (B) 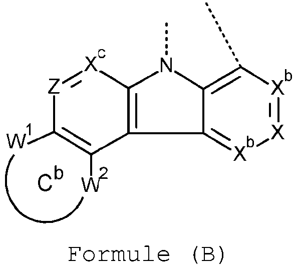
dans laquelle les liaisons en pointillés représentent les sites de condensation de la structure partielle B sur A, le cycle C^{b} représente en chaque occurrence, de manière identique ou différente, un cycle aliphatique ou hétéroaliphatique condensé ayant de 5 à 60 atomes de cycle, qui peut être substitué par un ou plusieurs radicaux R,
et, pour les autres symboles, ce qui suit est d'application :
Z représente en chaque occurrence, de manière identique ou différente, N, C-CN ou CR^{e} ;
W¹, W² représentent en chaque occurrence, de manière identique ou différente C(R)₂, O, S, Si(R)₂ ;
X représente en chaque occurrence, de manière identique ou différente, N ou CR , étant entendu que pas plus de deux des groupes X, X^{b} dans un cycle ne représentent N ;
X^{a} représente en chaque occurrence, de manière identique ou différente, N ou CR^{a} ;
X^{b} représente en chaque occurrence, de manière identique ou différente, N ou CR^{b}, étant entendu que pas plus de deux des groupes X, X^{b} dans un cycle ne représentent N ;
X^{c} représente en chaque occurrence, de manière identique ou différente, N ou CR^{c} ;
R représente en chaque occurrence, de manière identique ou différente, H, D, OH, F, Cl, Br, I, CN, NO₂, N(Ar)₂, N(R^{d})₂, C(=O)N(Ar)₂, C(=O)N(R^{d})₂, C(Ar)₃, C(R^{d})₃, Si(Ar)₃, Si(R^{d})₃, B(Ar)₂, B(R^{d})₂, C(=O)Ar, C(=O)R^{d}, P(=O)(Ar)₂, P(=O)(R^{d})₂, P(Ar)₂, P(R^{d})₂, S(=O)Ar, S(=O)R^{d}, S(=O)₂Ar, S (=O)₂R^{d}, OSO₂Ar, OSO₂R^{d}, un groupe alkyle, alcoxy ou thioalcoxy à chaîne droite ayant de 1 à 40 atomes de carbone ou un groupe alcényle ou alcynyle ayant de 2 à 40 atomes de carbone ou un groupe alkyle, alcoxy ou thioalcoxy ramifié ou cyclique ayant de 3 à 20 atomes de carbone, le groupe alkyle, alcoxy, thioalcoxy, alcényle ou alcynyle pouvant être respectivement substitué par un ou plusieurs radicaux R^{d}, où un ou plusieurs groupes CH₂ non voisins peuvent être remplacés par R^{d}C=CR^{d}, C=C, Si (R^{d})₂, C=O, C=S, C=Se, C=NR^{d}, -C(=O)O-, -C(=O)NR^{d}-, NR^{d}, P(=O)(R^{d}), -O-, -S-, SO ou SO₂ , ou un système cyclique aromatique ou hétéroaromatique ayant 5 à 60 atomes de cycle aromatique, qui peut être substitué respectivement par un ou plusieurs radicaux R^{d}, ou un groupe aryloxy ou hétéroaryloxy ayant 5 à 60 atomes de cycle aromatiques, qui peut être substitué par un ou plusieurs restes R^{d}, ou un groupe arylthio ou hétéroarylthio ayant 5 à 60 atomes de cycle aromatique, qui peut être substitué par un ou plusieurs radicaux R^{d}, ou un groupe diarylamino, arylhétéroarylamino, dihétéroarylamino ayant 5 à 60 atomes de cycle aromatique, qui peut être substitué par un ou plusieurs radicaux R^{d}, ou un groupe arylalkyle ou hétéroarylalkyle ayant 5 à 60 atomes de cycle aromatique et 1 à 10 atomes de carbone dans le radical alkyle, qui peut être substitué par un ou plusieurs radicaux R^{d} ; un radical R pouvant alors former un système cyclique avec un autre groupe ;
Ar représente en chaque occurrence, de manière identique ou différente, un système cyclique aromatique ou hétéroaromatique comprenant 5 à 60 atomes de cycle aromatique, qui peut être substitué par un ou plusieurs radicaux R^{d}, deux radicaux Ar, qui sont liés au même atome de C, de Si, de N, de P ou de B, pouvant également être pontés l'un avec l'autre par une simple liaison ou par un pont, choisis parmi B (R^{d}), C (R^{d})₂, Si(R^{d})₂, C=O, C=NR^{d}, C=C (R^{d})₂, O, S, S=O, SO₂, N (R^{d}), P(R^{d}) et P (=O) R^{d} ;
R^{a}, R^{b}, R^{c}, R^{d} représentent en chaque occurrence, de manière identique ou différente H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si (Ar')₃, Si(R¹)₃, B(Ar')₂, B(R¹)₂, C(=O)Ar', C(=O)R¹, P (=O) (Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, un groupe alkyle, alcoxy ou thioalcoxy à chaîne droite ayant de 1 à 40 atomes de carbone ou un groupe alcényle ou alcynyle ayant de 2 à 40 atomes de carbone ou un groupe alkyle, alcoxy ou thioalcoxy ramifié ou cyclique ayant de 3 à 20 atomes de carbone, où le groupe alkyle, alcoxy ou thioalcoxy peut respectivement être substitué par un ou plusieurs radicaux R¹, un ou plusieurs groupes CH₂ non voisins pouvant être remplacés par R¹C=CR¹, C=C, Si(R¹)₂, C=O, C=S, C=Se,
C=NR¹, -C(=O)O-, -C(=O)NR¹-, NR¹, P(=O)(R¹), -O-, -S-, SO ou SO₂, ou un système cyclique aromatique ou hétéroaromatique comprenant 5 à 60 atomes de cycle aromatique, qui peut respectivement être substitué par un ou plusieurs radicaux R¹, ou un groupe aryloxy ou hétéroaryloxy comprenant 5 à 60 atomes de cycle aromatique, qui peut être substitué par un ou plusieurs radicaux R¹ ; deux radicaux R^{a}, R^{b}, R^{c}, R^{d} pouvant également former, les uns avec les autres ou avec un autre groupe, un système cyclique ;
Ar' représente, en chaque occurrence, de manière identique ou différente, un système cyclique aromatique ou hétéroaromatique comprenant 5 à 60 atomes de cycle aromatique, qui peut être substitué par un ou plusieurs radicaux R¹, deux radicaux Ar', qui sont liés au même atome de C, de Si, de N, de P ou de B, également par une simple liaison ou par un pont, choisi parmi B(R¹), C(R¹)₂, Si(R¹)₂, C=O, C=NR¹, C=C(R¹)₂, O, S, S=O, SO₂, N(R¹), P(R¹) et P(=O)R¹, pouvant être pontés l'un avec l'autre ;
R¹ représente en chaque occurrence, de manière identique ou différente, H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, un groupe alkyle, alcoxy ou thioalcoxy à chaîne droite ayant de 1 à 40 atomes de carbone ou un groupe alkyle, alcoxy ou thioalcoxy ramifié ou cyclique ayant de 3 à 40 atomes de carbone, ou un groupe alcényle ayant de 2 à 40 atomes de carbone, qui peut respectivement être substitué par un ou plusieurs radicaux R², un ou plusieurs groupes CH₂ non voisins pouvant être remplacés par - R²C=CR²-, -C=C-, Si(R²)₂, C=O, C=S, C=Se, C=NR², -C(=O)O-, -C(=O)NR²-, NR², P (=O) (R²), -O-, -S-, SO ou SO₂ et un ou plusieurs atomes H pouvant être remplacés par D, F, Cl, Br, I, CN ou NO₂, ou un système cyclique aromatique ou hétéroaromatique ayant 5 à 60 atomes de cycle aromatique, qui peut respectivement être substitué par un ou plusieurs radicaux R², ou un groupe aryloxy ou hétéroaryloxy ayant 5 à 60 atomes de cycle aromatique, qui peut être substitué par un ou plusieurs radicaux R², ou un groupe aralkyle ou hétéroaralkyle ayant 5 à 60 atomes de cycle aromatique, qui peut être substitué par un ou plusieurs radicaux R², ou une combinaison de ces systèmes ; deux ou plus de deux radicaux R¹ pouvant former entre eux un système cyclique, un ou plusieurs radicaux R¹ pouvant former un système cyclique avec une autre partie du composé ;
Ar" représente en chaque occurrence, de manière identique ou différente, un système cyclique aromatique ou hétéroaromatique comprenant 5 à 30 atomes de cycle aromatique, qui peut être substitué par un ou plusieurs radicaux R², deux radicaux Ar", qui sont liés au même atome de C, de Si, de N, de P ou de B, également par une simple liaison ou par un pont, choisis parmi B(R²), C(R²)₂, Si(R²)₂, C=O, C=NR², C=C (R²)₂, O, S, S=O, SO₂, N(R²), P(R²) et P(=O)R², peuvent être pontés l'un avec l'autre ;
R² est choisi, en chaque occurrence, de manière identique ou différente, dans le groupe constitué par H, D, F, CN, un radical hydrocarboné aliphatique comprenant 1 à 20 atomes C ou un système cyclique aromatique ou hétéroaromatique comprenant 5 à 30 atomes de cycle aromatique, dans lequel un ou plusieurs atomes de H peuvent être remplacés par D, F, Cl, Br, I ou CN et qui peut être substitué par un ou plusieurs groupes alkyle comprenant à chaque fois 1 à 4 atomes de cycle aromatique, deux substituants R² ou plus pouvant former un système cyclique les uns avec les autres.
à condition que la structure de formule (I) comprenne au moins une structure partielle B dans laquelle le groupe Z représente N ou C-CN.

2. Composé selon la revendication 1, comprenant au moins une structure des formules (I-1) et/ou (I-2), 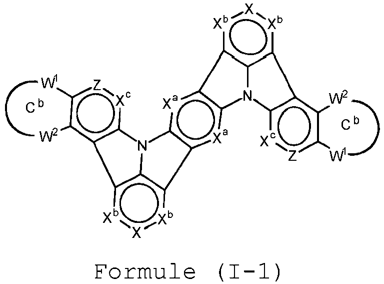 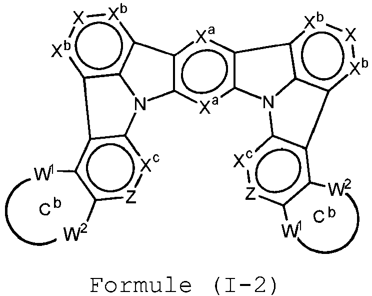 dans lesquelles les symboles C^{b}, W¹, W², Z, X, X^{a}, X^{b} et X^{c} ont les significations indiquées dans la revendication 1.

3. Composé selon la revendication 1 ou 2, **caractérisé en ce que** le composé comprend au moins une structure partielle de formules (B1-1) à (B1-30), 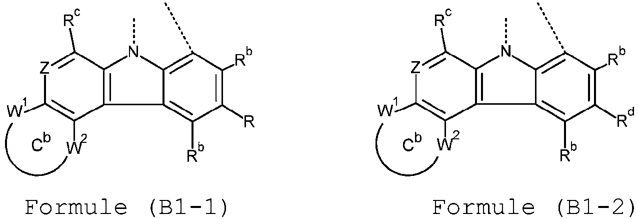 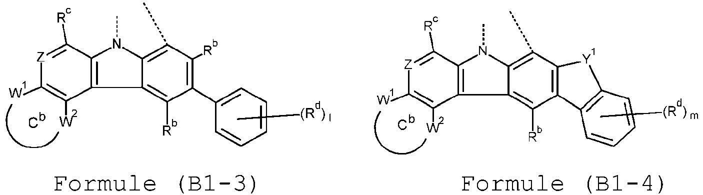 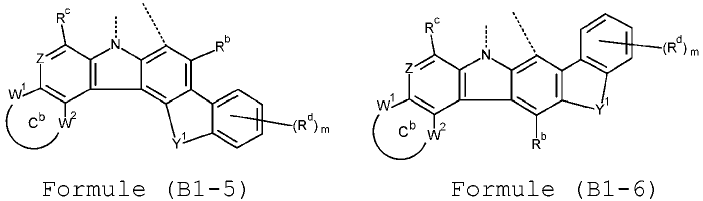 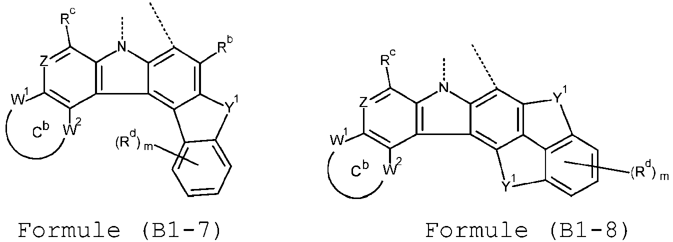 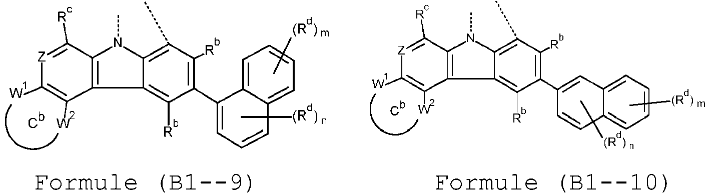 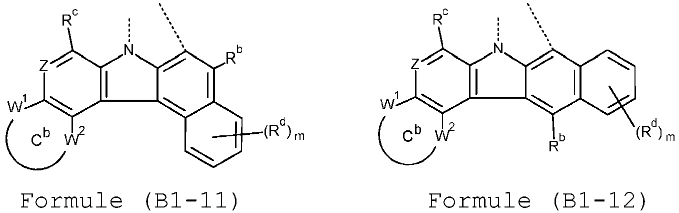 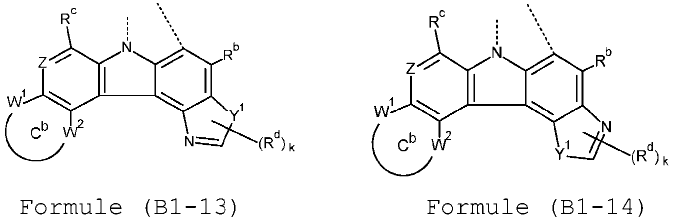 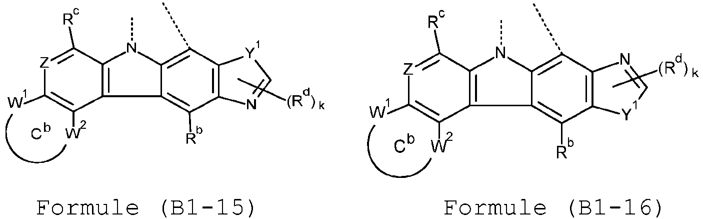 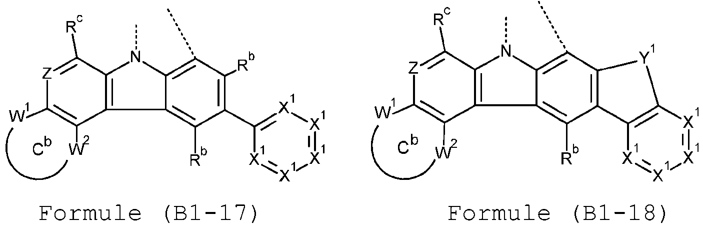 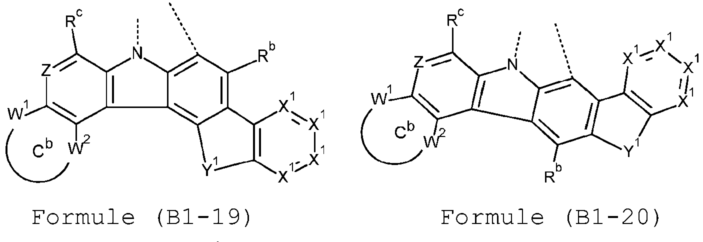 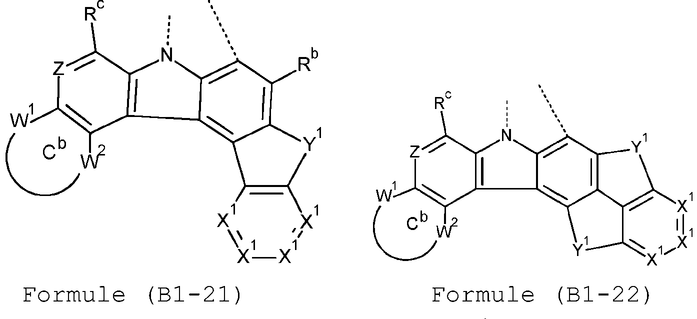 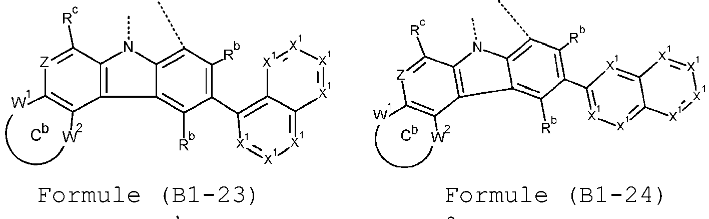 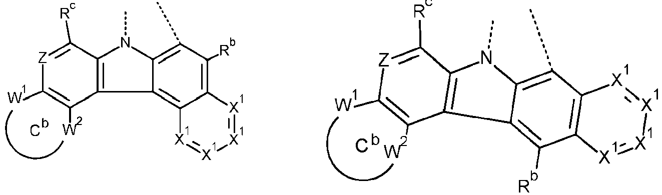 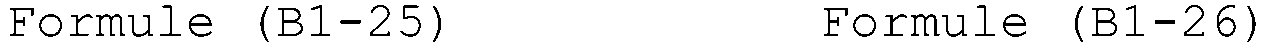 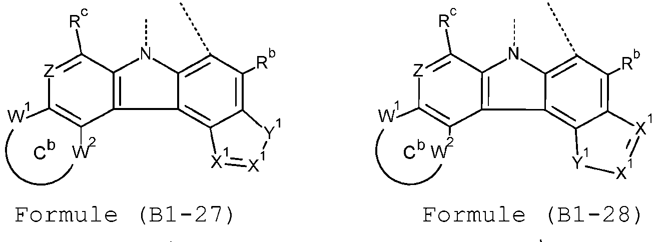 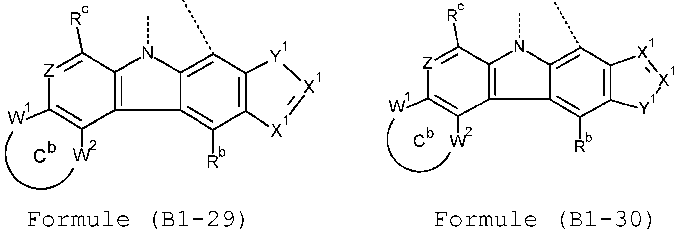 dans lesquelles les symboles C^{b}, W¹, W², Z, R, R^{b}, R^{c} et R^{d} ont les significations indiquées dans la revendication 1, les liaisons en pointillés représentent les sites de condensation de la structure partielle sur A, et pour les autres symboles et indices utilisés :
X¹ représente en chaque occurrence, de manière identique ou différente, N ou CR^{d}, étant entendu que pas plus de deux des groupes X¹ dans un cycle ne représentent N ;
Y¹ représente en chaque occurrence, de manière identique ou différente C(R^{d})₂, (R^{d})₂C-C(R^{d})₂, (R^{d})C=C (R^{d}), NR^{d}, NAr', O, S, SO, SO₂, Se, P(O)R^{d}, BR^{d} ou Si(R^{d})₂;
k représente 0 ou 1 ;
n représente 0, 1, 2 ou 3 ;
m représente 0, 1, 2, 3 ou 4 ;
l représente 0, 1, 2, 3, 4 ou 5.

4. Composé selon l'une ou plusieurs des revendications 1 à 3, comprenant au moins une structure des formules (II-1) à (II-15), 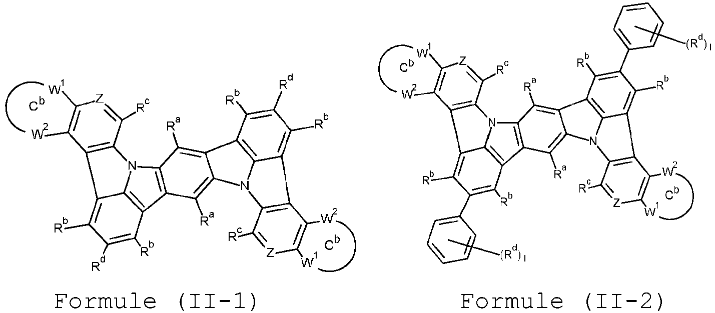 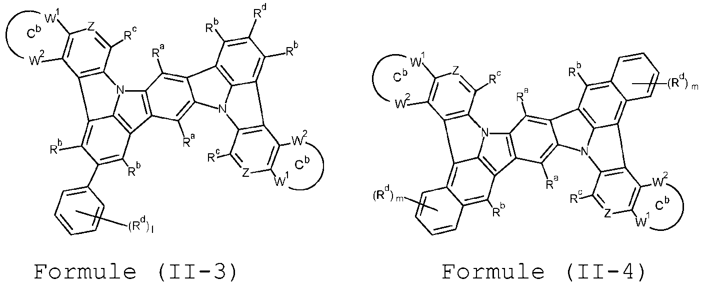 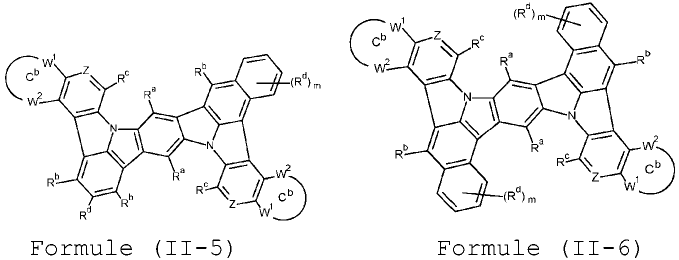 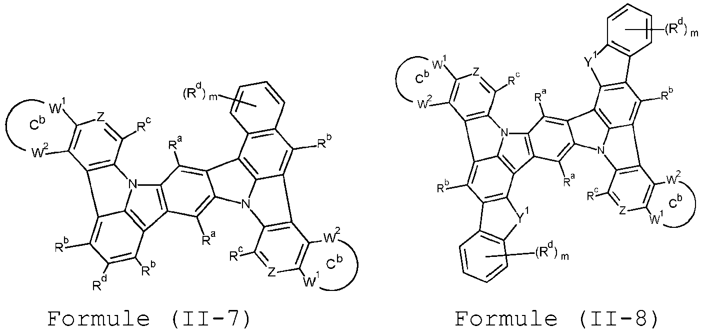 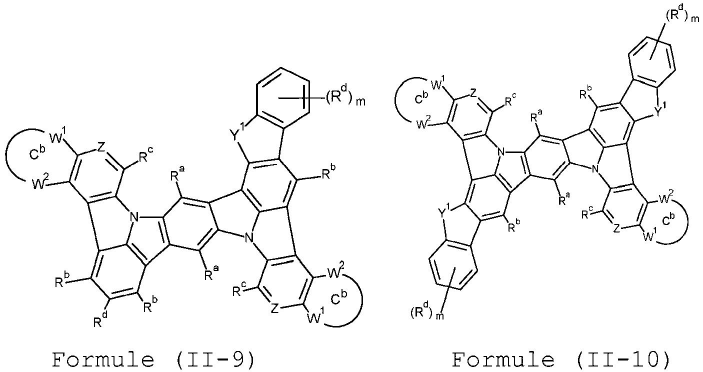 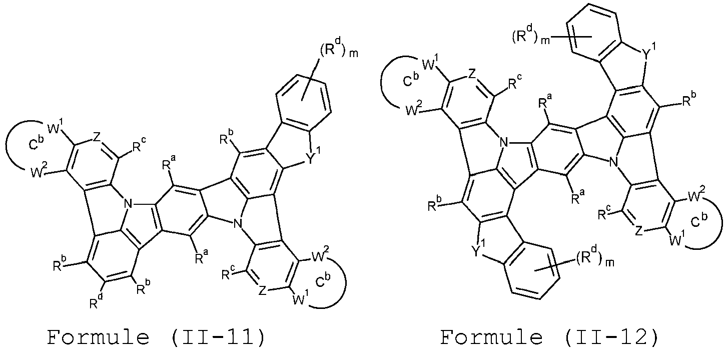 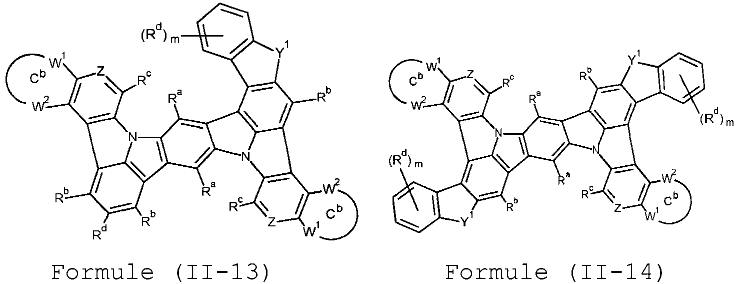 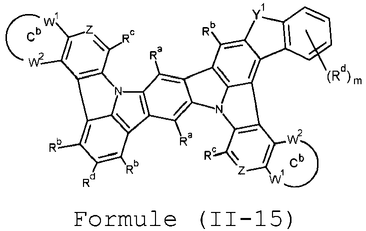 dans lesquelles les symboles C^{b}, W¹, W², Z, R, R^{a}, R^{b}, R^{c} et R^{d} ont les significations indiquées dans la revendication 1, le symbole Y¹ a les significations indiquées dans la revendication 3, et pour les autres indices utilisés :
m représente 0, 1, 2, 3 ou 4 ;
l représente 0, 1, 2, 3, 4 ou 5.

5. Composé selon une ou plusieurs des revendications 1 à 4, **caractérisé en ce que** le cycle condensé C^{b} est choisi parmi une structure de formules (BCY-1) à (BCY-10), 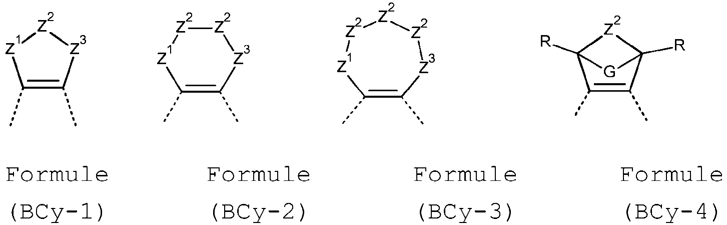 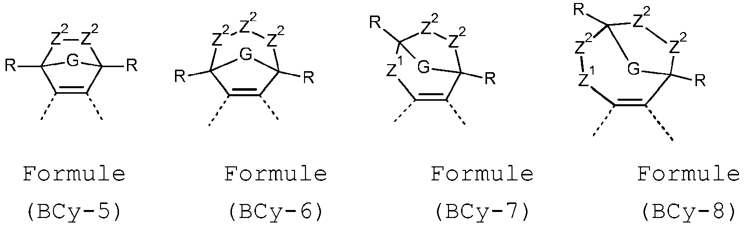 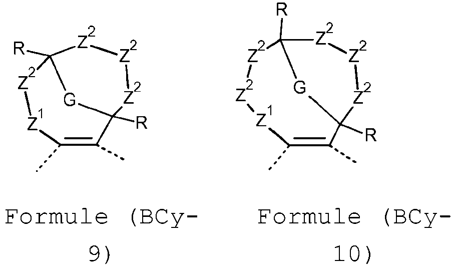 dans lesquelles R a la signification indiquée dans la revendication 1, les liaisons en pointillés représentent les sites de liaison du cycle condensé aux autres groupes et en outre :
Z¹, Z³ représentent en chaque occurrence, de manière identique ou différente, C(R³)₂, O, S ou Si(R³)₂ ;
Z² représente C(R)₂, O, S, NR ou C(=O), deux groupes Z² voisins pouvant représenter -CR=CR- ou un groupe arylène ou hétéroarylène lié en ortho ayant 5 à 14 atomes de cycle aromatique, qui peut être substitué par un ou plusieurs radicaux R ;
G représente un groupe alkylène ayant 1, 2 ou 3 atomes de carbone, qui peut être substitué par un ou plusieurs radicaux R, -CR=CR- ou un groupe arylène ou hétéroarylène lié en ortho ayant 5 à 14 atomes de cycle aromatique, qui peut être substitué par un ou plusieurs radicaux R ;
R³ représente en chaque occurrence, de manière identique ou différente, H, D, F, Cl, Br, I, CN, NO₂, N(Ar')₂, N(R^{d})₂, C(=O)Ar', C(=O)R^{d} P(=O)(Ar')₂, P(Ar')₂, B(Ar')₂, B(R^{d})₂, C(Ar')₃, C(R^{d})₃, Si(Ar')₃, Si(R^{d})₃, un groupe alkyle, alcoxy ou thioalcoxy à chaîne droite ayant de 1 à 40 atomes de carbone ou un groupe alkyle, alcoxy ou thioalcoxy ramifié ou cyclique ayant de 3 à 40 atomes de carbone ou un groupe alcényle ayant de 2 à 40 atomes de carbone, qui peut respectivement être substitué par un ou plusieurs radicaux R^{d}, un ou plusieurs groupes CH₂ non voisins pouvant être remplacés par -R^{d}C=CR^{d}-, -C=C-, Si(R^{d})₂, C=O, C=S, C=Se, C=NR^{d}, -C(=O)O-, -C(=O)NR^{d}-, NR^{d}, P(=O)(R^{d}), -O-, -S-, SO ou SO₂ et un ou plusieurs atomes H pouvant être remplacés par D, F, Cl, Br, I, CN ou NO₂, ou un système cyclique aromatique ou hétéroaromatique ayant 5 à 60 atomes de cyclique aromatique, qui peut respectivement être substitué par un ou plusieurs radicaux R^{d}, ou un groupe aryloxy ou hétéroaryloxy ayant 5 à 60 atomes de cycle aromatique, qui peut être substitué par un ou plusieurs radicaux R^{d}, ou un groupe aralkyle ou hétéroaralkyle ayant 5 à 60 atomes de cycle aromatique, qui peut être substitué par un ou plusieurs radicaux R^{d}, ou une combinaison de ces systèmes ; deux radicaux R³ liés au même atome de carbone peuvent former ensemble un système cyclique aliphatique ou aromatique et constituer ainsi un système spiro ; en outre, R³ pouvant former un système cyclique aliphatique avec un radical R, R^{a}, R^{c} ou R³, Ar' et R^{d} ayant les significations indiquées dans la revendication 1 ;
à condition que dans ces groupes, deux hétéroatomes ne soient pas directement liés l'un à l'autre et que deux groupes C=O ne soient pas directement liés l'un à l'autre.

6. Composé selon une ou plusieurs des revendications 1 à 5, **caractérisé en ce que** le cycle condensé C^{b} est choisi parmi une structure de formules (BRA-1) à (BRA-12), 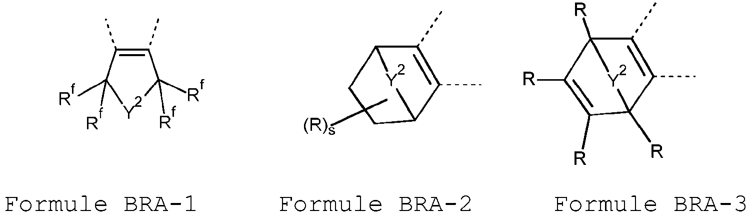 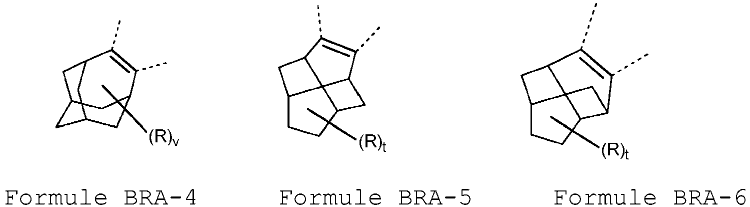 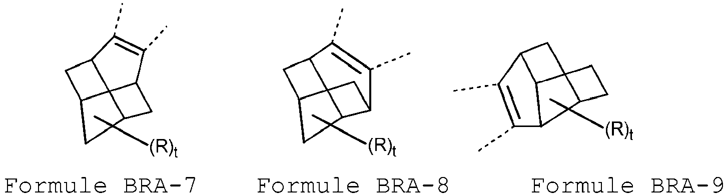 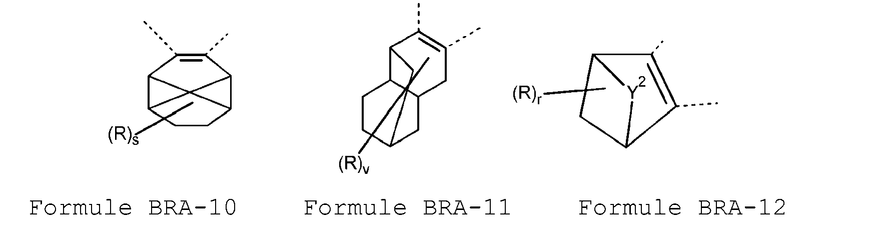 dans lesquelles R a la signification indiquée dans la revendication 1, les liaisons en pointillés représentent les sites de liaison du cycle condensé aux autres groupes et les autres symboles présentent la signification suivante :
Y² représente en chaque occurrence, de manière identique ou différente, C(R)₂, (R)₂C-C(R)₂, (R)C=C(R), NR, NAr", O ou S ;
R^{f} représente en chaque occurrence, de manière identique ou différente, F, un groupe alkyle, alcoxy ou thioalcoxy à chaîne droite comprenant 1 à 40 atomes de carbone ou un groupe alcényle ou alcynyle comprenant 2 à 40 atomes de carbone ou un groupe alkyle, alcoxy ou thioalcoxy ramifié ou cyclique comprenant 3 à 20 atomes de carbone, le groupe alkyle, alcoxy, thioalcoxy, alcényle ou alcynyle pouvant à chaque fois être substitué par un ou plusieurs radicaux R^{d}, un ou plusieurs groupes CH₂ non voisins pouvant être remplacés par R^{d}C=CR^{d}, C=C, Si(R^{d})₂, C=O, C=S, C=Se, C=NR^{d}, -C(=O)O-, -C(=O)NR^{d}-, NR^{d}, P(=O)(R^{d}), -O-, -S-, SO ou SO₂, ou un système cyclique aromatique ou hétéroaromatique comprenant 5 à 60 atomes de cycle aromatique, qui peut à chaque fois être substitué par un ou plusieurs radicaux R^{d}, ou un groupe aryloxy ou hétéroaryloxy comprenant 5 à 60 atomes de cycle aromatique, qui peut être substitué par un ou plusieurs radicaux R^{d} ; deux radicaux R^{f} l'un avec l'autre ou un radical R^{f} avec un radical R ou avec un autre groupe pouvant également former un système cyclique, R^{d} présentant la signification indiquée dans la revendication 1 ;
m représente 0, 1, 2, 3 ou 4 ;
s représente 0, 1, 2, 3, 4, 5 ou 6 ;
t représente 0, 1, 2, 3, 4, 5, 6, 7 ou 8 ;
v représente 0, 1, 2, 3, 4, 5, 6, 7, 8 ou 9.

7. Composé selon une ou plusieurs des revendications 1 à 6, en ce que le cycle condensé C^{b} est choisi parmi une structure de formules (BRA-1a) à (BRA-3f) 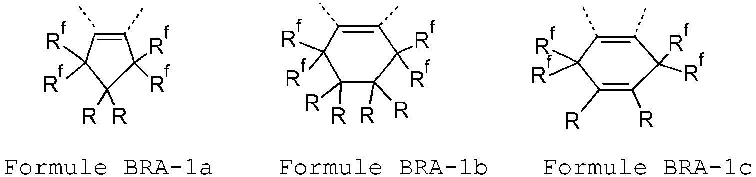 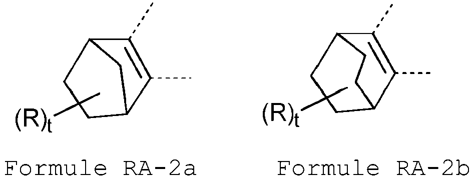 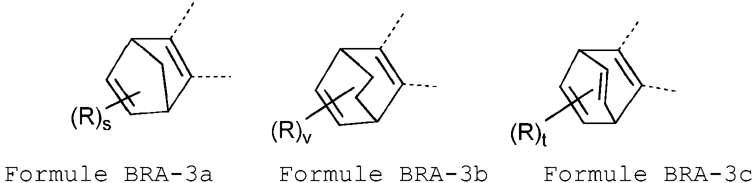 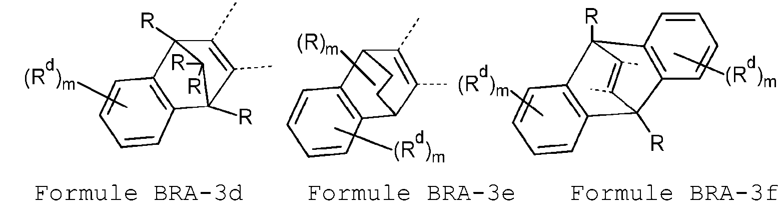 les liaisons en pointillés représentant les sites de liaison du cycle condensé aux autres groupes, l'indice m étant 0, 1, 2, 3 ou 4 et les symboles R, R^{d}, R^{f} et les indices s, t et v ayant la signification indiquée précédemment, notamment dans les revendications 1 et 6.

8. Composé selon une ou plusieurs des revendications 1 à 7, **caractérisé en ce qu'**au moins deux radicaux R, R^{a}, R^{b}, R^{c}, R^{d} forment, avec les autres groupes auxquels sont liés les deux radicaux R, R^{a}, R^{b}, R^{c}, R^{d} un cycle condensé, les deux radicaux R, R^{a}, R^{b}, R^{c}, R^{d} formant au moins une structure des formules Cy-1) à (Cy-10) suivantes, 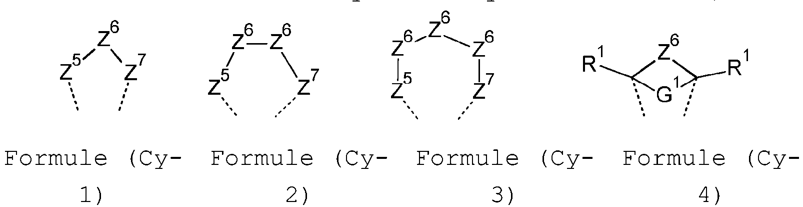 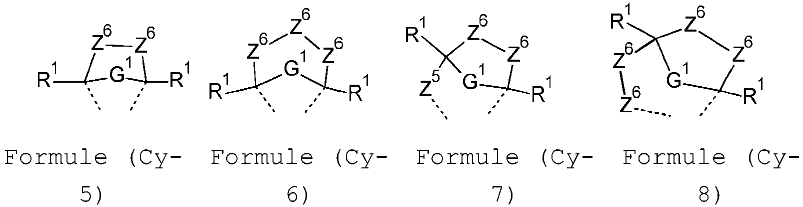 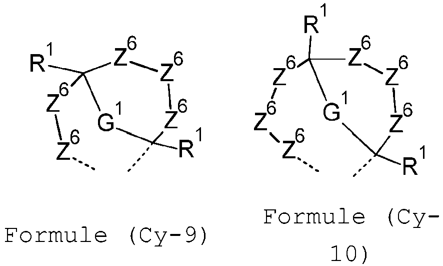 dans lesquelles R¹ présente la signification indiquée dans la revendication 1, les liaisons en pointillés représentent les sites de liaison aux atomes des groupes auxquels sont liés les deux radicaux R, R^{a}, R^{b}, R^{c}, R^{d} et en outre :
Z⁵, Z⁷ représentent en chaque occurrence, de manière identique ou différente, C(R⁴)₂, O, S, NR⁴ ou C(=O) ;
Z⁶ représente C(R¹)₂, O, S, NR¹ ou C(=O), deux groupes Z² voisins pouvant représenter -CR¹=CR¹- ou un groupe arylène ou hétéroarylène lié en ortho ayant 5 à 14 atomes de cycle aromatique, qui peut être substitué par un ou plusieurs radicaux R¹ ;
G¹ représente un groupe alkylène ayant 1, 2 ou 3 atomes de carbone, qui peut être substitué par un ou plusieurs radicaux R¹, -CR¹=CR¹- ou un groupe arylène ou hétéroarylène lié en ortho ayant 5 à 14 atomes de cycle aromatique, qui peut être substitué par un ou plusieurs radicaux R¹ ;
R⁴ représente en chaque occurrence, de manière identique ou différente, H, D, F, Cl, Br, I, CN, NO₂, N(Ar")₂, N(R²)₂, C(=O)Ar", C(=O)R², P(=O) (Ar")₂, P(Ar")₂, B(Ar")₂, B(R²)₂, C(Ar")₃, C(R²)₃, Si(Ar")₃, Si(R²)₃, un groupe alkyle, alcoxy ou thioalcoxy à chaîne droite ayant de 1 à 40 atomes de carbone ou un groupe alkyle, alcoxy ou thioalcoxy ramifié ou cyclique ayant de 3 à 40 atomes de carbone, ou un groupe alcényle ayant de 2 à 40 atomes de carbone, qui peut respectivement être substitué par un ou plusieurs radicaux R², un ou plusieurs groupes CH₂ non voisins étant remplacés par -R²C=CR²-, -C=C-, Si (R²)₂, C=O, C=S, C=Se, C=NR², -C(=O)O-, -C(=O)NR²-, NR², P(=O)(R²), -O-, -S-, SO ou SO₂ et un ou plusieurs atomes H pouvant être remplacés par D, F, Cl, Br, I, CN ou NO₂, ou un système cyclique aromatique ou hétéroaromatique ayant 5 à 60 atomes de cycle aromatique, qui peut respectivement être substitué par un ou plusieurs radicaux R², ou un groupe aryloxy ou hétéroaryloxy comportant 5 à 60 atomes de cycle aromatique, qui peut être substitué par un ou plusieurs radicaux R², ou un groupe aralkyle ou hétéroaralkyle comportant 5 à 60 atomes de cycle aromatique, qui peut être substitué par un ou plusieurs radicaux R², ou une combinaison de ces systèmes ; deux radicaux R⁴ liés au même atome de carbone peuvent former ensemble un système cyclique aliphatique ou aromatique et constituer ainsi un système spiro ; en outre, R⁴ pouvant former un système cyclique aliphatique avec un radical R, R^{a}, R^{b}, R^{c}, R^{d} ou R¹, les symboles R¹ et Ar" ayant les significations indiquées dans la revendication 1 ;
à condition que dans ces groupes, deux hétéroatomes ne soient pas directement liés l'un à l'autre et que deux groupes C=O ne soient pas directement liés l'un à l'autre.

9. Composé selon une ou plusieurs des revendications 1 à 8, **caractérisé en ce qu'**au moins deux radicaux R, R^{a}, R^{b}, R^{c}, R^{d} forment, avec les autres groupes auxquels sont liés les deux radicaux R, R^{a}, R^{b}, R^{c}, R^{d}, un cycle condensé, les deux radicaux R, R^{a}, R^{b}, R^{c}, R^{d} formant au moins une structure des formules (RA-1) à (RA-13) 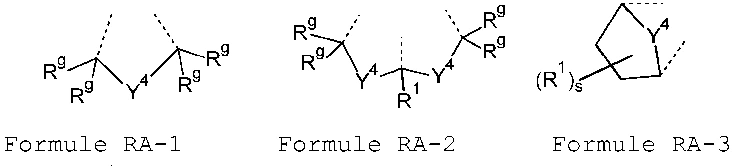 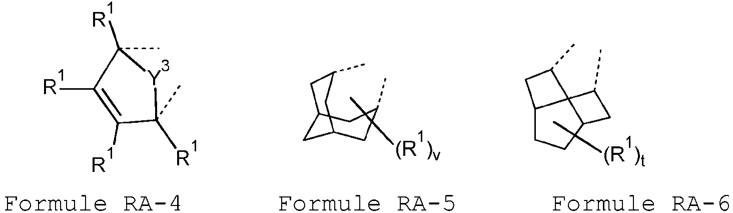 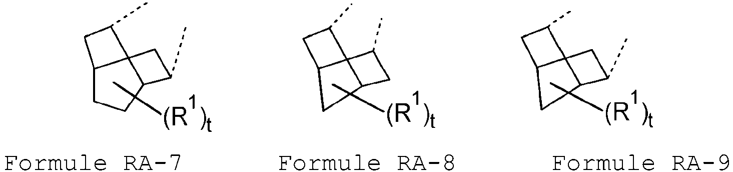 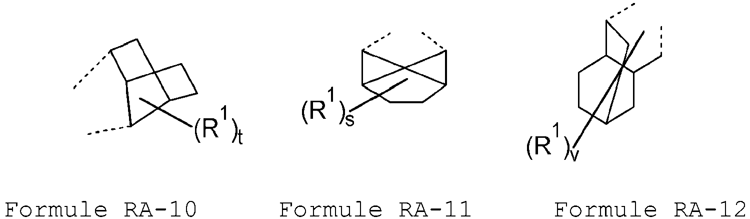 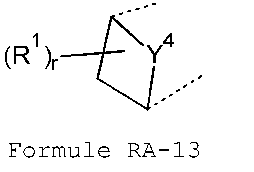 dans lesquelles R¹ présente la signification indiquée ci-dessus, les liaisons en pointillés représentent les sites de liaison aux atomes des groupes auxquels sont liés les deux radicaux R, R^{a}, R^{b}, R^{c}, R^{d}, et les autres symboles présentent la signification suivante :
Y⁴ représente, en chaque occurrence, de manière identique ou différente, C(R¹)₂, (R¹)₂C-C(R¹)₂, (R¹)C=C(R¹), NR¹, NAr', O ou S ;
R^{g} représente en chaque occurrence, de manière identique ou différente, F, un groupe alkyle, alcoxy ou thioalcoxy linéaire comprenant 1 à 40 atomes de carbone ou un groupe alcényle ou alcynyle comprenant 2 à 40 atomes de carbone ou un groupe alkyle, alcoxy ou thioalcoxy ramifié ou cyclique comprenant 3 à 20 atomes de carbone, le groupe alkyle, alcoxy, thioalcoxy, alcényle ou alcynyle pouvant à chaque fois être substitué par un ou plusieurs radicaux R², un ou plusieurs groupes CH₂ non voisins pouvant être remplacés par R²C=CR², C=C, Si (R²) ₂, C=O, C=S, C=Se, C=NR², -C(=O)O-, -C(=O)NR²-, NR², P(=O)(R²), -O-, -S-, SO ou SO₂, ou un système cyclique aromatique ou hétéroaromatique comprenant 5 à 60 atomes de cycle aromatique, qui peut à chaque fois être substitué par un ou plusieurs radicaux R², ou un groupe aryloxy ou hétéroaryloxy comprenant 5 à 60 atomes de cycle aromatique, qui peut être substitué par un ou plusieurs radicaux R² ; deux radicaux R^{g} l'un avec l'autre ou un radical R^{g} avec un radical R¹ ou avec un autre groupe peuvent également former un système cyclique, R² présentant la signification indiquée dans la revendication 1 ;
m représente 0, 1, 2, 3 ou 4 ;
s représente 0, 1, 2, 3, 4, 5 ou 6 ;
t représente 0, 1, 2, 3, 4, 5, 6, 7 ou 8 ;
v représente 0, 1, 2, 3, 4, 5, 6, 7, 8 ou 9.

10. Composé selon une ou plusieurs des revendications 1 à 9, **caractérisé en ce qu'**au moins deux radicaux R, R^{a}, R^{b}, R^{c}, R^{d} forment, avec les autres groupes auxquels sont liés les deux radicaux R, R^{a}, R^{b}, R^{c}, R^{d} un cycle condensé, les deux radicaux R, R^{a}, R^{b}, R^{c}, R^{d} formant les structures de formule (RB), 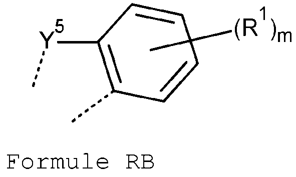 dans laquelle R¹ présente la signification indiquée dans la revendication 1, les liaisons en pointillés représentent les sites de liaison par l'intermédiaire desquels les deux radicaux R, R^{a}, R^{b}, R^{c}, R^{d} se lient, l'indice m représente 0, 1, 2, 3 ou 4 et Y⁵ représente C(R¹)₂, NR¹, NAr', BR¹, BAr', O ou S.

11. Composé selon une ou plusieurs des revendications 1 à 10, **caractérisé en ce que** R ou Ar sont choisis de manière identique ou différente en chaque occurrence parmi les groupes phényle, biphényle, terphényle, quaterphényle, fluorène, spirobifluorène, naphtalène, indole, benzofurane, benzothiophène, carbazole, dibenzofurane, dibenzothiophène, indénocarbazole, indolocarbazole, pyridine, pyrimidine, pyrazine, pyridazine, triazine, quinoléine, isoquinoléine, quinazoline, quinoxaline, phénanthrène ou triphénylène, qui peuvent respectivement être substitués par un ou plusieurs radicaux R^{d}.

12. Composé selon au moins l'une quelconque des revendications précédentes, **caractérisé en ce que** le radical R^{b} et/ou R^{d} représente au moins un groupe choisi parmi C(Ar')₃, C(R¹)₃, Si(Ar')₃, Si(R¹)₃, B(R¹)₂ ou comprend, représente ou forme avec un radical R^{b} ou R^{d} un groupe fluorène qui peut être substitué par un ou plusieurs radicaux R¹.

13. Composé selon au moins l'une quelconque des revendications précédentes, **caractérisé en ce que** le composé comprend exactement deux ou exactement trois structures selon la formule (I), (I-1), (I-2) et/ou (II-1) bis (II-15).

14. Oligomère, polymère ou dendrimère contenant un ou plusieurs composés selon l'une des revendications 1 à 13, dans lequel il existe, au lieu d'un atome d'hydrogène ou d'un substituant, une ou plusieurs liaisons des composés au polymère, à l'oligomère ou au dendrimère.

15. Formulation contenant au moins un composé selon l'une ou plusieurs des revendications 1 à 13 ou un oligomère, un polymère ou un dendrimère selon la revendication 14 et au moins un autre composé, l'autre composé étant de préférence choisi parmi un ou plusieurs solvants.

16. Composition contenant au moins un composé selon l'une ou plusieurs des revendications 1 à 13 ou un oligomère, un polymère ou un dendrimère selon la revendication 14 et au moins un autre composé choisi dans le groupe constitué par les émetteurs fluorescents, les émetteurs phosphorescents, les émetteurs présentant une TADF (fluorescence retardée activée thermiquement), les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous, de préférence les matériaux hôtes.

17. Composition selon la revendication 16, **caractérisée en ce que** la composition au moins un autre composé est un matériau hôte TADF et/ou au moins un autre composé est un émetteur phosphorescent (émetteur triplet), les autres composés formant de préférence un système hyperfluorescent et/ou hyperphosphorescent avec un composé selon une ou plusieurs des revendications 1 à 13 ou un oligomère, polymère ou dendrimère selon la revendication 14.

18. Procédé de préparation d'un composé selon une ou plusieurs des revendications 1 à 13, **caractérisé en ce qu'**on synthétise une structure de base présentant un groupe amino aromatique et on introduit au moins un radical aromatique ou hétéroaromatique, de préférence au moyen d'une réaction de substitution aromatique nucléophile ou d'une réaction de couplage.

19. Utilisation d'un composé selon l'une ou plusieurs des revendications 1 à 13 ou d'un oligomère, d'un polymère ou d'un dendrimère selon la revendication 14 dans un dispositif électronique.

20. Dispositif électronique contenant au moins un composé selon l'une ou plusieurs des revendications 1 à 13 ou un oligomère, un polymère ou un dendrimère selon la revendication 14.
